(19) **Europäisches Patentamt / European Patent Office / Office européen des brevets**

(11) **EP 1 562 982 B1**

(12) **EUROPEAN PATENT SPECIFICATION**

(45) Date of publication and mention
of the grant of the patent:
**05.05.2010 Bulletin 2010/18**

(21) Application number: **03780503.3**

(22) Date of filing: **17.11.2003**

(51) Int Cl.:
**C07K 14/22** $^{(2006.01)}$ **A61K 39/095** $^{(2006.01)}$

(86) International application number:
**PCT/IB2003/006281**

(87) International publication number:
**WO 2004/046177 (03.06.2004 Gazette 2004/23)**

(54) **UNEXPECTED SURFACE PROTEINS IN NEISSERIA MENINGITIDIS**

UNERWARTETE OBERFLÄCHENPROTEINE IN NEISSERIA MENINGITIDIS

PROTEINES DE SURFACE INATTENDUES DU NEISSERIA MENINGITIDIS

(84) Designated Contracting States:
**AT BE BG CH CY CZ DE DK EE ES FI FR GB GR HU IE IT LI LU MC NL PT RO SE SI SK TR**

(30) Priority: **15.11.2002 GB 0226734**
**27.03.2003 GB 0307131**

(43) Date of publication of application:
**17.08.2005 Bulletin 2005/33**

(73) Proprietor: **Novartis Vaccines and Diagnostics S.r.l.**
**53100 Siena (SI) (IT)**

(72) Inventors:
• **NORAIS, Nathalie**
**53100 Siena (IT)**
• **GRANDI, Guido**
**53100 Siena (IT)**

(74) Representative: **Marshall, Cameron John et al**
**Carpmaels & Ransford**
**43-45 Bloomsbury Square**
**London WC1A 2RA (GB)**

(56) References cited:
**WO-A-00/25811 WO-A-01/09350**
**WO-A-01/52885**

• **CLAASSEN I ET AL: "Production, characterization and control of a Neisseria meningitidis hexavalent class 1 outer membrane protein containing vesicle vaccine" VACCINE, BUTTERWORTH SCIENTIFIC. GUILDFORD, GB, vol. 14, no. 10, 1 July 1996 (1996-07-01), pages 1001-1008, XP004057632 ISSN: 0264-410X - & VAN DER LEY P ET AL: "Construction of Neisseria meningitidis strains carrying multiple chromosomal copies of the porA gene for use in the production of a multivalent outer membrane vesicle vaccine" VACCINE, BUTTERWORTH SCIENTIFIC. GUILDFORD, GB, vol. 13, no. 4, 1995, pages 401-407, XP004057740 ISSN: 0264-410X**
• **NORAIS NATHALIE ET AL: "Combined automated PCR cloning, in vitro transcription/translation and two-dimensional electrophoresis for bacterial proteome analysis" PROTEOMICS, vol. 1, no. 11, November 2001 (2001-11), pages 1378-1389, XP009032238 ISSN: 1615-9853**
• **DATABASE UNIPROT [Online] EBI; 1 October 2000 (2000-10-01), TETTELIN ET AL.: "Cell division ATP binding protein FtsE" XP002284894 Database accession no. Q9K1R3**
• **GRIFANTINI R ET AL: "Previously unrecognized vaccine candidates against group B meningococcus identified by DNA microarrays" NATURE BIOTECHNOLOGY, NATURE PUBLISHING, US, vol. 20, no. 9, September 2002 (2002-09), pages 914-921, XP002272872 ISSN: 1087-0156**

- PIZZA M ET AL: "IDENTIFICATION OF VACCINE CANDIDATES AGAINST SEROGROUP B MENINGOCOCCUS BY WHOLE-GENOME SEQUENCING" SCIENCE, AMERICAN ASSOCIATION FOR THE ADVANCEMENT OF SCIENCE,, US, vol. 287, no. 5459, 10 March 2000 (2000-03-10), pages 1816-1820, XP000986271 ISSN: 0036-8075
- TETTELIN H ET AL: "COMPLETE GENOME SEQUENCE OF NEISSERIA MENINGITIDIS SEROGROUP B STRAIN MC58" SCIENCE, AMERICAN ASSOCIATION FOR THE ADVANCEMENT OF SCIENCE,, US, vol. 287, 2000, pages 1809-1815, XP000914963 ISSN: 0036-8075
- JOLLEY KEITH A ET AL: "Immunization with recombinant Opc outer membrane protein from Neisseria meningitidis: Influence of sequence variation and levels of expression on the bactericidal immune response against meningococci" INFECTION AND IMMUNITY, vol. 69, no. 6, June 2001 (2001-06), pages 3809-3916, XP002284891 ISSN: 0019-9567
- WRIGHT J CLAIRE ET AL: "Immunization with the recombinant PorB outer membrane protein induces a bactericidal immune response against Neisseria meningitidis" INFECTION AND IMMUNITY, vol. 70, no. 8, August 2002 (2002-08), pages 4028-4034, XP002284892 ISSN: 0019-9567
- POLLARD A J ET AL: "The meningococcus tamed?" ARCHIVES OF DISEASE IN CHILDHOOD. ENGLAND JUL 2002, vol. 87, no. 1, July 2002 (2002-07), pages 13-17, XP002284893 ISSN: 1468-2044

Description

**TECHNICAL FIELD**

This invention is in the field of meningococcal biochemistry, in particular the trafficking and localisation of meningococcal proteins.

**BACKGROUND ART**

The complete genome sequence of serogroup B *N.meningitidis* has been published [1] and has been subjected to analysis in order to identify candidate vaccine antigens [2]. The complete genome sequence of serogroup A *N.meningitidis* is also known [3].

Footnote 12 of reference 2 describes the authors' approach to antigen selection. Briefly, a first screening for coding capacity of the genome sequence was performed using computer programs included in the Wisconsin package version 10.0, Genetics Computer Group (GCG). BLASTX was used to classify ORFs as coding either for known cytoplasmic functions or for other functions. The cytoplasmic ORFs were "not further investigated" as candidate antigens.

A second screening step aimed at identifying putative proteins with a cellular localization spanning from the inner membrane to outside the bacterium was also used. This screening involved F-BLAST, FASTA, MOTIFS, FINDPATTERNS, and PSORT, as well as the ProDom, Pfam, and Blocks databases. ORFs were thus selected on the basis of features typical of surface-associated proteins such as transmembrane domains, leader peptides, homologies to known surface proteins, lipoprotein signature, outer membrane anchoring motifs, and host cell binding domains such as RGD.

In total, reference 2 identified 570 ORFs within the genome as candidate vaccine antigens. 98.8% of these selected serogroup B ORFs were also found and conserved in serogroup A, and 95.3% were found and conserved in *N. gonorrhoeae.*

This "reverse genomics" approach to vaccine candidate selection is undoubtedly powerful, and has been copied for other organisms [*e.g.* refs. 4 to 10], but it does not identify all surface-exposed proteins and vaccine candidates [11]. It is therefore an object of the invention to identify surface-exposed meningococcal proteins which are not identified by computer prediction methods.

**DISCLOSURE OF THE INVENTION**

***Outer membrane vesicles (OMVs)***

One of the various approaches to immunising against *N.meningitidis* infection is to use outer membrane vesicles (OMVs). An efficacious OMV vaccine against serogroup B has been produced by the Norwegian National Institute of Public Health [*e.g.* ref. 16] but, although this vaccine is safe and prevents meningococcal disease, its efficacy is limited to the strain used to make the vaccine.

To increase the efficacy of OMV vaccines, it has been proposed to supplement them with additional immunogens. Reference 17 discloses compositions comprising OMVs supplemented with transferrin binding proteins (*e.g.* TbpA and TbpB) and/or Cu,Zn-superoxide dismutase. Reference 18 discloses compositions comprising OMVs supplemented by various proteins.

The inventors have found that the portion of the proteome present in OMVs prepared from one strain of *N.meningitidis* can be very different from that of another strain. However, the proteins present in OMVs from any given strain must be compatible with the OMV environment, and so such proteins can be used to supplement OMVs from other strains without needing to be concerned that the proteins might lose immunogenicity, or that they might destabilise the OMVs, *etc.*

In general, therefore, the invention provides a composition comprising: (a) OMVs prepared from a first strain of *N.meningitidis;* and (b) one or more proteins which are present in OMVs prepared from a second strain of *N.meningitidis,* but which are not present in OMVs prepared from said first strain

wherein said one or more proteins includes NMB0007 disclosed by Tettelin et al. [Science (2000) 287:1809-1815] and deposited in the sequence databases under accession number Q9K1 R3, and having the sequence of SEQ ID NO: 218:

| 10 | 20 | 30 | 40 | 50 | 60 |
|---|---|---|---|---|---|
| MIRFEQVSKT | YPGGFEALKN | VSFQINKGEM | IFIAGHSGSG | KSTILKLISG | ITKPSRGKIL |
| 70 | 80 | 90 | 100 | 110 | 120 |
| FNGQDLGTLS | DNQIGFMRQH | IGIVFQDHKI | LYDRNVLQNV | ILPLRIIGYP | PRKAEERARI |

(continued)

| 130 | 140 | 150 | 160 | 170 | 180 |
|---|---|---|---|---|---|
| AIEKVGLKGR | ELDDPVTLSG | GEQQRLCIAR | AVVHQPGLLI | ADEPSANLDR | AYALDIMELF |

| 190 | 200 | 210 | | | |
|---|---|---|---|---|---|
| KTFHEAGTTV | IVAAHDETLM | ADYGHRILRL | SKGRLA | | |

Thus the invention allows the inter-strain reactivity of OMVs to be improved, without requiring the mixing of OMVs from different strains.

The protein(s) of component (b) can be included in the composition in various ways. For example: (i) they can be purified from the second strain and added to component (a); (ii) they can be expressed recombinantly, purified, and added to component (a); or (iii) the first strain can be engineered such that it expresses said protein(s), either from its chromosomal DNA or from extrachromosomal DNA *(e.g.* a plasmid), or such that existing expression of said protein(s) is up-regulated, or such that trafficking of said protein(s) already expressed by the first strain is altered to direct it/them to a different cellular location, thereby causing it/them to be present in OMVs. In situation (iii), the OMVs of component (a), prepared from the manipulated first strain, will already include the proteins of component (b), but these proteins are not present in OMVs prepared from the un-manipulated or wild-type first strain. Thus the invention provides a composition comprising OMVs prepared from a genetically modified first strain of *N.meningitidis,* wherein said OMVs include one or more proteins which are (a) not present in OMVs prepared from said first strain prior to its being genetically modified, but which are (b) present in OMVs prepared from a second strain of *N.meningitidis.*

The *N.meningitidis* strains are preferably from serogroup B of *N.meningitidis.*

Methods for preparing OMVs are well known [*e.g.* refs. 16 to 26]. also describe OMV preparations from meningococcus. A preferred method involves deoxycholate extraction.

The proteins present in component (b) will depend on the meningococcal strain used to prepare the OMVs of component (a) but included in component (b) is always NMB0007.

It will be appreciated that these references to NMB proteins are based on the genomic sequence of serogroup B strain MC58 [1], and that the corresponding proteins in any particular strain, and the genes encoding them, can readily be determined.

## *Compositions*

According to a further aspect, the invention provides compositions comprising OMVs according to the invention. These compositions are preferably immunogenic compositions, such as vaccines, and are suitable for immunisation and vaccination purposes. Vaccines of the invention may be prophylactic or therapeutic, and will typically comprise an antigen which can induce antibodies which are (a) capable of binding to a meningococcal membrane component and/or (b) bactericidal against meningococcus.

Compositions of the invention will generally be administered directly to a patient. Direct delivery may be accomplished by parenteral injection (*e.g.* subcutaneously, intraperitoneally, intravenously, intramuscularly, or to the interstitial space of a tissue), or by rectal, oral, vaginal, topical, transdermal, intranasal, ocular, aural, or pulmonary or other mucosal administration. Electrical methods may be used to for delivery *e.g.* delivery via electroporation.

The invention may be used to elicit systemic and/or mucosal immunity.

Dosage treatment can be a single dose schedule or a multiple dose schedule.

The immunogenic composition of the invention will generally include a pharmaceutically acceptable carrier, which can be any substance that does not itself induce the production of antibodies harmful to the patient receiving the composition, and which can be administered without undue toxicity.

Suitable carriers can be large, slowly-metabolised macromolecules such as proteins, polysaccharides, polylactic acids, polyglycolic acids, polymeric amino acids, amino acid copolymers, and inactive virus particles. Such carriers are well known to those of ordinary skill in the art. Pharmaceutically acceptable carriers can include liquids such as water, saline, glycerol and ethanol. Auxiliary substances, such as wetting or emulsifying agents, pH buffering substances, and the like, can also be present in such vehicles. Liposomes are suitable carriers. A thorough discussion of pharmaceutical carriers is available in ref. 27.

Neisserial infections affect various areas of the body and so the compositions of the invention may be prepared in various forms. For example, the compositions may be prepared as injectables, either as liquid solutions or suspensions. Solid forms suitable for solution in, or suspension in, liquid vehicles prior to injection can also be prepared. The composition may be prepared for topical administration *e.g.* as an ointment, cream or powder. The composition be prepared for oral administration *e.g.* as a tablet or capsule, or as a syrup (optionally flavoured). The composition may be prepared for pulmonary administration *e.g.* as an inhaler, using a fine powder or a spray. The composition may be prepared as a suppository or pessary. The composition may be prepared for nasal, aural or ocular administration *e.g.* as drops.

The composition is preferably sterile. It is preferably pyrogen-free. It is preferably buffered *e.g.* at between pH 6 and pH 8, generally around pH 7.

*Further components of compositions*

Immunogenic compositions comprise an immunologically effective amount of immunogen, as well as any other of other specified components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (*e.g.* non-human primate, primate, *etc.),* the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials. Dosage treatment may be a single dose schedule or a multiple dose schedule (*e.g.* including booster doses). The composition may be administered in conjunction with other immunoregulatory agents.

The composition will generally comprise an adjuvant. Preferred adjuvants to enhance effectiveness of the composition include, but are not limited to: (A) MF59 (5% Squalene, 0.5% Tween 80, and 0.5% Span 85, formulated into submicron particles using a microfluidizer) [see Chapter 10 of ref. 28; see also ref. 29]; (B) microparticles (*i.e.* a particle of ~100nm to ~150$\mu$m in diameter, more preferably ~200nm to ~30$\mu$m in diameter, and most preferably ~500nm to ~10$\mu$m in diameter) formed from materials that are biodegradable and non-toxic (*e.g.* a poly($\alpha$-hydroxy acid), a polyhydroxybutyric acid, a polyorthoester, a polyanhydride, a polycaprolactone *etc.),* with poly(lactide-co-glycolide) being preferred ('PLG'), optionally being charged surface (*e.g.* by adding a cationic, anionic, or nonionic detergent such as SDS (negative) or CTAB (positive) [*e.g.* refs. 30 & 31]); (C) liposomes [see Chapters 13 and 14 of ref. 28]; (D) ISCOMs [see Chapter 23 of ref. 28], which may be devoid of additional detergent [32]; (E) SAF, containing 10% Squalane, 0.4% Tween 80, 5% pluronic-block polymer L121, and thr-MDP, either microfluidized into a submicron emulsion or vortexed to generate a larger particle size emulsion [see Chapter 12 of ref. 28]; (F) Ribi™ adjuvant system (RAS), (Ribi Immunochem) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); (G) saponin adjuvants, such as QuilA or QS21 [see Chapter 22 of ref. 28], also known as Stimulon™; (H) chitosan [*e.g.* 33]; (I) complete Freund's adjuvant (CFA) and incomplete Freund's adjuvant (IFA); (J) cytokines, such as interleukins (*e.g.* IL-1, IL-2, IL-4, IL-5, IL-6, IL-7, IL-12, *etc.),* interferons (*e.g.* interferon-y), macrophage colony stimulating factor, tumor necrosis factor, *etc.* [see Chapters 27 & 28 of ref. 28], RC529; (K) a saponin (*e.g.* QS21) + 3dMPL + IL-12 (optionally + a sterol) [34]; (L) monophosphoryl lipid A (MPL) or 3-O-deacylated MPL (3dMPL) [*e.g.* chapter 21 of ref. 28]; (M) combinations of 3dMPL with, for example, QS21 and/or oil-in-water emulsions [35]; (N) oligonucleotides comprising CpG motifs [36] *i.e.* containing at least one CG dinucleotide, with 5-methylcytosine optionally being used in place of cytosine; (O) a polyoxyethylene ether or a polyoxyethylene ester [37]; (P) a polyoxyethylene sorbitan ester surfactant in combination with an octoxynol [38] or a polyoxyethylene alkyl ether or ester surfactant in combination with at least one additional non-ionic surfactant such as an octoxynol [39]; (Q) an immunostimulatory oligonucleotide (*e.g.* a CpG oligonucleotide) and a saponin [40]; (R) an immunostimulant and a particle of metal salt [41]; (S) a saponin and an oil-in-water emulsion [42]; (T) *E.coli* heat-labile enterotoxin ("LT"), or detoxified mutants thereof, such as the K63 or R72 mutants [*e.g.* Chapter 5 of ref. 43]; (U) cholera toxin ("CT"), or detoxified mutants thereof [*e.g.* Chapter 5 of ref. 43]; (V) double-stranded RNA; (W) aluminium salts, such as aluminium hydroxides (including oxyhydroxides), aluminium phosphates (including hydroxyphosphates), aluminium sulfate, *etc* [Chapters 8 & 9 in ref. 44]; (X) monophosphoryl lipid A mimics, such as aminoalkyl glucosaminide phosphate derivatives *e.g.* RC-529 [45]; and (Y) other substances that act as immunostimulating agents to enhance the effectiveness of the composition [*e.g.* see Chapter 7 of ref. 28], such as calcium phosphate. Aluminium salts (aluminium phosphates and particularly hydroxyphosphates, and/or hydroxides and particularly oxyhydroxide) are preferred adjuvants for parenteral immunisation. Toxin mutants are preferred mucosal adjuvants.

Muramyl peptides include N-acetyl-muramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl-L-alanine-2-(1'-2'-dipalmitoyl-*sn*-glycero-3-hydroxyphosphoryloxy)-ethylamine MTP-PE), *etc.*

Compositions of the invention may comprise antigens (*e.g.* protective antigens against *N.meningitidis* or against other organisms) in addition to the proteins mentioned above *e.g.* DTP antigens, Hib antigen *etc.* The composition may comprise one or more of the following further antigens:

- antigens from *Helicobacter pylori* such as CagA [46 to 49], VacA [50, 51], NAP [52, 53, 54], HopX [*e.g.* 55], HopY [*e.g.* 55] and/or urease.
- a saccharide antigen from *N.meningitidis* serogroup A, C, W135 and/or Y, such as the oligosaccharide disclosed in ref. 56 from serogroup C [see also ref. 57] or the oligosaccharides of ref. 58.

- a saccharide antigen from *Streptococcus pneumoniae* [*e.g.* 59, 60, 61].
- an antigen from hepatitis A virus, such as inactivated virus [*e.g.* 62, 63].
- an antigen from hepatitis B virus, such as the surface and/or core antigens [*e.g.* 63, 64].
- an antigen from *Bordetella pertussis,* such as pertussis holotoxin (PT) and filamentous haemagglutinin (FHA) from *B.pertussis,* optionally also in combination with pertactin and/or agglutinogens 2 and 3 [*e.g.* refs. 65 & 66].
- a diphtheria antigen, such as a diphtheria toxoid [*e.g.* chapter 3 of ref. 67] *e.g.* the CRM$_{197}$ mutant [*e.g.* 68].
- a tetanus antigen, such as a tetanus toxoid [*e.g.* chapter 4 of ref. 87].
- a saccharide antigen from *Haemophilus influenzae B* [*e.g.* 57].
- an antigen from hepatitis C virus [*e.g.* 69].
- an antigen from *N.gonorrhoeae* [*e.g.* 70, 71, 72, 73].
- an antigen from *Chlamydia pneumoniae* [*e.g.* refs. 74 to 80].
- an antigen from *Chlamydia trachomatis* [*e.g.* 81].
- an antigen from *Porphyromonas gingivalis* [*e.g.* 82].
- polio antigen(s) [*e.g.* 83, 84] such as IPV.
- rabies antigen(s) *[e.g.* 85] such as lyophilised inactivated virus *[e.g.* 86, RabAvert™].
- measles, mumps and/or rubella antigens [*e.g.* chapters 9, 10 & 11 of ref. 87].
- influenza antigen(s) [*e.g.* chapter 19 of ref. 87], such as the haemagglutinin and/or neuraminidase surface proteins.
- an antigen from *Moraxella catarrhalis* [*e.g.* 88].
- an protein antigen from *Streptococcus agalactiae* (group B streptococcus) [*e.g.* 89, 90].
- a saccharide antigen from *Streptococcus agalactiae* (group B streptococcus).
- an antigen from *Streptococcus pyogenes* (group A streptococcus) [*e.g.* 90, 91, 92].
- an antigen from *Staphylococcus aureus* [*e.g.* 93].
- an antigen from *Bacillus anthracis* [*e.g.* 94, 95, 96].
- an antigen from a virus in the flaviviridae family (genus flavivirus), such as from yellow fever virus, Japanese encephalitis virus, four serotypes of Dengue viruses, tick-bome encephalitis virus, West Nile virus.
- a pestivirus antigen, such as from classical porcine fever virus, bovine viral diarrhoea virus, and/or border disease virus.
- a parvovirus antigen *e.g*, from parvovirus B19.
- a prion protein (*e.g.* the CJD prion protein)
- an amyloid-protein, such as a beta peptide [97]
- a cancer antigen, such as those listed in Table 1 of ref. 98 or in tables 3 & 4 of ref. 99.

The composition may comprise one or more of these further antigens.

Toxic protein antigens may be detoxified where necessary (*e.g.* detoxification of pertussis toxin by chemical and/or genetic means [66]).

Where a diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens. DTP combinations are thus preferred.

Saccharide antigens are preferably in the form of conjugates. Carrier proteins for the conjugates include the *N.meningitidis* outer membrane protein [100], synthetic peptides [101,102], heat shock proteins [103,104], pertussis proteins [105,106], protein D from *Hinfluenzae* [107], cytokines [108], lymphokines [108], streptococcal proteins, hormones [108], growth factors [108], toxin A or B from *C.difficile* [109], iron-uptake proteins [110], *etc.* A preferred carrier protein is the CRM197 diphtheria toxoid [111].

Antigens in the composition will typically be present at a concentration of at least 1μg/ml each. In general, the concentration of any given antigen will be sufficient to elicit an immune response against that antigen.

Immunogenic compositions of the invention may be used therapeutically (*i.e.* to treat an existing infection) or prophylactically (*i.e.* to prevent future infection).

As an alternative to using proteins antigens in the immunogenic compositions of the invention, nucleic acid (preferably DNA *e.g.* in the form of a plasmid) encoding the antigen may be used.

***Medical methods* etc.**

The invention also provides nucleic acids or proteins or OMVs of the invention, or antibodies which bind to proteins of the invention, for use as medicaments (*e.g.* as vaccines). It also provides the use of nucleic acid or protein or OMVs according to the invention in the manufacture of a medicament (*e.g.* a vaccine or an immunogenic composition) for treating or preventing infection due to a *Neisseria.* This will generally be *N.meningitidis* but, due to inter-species cross-reactivity, it may also be *N.gonorrhoeae.*

The invention also provides a method of treating *(e.g.* immunising) a patient *(e.g.* a human), comprising administering to the patient a therapeutically effective amount of nucleic acid and/or protein and/or OMVs according to the invention.

The invention also provides a method of raising antibodies in an animal, comprising administering to the patient an immunologically effective amount of nucleic acid and/or protein and/or OMVs according to the invention.

The invention also provides a method of detecting *N.meningitidis* bacteria in a sample, comprising the steps of: (a) contacting an antibody *(e.g.* monoclonal or polyclonal) which binds to a protein of the invention with a biological sample under conditions suitable for the formation of an antibody-antigen complexes; and (b) detecting said complexes. The method is particularly suitable for detecting intact bacteria because the proteins of the invention are present in the membrane. If the correct protein is used, it is also useful for distinguishing between *N.meningitidis* strains.

The invention also provides nucleic acids or proteins or OMVs of the invention, or antibodies which bind to proteins of the invention, for use as diagnostic reagents. It also provides the use of nucleic acid or protein or OMVs according to the invention, or antibodies which bind to proteins of the invention, in the manufacture of a reagent for diagnosing infection due to a *Neisseria.*

The invention also provides a method for investigating *N.meningitidis,* wherein the method includes a step of performing 2D electrophoresis on a membrane fraction from the bacterium.

### *Techniques*

A summary of standard techniques and procedures which may be employed in order to perform the invention *(e.g.* to utilise the disclosed sequences for immunisation or diagnosis) follows. This summary is not a limitation on the invention but, rather, gives examples that may be used, but are not required.

### *General*

The practice of the present invention will employ, unless otherwise indicated, conventional techniques of molecular biology, microbiology, recombinant DNA, and immunology, which are within the skill of the art.

Such techniques are explained fully in the literature *e.g.* Sambrook Molecular Cloning; A Laboratory Manual Second Edition (1989) and Third Edition (2001); DNA Cloning, Volumes I and ii (D.N Glover ed. 1985); Oligonucleotide Synthesis (M.J. Gait ed, 1984); Nucleic Acid Hybridization (B.D. Hames & S.J. Higgins eds. 1984); Transcription and Translation (B.D. Hames & S.J. Higgins eds. 1984); Animal Cell Culture (R.I. Freshney ed. 1986); Immobilized Cells and Enzymes (IRL Press, 1986); B. Perbal, A Practical Guide to Molecular Cloning (1984); the Methods in Enzymology series (Academic Press, Inc.), especially volumes 154 & 155; Gene Transfer Vectors for Mammalian Cells (J.H. Miller and M.P. Calos eds. 1987, Cold Spring Harbor Laboratory); Mayer and Walker, eds. (1987), Immunochemical Methods in Cell and Molecular Biology (Academic Press, London); Scopes, (1987) Protein Purification: Principles and Practice, Second Edition (Springer-Verlag, N.Y.), and Handbook of Experimental Immunology, Volumes I-IV (D.M. Weir and C. C. Blackwell eds 1986).

Standard abbreviations for nucleotides and amino acids are used in this specification.

### *Definitions*

A composition containing X is "substantially free of" Y when at least 85% by weight of the total X+Y in the composition is X. Preferably, X comprises at least about 90% by weight of the total of X+Y in the composition, more preferably at least about 95% or even 99% by weight.

The term "comprising" means "including" as well as "consisting" *e.g.* a composition "comprising" X may consist exclusively of X or may include something additional to X, such as X+Y.

The term "heterologous" refers to two biological components that are not found together in nature. The components may be host cells, genes, or regulatory regions, such as promoters. Although the heterologous components are not found together in nature, they can function together, as when a promoter heterologous to a gene is operably linked to the gene. Another example is where a Neisserial sequence is heterologous to a mouse host cell. A further examples would be two epitopes from the same or different proteins which have been assembled in a single protein in an arrangement not found in nature.

An "origin of replication" is a polynucleotide sequence that initiates and regulates replication of polynucleotides, such as an expression vector. The origin of replication behaves as an autonomous unit of polynucleotide replication within a cell, capable of replication under its own control. An origin of replication may be needed for a vector to replicate in a particular host cell. With certain origins of replication, an expression vector can be reproduced at a high copy number in the presence of the appropriate proteins within the cell. Examples of origins are the autonomously replicating sequences, which are effective in yeast; and the viral T-antigen, effective in COS-7 cells.

A "mutant" sequence is defined as DNA, RNA or amino acid sequence differing from but having sequence identity with the native or disclosed sequence. Depending on the particular sequence, the degree of sequence identity between the native or disclosed sequence and the mutant sequence is preferably greater than 50% *(e.g.* 60%, 70%, 80%, 90%, 95%, 99% or more, calculated using the Smith-Waterman algorithm as described above). As used herein, an "allelic variant" of a nucleic acid molecule, or region, for which nucleic acid sequence is provided herein is a nucleic acid molecule, or region, that occurs essentially at the same locus in the genome of another or second isolate, and that, due to natural variation caused by, for example, mutation or recombination, has a similar but not identical nucleic acid sequence. A coding region allelic variant typically encodes a protein having similar activity to that of the protein encoded by the gene to which it is being compared. An allelic variant can also comprise an alteration in the 5' or 3' untranslated regions of the gene, such as in regulatory control regions (*e.g.* see US patent 5,753,235).

*Expression systems*

The Neisserial nucleotide sequences can be expressed in a variety of different expression systems; for example those used with mammalian cells, baculoviruses, plants, bacteria, and yeast.

i. Mammalian Systems

Mammalian expression systems are known in the art. A mammalian promoter is any DNA sequence capable of binding mammalian RNA polymerase and initiating the downstream (3') transcription of a coding sequence (*e.g.* structural gene) into mRNA. A promoter will have a transcription initiating region, which is usually placed proximal to the 5' end of the coding sequence, and a TATA box, usually located 25-30 base pairs (bp) upstream of the transcription initiation site. The TATA box is thought to direct RNA polymerase II to begin RNA synthesis at the correct site. A mammalian promoter will also contain an upstream promoter element, usually located within 100 to 200 bp upstream of the TATA box. An upstream promoter element determines the rate at which transcription is initiated and can act in either orientation [Sambrook et al. (1989) "Expression of Cloned Genes in Mammalian Cells." In Molecular Cloning: A Laboratory Manual, 2nd ed.*].*

Mammalian viral genes are often highly expressed and have a broad host range; therefore sequences encoding mammalian viral genes provide particularly useful promoter sequences. Examples include the SV40 early promoter, mouse mammary tumor virus LTR promoter, adenovirus major late promoter (Ad MLP), and herpes simplex virus promoter. In addition, sequences derived from non-viral genes, such as the murine metallotheionein gene, also provide useful promoter sequences. Expression may be either constitutive or regulated (inducible), depending on the promoter can be induced with glucocorticoid in hormone-responsive cells.

The presence of an enhancer element (enhancer), combined with the promoter elements described above, will usually increase expression levels. An enhancer is a regulatory DNA sequence that can stimulate transcription up to 1000-fold when linked to homologous or heterologous promoters, with synthesis beginning at the normal RNA start site. Enhancers are also active when they are placed upstream or downstream from the transcription initiation site, in either normal or flipped orientation, or at a distance of more than 1000 nucleotides from the promoter [Maniatis et al. (1987) Science 236:1237; Alberts et al. (1989) Molecular Biology of the Cell, 2nd ed.]. Enhancer elements derived from viruses may be particularly useful, because they usually have a broader host range. Examples include the SV40 early gene enhancer [Dijkema et al (1985) EMBO J. 4:761] and the enhancer/promoters derived from the long terminal repeat (LTR) of the Rous Sarcoma Virus [Gorman et al. (1982) PNAS USA 79:6777] and from human cytomegalovirus [Boshart et al. (1985) Cell 41:521]. Additionally, some enhancers are regulatable and become active only in the presence of an inducer, such as a hormone or metal ion [Sassone-Corsi and Borelli (1986) Trends Genet. 2:215; Maniatis et al. (1987) Science 236:1237].

A DNA molecule may be expressed intracellularly in mammalian cells. A promoter sequence may be directly linked with the DNA molecule, in which case the first amino acid at the N-terminus of the recombinant protein will always be a methionine, which is encoded by the ATG start codon. If desired, the N-terminus may be cleaved from the protein by *in vitro* incubation with cyanogen bromide.

Alternatively, foreign proteins can also be secreted from the cell into the growth media by creating chimeric DNA molecules that encode a fusion protein comprised of a leader sequence fragment that provides for secretion of the foreign protein in mammalian cells. Preferably, there are processing sites encoded between the leader fragment and the foreign gene that can be cleaved either *in vivo* or *in vitro.* The leader sequence fragment usually encodes a signal peptide comprised of hydrophobic amino acids which direct the secretion of the protein from the cell. The adenovirus triparite leader is an example of a leader sequence that provides for secretion of a foreign protein in mammalian cells.

Usually, transcription termination and polyadenylation sequences recognized by mammalian cells are regulatory regions located 3' to the translation stop codon and thus, together with the promoter elements, flank the coding sequence. The 3' terminus of the mature mRNA is formed by site-specific post-transcriptional cleavage and polyadenylation [Birnstiel et al. (1985) Cell 41:349; Proudfoot and Whitelaw (1988) "Termination and 3' end processing of eukaryotic RNA. In Transcription and splicing (ed. B.D. Hames and D.M. Glover); Proudfoot (1989) Trends Biochem. Sci. 14:105]. These sequences direct the transcription of an mRNA which can be translated into the polypeptide encoded by the DNA. Examples of transcription terminater/polyadenylation signals include those derived from SV40 [Sambrook et al (1989) "Expression of cloned genes in cultured mammalian cells." In Molecular Cloning: A Laboratory Manual].

Usually, the above described components, comprising a promoter, polyadenylation signal, and transcription termination sequence are put together into expression constructs. Enhancers, introns with functional splice donor and acceptor sites, and leader sequences may also be included in an expression construct, if desired. Expression constructs are often maintained in a replicon, such as an extrachromosomal element (*e.g.* plasmids) capable of stable maintenance in a host, such as mammalian cells or bacteria. Mammalian replication systems include those derived from animal viruses, which require trans-acting factors to replicate. For example, plasmids containing the replication systems of papovaviruses, such as SV40 [Gluzman (1981) Cell 23:175] or polyomavirus, replicate to extremely high copy number in the presence of the appropriate viral T antigen. Additional examples of mammalian replicons include those derived from bovine papillomavirus and Epstein-Barr virus. Additionally, the replicon may have two replicaton systems, thus allowing it to be maintained, for example, in mammalian cells for expression and in a prokaryotic host for cloning and amplification. Examples of such mammalian-bacteria shuttle vectors include pMT2 [Kaufman et al. (1989) Mol. Cell. Biol. 9:946] and pHEBO [Shimizu et al. (1986) Mol. Cell. Biol. 6:1074].

The transformation procedure used depends upon the host to be transformed. Methods for introduction of heterologous polynucleotides into mammalian cells are known in the art and include dextran-mediated transfection, calcium phosphate precipitation, polybrene-mediated transfection, protoplast fusion, electroporation, encapsulation of polynucleotide(s) in liposomes, direct microinjection of the DNA into nuclei.

Mammalian cell lines available as hosts for expression are known in the art and include many immortalized cell lines available from the American Type Culture Collection (ATCC), including but not limited to, Chinese hamster ovary (CHO) cells, HeLa cells, baby hamster kidney (BHK) cells, monkey kidney cells (COS), human hepatocellular carcinoma cells (*e.g.* Hep G2), and a number of other cell lines.

### ii. Baculovirus Systems

The polynucleotide encoding the protein can also be inserted into a suitable insect expression vector, and is operably linked to the control elements within that vector. Vector construction employs techniques which are known in the art. Generally, the components of the expression system include a transfer vector, usually a bacterial plasmid, which contains both a fragment of the baculovirus genome, and a convenient restriction site for insertion of the heterologous gene or genes to be expressed; a wild type baculovirus with a sequence homologous to the baculovirus-specific fragment in the transfer vector (this allows for the homologous recombination of the heterologous gene in to the baculovirus genome); and appropriate insect host cells and growth media.

After inserting the DNA sequence encoding the protein into the transfer vector, the vector and the wild type viral genome are transfected into an insect host cell where the vector and viral genome are allowed to recombine. The packaged recombinant virus is expressed and recombinant plaques are identified and purified. Materials and methods for baculovirus/insect cell expression systems are commercially available in kit form from, *inter alia,* Invitrogen, San Diego CA ("MaxBac" kit). These techniques are generally known to those skilled in the art and fully described in Summers and Smith, *Texas Agricultural Experiment Station Bulletin No. 1555* (1987) (hereinafter "Summers and Smith").

Prior to inserting the DNA sequence encoding the protein into the baculovirus genome, the above described components, comprising a promoter, leader (if desired), coding sequence of interest, and transcription termination sequence, are usually assembled into an intermediate transplacement construct (transfer vector). This construct may contain a single gene and operably linked regulatory elements; multiple genes, each with its owned set of operably linked regulatory elements; or multiple genes, regulated by the same set of regulatory elements. Intermediate transplacement constructs are often maintained in a replicon, such as an extrachromosomal element (*e.g.* plasmids) capable of stable maintenance in a host, such as a bacterium. The replicon will have a replication system, thus allowing it to be maintained in a suitable host for cloning and amplification.

Currently, the most commonly used transfer vector for introducing foreign genes into AcNPV is pAc373. Many other vectors, known to those of skill in the art, have also been designed. These include, for example, pVL985 (which alters the polyhedrin start codon from ATG to ATT, and which introduces a BamHI cloning site 32 basepairs downstream from the ATT; see Luckow and Summers, Virology (1989) 17:31.

The plasmid usually also contains the polyhedrin polyadenylation signal (Miller et al. (1988) Ann. Rev. Microbiol., 42:177) and a prokaryotic ampicillin-resistance (*amp*) gene and origin of replication for selection and propagation in *E. coli.*

Baculovirus transfer vectors usually contain a baculovirus promoter. A baculovirus promoter is any DNA sequence capable of binding a baculovirus RNA polymerase and initiating the downstream (5' to 3') transcription of a coding sequence (*e.g.* structural gene) into mRNA. A promoter will have a transcription initiation region which is usually placed proximal to the 5' end of the coding sequence. This transcription initiation region usually includes an RNA polymerase binding site and a transcription initiation site. A baculovirus transfer vector may also have a second domain called an enhancer, which, if present, is usually distal to the structural gene. Expression may be either regulated or constitutive.

Structural genes, abundantly transcribed at late times in a viral infection cycle, provide particularly useful promoter sequences. Examples include sequences derived from the gene encoding the viral polyhedron protein, Friesen et al., (1986) "The Regulation of Baculovirus Gene Expression," in: The Molecular Biology of Baculoviruses (ed. Walter Doerfler); EPO Publ. Nos. 127 839 and 155 476; and the gene encoding the p10 protein, Vlak et al., (1988), J. Gen. Virol. 69:765.

DNA encoding suitable signal sequences can be derived from genes for secreted insect or baculovirus proteins, such as the baculovirus polyhedrin gene (Carbonell et al. (1988) Gene, 73:409). Alternatively, since the signals for mammalian cell posttranslational modifications (such as signal peptide cleavage, proteolytic cleavage, and phosphorylation) appear to be recognized by insect cells, and the signals required for secretion and nuclear accumulation also appear to be conserved between the invertebrate cells and vertebrate cells, leaders of non-insect origin, such as those derived from genes encoding human $\alpha$-interferon, Maeda et al., (1985), Nature 315:592; human gastrin-releasing peptide, Lebacq-Verheyden et al., (1988), Molec. Cell. Biol. 8:3129; human IL-2, Smith et al., (1985) Proc. Nat'l Acad. Sci. USA, 82:8404; mouse IL-3, (Miyajima et al., (1987) Gene 58:273; and human glucocerebrosidase, Martin et al. (1988) DNA, 7:99, can also be used to provide for secretion in insects.

A recombinant polypeptide or polyprotein may be expressed intracellularly or, if it is expressed with the proper regulatory sequences, it can be secreted. Good intracellular expression of nonfused foreign proteins usually requires heterologous genes that ideally have a short leader sequence containing suitable translation initiation signals preceding an ATG start signal. If desired, methionine at the N-terminus may be cleaved from the mature protein by *in vitro* incubation with cyanogen bromide.

Alternatively, recombinant polyproteins or proteins which are not naturally secreted can be secreted from the insect cell by creating chimeric DNA molecules that encode a fusion protein comprised of a leader sequence fragment that provides for secretion of the foreign protein in insects. The leader sequence fragment usually encodes a signal peptide comprised of hydrophobic amino acids which direct the translocation of the protein into the endoplasmic reticulum.

After insertion of the DNA sequence and/or the gene encoding the expression product precursor of the protein, an insect cell host is co-transformed with the heterologous DNA of the transfer vector and the genomic DNA of wild type baculovirus -- usually by co-transfection. The promoter and transcription termination sequence of the construct will usually comprise a 2-5kb section of the baculovirus genome. Methods for introducing heterologous DNA into the desired site in the baculovirus virus are known in the art. (See Summers and Smith *supra;* Ju et al. (1987); Smith et al., Mol. Cell. Biol. (1983) 3:2156; and Luckow and Summers (1989)). For example, the insertion can be into a gene such as the polyhedrin gene, by homologous double crossover recombination; insertion can also be into a restriction enzyme site engineered into the desired baculovirus gene. Miller et al., (1989), Bioessays 4:91.The DNA sequence, when cloned in place of the polyhedrin gene in the expression vector, is flanked both 5' and 3' by polyhedrin-specific sequences and is positioned downstream of the polyhedrin promoter.

The newly formed baculovirus expression vector is subsequently packaged into an infectious recombinant baculovirus. Homologous recombination occurs at low frequency (between ~1% and ~5%); thus, the majority of the virus produced after cotransfection is still wild-type virus. Therefore, a method is necessary to identify recombinant viruses. An advantage of the expression system is a visual screen allowing recombinant viruses to be distinguished. The polyhedrin protein, which is produced by the native virus, is produced at very high levels in the nuclei of infected cells at late times after viral infection. Accumulated polyhedrin protein forms occlusion bodies that also contain embedded particles. These occlusion bodies, up to 15$\mu$m in size, are highly refractile, giving them a bright shiny appearance that is readily visualized under the light microscope. Cells infected with recombinant viruses lack occlusion bodies. To distinguish recombinant virus from wild-type virus, the transfection supernatant is plaqued onto a monolayer of insect cells by techniques known to those skilled in the art. Namely, the plaques are screened under the light microscope for the presence (indicative of wild-type virus) or absence (indicative of recombinant virus) of occlusion bodies. "Current Protocols in Microbiology" Vol. 2 (Ausubel et al. eds) at 16.8 (Supp. 10, 1990); Summers & Smith, *supra;* Miller et al. (1989).

Recombinant baculovirus expression vectors have been developed for infection into several insect cells. For example, recombinant baculoviruses have been developed for, *inter alia: Aedes aegypti, Autographa californica, Bombyx mori, Drosophila melanogaster, Spodoptera frugiperda,* and *Trichoplusia ni* (WO 89/046699; Carbonell et al., (1985) J. Virol. 56:153; Wright (1986) Nature 321:718; Smith et al., (1983) Mol. Cell. Biol. 3:2156; and see generally, Fraser, et al. (1989) In Vitro Cell. Dev. Biol. 25:225).

Cells and cell culture media are commercially available for both direct and fusion expression of heterologous polypeptides in a baculovirus/expression system; cell culture technology is generally known to those skilled in the art. *See, e.g.* Summers and Smith *supra.*

The modified insect cells may then be grown in an appropriate nutrient medium, which allows for stable maintenance of the plasmid(s) present in the modified insect host. Where the expression product gene is under inducible control, the host may be grown to high density, and expression induced. Alternatively, where expression is constitutive, the product will be continuously expressed into the medium and the nutrient medium must be continuously circulated, while removing the product of interest and augmenting depleted nutrients. The product may be purified by such techniques as chromatography, e.g. HPLC, affinity chromatography, ion exchange chromatography, etc.; electrophoresis; density gradient centrifugation; solvent extraction, or the like. As appropriate, the product may be further purified, as required, so as to remove substantially any insect proteins which are also secreted in the medium or result from lysis of insect cells, so as to provide a product which is at least substantially free of host debris, e.g. proteins, lipids and polysaccharides.

In order to obtain protein expression, recombinant host cells derived from the transformants are incubated under conditions which allow expression of the recombinant protein encoding sequence. These conditions will vary, dependent upon the host cell selected. However, the conditions are readily ascertainable to those of ordinary skill in the art, based upon what is known in the art.

iii. Plant Systems

There are many plant cell culture and whole plant genetic expression systems known in the art. Exemplary plant cellular genetic expression systems include those described in patents, such as: US 5,693,506; US 5,659,122; and US 5,608,143. Additional examples of genetic expression in plant cell culture has been described by Zenk, Phytochemistry 30:3861-3863 (1991). Descriptions of plant protein signal peptides may be found in addition to the references described above in Vaulcombe et al., Mol. Gen. Genet. 209:33-40 (1987); Chandler et al., Plant Molecular Biology 3: 407-418 (1984); Rogers, J. Biol. Chem. 260:3731-3738 (1985); Rothstein et al., Gene 55:353-356 (1987); Whittier et al., Nucleic Acids Research 15:2515-2535 (1987); Wirsel et al., Molecular Microbiology 3:3-14 (1989); Yu et al., Gene 122:247-253 (1992). A description of the regulation of plant gene expression by the phytohormone, gibberellic acid and secreted enzymes induced by gibberellic acid can be found in R.L. Jones and J. MacMillin, Gibberellins: in: Advanced Plant Physiology,. Malcolm B. Wilkins, ed., 1984 Pitman Publishing Limited, London, pp. 21-52. References that describe other metabolically-regulated genes: Sheen, Plant Cell, 2:1027-1038(1990); Maas et al., EMBO J. 9:3447-3452 (1990); Benkel and Hickey, Proc. Natl. Acad. Sci. 84:1337-1339 (1987)

Typically, using techniques known in the art, a desired polynucleotide sequence is inserted into an expression cassette comprising genetic regulatory elements designed for operation in plants. The expression cassette is inserted into a desired expression vector with companion sequences upstream and downstream from the expression cassette suitable for expression in a plant host. The companion sequences will be of plasmid or viral origin and provide necessary characteristics to the vector to permit the vectors to move DNA from an original cloning host, such as bacteria, to the desired plant host. The basic bacterial/plant vector construct will preferably provide a broad host range prokaryote replication origin; a prokaryote selectable marker; and, for Agrobacterium transformations, T DNA sequences for Agrobacterium-mediated transfer to plant chromosomes. Where the heterologous gene is not readily amenable to detection, the construct will preferably also have a selectable marker gene suitable for determining if a plant cell has been transformed. A general review of suitable markers, for example for the members of the grass family, is found in Wilmink and Dons, 1993, Plant Mol. Biol. Reptr, 11(2):165-185.

Sequences suitable for permitting integration of the heterologous sequence into the plant genome are also recommended. These might include transposon sequences and the like for homologous recombination as well as Ti sequences which permit random insertion of a heterologous expression cassette into a plant genome. Suitable prokaryote selectable markers include resistance toward antibiotics such as ampicillin or tetracycline. Other DNA sequences encoding additional functions may also be present in the vector, as is known in the art.

The nucleic acid molecules of the subject invention may be included into an expression cassette for expression of the protein(s) of interest. Usually, there will be only one expression cassette, although two or more are feasible. The recombinant expression cassette will contain in addition to the heterologous protein encoding sequence the following elements, a promoter region, plant 5' untranslated sequences, initiation codon depending upon whether or not the structural gene comes equipped with one, and a transcription and translation termination sequence. Unique restriction enzyme sites at the 5' and 3' ends of the cassette allow for easy insertion into a pre-existing vector.

A heterologous coding sequence may be for any protein relating to the present invention. The sequence encoding the protein of interest will encode a signal peptide which allows processing and translocation of the protein, as appropriate, and will usually lack any sequence which might result in the binding of the desired protein of the invention to a membrane. Since, for the most part, the transcriptional initiation region will be for a gene which is expressed and translocated during germination, by employing the signal peptide which provides for translocation, one may also provide for translocation of the protein of interest. In this way, the protein(s) of interest will be translocated from the cells in which they are expressed and may be efficiently harvested. Typically secretion in seeds are across the aleurone or scutellar epithelium layer into the endosperm of the seed.

While it is not required that the protein be secreted from the cells in which the protein is produced, this facilitates the isolation and purification of the recombinant protein.

Since the ultimate expression of the desired gene product will be in a eucaryotic cell it is desirable to determine whether any portion of the cloned gene contains sequences which will be processed out as introns by the host's splicosome machinery. If so, site-directed mutagenesis of the "intron" region may be conducted to prevent losing a portion of the genetic message as a false intron code, Reed and Maniatis, Cell 41:95-105, 1985.

The vector can be microinjected directly into plant cells by use of micropipettes to mechanically transfer the recombinant DNA. Crossway, Mol. Gen. Genet, 202:179-185, 1985. The genetic material may also be transferred into the plant cell by using polyethylene glycol, Krens, et al., Nature, 296, 72-74, 1982. Another method of introduction of nucleic acid segments is high velocity ballistic penetration by small particles with the nucleic acid either within the matrix of small beads or particles, or on the surface, Klein, et al., Nature, 327, 70-73, 1987 and Knudsen and Muller, 1991, Planta, 185:330-336 teaching particle bombardment of barley endosperm to create transgenic barley. Yet another method of introduction would be fusion of protoplasts with other entities, either minicells, cells, lysosomes or other fusible lipid-surfaced bodies, Fraley, et al., Proc. Natl. Acad Sci. USA, 79, 1859-1863,1982.

The vector may also be introduced into the plant cells by electroporation. (Fromm et al., Proc. Natl Acad Sci. USA 82:5824, 1985). In this technique, plant protoplasts are electroporated in the presence of plasmids containing the gene construct. Electrical impulses of high field strength reversibly permeabilize biomembranes allowing the introduction of the plasmids. Electroporated plant protoplasts reform the cell wall, divide, and form plant callus.

All plants from which protoplasts can be isolated and cultured to give whole regenerated plants can be transformed by the present invention so that whole plants are recovered which contain the transferred gene. It is known that practically all plants can be regenerated from cultured cells or tissues, including but not limited to all major species of sugarcane, sugar beet, cotton, fruit and other trees, legumes and vegetables. Some suitable plants include, for example, species from the genera *Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Asparagus, Antirrhinum, Hererocallis, Nemesia, Pelargonium, Panicum, Pennisetum, Ranunculus, Senecio, Salpiglossis, Cucumis, Browaalia, Glycine, Lolium, Zea, Triticum, Sorghum,* and *Datura.*

Means for regeneration vary from species to species of plants, but generally a suspension of transformed protoplasts containing copies of the heterologous gene is first provided. Callus tissue is formed and shoots may be induced from callus and subsequently rooted. Alternatively, embryo formation can be induced from the protoplast suspension. These embryos germinate as natural embryos to form plants. The culture media will generally contain various amino acids and hormones, such as auxin and cytokinins. It is also advantageous to add glutamic acid and proline to the medium, especially for such species as corn and alfalfa. Shoots and roots normally develop simultaneously. Efficient regeneration will depend on the medium, on the genotype, and on the history of the culture. If these three variables are controlled, then regeneration is fully reproducible and repeatable.

In some plant cell culture systems, the desired protein of the invention may be excreted or alternatively, the protein may be extracted from the whole plant. Where the desired protein of the invention is secreted into the medium, it may be collected. Alternatively, the embryos and embryoless-half seeds or other plant tissue may be mechanically disrupted to release any secreted protein between cells and tissues. The mixture may be suspended in a buffer solution to retrieve soluble proteins. Conventional protein isolation and purification methods will be then used to purify the recombinant protein. Parameters of time, temperature pH, oxygen, and volumes will be adjusted through routine methods to optimize expression and recovery of heterologous protein.

iv. Bacterial Systems

Bacterial expression techniques are known in the art. A bacterial promoter is any DNA sequence capable of binding bacterial RNA polymerase and initiating the downstream (3') transcription of a coding sequence (*e.g.* structural gene) into mRNA. A promoter will have a transcription initiation region which is usually placed proximal to the 5' end of the coding sequence. This transcription initiation region usually includes an RNA polymerase binding site and a transcription initiation site. A bacterial promoter may also have a second domain called an operator, that may overlap an adjacent RNA polymerase binding site at which RNA synthesis begins. The operator permits negative regulated (inducible) transcription, as a gene repressor protein may bind the operator and thereby inhibit transcription of a specific gene. Constitutive expression may occur in the absence of negative regulatory elements, such as the operator. In addition, positive regulation may be achieved by a gene activator protein binding sequence, which, if present is usually proximal (5') to the RNA polymerase binding sequence. An example of a gene activator protein is the catabolite activator protein (CAP), which helps initiate transcription of the lac operon in Escherichia coli (E. coli) [Raibaud et al. (1984) Annu. Rev. Genet. 18:173]. Regulated expression may therefore be either positive or negative, thereby either enhancing or reducing transcription.

Sequences encoding metabolic pathway enzymes provide particularly useful promoter sequences. Examples include promoter sequences derived from sugar metabolizing enzymes, such as galactose, lactose (*lac*) [Chang et al. (1977) Nature 198:1056], and maltose. Additional examples include promoter sequences derived from biosynthetic enzymes such as tryptophan (*trp*) [Goeddel et al. (1980) Nuc. Acids Res. 8:4057; Yelverton et al. (1981) Nucl. Acids Res. 9:731; US patent 4,738,921; EP-A-0036776 and EP-A-0121775]. The g-laotamase (*bla*) promoter system [Weissmann (1981) "The cloning of interferon and other mistakes." In Interferon 3 (ed. I. Gresser)], bacteriophage lambda PL [Shimatake et al. (1981) Nature 292:128] and T5 [US patent 4,689,406] promoter systems also provide useful promoter sequences.

In addition, synthetic promoters which do not occur in nature also function as bacterial promoters. For example, transcription activation sequences of one bacterial or bacteriophage promoter may be joined with the operon sequences of another bacterial or bacteriophage promoter, creating a synthetic hybrid promoter [US patent 4,551,433]. For example, the *tac* promoter is a hybrid *trp-lac* promoter comprised of both trp promoter and *lac* operon sequences that is regulated by the *lac* repressor [Amann et al. (1983) Gene 25:167; de Boer et al. (1983) Proc. Natl. Acad. Sci. 80:21]. Furthermore, a bacterial promoter can include naturally occurring promoters of non-bacterial origin that have the ability to bind bacterial RNA polymerase and initiate transcription. A naturally occurring promoter of non-bacterial origin can also be coupled with a compatible RNA polymerase to produce high levels of expression of some genes in prokaryotes. The bacteriophage T7 RNA polymerase/promoter system is an example of a coupled promoter system [Studier et al. (1986) J. Mol. Biol. 189:113; Tabor et al. (1985) Proc Natl. Acad Sci. 82:1074]. In addition, a hybrid promoter can also be comprised of a bacteriophage promoter and an *E. coli* operator region (EPO-A-0 267 851).

In addition to a functioning promoter sequence, an efficient ribosome binding site is also useful for the expression of foreign genes in prokaryotes. In *E. coli,* the ribosome binding site is called the Shine-Dalgarno (SD) sequence and includes an initiation codon (ATG) and a sequence 3-9 nucleotides in length located 3-11 nucleotides upstream of the initiation codon [Shine et al. (1975) Nature 254:34]. The SD sequence is thought to promote binding of mRNA to the ribosome by the pairing of bases between the SD sequence and the 3' and of *E. coli* 16S rRNA [Steitz et al. (1979) "Genetic signals and nucleotide sequences in messenger RNA." In Biological Regulation and Development: Gene Expression (ed. R.F. Goldberger)]. To express eukaryotic genes and prokaryotic genes with weak ribosome-binding site [Sambrook et al. (1989) "Expression of cloned genes in Escherichia coli." In Molecular Cloning: A Laboratry Manual].

A DNA molecule may be expressed intracellularly. A promoter sequence may be directly linked with the DNA molecule, in which case the first amino acid at the N-terminus will always be a methionine, which is encoded by the ATG start codon. If desired, methionine at the N-terminus may be cleaved from the protein by *in vitro* incubation with cyanogen bromide or by either *in vivo* on *in vitro* incubation with a bacterial methionine N-terminal peptidase (EPO-A-0 219 237).

Fusion proteins provide an alternative to direct expression. Usually, a DNA sequence encoding the N-terminal portion of an endogenous bacterial protein, or other stable protein, is fused to the 5' end of heterologous coding sequences. Upon expression, this construct will provide a fusion of the two amino acid sequences. For example, the bacteriophage lambda cell gene can be linked at the 5' terminus of a foreign gene and expressed in bacteria. The resulting fusion protein preferably retains a site for a processing enzyme (factor Xa) to cleave the bacteriophage protein from the foreign gene [Nagai et al. (1984) Nature 309:810]. Fusion proteins can also be made with sequences from the *lac*Z [Jia et al. (1987) Gene 60:197], *trp*E [Allen et al. (1987) J. Biotechnol. 5:93; Makoff et al. (1989) J. Gen. Microbiol. 135:11], and Chey [EP-A-0 324 647] genes. The DNA sequence at the junction of the two amino acid sequences may or may not encode a cleavable site. Another example is a ubiquitin fusion protein. Such a fusion protein is made with the ubiquitin region that preferably retains a site for a processing enzyme (*e.g.* ubiquitin specific processing-protease) to cleave the ubiquitin from the foreign protein. Through this method, native foreign protein can be isolated [Miller et al. (1989) Bio/ Technology 7:698].

Alternatively, foreign proteins can also be secreted from the cell by creating chimeric DNA molecules that encode a fusion protein comprised of a signal peptide sequence fragment that provides for secretion of the foreign protein in bacteria [US patent 4,336,336]. The signal sequence fragment usually encodes a signal peptide comprised of hydrophobic amino acids which direct the secretion of the protein from the cell. The protein is either secreted into the growth media (gram-positive bacteria) or into the periplasmic space, located between the inner and outer membrane of the cell (gram-negative bacteria). Preferably there are processing sites, which can be cleaved either *in vivo* or *in vitro* encoded between the signal peptide fragment and the foreign gene.

DNA encoding suitable signal sequences can be derived from genes for secreted bacterial proteins, such as the *E. coli* outer membrane protein gene (*ompA*) [Masui et al. (1983), in: Experimental Manipulation of Gene Expression; Ghrayeb et al. (1984) EMBO J. 3:2437] and the *E. coli* alkaline phosphatase signal sequence (*phoA*) [Oka et al. (1985) Proc. Natl. Acad. Sci. 82:7212]. As an additional example, the signal sequence of the alpha-amylase gene from various Bacillus strains can be used to secrete heterologous proteins from *B. subtilis* [Palva et al. (1982) Proc. Natl. Acad. Sci. USA 79:5582; EP-A-0 244 042].

Usually, transcription termination sequences recognized by bacteria are regulatory regions located 3' to the translation stop codon, and thus together with the promoter flank the coding sequence. These sequences direct the transcription of an mRNA which can be translated into the polypeptide encoded by the DNA. Transcription termination sequences frequently include DNA sequences of about 50 nucleotides capable of forming stem loop structures that aid in terminating transcription. Examples include transcription termination sequences derived from genes with strong promoters, such as the trp gene in *E. coli* as well as other biosynthetic genes.

Usually, the above described components, comprising a promoter, signal sequence (if desired), coding sequence of interest, and transcription termination sequence, are put together into expression constructs. Expression constructs are often maintained in a replicon, such as an extrachromosomal element (*e.g.* plasmids) capable of stable maintenance in a host, such as bacteria. The replicon will have a replication system, thus allowing it to be maintained in a prokaryotic host either for expression or for cloning and amplification. In addition, a replicon may be either a high or low copy number plasmid. A high copy number plasmid will generally have a copy number ranging from about 5 to about 200, and usually about 10 to about 150. A host containing a high copy number plasmid will preferably contain at least about 10, and more preferably at least about 20 plasmids. Either a high or low copy number vector may be selected, depending upon the effect of the vector and the foreign protein on the host.

Alternatively, the expression constructs can be integrated into the bacterial genome with an integrating vector. Integrating vectors usually contain at least one sequence homologous to the bacterial chromosome that allows the vector to integrate. Integrations appear to result from recombinations between homologous DNA in the vector and the bacterial chromosome. For example, integrating vectors constructed with DNA from various Bacillus strains integrate into the Bacillus chromosome (EP-A- 0 127 328). Integrating vectors may also be comprised of bacteriophage or transposon sequences.

Usually, extrachromosomal and integrating expression constructs may contain selectable markers to allow for the selection of bacterial strains that have been transformed. Selectable markers can be expressed in the bacterial host and may include genes which render bacteria resistant to drugs such as ampicillin, chloramphenicol, erythromycin, kanamycin (neomycin), and tetracycline [Davies et al. (1978) Annu. Rev. Microbiol. 32:469]. Selectable markers may also include biosynthetic genes, such as those in the histidine, tryptophan, and leucine biosynthetic pathways.

Alternatively, some of the above described components can be put together in transformation vectors. Transformation vectors are usually comprised of a selectable market that is either maintained in a replicon or developed into an integrating vector, as described above.

Expression and transformation vectors, either extra-chromosomal replicons or integrating vectors, have been developed for transformation into many bacteria. For example, expression vectors have been developed for, *inter alia,* the following bacteria: Bacillus subtilis [Palva et al. (1982) Proc. Natl. Acad. Sci. USA 79:5582; EP-A-0 036 259 and EP-A-0 063 953; WO 84/04541], Escherichia coli [Shimatake et al. (1981) Nature 292:128; Amann et al. (1985) Gene 40: 183; Studier et al. (1986) J. Mol. Biol. 189:113; EP-A-0 036 776,EP-A-0 136 829 and EP-A-0 136 907], Streptococcus cremoris [Powell et al. (1988) Appl. Environ. Microbiol. 54:655]; Streptococcus lividans [Powell et al. (1988) Appl. Environ. Microbiol. 54:655], Streptomyces lividans [US patent 4,745,056].

Methods of introducing exogenous DNA into bacterial hosts are well-known in the art, and usually include either the transformation of bacteria treated with $CaCl_2$ or other agents, such as divalent cations and DMSO. DNA can also be introduced into bacterial cells by electroporation. Transformation procedures usually vary with the bacterial species to be transformed. See *e.g.* [Masson et al. (1989) FEMS Microbiol. Lett. 60:273; Palva et al. (1982) Proc. Natl. Acad. Sci. USA 79:5582; EP-A-0 036 259 and EP-A-0 063 953; WO 84/04541, Bacillus], [Miller et al. (1988) Proc. Natl. Acad. Sci. 85:856; Wang et al. (1990) J. Bacteriol. 172:949, Campylobacter], [Cohen et al. (1973) Proc. Natl. Acad. Sci. 69: 2110; Dower et al. (1988) Nucleic Acids Res. 16:6127; Kushner (1978) "An improved method for transformation of Escherichia coli with ColE1-derived plasmids. In Genetic Engineering: Proceedings of the International Symposium on Genetic Engineering (eds. H.W. Boyer and S. Nicosia); Mandel et al. (1970) J. Mol. Biol. 53:159; Taketo (1988) Biochim. Biophys. Acta 949:318; Escherichia], [Chassy et al. (1987) FEMS Microbiol. Lett. 44:173 Lactobacillus]; [Fiedler et al. (1988) Anal. Biochem 170:38, Pseudomonas]; [Augustin et al. (1990) FEMS Microbiol. Lett. 66:203, Staphylococcus], [Barany et al. (1980) J. Bacteriol. 144:698; Harlander (1987) "Transformation of Streptococcus lactis by electroporation, in: Streptococcal Genetics (ed. J. Ferretti and R. Curtiss III); Perry et al. (1981) Infect. Immun. 32:1295; Powell et al. (1988) Appl. Environ. Microbiol. 54:655; Somkuti et al. (1987) Proc. 4th Evr. Cong. Biotechnology 1:412, Streptococcus].

v. Yeast Expression

Yeast expression systems are also known to one of ordinary skill in the art. A yeast promoter is any DNA sequence capable of binding yeast RNA polymerase and initiating the downstream (3') transcription of a coding sequence (*e.g.* structural gene) into mRNA. A promoter will have a transcription initiation region which is usually placed proximal to the 5' end of the coding sequence. This transcription initiation region usually includes an RNA polymerase binding site (the "TATA Box") and a transcription initiation site. A yeast promoter may also have a second domain called an upstream activator sequence (UAS), which, if present, is usually distal to the structural gene. The UAS permits regulated (inducible) expression. Constitutive expression occurs in the absence of a UAS. Regulated expression may be either positive or negative, thereby either enhancing or reducing transcription.

Yeast is a fermenting organism with an active metabolic pathway, therefore sequences encoding enzymes in the metabolic pathway provide particularly useful promoter sequences. Examples include alcohol dehydrogenase (ADH) (EP-A-0 284 044), enolase, glucokinase, glucose-6-phosphate isomerase, glyceraldehyde-3-phosphate-dehydrogenase (GAP or GAPDH), hexokinase, phosphofructokinase, 3-phosphoglycerate mutase, and pyruvate kinase (PyK) (EPO-A-0 329 203). The yeast *PHO5* gene, encoding acid phosphatase, also provides useful promoter sequences [Myanohara et al. (1983) Proc. Natl. Acad. Sci. USA 80:1].

In addition, synthetic promoters which do not occur in nature also function as yeast promoters. For example, UAS sequences of one yeast promoter may be joined with the transcription activation region of another yeast promoter, creating a synthetic hybrid promoter. Examples of such hybrid promoters include the ADH regulatory sequence linked to the GAP transcription activation region (US Patent Nos. 4,876,197 and 4,880,734). Other examples of hybrid promoters include promoters which consist of the regulatory sequences of either the *ADH2, GAL4, GAL10,* OR *PHO5* genes, combined with the transcriptional activation region of a glycolytic enzyme gene such as GAP or PyK (EP-A-0 164 556). Furthermore, a yeast promoter can include naturally occurring promoters of non-yeast origin that have the ability to bind yeast RNA polymerase and initiate transcription. Examples of such promoters include, *inter alia*, [Cohen et al. (1980) Proc. Natl. Acad. Sci. USA 77:1078; Henikoff et al. (1981) Nature 283:835; Hollenberg et al. (1981) Curr. Topics Microbiol. Immunol. 96:119; Hollenberg et al. (1979) "The Expression of Bacterial Antibiotic Resistance Genes in the Yeast Saccharomyces cerevisiae," in: Plasmids of Medical, Environmental and Commercial Importance (eds. K.N. Timmis and A. Puhler); Mercerau-Puigalon et al. (1980) Gene 11:163; Panthier et al. (1980) Curr. Genet. 2:109;].

A DNA molecule may be expressed intracellularly in yeast. A promoter sequence may be directly linked with the DNA molecule, in which case the first amino acid at the N-terminus of the recombinant protein will always be a methionine, which is encoded by the ATG start codon. If desired, methionine at the N-terminus may be cleaved from the protein by *in vitro* incubation with cyanogen bromide.

Fusion proteins provide an alternative for yeast expression systems, as well as in mammalian, baculovirus, and bacterial expression systems. Usually, a DNA sequence encoding the N-terminal portion of an endogenous yeast protein, or other stable protein, is fused to the 5' end of heterologous coding sequences. Upon expression, this construct will provide a fusion of the two amino acid sequences. For example, the yeast or human superoxide dismutase (SOD) gene, can be linked at the 5' terminus of a foreign gene and expressed in yeast. The DNA sequence at the junction of the two amino acid sequences may or may not encode a cleavable site. See *e.g.* EP-A-0 196 056. Another example is a ubiquitin fusion protein. Such a fusion protein is made with the ubiquitin region that preferably retains a site for a processing enzyme (*e.g.* ubiquitin-specific processing protease) to cleave the ubiquitin from the foreign protein. Through this method, therefore, native foreign protein can be isolated (*e.g.* WO88/024066).

Alternatively, foreign proteins can also be secreted from the cell into the growth media by creating chimeric DNA molecules that encode a fusion protein comprised of a leader sequence fragment that provide for secretion in yeast of the foreign protein. Preferably, there are processing sites encoded between the leader fragment and the foreign gene that can be cleaved either *in vivo* or *in vitro.* The leader sequence fragment usually encodes a signal peptide comprised of hydrophobic amino acids which direct the secretion of the protein from the cell.

DNA encoding suitable signal sequences can be derived from genes for secreted yeast proteins, such as the genes for invertase (EP-A-0012873; JPO 62,096,086) and A-factor (US patent 4,588,684). Alternatively, leaders of non-yeast origin exit, such as an interferon leader, that also provide for secretion in yeast (EP-A-0060057).

A preferred class of secretion leaders are those that employ a fragment of the yeast alpha-factor gene, which contains both a "pre" signal sequence, and a "pro" region. The types of alpha-factor fragments that can be employed include the full-length pre-pro alpha factor leader (about 83 amino acid residues) as well as truncated alpha-factor leaders (usually about 25 to about 50 amino acid residues) (US Patents 4,546,083 and 4,870,008; EP-A-0 324 274). Additional leaders employing an alpha-factor leader fragment that provides for secretion include hybrid alpha-factor leaders made with a presequence of a first yeast, but a pro-region from a second yeast alphafactor. (*e.g.* see WO 89/02463.)

Usually, transcription termination sequences recognized by yeast are regulatory regions located 3' to the translation stop codon, and thus together with the promoter flank the coding sequence. These sequences direct the transcription of an mRNA which can be translated into the polypeptide encoded by the DNA. Examples of transcription terminator sequence and other yeast-recognized termination sequences, such as those coding for glycolytic enzymes.

Usually, the above described components, comprising a promoter, leader (if desired), coding sequence of interest, and transcription termination sequence, are put together into expression constructs. Expression constructs are often maintained in a replicon, such as an extrachromosomal element (*e.g.* plasmids) capable of stable maintenance in a host, such as yeast or bacteria. The replicon may have two replication systems, thus allowing it to be maintained, for example, in yeast for expression and in a prokaryotic host for cloning and amplification. Examples of such yeast-bacteria shuttle vectors include YEp24 [Botstein et al. (1979) Gene 8:17-24], pCl/l [Brake et al. (1984) Proc. Natl. Acad Sci USA 81:4642-4646], and YRp17 [Stinchcomb et al. (1982) J. Mol. Biol. 158:157]. In addition, a replicon may be either a high or low copy number plasmid. A high copy number plasmid will generally have a copy number ranging from about 5 to about 200, and usually about 10 to about 150. A host containing a high copy number plasmid will preferably have at least about 10, and more preferably at least about 20. Enter a high or low copy number vector may be selected, depending upon the effect of the vector and the foreign protein on the host. See *e.g.* Brake *et al., supra.*

Alternatively, the expression constructs can be integrated into the yeast genome with an integrating vector. Integrating vectors usually contain at least one sequence homologous to a yeast chromosome that allows the vector to integrate, and preferably contain two homologous sequences flanking the expression construct. Integrations appear to result from recombinations between homologous DNA in the vector and the yeast chromosome [Orr-Weaver et al. (1983) Methods in Enzymol. 101:228-245]. An integrating vector may be directed to a specific locus in yeast by selecting the appropriate homologous sequence for inclusion in the vector. See Orr-Weaver *et al., supra.* One or more expression construct may integrate, possibly affecting levels of recombinant protein produced [Rine et al. (1983) Proc. Natl. Acad. Sci. USA 80:6750]. The chromosomal sequences included in the vector can occur either as a single segment in the vector, which results in the integration of the entire vector, or two segments homologous to adjacent segments in the chromosome and flanking the expression construct in the vector, which can result in the stable integration of only the expression construct.

Usually, extrachromosomal and integrating expression constructs may contain selectable markers to allow for the selection of yeast strains that have been transformed. Selectable markers may include biosynthetic genes that can be expressed in the yeast host, such as *ADE2, HIS4, LEU2, TRP1*, and *ALG7,* and the G418 resistance gene, which confer resistance in yeast cells to tunicamycin and G418, respectively. In addition, a suitable selectable marker may also provide yeast with the ability to grow in the presence of toxic compounds, such as metal. For example, the presence of *CUP1* allows yeast to grow in the presence of copper ions [Butt et al. (1987) Microbiol, Rev. 51:351].

Alternatively, some of the above described components can be put together into transformation vectors. Transformation vectors are usually comprised of a selectable marker that is either maintained in a replicon or developed into an integrating vector, as described above.

Expression and transformation vectors, either extrachromosomal replicons or integrating vectors, have been developed for transformation into many yeasts. For example, expression vectors have been developed for, *inter alia,* the following yeasts:Candida albicans [Kurtz, et al. (1986) Mol. Cell. Biol. 6:142], Candida maltosa [Kunze, et al. (1985) J. Basic Microbiol. 25:141]. Hansenula polymorpha [Gleeson, et al. (1986) J. Gen. Microbiol. 132:3459; Roggenkamp et al. (1986) Mol. Gen. Genet. 202:302], Kluyveromyces fragilis [Das, et al. (1984) J. Bacteriol. 158:1165], Kluyveromyces lactis [De Louvencourt et al. (1983) J. Bacteriol. 154:737; Van den Berg et al. (1990) Bio/Technology 8:135], Pichia guillerimondii [Kunze et al. (1985) J. Basic Microbiol. 25:141], Pichia pastoris [Cregg, et al. (1985) Mol. Cell. Biol. 5: 3376; US Patent Nos. 4,837,148 and 4,929,555], Saccharomyces cerevisiae [Hinnen et al. (1978) Proc. Natl. Acad. Sci. USA 75:1929; Ito et al. (1983) J. Bacteriol. 153:163], Schizosaccharomyces pombe [Beach and Nurse (1981) Nature 300:706], and Yarrowia lipolytica [Davidow, et al. (1985) Curr. Genet. 10:380471 Gaillardin, et al. (1985) Curr. Genet. 10:49].

Methods of introducing exogenous DNA into yeast hosts are well-known in the art, and usually include either the transformation of spheroplasts or of intact yeast cells treated with alkali cations. Transformation procedures usually vary with the yeast species to be transformed. See *e.g.* [Kurtz et al. (1986) Mol. Cell. Biol. 6:142; Kunze et al. (1985) J. Basic Microbiol. 25:141; Candida]; [Gleeson et al. (1986) J. Gen. Microbiol. 132:3459; Roggenkamp et al. (1986) Mol. Gen. Genet. 202:302; Hansenula]; [Das et al. (1984) J. Bacteriol. 158:1165; De Louvencourt et al. (1983) J. Bacteriol. 154:1165; Van den Berg et al. (1990) Bio/Technology 8:135; Kluyveromyces]; [Cregg et al. (1985) Mol. Cell. Biol. 5: 3376; Kunze et al. (1985) J. Basic Microbiol. 25:141; US Patents 4,837,148 & 4,929,555; Pichia]; [Hinnen et al. (1978) Proc. Natl. Acad. Sci. USA 75;1929; Ito et al. (1983) J. Bacteriol. 153:163 Saccharomyces]; [Beach & Nurse (1981) Nature 300:706; Schizosaccharomyces]; [Davidow et al. (1985) Curr. Genet. 10:39; Gaillardin et al. (1985) Curr. Genet. 10:49; Yarrowia].

*Pharmaceutical Compositions*

Pharmaceutical compositions can comprise polypeptides and/or nucleic acid of the invention. The pharmaceutical compositions will comprise a therapeutically effective amount of either polypeptides, antibodies, or polynucleotides of the claimed invention.

The term "therapeutically effective amount" as used herein refers to an amount of a therapeutic agent to treat, ameliorate, or prevent a desired disease or condition, or to exhibit a detectable therapeutic or preventative effect.

The effect can be detected by, for example, chemical markers or antigen levels. Therapeutic effects also include reduction in physical symptoms, such as decreased body temperature. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgement of the clinician.

For purposes of the present invention, an effective dose will be from about 0.01 mg/ kg to 50 mg/kg or 0.05 mg/kg to about 10 mg/kg of the DNA constructs in the individual to which it is administered.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

*Delivery Methods*

Once formulated, the compositions of the invention can be administered directly to the subject. The subjects to be treated can be animals; in particular, human subjects can be treated.

Direct delivery of the compositions will generally be accomplished by injection, either subcutaneously, intraperitoneally, intravenously or intramuscularly or delivered to the interstitial space of a tissue. The compositions can also be administered into a lesion. Other modes of administration include oral and pulmonary administration, suppositories, and transdermal or transcutaneous applications (*e.g.* see WO98/20734), needles, and gene guns or hyposprays. Dosage treatment may be a single dose schedule or a multiple dose schedule.

*Vaccines*

Vaccines according to the invention may either be prophylactic (ie. to prevent infection) or therapeutic (ie. to treat disease after infection).

Such vaccines comprise immunising antigen(s), immunogen(s), polypeptide(s), protein(s) or nucleic acid, usually in combination with "pharmaceutically acceptable carriers," which include any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. Such carriers are well known to those of ordinary skill in the art. Additionally, these carriers may function as immunostimulating agents ("adjuvants"). Furthermore, the antigen or immunogen may be conjugated to a bacterial toxoid, such as a toxoid from diphtheria, tetanus, cholera, H. *pylori, etc.* pathogens.

Preferred adjuvants to enhance effectiveness of the composition include, but are not limited to: (1) aluminum salts (alum), such as aluminum hydroxide, aluminum phosphate, aluminum sulfate, etc; (2) oil-in-water emulsion formulations (with or without other specific immunostimulating agents such as muramyl peptides (see below) or bacterial cell wall components), such as for example (a) MF59™ (WO 90/14837; Chapter 10 in *Vaccine design: the subunit and adjuvant approach,* eds. Powell & Newman, Plenum Press 1995), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); (3) saponin adjuvants, such as Stimulon™ (Cambridge Bioscience, Worcester, MA) may be used or particles generated therefrom such as ISCOMs (immunostimulating complexes); (4) Complete Freund's Adjuvant (CFA) and Incomplete Freund's Adjuvant (IFA); (5) cytokines, such as interleukins (*e.g.* IL-1, IL-2, IL-4, IL-5, IL-6, IL-7, IL-12, *etc.),* interferons (*e.g.* gamma interferon), macrophage colony stimulating factor (M-CSF), tumor necrosis factor (TNF), etc; and (6) other substances that act as immunostimulating agents to enhance the effectiveness of the composition. Alum and MF59™ are preferred.

As mentioned above, muramyl peptides include, but are not limited to, N-acetyl-muramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl-L-alanine-2-(1'-2'-dipalmitoyl-*sn*-glycero-3-hydroxyphosphoryloxy)-ethylamine (MTP-PE), *etc.*

The immunogenic compositions (*e.g.* the immunising antigen/immunogen/polypeptide/protein/ nucleic acid, pharmaceutically acceptable carrier, and adjuvant) typically will contain diluents, such as water, saline, glycerol, ethanol, etc. Additionally, auxiliary substances, such as wetting or emulsifying agents, pH buffering substances, and the like, may be present in such vehicles.

Typically, the immunogenic compositions are prepared as injectables, either as liquid solutions or suspensions; solid forms suitable for solution in, or suspension in, liquid vehicles prior to injection may also be prepared. The preparation also may be emulsified or encapsulated in liposomes for enhanced adjuvant effect, as discussed above under pharmaceutically acceptable carriers.

Immunogenic compositions used as vaccines comprise an immunologically effective amount of the antigenic or immunogenic polypeptides, as well as any other of the above-mentioned components, as needed. By "immunologically effective amount", it is meant that the administration of that amount to an individual, either in a single dose or as part of a series, is effective for treatment or prevention. This amount varies depending upon the health and physical condition of the individual to be treated, the taxonomic group of individual to be treated (*e.g.* nonhuman primate, primate, *etc.*), the capacity of the individual's immune system to synthesize antibodies, the degree of protection desired, the formulation of the vaccine, the treating doctor's assessment of the medical situation, and other relevant factors. It is expected that the amount will fall in a relatively broad range that can be determined through routine trials.

The immunogenic compositions are conventionally administered parenterally, *e.g.* by injection, either subcutaneously, intramuscularly, or transdermally/transcutaneously (*e.g.* WO98/20734). Additional formulations suitable for other modes of administration include oral and pulmonary formulations, suppositories, and transdermal applications. Dosage treatment may be a single dose schedule or a multiple dose schedule. The vaccine may be administered in conjunction with other immunoregulatory agents.

As an alternative to protein-based vaccines, DNA vaccination may be employed [*e.g.* Robinson & Torres (1997) Seminars in Immunology 9:271-283; Donnelly et al. (1997) Annu Rev Immunol 15:617-648; see later herein].

*Gene Delivery Vehicles*

Gene therapy vehicles for delivery of constructs including a coding sequence of a therapeutic of the invention, to be delivered to the mammal for expression in the mammal, can be administered either locally or systemically.

These constructs can utilize viral or non-viral vector approaches in *in vivo* or *ex vivo* modality. Expression of such coding sequence can be induced using endogenous mammalian or heterologous promoters. Expression of the coding sequence in vivo can be either constitutive or regulated.

The invention includes gene delivery vehicles capable of expressing the contemplated nucleic acid sequences.

The gene delivery vehicle is preferably a viral vector and, more preferably, a retroviral, adenoviral, adeno-associated viral (AAV), herpes viral, or alphavirus vector. The viral vector can also be an astrovirus, coronavirus, orthomyxovirus, papovavirus, paramyxovirus, parvovirus, picornavirus, poxvirus, or togavirus viral vector. See generally, Jolly (1994) Cancer Gene Therapy 1:51-64; Kimura (1994) Human Gene Therapy 5:845-852; Connelly (1995) Human Gene Therapy 6:185-193; and Kaplitt (1994) Nature Genetics 6:148-153.

These recombinant retroviral vectors may be used to generate transduction competent retroviral vector particles by introducing them into appropriate packaging cell lines (see US patent 5,591,624). Retrovirus vectors can be constructed for site-specific integration into host cell DNA by incorporation of a chimeric integrase enzyme into the retroviral particle (see WO96/37626). It is preferable that the recombinant viral vector is a replication defective recombinant virus.

Packaging cell lines suitable for use with the above-described retrovirus vectors are well known in the art, are readily prepared (see WO95/30763 and WO92/05266), and can be used to create producer cell lines (also termed vector cell lines or "VCLs") for the production of recombinant vector particles. Preferably, the packaging cell lines are made from human parent cells (*e.g.* HT1080 cells) or mink parent cell lines, which eliminates inactivation in human serum.

Preferred retroviruses for the construction of retroviral gene therapy vectors include Avian Leukosis Virus, Bovine Leukemia, Virus, Murine Leukemia Virus, Mink-Cell Focus-Inducing Virus, Murine Sarcoma Virus, Reticuloendotheliosis Virus and Rous Sarcoma Virus. Particularly preferred Murine Leukemia Viruses include 4070A and 1504A (Hartley and Rowe (1976) J Virol 19:19-25), Abelson (ATCC No. VR-999), Friend (ATCC No. VR-245), Graffi, Gross (ATCC Nol VR-590), Kirsten, Harvey Sarcoma Virus and Rauscher (ATCC No. VR-998) and Moloney Murine Leukemia Virus (ATCC No. VR-190). Such retroviruses may be obtained from depositories or collections such as the American Type Culture Collection ("ATCC") in Rockville, Maryland or isolated from known sources using commonly available techniques.

The gene therapy vectors of the invention also include herpes vectors. Leading and preferred examples are herpes simplex virus vectors containing a sequence encoding a thymidine kinase polypeptide such as those disclosed in US 5,288,641 and EP0176170 (Roizman). Additional exemplary herpes simplex virus vectors include HFEM/ICP6-LacZ disclosed in WO95/04139 (Wistar), pHSVlac described in Geller (1988) Science 241:1667-1669 and in WO90/09441 & WO92/07945, HSV Us3::pgC-lacZ described in Fink (1992) Human Gene Therapy 3:11-19 and HSV 7134, 2 RH 105 and GAL4 described in EP 0453242 (Breakefield), and those deposited with ATCC as accession numbers ATCC VR-977 and ATCC VR-260.

Also contemplated are alpha virus gene therapy vectors that can be employed in this invention. Preferred alpha virus vectors are Sindbis viruses vectors. Togaviruses, Semliki Forest virus (ATCC VR-67; ATCC VR-1247), Middleberg virus (ATCC VR-370), Ross River virus (ATCC VR-373; ATCC VR-1246), Venezuelan equine encephalitis virus (ATCC VR923; ATCC VR-1250; ATCC VR-1249; ATCC VR-532), and those described in US patents 5,091,309, 5,217,879, and WO92/10578. More particularly, those alpha virus vectors described in US Serial No. 08/405,627, filed March 15, 1995, WO94/21792, WO92/10578, WO95/07994, US 5,091,309 and

US 5,217,879 are employable. Such alpha viruses may be obtained from depositories or collections such as the ATCC in Rockville, Maryland or isolated from known sources using commonly available techniques. Preferably, alphavirus vectors with reduced cytotoxicity are used (see USSN 08/679640).

DNA vector systems such as eukaryotic layered expression systems are also useful for expressing the nucleic acids of the invention. See WO95/07994 for a detailed description of eukaryotic layered expression systems. Preferably, the eukaryotic layered expression systems of the invention are derived from alphavirus vectors and most preferably from Sindbis viral vectors.

Other viral vectors suitable for use in the present invention include those derived from poliovirus, for example ATCC VR-58 and those described in Evans, Nature 339 (1989) 385 and Sabin (1973) J. Biol. Standardization 1:115; rhinovirus, for example ATCC VR-1110 and those described in Arnold (1990) J Cell Biochem L401; pox viruses such as canary pox virus or vaccinia virus, for example ATCC VR-111 and ATCC VR-2010 and those described in Fisher-Hoch (1989) Proc Natl Acad Sci 86:317; Flexner (1989) Ann NY Acad Sci 569:86, Flexner (1990) Vaccine 8:17; in US 4,603,112 and US 4,769,330 and WO89/01973; SV40 virus, for example ATCC VR-305 and those described in Mulligan (1979) Nature 277:108 and Madzak (1992) J Gen Virol 73:1533; influenza virus, for example ATCC VR-797 and recombinant influenza viruses made employing reverse genetics techniques as described in US 5,166,057 and in Enami (1990) Proc Natl Acad Sci 87:3802-3805; Enami & Palese (1991) J Virol 65:2711-2713 and Luytjes (1989) Cell 59:110, (see also McMichael (1983) NEJ Med 309:13, and Yap (1978) Nature 273:238 and Nature (1979) 277:108); human immunodeficiency virus as described in EP-0386882 and in Buchschacher (1992) J. Virol. 66:2731; measles virus, for example ATCC VR-67 and VR-1247 and those described in EP-0440219; Aura virus, for example ATCC VR-368; Bebaru virus, for example ATCC VR-600 and ATCC VR-1240; Cabassou virus, for example ATCC VR-922; Chikungunya virus, for example ATCC VR-64 and ATCC VR-1241; Fort Morgan Virus, for example ATCC VR-924; Getah virus, for example ATCC VR-369 and ATCC VR-1243; Kyzylagach virus, for example ATCC VR-927; Mayaro virus, for example ATCC VR-66; Mucambo virus, for example ATCC VR-580 and ATCC VR-1244; Ndumu virus, for example ATCC VR-371; Pixuna virus, for example ATCC VR-372 and ATCC VR-1245; Tonate virus, for example ATCC VR-925; Triniti virus, for example ATCC VR-469; Una virus, for example ATCC VR-374; Whataroa virus, for example ATCC VR-926; Y-62-33 virus, for example ATCC VR-375; O'Nyong virus, Eastern encephalitis virus, for example ATCC VR-65 and ATCC VR-1242; Western encephalitis virus, for example pATCC VR-70, ATCC VR-1251, ATCC VR-622 and ATCC VR-1252; and coronavirus, for example ATCC VR-740 and those described in Hamre (1966) Proc Soc Exp Biol Med 121:190.

Delivery of the compositions of this invention into cells is not limited to the above mentioned viral vectors. Other delivery methods and media may be employed such as, for example, nucleic acid expression vectors, polycationic condensed DNA linked or unlinked to killed adenovirus alone, for example see US Serial No. 08/366,787, filed December 30, 1994 and Curiel (1992) Hum Gene Ther 3:147-154 ligand linked DNA, for example see Wu (1989) J Biol Chem 264: 16985-16987, eucaryotic cell delivery vehicles cells, for example see US Serial No.08/240,030, filed May 9, 1994, and US Serial No. 08/404,796, deposition of photopolymerized hydrogel materials, hand-held gene transfer particle gun, as described in US Patent 5,149,655, ionizing radiation as described in US5,206,152 and in WO92/11033, nucleic charge neutralization or fusion with cell membranes. Additional approaches are described in Philip (1994) Mol Cell Biol 14: 2411-2418 and in Woffendin (1994) Proc Natl Acad Sci 91:1581-1585.

Particle mediated gene transfer may be employed, for example see US Serial No. 60/023,867. Briefly, the sequence can be inserted into conventional vectors that contain conventional control sequences for high level expression, and then incubated with synthetic gene transfer molecules such as polymeric DNA-binding cations like polylysine, protamine, and albumin, linked to cell targeting ligands such as asialoorosomucoid, as described in Wu & Wu (1987) J. Biol. Chem. 262:4429-4432, insulin as described in Hucked (1990) Biochem Pharmacol 40:253-263, galactose as described in Plank (1992) Bioconjugate Chem 3:533-539, lactose or transferrin.

Naked DNA may also be employed. Exemplary naked DNA introduction methods are described in WO90/11092 and US 5,580,859. Uptake efficiency may be improved using biodegradable latex beads. DNA coated latex beads are efficiently transported into cells after endocytosis initiation by the beads. The method may be improved further by treatment of the beads to increase hydrophobicity and thereby facilitate disruption of the endosome and release of the DNA into the cytoplasm.

Liposomes that can act as gene delivery vehicles are described in US 5,422,120, WO95/13796, WO94/23697, WO91/14445 and EP-524,968. As described in USSN. 60/023,867, on non-viral delivery, the nucleic acid sequences encoding a polypeptide can be inserted into conventional vectors that contain conventional control sequences for high level expression, and then be incubated with synthetic gene transfer molecules such as polymeric DNA-binding cations like polylysine, protamine, and albumin, linked to cell targeting ligands such as asialoorosomucoid, insulin, galactose, lactose, or transferrin. Other delivery systems include the use of liposomes to encapsulate DNA comprising the gene under the control of a variety of tissue-specific or ubiquitously-active promoters. Further non-viral delivery suitable for use includes mechanical delivery systems such as the approach described in Woffendin et al (1994) Proc. Natl. Acad. Sci. USA 91(24):11581-11585. Moreover, the coding sequence and the product of expression of such can be delivered through deposition of photopolymerized hydrogel materials. Other conventional methods for gene delivery that can be used for delivery of the coding sequence include, for example, use of hand-held gene transfer particle gun, as described in US 5,149,655; use of ionizing radiation for activating transferred gene, as described in US 5,206,152 and WO92/11033

Exemplary liposome and polycationic gene delivery vehicles are those described in US 5,422,120 and 4,762,915; in WO 95/13796; WO94/23697; and WO91/14445; in EP-0524968; and in Stryer, Biochemistry, pages 236-240 (1975) W.H. Freeman, San Francisco; Szoka (1980) Biochem Biophys Acta 600:1; Bayer (1979) Biochem Biophys Acta 550:464; Rivnay (1987) Meth Enzymol 149:119; Wang (1987) Proc Natl Acad Sci 84:7851; Plant (1989) Anal Biochem 176:420.

A polynucleotide composition can comprises therapeutically effective amount of a gene therapy vehicle, as the term is defined above. For purposes of the present invention, an effective dose will be from about 0.01 mg/ kg to 50 mg/kg or 0.05 mg/kg to about 10 mg/kg of the DNA constructs in the individual to which it is administered.

*Delivery Methods*

Once formulated, the polynucleotide compositions of the invention can be administered (1) directly to the subject; (2) delivered *ex vivo,* to cells derived from the subject; or (3) *in vitro* for recombinant protein expression. The subjects to be treated can be mammals or birds. Also, human subjects can be treated.

Direct delivery of the compositions will generally be accomplished by injection, either subcutaneously, intraperitoneally, intravenously or intramuscularly or delivered to the interstitial space of a tissue. The compositions can also be administered into a lesion. Other modes of administration include oral and pulmonary administration, suppositories, and transdermal or transcutaneous applications (*e.g.* see WO98/20734), needles, and gene guns or hyposprays. Dosage treatment may be a single dose schedule or a multiple dose schedule.

Methods for the *ex vivo* delivery and reimplantation of transformed cells into a subject are known in the art and described in *e.g*. WO93/14778. Examples of cells useful in *ex* vivo applications include, for example, stem cells, particularly hematopoetic, lymph cells, macrophages, dendritic cells, or tumor cells.

Generally, delivery of nucleic acids for both *ex vivo* and *in vitro* applications can be accomplished by the following procedures, for example, dextran-mediated transfection, calcium phosphate precipitation, polybrene mediated transfection, protoplast fusion, electroporation, encapsulation of the polynucleotide(s) in liposomes, and direct microinjection of the DNA into nuclei, all well known in the art.

*Polynucleotide and polypeptide pharmaceutical compositions*

In addition to the pharmaceutically acceptable carriers and salts described above, the following additional agents can be used with polynucleotide and/or polypeptide compositions.

A.Polypeptides

One example are polypeptides which include, without limitation: asioloorosomucoid (ASOR); transferrin; asialoglycoproteins; antibodies; antibody fragments; ferritin; interleukins; interferons, granulocyte, macrophage colony stimulating factor (GM-CSF), granulocyte colony stimulating factor (G-CSF), macrophage colony stimulating factor (M-CSF), stem cell factor and erythropoietin. Viral antigens, such as envelope proteins, can also be used. Also, proteins from other invasive organisms, such as the 17 amino acid peptide from the circumsporozoite protein of plasmodium falciparum known as RII.

B.Hormones. Vitamins, *etc.*

Other groups that can be included are, for example: hormones, steroids, androgens, estrogens, thyroid hormone, or vitamins, folic acid.

## C.Polyalkylenes, Polysaccharides, *etc.*

Also, polyalkylene glycol can be included with the desired polynucleotides/polypeptides. In a preferred embodiment, the polyalkylene glycol is polyethlylene glycol. In addition, mono-, di-, or polysaccharides can be included. In a preferred embodiment of this aspect, the polysaccharide is dextran or DEAE-dextran. Also, chitosan and poly(lactide-co-glycolide)

## D.Lipids, and Liposomes

The desired polynucleotide/polypeptide can also be encapsulated in lipids or packaged in liposomes prior to delivery to the subject or to cells derived therefrom.

Lipid encapsulation is generally accomplished using liposomes which are able to stably bind or entrap and retain nucleic acid. The ratio of condensed polynucleotide to lipid preparation can vary but will generally be around 1:1 (mg DNA:micromoles lipid), or more of lipid. For a review of the use of liposomes as carriers for delivery of nucleic acids, see, Hug and Sleight (1991) Biochim. Biophys. Acta. 1097:1-17; Straubinger (1983) Meth. Enzymol. 101:512-527.

Liposomal preparations for use in the present invention include cationic (positively charged), anionic (negatively charged) and neutral preparations. Cationic liposomes have been shown to mediate intracellular delivery of plasmid DNA (Felgner (1987) Proc. Natl. Acad. Sci. USA 84:7413-7416); mRNA (Malone (1989) Proc. Natl. Acad. Sci. USA 86:6077-6081); and purified transcription factors (Debs (1990) J. Biol. Chem. 265:10189-10192), in functional form.

Cationic liposomes are readily available. For example, N[1-2,3-dioleyloxy)propyl]-N,N,N-triethylammonium (DOTMA) liposomes are available under the trademark Lipofectin, from GIBCO BRL, Grand Island, NY. (See, also, Felgner *supra*). Other commercially available liposomes include transfectace (DDAB/DOPE) and

DOTAP/DOPE (Boerhinger). Other cationic liposomes can be prepared from readily available materials using techniques well known in the art. See, *e.g.* Szoka (1978) Proc. Natl. Acad. Sci. USA 75:4194-4198; WO90/11092 for a description of the synthesis of DOTAP (1,2-bis(oleoyloxy)-3-(trimethylammonio)propane) liposomes.

Similarly, anionic and neutral liposomes are readily available, such as from Avanti Polar Lipids (Birmingham, AL), or can be easily prepared using readily available materials. Such materials include phosphatidyl choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with the DOTMA and DOTAP starting materials in appropriate ratios. Methods for making liposomes using these materials are well known in the art.

The liposomes can comprise multilammelar vesicles (MLVs), small unilamellar vesicles (SUVs), or large unilamellar vesicles (LUVs). The various liposome-nucleic acid complexes are prepared using methods known in the art. See *e.g.* Straubinger (1983) Meth. Immunol. 101:512-527; Szoka (1978) Proc. Natl. Acad. Sci. USA 75:4194-4198; Papahadjopoulos (1975) Biochim. Biophys. Acta 394:483; Wilson (1979) Cell 17:77); Deamer & Bangham (1976) Biochim. Biophys. Acta 443:629; Ostro (1977) Biochem. Biophys. Res. Commun. 76:836; Fraley (1979) Proc. Natl. Acad. Sci. USA 76:3348); Enoch & Strittmatter (1979) Proc. Natl. Acad. Sci. USA 76:145; Fraley (1980) J. Biol. Chem. (1980) 255:10431; Szoka & Papahadjopoulos (1978) Proc. Natl. Acad. Sci. USA 75:145; and Schaefer-Ridder (1982) Science 215:166.

## E.Lipoproteins

In addition, lipoproteins can be included with the polynucleotide/polypeptide to be delivered. Examples of lipoproteins to be utilized include: chylomicrons, HDL, IDL, LDL, and VLDL. Mutants, fragments, or fusions of these proteins can also be used. Also, modifications of naturally occurring lipoproteins can be used, such as acetylated LDL. These lipoproteins can target the delivery of polynucleotides to cells expressing lipoprotein receptors. Preferably, if lipoproteins are including with the polynucleotide to be delivered, no other targeting ligand is included in the composition.

Naturally occurring lipoproteins comprise a lipid and a protein portion. The protein portion are known as apoproteins. At the present, apoproteins A, B, C, D, and E have been isolated and identified. At least two of these contain several proteins, designated by Roman numerals, AI, AII, AIV; CI, CII, CIII.

A lipoprotein can comprise more than one apoprotein. For example, naturally occurring chylomicrons comprises of A, B, C, & E, over time these lipoproteins lose A and acquire C and E apoproteins. VLDL comprises A, B, C, & E apoproteins, LDL comprises apoprotein B; HDL comprises apoproteins A, C, & E.

The amino acid of these apoproteins are known and are described in, for example, Breslow (1985) Annu Rev. Biochem 54:699; Law (1986) Adv. Exp Med. Biol. 151:162; Chen (1986) J Biol Chem 261:12918; Kane (1980) Proc Natl Acad Sci USA 77:2465; and Utermann (1984) Hum Genet 65:232.

Lipoproteins contain a variety of lipids including, triglycerides, cholesterol (free and esters), and phospholipids.

The composition of the lipids varies in naturally occurring lipoproteins. For example, chylomicrons comprise mainly triglycerides. A more detailed description of the lipid content of naturally occurring lipoproteins can be found, for example, in Meth. Enzymol. 128 (1986). The composition of the lipids are chosen to aid in conformation of the apoprotein for receptor binding activity. The composition of lipids can also be chosen to facilitate hydrophobic interaction and association with the polynucleotide binding molecule.

Naturally occurring lipoproteins can be isolated from serum by ultracentrifugation, for instance. Such methods are described in *Meth. Enzymol.* (*supra*); Pitas (1980) J. Biochem. 255:5454-5460 and Mahey (1979) J Clin. Invest 64: 743-750. Lipoproteins can also be produced by *in vitro* or recombinant methods by expression of the apoprotein genes in a desired host cell. See, for example, Atkinson (1986) Annu Rev Biophys Chem 15:403 and

Radding (1958) Biochim Biophys Acta 30: 443. Lipoproteins can also be purchased from commercial suppliers, such as Biomedical Techniologies, Inc., Stoughton, Massachusetts, USA. Further description of lipoproteins can be found in Zuckermann et al. WO98/06437.

F.Polycationic Agents

Polycationic agents can be included, with or without lipoprotein, in a composition with the desired polynucleotide/polypeptide to be delivered.

Polycationic agents, typically, exhibit a net positive charge at physiological relevant pH and are capable of neutralizing the electrical charge of nucleic acids to facilitate delivery to a desired location. These agents have both in vitro, ex vivo, and in vivo applications. Polycationic agents can be used to deliver nucleic acids to a living subject either intramuscularly, subcutaneously, etc.

The following are examples of useful polypeptides as polycationic agents: polylysine, polyarginine, polyornithine, and protamine. Other examples include histones, protamines, human serum albumin, DNA binding proteins, non-histone chromosomal proteins, coat proteins from DNA viruses, such as (X174, transcriptional factors also contain domains that bind DNA and therefore may be useful as nucleic aid condensing agents. Briefly, transcriptional factors such as C/CEBP, c-jun, c-fos, AP-1, AP-2, AP-3, CPF, Prot-1, Sp-1, Oct-1, Oct-2, CREP, and TFIID contain basic domains that bind DNA sequences.

Organic polycationic agents include: spermine, spermidine, and purtrescine.

The dimensions and of the physical properties of a polycationic agent can be extrapolated from the list above, to construct other polypeptide polycationic agents or to produce synthetic polycationic agents.

Synthetic polycationic agents which are useful include, for example, DEAE-dextran, polybrene. Lipofectin™ and lipofectAMINE™ are monomers that form polycationic complexes when combined with polynucleotides/polypeptides.

*Nucleic Acid Hybridisation*

"Hybridization" refers to the association of two nucleic acid sequences to one another by hydrogen bonding. Typically, one sequence will be fixed to a solid support and the other will be free in solution. Then, the two sequences will be placed in contact with one another under conditions that favor hydrogen bonding. Factors that affect this bonding include: the type and volume of solvent; reaction temperature; time of hybridization; agitation; agents to block the non-specific attachment of the liquid phase sequence to the solid support (Denhardt's reagent or BLOTTO); concentration of the sequences; use of compounds to increase the rate of association of sequences (dextran sulfate or polyethylene glycol); and the stringency of the washing conditions following hybridization. See Sambrook *et al*. [*supra*] vol.2, chapt. 9, pp.9.47 to 9.57.

"Stringency" refers to conditions in a hybridization reaction that favor association of very similar sequences over sequences that differ. For example, the combination of temperature and salt concentration should be chosen that is approximately 120 to 200°C below the calculated Tm of the hybrid under study. The temperature and salt conditions can often be determined empirically in preliminary experiments in which samples of genomic DNA immobilized on filters are hybridized to the sequence of interest and then washed under conditions of different stringencies. See Sambrook *et al.* at page 9.50.

Variables to consider when performing, for example, a Southern blot are (1) the complexity of the DNA being blotted and (2) the homology between the probe and the sequences being detected. The total amount of the fragment (s) to be studied can vary a magnitude of 10, from 0.1 to 1$\mu$g for a plasmid or phage digest to $10^{-9}$ to $10^{-8}$ g for a single copy gene in a highly complex eukaryotic genome. For lower complexity polynucleotides, substantially shorter blotting, hybridization, and exposure times, a smaller amount of starting polynucleotides, and lower specific activity of probes can be used. For example, a single-copy yeast gene can be detected with an exposure time of only 1 hour starting with 1 $\mu$g of yeast DNA, blotting for two hours, and hybridizing for 4-8 hours with a probe of $10^8$ cpm/$\mu$g. For a single-copy mammalian gene a conservative approach would start with 10 $\mu$g of DNA, blot overnight, and hybridize overnight in the presence of 10% dextran sulfate using a probe of greater than $10^8$ cpm/$\mu$g, resulting in an exposure time of ~24 hours.

Several factors can affect the melting temperature (Tm) of a DNA-DNA hybrid between the probe and the fragment of interest, and consequently, the appropriate conditions for hybridization and washing. In many cases the probe is not 100% homologous to the fragment. Other commonly encountered variables include the length and total G+C content of the hybridizing sequences and the ionic strength and formamide content of the hybridization buffer. The effects of all of these factors can be approximated by a single equation:

$$Tm = 81 + 16.6(\log_{10}Ci) + 0.4[\%(G + C)] - 0.6(\% formamide) - 600/n - 1.5(\% mismatch).$$

where Ci is the salt concentration (monovalent ions) and n is the length of the hybrid in base pairs (slightly modified from Meinkoth & Wahl (1984) Anal. Biochem. 138: 267-284).

In designing a hybridization experiment, some factors affecting nucleic acid hybridization can be conveniently altered. The temperature of the hybridization and washes and the salt concentration during the washes are the simplest to adjust. As the temperature of the hybridization increases (ie. stringency), it becomes less likely for hybridization to occur between strands that are nonhomologous, and as a result, background decreases. If the radiolabelled probe is not completely homologous with the immobilized fragment (as is frequently the case in gene family and interspecies hybridization experiments), the hybridization temperature must be reduced, and background will increase. The temperature of the washes affects the intensity of the hybridizing band and the degree of background in a similar manner. The stringency of the washes is also increased with decreasing salt concentrations.

In general, convenient hybridization temperatures in the presence of 50% formamide are 42°C for a probe with is 95% to 100% homologous to the target fragment, 37°C for 90% to 95% homology, and 32°C for 85% to 90% homology. For lower homologies, formamide content should be lowered and temperature adjusted accordingly, using the equation above. If the homology between the probe and the target fragment are not known, the simplest approach is to start with both hybridization and wash conditions which are nonstringent. If non-specific bands or high background are observed after autoradiography, the filter can be washed at high stringency and reexposed. If the time required for exposure makes this approach impractical, several hybridization and/or washing stringencies should be tested in parallel.

*Nucleic Acid Probe Assays*

Methods such as PCR, branched DNA probe assays, or blotting techniques utilizing nucleic acid probes according to the invention can determine the presence of cDNA or mRNA. A probe is said to "hybridize" with a sequence of the invention if it can form a duplex or double stranded complex, which is stable enough to be detected.

The nucleic acid probes will hybridize to the Neisserial nucleotide sequences of the invention (including both sense and antisense strands). Though many different nucleotide sequences will encode the amino acid sequence, the native Neisserial sequence is preferred because it is the actual sequence present in cells. mRNA represents a coding sequence and so a probe should be complementary to the coding sequence; single-stranded cDNA is complementary to mRNA, and so a cDNA probe should be complementary to the non-coding sequence.

The probe sequence need not be identical to the Neisserial sequence (or its complement) - some variation in the sequence and length can lead to increased assay sensitivity if the nucleic acid probe can form a duplex with target nucleotides, which can be detected. Also, the nucleic acid probe can include additional nucleotides to stabilize the formed duplex. Additional Neisserial sequence may also be helpful as a label to detect the formed duplex. For example, a non-complementary nucleotide sequence may be attached to the 5' end of the probe, with the remainder of the probe sequence being complementary to a Neisserial sequence. Alternatively, non-complementary bases or longer sequences can be interspersed into the probe, provided that the probe sequence has sufficient complementarity with the a Neisserial sequence in order to hybridize therewith and thereby form a duplex which can be detected.

The exact length and sequence of the probe will depend on the hybridization conditions, such as temperature, salt condition and the like. For example, for diagnostic applications, depending on the complexity of the analyte sequence, the nucleic acid probe typically contains at least 10-20 nucleotides, preferably 15-25, and more preferably >30 nucleotides, although it may be shorter than this. Short primers generally require cooler temperatures to form sufficiently stable hybrid complexes with the template.

Probes may be produced by synthetic procedures, such as the triester method of Matteucci et al. [J. Am. Chem. Soc. (1981) 103:3185], or according to Urdea et al. [Proc. Natl. Acad. Sci. USA (1983) 80: 7461], or using commercially available automated oligonucleotide synthesizers.

The chemical nature of the probe can be selected according to preference. For certain applications, DNA or RNA are appropriate. For other applications, modifications may be incorporated *e.g.* backbone modifications, such as phosphorothioates or methylphosphonates, can be used to increase *in vivo* half-life, alter RNA affinity, increase nuclease resistance *etc*. [*e.g.* see Agrawal & Iyer (1995) Curr Opin Biotechnol 6:12-19; Agrawal (1996) TIBTECH 14:376-387];

analogues such as peptide nucleic acids may also be used [*e.g.* see Corey (1997) TIBTECH 15:224-229; Buchardt et al. (1993) TIBTECH 11:384-386].

Alternatively, the polymerase chain reaction (PCR) is another well-known means for detecting small amounts of target nucleic acids. The assay is described in: Mullis et al. [Meth. Enzymol. (1987) 155: 335-350]; US patents 4,683,195 & 4,683,202. Two 'primers' hybridize with the target nucleic acids and are used to prime the reaction. The primers can comprise sequence that does not hybridize to the sequence of the amplification target (or its complement) to aid with duplex stability or, for example, to incorporate a convenient restriction site. Typically, such sequence will flank the desired Neisserial sequence.

A thermostable polymerase creates copies of target nucleic acids from the primers using the original target nucleic acids as a template. After a threshold amount of target nucleic acids are generated by the polymerase, they can be detected by more traditional methods, such as Southern blots. When using the Southern blot method, the labelled probe will hybridize to the Neisserial sequence (or its complement).

Also, mRNA or cDNA can be detected by traditional blotting techniques described in Sambrook *et al* [*supra*]. mRNA, or cDNA generated from mRNA using a polymerase enzyme, can be purified and separated using gel electrophoresis. The nucleic acids on the gel are then blotted onto a solid support, such as nitrocellulose. The solid support is exposed to a labelled probe and then washed to remove any unhybridized probe. Next, the duplexes containing the labelled probe are detected. Typically, the probe is labelled with a radioactive moiety.

## BRIEF DESCRIPTION OF THE DRAWINGS

Figure 1 shows the overall strategy for 2D-PAGE. The individual gels are shown as Figures 2 to 4.

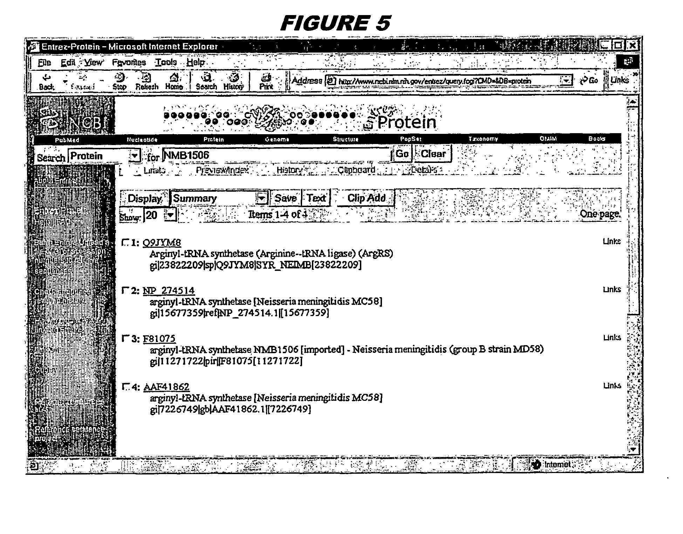

Figure 5 shows the results of a protein query of the NCBI Entrez system using the term 'NMB1506'.

## MODES FOR CARRYING OUT THE INVENTION

The overall strategy for identifying membrane proteins was to use two dimensional electrophoresis to separate the proteins in a *N.meningitidis* membrane preparation, followed by identification of proteins found in the electrophoretic gel. This experiment was not straightforward because, as is well known, preparing a membrane fraction which is not contaminated with cytoplasmic proteins is extremely difficult.

Some of the proteins identified by electrophoresis were expected to be there, but many were not. Furthermore, several proteins which were annotated as "hypothetical" have now been shown to exist *in vivo* - they are not artefacts. A further point to note is that proteins visible on a 2D gel must be expressed at least at a moderate level, thus making them useful targets for binding.

### *Preparation of membrane fraction*

To prepare the membrane fraction, *N.meningitidis* strain MC58 was growth in liquid "base for the isolation of pathogenic Neisseria" (GC, Difco), supplemented with 4g/l glucose, 0.1 g/l glutamine, and 2.2 mg/l cocarboxylase, at 37˚C in a humidified atmosphere containing 5% $CO_2$. Bacteria were harvested at $OD_{600nm}$=0.5 by centrifugation at 10,000 g for 20 minutes. The pellet was resuspended with 10 ml Tris-base containing the EDTA-free protease inhibitor cocktail used according to the manufacturer (Roche Diagnostic Mannhein, Germany). The bacteria were inactivated 45 minutes at 56˚C, cooled in ice in presence of 1,000 U benzonase, and were disrupted with a French Press at 18000 psi (4 passages). The lysate was centrifuged at 70,000 g overnight at 5˚C. The pellet was washed twice with 40mM Tris-base and resuspended in 10 ml of 5M urea, 2M thiourea, 2% (w/v) CHAPS, 2% (w/v) ASB 14, 0.5% (v/v) C.A., 2mM TBP and centrifuged at 100,000 g at 10˚C for 3 hours. Supernatant containing solubilised membrane proteins was aliquoted and stored at -80˚C.

This process is quick, easy to use, and results in a membrane preparation with little or no contamination from cytosolic proteins [*e.g.* refs. 112 to 115]. It was improved by using new techniques for the solubilisation of bacterial membrane proteins prior and during to isoelectric focusing, in order to optimise the separation of outer membrane proteins by two-dimensional electrophoresis (2DE). Thirourea was thus used with the urea as chaotropic agents [112], the zwitterionic detergent ASB-14 was used in association with CHAPS [116], and tributyl phosphine was added prior to 2DE [117].

### *2D electrophoresis*

200μg of the membrane proteins were brought to a final volume of 125 μl with reswelling solution (7M urea, 2M thiourea, 2% (w/v) CHAPS, 2% (w/v) ASB 14, 2% (v/v) adequate ampholine (Amersham Pharmacia Biotech (Piscataway, NJ), 2 mM thiobutyl phosphine). The proteins were absorbed overnight onto an Immobiline DryStrip (7 cm; pH-gradient 3-10 non linear, pH-gradient, 4-7 or 6-11 linear) using the Immobiline Dry-Strip Reswelling Tray (Amersham Pharmacia Biotech (Piscataway, NJ). Proteins were then separated by 2D electrophoresis. The first dimension was run on an IPGphor Isoelectric Focusing Unit (Amersham Pharmacia Biotech, Piscataway, NJ), The proteins were separated applying sequentially 150 V for 35 min., 500 V for 35 min., 1000 V for 30 min, 2600 V for 10 min., 3500 V for 15 min., 4200 V for 15 min., and then 5000 V to reach 10kVh. For the second dimension, the strips were equilibrated as described in ref. 117 and the proteins were separated on a linear 9-16% acrylamide SDS-PAGE (1.5 mm thick, 6 cm high) using the Mini Proten II Cell from BioRad. The 2D gel was stained with colloidal Coomassie [118]. Gels were scanned with a Personal densitometer SI (Molecular Dynamics) at 12 bits and 50 $\mu$m per pixel.

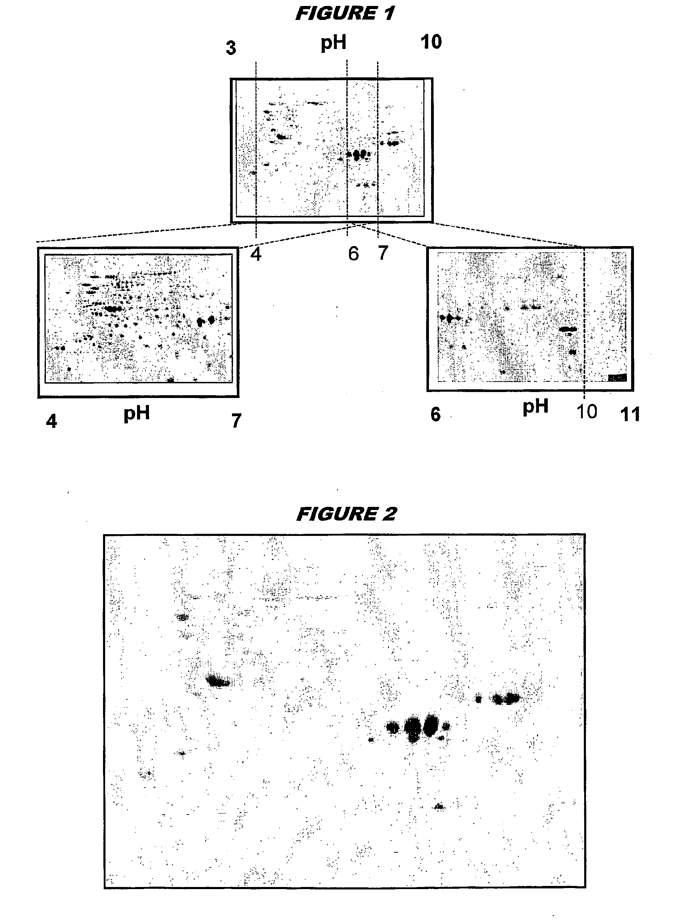

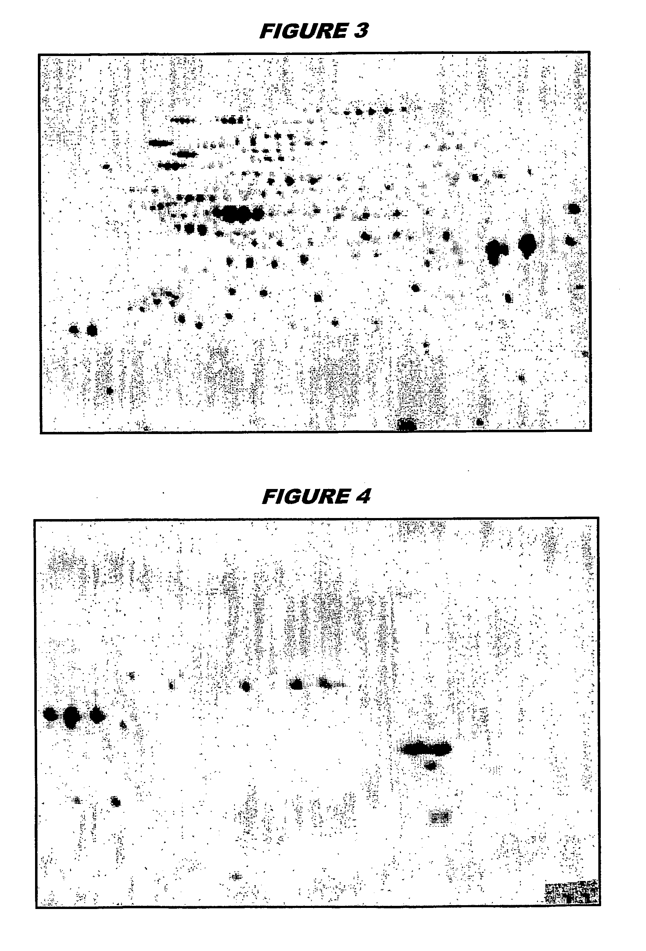

The initial 2D IPG gradient gel is shown in Figure 2. 325 protein spots were visualised by colloidal Coomassie blue. In order to increase the spot number, 200$\mu$g of the same protein fraction was separated on 2D gels with a pH gradients of 4-7 (Figure 3) and 6-11 (Figure 4). 498 and 187 spots were visualised by colloidal Coomassie blue in these gels, respectively. Each gel was performed and analysed in duplicate, with no major differences observed between duplicates.

### Spot excision

A total of 1,867 protein spots were excised from the three gels, plus the duplicated gels, in-gel digested with trypsin, and the generated peptides were analysed by MALDI-TOF mass spectrometry. Proteins were identified by interpreting their peptide mass fingerprint using the software Mascot.

The protocol used for in-gel digestion was a modification of reference 119. Protein spots were excised from the gel, washed sequentially with Milli-Q water and acetonitrile (1/1, v/v), and dried by a Speed Vac vacuum centrifuge apparatus (Savant, Holbrook, NY). The pieces of gel were re-hydrated by adding 7-10 $\mu$l of a solution of 50 mM ammonium bicarbonate, 5 mM CaCl$_2$ containing 12 mg/l trypsin sequencing grade (Roche Diagnostic Corporation, Indianapolis, IN). The tryptic digestion was allowed to run for 18 hours at 37˚C. Following digestion, the peptides were extracted by sonication in a sonicator bath with 50 $\mu$l 50% acetonitrile, 5% TFA for 30 min. The extraction was repeated, extract solutions were pooled together and the volume was reduced to 10 $\mu$l in a Speed Vac vacuum centrifuge apparatus. The samples were automatically prepared for mass spectrometry using the MAP II (Bruker, Bremen, Germany). The instrument was programmed to perform a desalting of the sample with ZIP-TIP (C18, Millipore). The peptides were eluted from the ZIP TIP with a solution of 5 g/l of 2,5-dihydroxybenzoic acid in 50% acetonitrile/0,1% TFA and directly loaded onto the Anchorchip (400 $\mu$m, Bruker, Bremen, Germany), The samples were allowed to air dry at room temperature.

### Excised spot analysis

MALDI-TOF spectra were acquired on a Bruker Biflex II MALDI-TOF (Bremen, Germany) equipped with the SCOUT 381 multiprobe ion source in a positive-ion reflector mode. The acceleration voltage was set to 19 kV and the reflector voltage was set to 20 kV. Typically about 100 laser shots were average per spectrum from a 337 nm N2 laser. Spectra were externally calibrated using a combination of angiotensin II (1,046.54 Da), substance P (1,347.74 Da), Bombensin (1,619.82 Da) and ACTH18-39 (Clip human, 2,465.20 Da). Peptides were selected in the mass range of 700-3000 Da. Resulting values for monoisotopic peaks were used for search using the software Mascot run on a database containing the genome information of *N.meningitidis* and *C.pneumoniae.* All the searches were performed using a window of 500 ppm as constraint, allowing one missed cleavage, and considering propiamide cysteines as fixed modification, and oxidized methionine as variable modification. Signals generated from keratin digestion or trypsin autolysis were not considered for the search in the database. Proteins were successfully identified when the MOWSE score was superior to 47. When proteins were identified with an inferior Mowse score, Post Source Decay (PSD) experiments were performed to confirm the identification. PSD spectra of the parent ions were built by pasting the spectra obtain with successive reflector voltage from 20 to 0.59 V.

Within the Figure 2 gel, 261/325 spots were identified by peptide mass fingerprint. When the same protein was identified in two or more samples from a train of spots clearly arising from isoforms of the same protein, the identification was extended to cover all spots in this row. When needed, the identification of the proteins were confirmed by PSD experiments, mainly for protein with a Mr inferior to 15 kDa. The remaining 64/325 spots contained too little protein material to give an unambiguous identification. The identified 261 spots were encoded by 138 separate genes, of which 11 were confirmed by PSD experiments. An additional 18 proteins were identified from the duplicated gel.

The Figure 3 gel allowed the identification of 194 unique proteins, of which 72 were not identified from the Figure 2 gel. Similarly, the Figure 4 gel allowed the identification of 87 unique proteins of which 26 were not identified from the Figure 2 gel.

The identified proteins are listed in Table 1, with columns indicating which gel they were found in. In summary, among the 1,867 proteins spots processed, 1,433 proteins were identified. The identified spots represent 250 unique proteins. Only 8.8% of the proteins were identified from the pH 6-11 gels which confirms the difficulty of separating very basic proteins by 2DE. The identified proteins cover 11.58% of the predicted ORFs encoded by the genome [1].

Within the 250 proteins, 45 acted as positive controls, as they had previously been identified as useful vaccine/ diagnostic antigens in references 120 to 122. These 45 proteins are indicated with a '+' in Table 1. The remaining 205 proteins are listed as SEQ ID NOs 1 to 205.

Within the remaining 205 proteins, 138 completely elude the PSORT subcellular localisation algorithm [123, 124]. These 138 are marked with a '*' in Table 1, and it is particularly surprising to find these proteins in the membrane. Of these 138, 76 elude various algorithmic predictions, and this subset is marked '**'.

*OMV analysis*

In a separate series of experiments, outer membrane vesicles from serogroup B strains 394/98 (New Zealand) and H44/76 (Norway) were proteomically analysed. Sera from humans immunised with one of the two OMV preparations were analysed, and nine specific immunogenic proteins were identified: NMB 0182, 0382, 0634, 0763, 1126, 1342, 1429, 1799 & 2039. Of these 9 proteins: NMB 0382, 0634, 0763, 1342, 1429 & 2039 are in common with SEQ IDs 1 to 205; NMB0182 is a known immunogen [125]; NMB1126 (gi:7226362; SEQ ID 206) is annotated as 'hypothetical protein', having been identified only through GLIMMER2 prediction, and was not previously recognised as an immunogen; NMB 1799 (gi: 7227052; SEQ ID 207) is annotated as 'S-adenosylmethionine synthetase', which is a metabolic enzyme which would not normally be expected to be immunoaccessible. SEQ IDs 208-217 were not previously recognised as immunogens.

Proteins which were present in the 394/98 OMVs, but not in the H44/76 OMVs, were: 0007, 0018, 0031, 0051, 0052, 0089, 0110, 0130, 0132, 0168, 0214, 0336, 0375, 0423, 0427, 0462, 0554, 0586, 0604, 0610, 0623, 0626, 0634, 0652, 0758, 0854, 0920, 0944, 0954, 0955, 0959, 0983, 1046, 1055, 1127, 1153, 1191, 1199, 1201, 1228, 1240, 1285, 1313, 1332, 1339, 1358, 1392, 1437, 1457, 1460, 1506, 1518, 1533, 1554, 1572, 1577, 1594, 1612, 1642, 1668, 1684, 1710, 1762, 1796, 1812, 1849, 1854, 1855, 1861, 1874, 1903, 1921, 1949, 1953, 2086, 2102, 2129, 2134, 2138, 2159.

Proteins which were present in the H44/76 OMVs, but not in the 394/98 OMVs, were: 0143, 0204, 0280, 0313, 0410, 0477, 0546, 0638, 0889, 1011, 1124, 1162, 1342, 1540, 1714, 1799, 1808, 1969, 1998, 2069, 2103, 2154.

Proteins which were present in both OMV preparations were: 0035, 0088, 0109, 0124, 0126, 0138, 0139, 0154, 0157, 0171, 0182, 0219, 0359, 0382, 0387, 0426, 0461, 0595, 0617, 0618, 0631, 0663, 0703, 0757, 0763, 0787, 0875, 0876, 0943, 0946, 0957, 1053, 1126, 1131, 1252, 1301, 1323, 1341, 1429, 1445, 1497, 1567, 1574, 1576, 1869, 1934, 1936, 1946, 1972, 1988, 2039, 2096, 2101.

**TABLE 1**

| | NMB | ID (gi:722xxxx): name / MW / pI | Gel (Fig.) | | | Note |
|---|---|---|---|---|---|---|
| | | | 2 | 3 | 4 | |
| 1 | 0010 | 5235: phosphoglycerate kinase / 41902 / 5.12 | | X | | |
| 2 | 0018 | 5244: pilin PilE /15 246 / 9.21 | X | | X | |
| 3 | 0030 | 5249: methionyl-tRNA synthetase / 76 853 / 5.44 | | X | | |
| 4 | 0035 | 5255: conserved hypothetical protein / 40 218/4.74 | X | X | | + |
| 5 | 0038 | 5258: UDP-N-acetylglucosamine pyrophosphorylase /49 013 / 6.48 | X | | X | |
| 6 | 0045 | 5266: signal recognition particle protein / 45142/4.85 | | X | | * |
| 7 | 0051 | 5271: twitching motility protein / 45 675 / 6.51 | X | | X | * |
| 8 | 0052 | 5272: twitching motility protein PilT /38 05016.61 | X | | X | ** |
| 9 | 0068 | 5288: polysialic acid capsule biosynthesis protein SiaC / 38821 / 5.35 | | X | | * |
| 10 | 0088 | 5306. outer membrane protein P1, putative / 45 902/9.35 | X | | X | + |
| 11 | 0089 | 5307: pyruvate kinase II / 52 435 / 5;27 | X | X | | * |
| 12 | 0109 | 5324: conserved hypothetical protein / 43188/6.77 | X | | X | * |
| 13 | 0115 | 5331: nitrogen assimilation regulatory protein NtrX / 46373 / 4.98 | X | | | ** |
| 14 | 0124/ 0139 | 5341-5357: translation elongation factor TU / 42 909-42 925/5.07 | X | X | X | ** |

(continued)

| | NMB | ID (gi:722xxxx): name / MW / pI | Gel (Fig.) | | | Note |
|---|---|---|---|---|---|---|
| | | | 2 | 3 | 4 | |
| 15 | 0126 | 5343: transcription antitermination protein NusG / 20 550 / 6.03 | X | X | | ** |
| 16 | 0128 | 5345: 50S ribosomal protein L1 / 2410219.59 | X | X | X | * |
| 17 | 0130 | 5347: 50S ribosomal protein L10 / 17 594 / 7.91 | X | X | X | ** |
| 18 | 0131 | 5348 : 50S ribosomal protein L7/L12 / 12623 / 4.60 | | X | | |
| 19 | 0132 | 5349: DNA-directed RNA polymerase, beta subunit / 155 945 / 5.34, | X | X | X | |
| 20 | 0133 | 5351: DNA-directed RNA polymerase, beta' subunit / 154660 / 6.86 | | | X | * |
| 21 | 0137 | 5355: 30S ribosomal protein S7 / 10646 / 10.33 | | | X | * |
| 22 | 0138 | 5356: elongation factor G (EF-G) / 77 244 / 5.08 | X | X | X | ** |
| 23 | 0140 | 5358: 30S ribosomal protein S10 / 11799 / 7.57 | | | X | ** |
| 24 | 0142 | 5360: 50S ribosomal protein L3 / 22664 / 10.03 | | | X | ** |
| 25 | 0143 | 5361: 50S ribosomal protein L4 / 23248 / 9.92 | | | X | ** |
| 26 | 0144 | 5362: 50S ribosomal protein L23 / 11268 / 9.85 | | | X | |
| 27 | 0154 | 5372: 50S ribosomal protein L5 / 20 32319.49 | X | X | X | ** |
| 28 | 0156 | 5374: 30S ribosomal protein S8 / 14257 / 9.73 | | | X | * |
| 29 | 0157 | 5375: 50S ribosomal protein L6 / 18 892 / 9.63 | | X | X | * |
| 30 | 0159 | 5377: 30s ribosomal protein S5 / 18235 / 10.04 | | | X | * |
| 31 | 0168 | 5386: DNA-directed RNA polymerase, alpha subunit / 36 076 / 4.94 | X | X | X | ** |
| 32 | 0171 | 5389: septum site-determining protein MinD / 29 559 / 5.70 | X | X | | ** |
| 33 | 0173 | 5391: transciptional regulator, LysR family / 34 071 / 5.74 | X | X | | |
| 34 | 0181 | 5400: outer membrane protein OmpH, putative / 19261 / 9.23 | | X | | + |
| 35 | 0182 | 5401: outer membrane protein Omp85 / 86 254 / 8.37 | X | | X | + |
| 36 | 0191 | 5411. ParA family protein / 27150/5.53 | X | | | + |
| 37 | 0193 | 5413: glucose inhibited division protein A / 69 808 / 6.57 | X | | X | * |
| 38 | 0196 | 5417: ribonuclease E / 102087 / 5.97 | | X | X | * |
| 39 | 0212 | 5433: DNA gyrase subunit B / 88188 / 5.46 | X | X | | * |
| 40 | 0214 | 5436: oligopeptidase A 176 054/5.16 | X | X | | + |
| 41 | 0219 | 5441: 3-oxoacyl-(acyl-carrier-protein) synthase II / 43 221 / 5.36 | | X | | |
| 42 | 0246 | 5468: NADH dehydrogenase I, F subunit / 48007 / 5.66 | | X | | * |
| 43 | 0278 | 5503. thiol:disulfide interchange protein DsbA /23 228 / 5.16 / 18 | X | X | X | + |
| 44 | 0286 | 5510: conserved hypothetical protein / 22120/5.04 | | X | | * |
| 45 | 0292 | 5516: conserved hypothetical protein / 22080/5.37 | | X | | + |
| 46 | 0294 | 5518: thiol:disulfide interchange protein DsbA/23 566 / 5.09 lipo | X | X | X | |
| 47 | 0329 | 5549: type IV pilus assembly protein / 61 965 / 5.42 | X | X | | * |
| 48 | 0335 | 5555: 2,3,4,5-tetrahydropyridine-2-arboxylate N-succinyltransferase / 29 410 / 5.42 | X | X | | ** |

(continued)

| | NMB | ID (gi:722xxxx): name / MW / pI | Gel (Fig.) | | | Note |
|---|---|---|---|---|---|---|
| | | | 2 | 3 | 4 | |
| 49 | 0336 | 5556: enoyl-(acyl-carrier-protein) reductase / 125 252 / 5.45 / 22 | | X | | |
| 50 | 0337 | 5557: branched-chain amino acid aminotransferase, putative / 36421 / 5.80 | | X | | |
| 51 | 0345 | 5566: cell-binding factor, putative / 31507 / 9.23 | | | X | + |
| 52 | 0351 | 5572: transaldolase / 37 578 / 5.09 | | X | | ** |
| 53 | 0359 | 5581: glutamate-ammonia ligase / 52137 / 5.20 | X | X | X | * |
| 54 | 0382 | 5603: outer membrane protein class 4 / 23 969 / 6.26 | X | X | X | |
| 55 | 0387 | 5608:ABC transporter, ATP-binding protein / 62 073 / 5.23 | | X | X | + |
| 56 | 0390 | 5612: maltose phosphorylase / 85 490 / 5.36 | X | X | | * |
| 57 | 0391 | 5613: beta-phosphoglucomutase / 23728 / 5.08 | | X | | |
| 58 | 0426 | 5649: cell division protein FtsA / 44 061 / 5,33 | X | X | | |
| 59 | 0427 | 5651: cell division protein FtsZ/41 487/4.94 | X | | | |
| 60 | 0446 | 5671: chorismate mutase/prephenate dehydratase / 39778 / 5.65 | | X | | |
| 61 | 0462 | 5689: spermidine/putrescine ABC transporter, periplasmic ... / 50 556 / 6.92 | X | X | | |
| 62 | 0466 | 5693. aspartyl-tRNA synthetase / 68 125 / 5.28 | X | X | X | ** |
| 63 | 0477 | 5705: conserved hypothetical protein / 19758 / 5.19 | | X | | ** |
| 64 | 0530 | 5755: glycosyl hydrolase, family 3 / 39554 / 5.39 | | X | | ** |
| 65 | 0537 | 5762: conserved hypothetical protein / 31 150 / 4.77 | | X | | + |
| 66 | 0540 | 5765: aspartate aminotransferase / 43 929 / 6.52 | X | X | X | ** |
| 67 | 0546 | 5772: alcohol dehydrogenase, propanol-preferring / 36 548 / 5.65 | X | X | | * |
| 68 | 0550 | 5777: thiol:disulfide interchange protein DsbC / 26 451 / 6.93 | X | | | + |
| 69 | 0554 | 5780: dnaK protein / 68 792/4.85 | X | X | X | + |
| 70 | 0564 | 5791: Na(+)-translocating NADH-quinone reductase, subunit F / 42 303 / 4.95 | X | X | | |
| 71 | 0569 | 5796: Na(+)-translocating NADH-quinone reductase, subunit A /48 636 /6.20 | X | X | X | + |
| 72 | 0589 | 5819: 50s ribosomal protein L19 / 13760 / 10.45 | | | X | ** |
| 73 | 0595 | 5825: DNA-binding response regulator / 24 780 / 5.44 | X | X | | ** |
| 74 | 0604 | 5834: alcohol dehydrogenase, zinc-containing / 38394 / 5.47 | | X | | |
| 75 | 0610 | 5840: spermidine/putrescine ABC transporter, ATP-binding protein / 47 425 / 6.03 | X | X | X | * |
| 76 | 0617 | 5847: transcription termination factor Rho / 47 306 / 6.23 | X | | X | ** |
| 77 | 0618 | 5848:phosphoenolpyruvate synthase /,87 170 / 5.01 | X | X | X | ** |
| 78 | 0623 | 5854: spermidine/putrescine ABC transporter, periplasmic ... / 39 511 / 5.38 | X | X | | |
| 79 | 0626 | 5856: peptide chain release factor 3/59 589/5.43 | | X | | + |
| 80 | 0631 | 5861: phosphate acetyltransferase Pta, putative / 50306 / 4.83 | X | X | | |

(continued)

| | NMB | ID (gi:722xxxx): name / MW / pl | Gel (Fig.) | | | Note |
|---|---|---|---|---|---|---|
| | | | 2 | 3 | 4 | |
| 81 | 0634 | 5862: iron(III) ABC transporter, periplasmic binding protein / 35806 / 9.60 | | | X | |
| 82 | 0637 | 5866: argininosuccinate lyase /51245 / 5,22 | X | X | | ** |
| 83 | 0641 | 5870: inorganic pyrophosphatase / 19 812 / 4.71 | | X | | ** |
| 84 | 0663 | 5888: outer membrane protein NsgA / 18386 / 9.64 | | | X | |
| 85 | 0667 | 5893: hypothetical protein / 45 292 / 5,97 / 16 | X | X | X | + |
| 86 | 0697 | 5925: dimethyladenosine transferase / 29269 / 7.11 | | | X | ** |
| 87 | 0703 | 5932: competence lipoprotein ComL / 29 273 / 8.72 | X | | X | |
| 88 | 0711 | 5938: conserved hypothetical protein / 33516 / 6.11 | | X | | ** |
| 89 | 0720 | 5946: threonyl-tRNA synthetase /72 692 / 5,86 | X | X | | * |
| 90 | 0724 | 5951: phenylalanyl-tRNA synthetase, alpha chain / 37 334 / 5.46 | X | X | X | ** |
| 91 | 0728 | 5955: phenylalanyl-tRNA synthetase, beta chain / 86 050 / 5.19 | X | X | | * |
| 92 | 0743 | 5972: ubiquinone/menaquinone biosynthesis methlytransferase UbiE / 27 366 / 7.14 | X | | X | ** |
| 93 | 0757 | 5987: phosphoribosylaminoimidazole-succinocarboxamide synthase / 32476 / 5.26 | | X | | ** |
| 94 | 0758 | 5988: polyribonucleotide nucleotidyltransferase 176 422 15.35 | X | X | X | |
| 95 | 0763 | 5993: cysteine synthase / 32 821 / 6.06 | X | X | | * |
| 96 | 0768 | 5999: twitching motility protein PilT / 41508 / 7.64 | X | X | X | + |
| 97 | 0778 | 6009: uroporphyrin-III C-methyltransferase HemX, putative / 46 320 /5,33 | X | X | | |
| 98 | 0787 | 6019: amino add ABC transporter, periplasmic amino acid-binding protein / 26 995 / 5.42 | X | X | | + |
| 99 | 0791 | 6024 : peptidyl-prolyl cis-trans isomerase / 18185315.05 | | X | | ** |
| 100 | 0798 | 6031: cell division protein FtsH / 70 058 / 5,07 / 20 | X | | | + |
| 101 | 0801 | 6034: delta-aminolevulinic acid dehydratase / 36878 / 5.23 | | X | | + |
| 102 | 0804 | 6038: NAD(P)H nitroreductase, putative / 24687 / 6.17 | | X | | * |
| 103 | 0814 | 6048: histidyl-tRNA synthetase / 41 746 / 5,45 | X | X | | ** |
| 104 | 0815 | 6050: adenylosuccinate synthetase / 46 250 / 5,77 | X | X | X | * |
| 105 | 0828 | 6063: ADP-L-glycero-D-mannoheptose-6-epimerase / 38 437 / 5,68 | X | X | | |
| 106 | 0853 | 6089: conserved hypothetical protein / 23136 / 5.67 | | X | | + |
| 107 | 0854 | 6090: histidyl-tRNA synthetase / 48 465 / 5.57 | X | X | | ** |
| 108 | 0875 | 6113: ribose-phosphate pyrophosphokinase / 35 598 / 5.41 | X | X | | ** |
| 109 | 0876 | 6114: 50S ribosomal protein L25 / 20 956 / 6.60 | X | X | X | * |
| 110 | 0878 | 6116: threonine dehydratase / 55585 / 5.41 | | X | | |
| 111 | 0884 | 6122: superoxide dismutase / 21893 / 5.78 | X | X | | * |
| 112 | 0885 | 6124: replicative DNA helicase / 52 089 / 5,04 | X | X | | ** |
| 113 | 0889 | 6127: hypothetical protein / 21970/6.40 | | X | | + |

(continued)

| | NMB | ID (gi:722xxxx): name / MW / pI | Gel (Fig.) | | | Note |
|---|---|---|---|---|---|---|
| | | | 2 | 3 | 4 | |
| 114 | 0894 | 6131: aminotransferase, class I / 44 013 / 5.79 | | X | | ** |
| 115 | 0920 | 6158 : isocitrate dehydrogenase / 80163 / 5.57 | X | X | X | * |
| 116 | 0928 | 6166: hypothetical protein /43 815 / 9.01 | X | | | + |
| 17 7 | 0944 | 6182. 5-melhyltetrahydropteroyltriglulamate-homocysteine methyltransferase / 85 078 / 5.27 | X | X | | |
| 118 | 0946 | 6184: peroxiredoxin 2 family protein/glutaredoxin /26 912 / 4.80 | X | X | | * |
| 119 | 0947 | 6186 : lipoamide dehydrogenase, putative / 44527 / 5.66 | X | X | | |
| 120 | 0950 | 6189: succinate dehydrogenase, flavoprotein subunit / 66 787 / 5,84 | X | X | | + |
| 121 | 0955 | 6194: 2-oxoglutarate dehydrogenase, E1 component / 105 082 / 6.24 | X | X | X | |
| 122 | 0956 | 6195: 2-oxoglutarate dehydrogenase, E2, dihydrolipoamide succinyltransferase / 41491 / 5.14 | | X | | |
| 123 | 0959 | 6199: succinyl-CoA synthetase, beta subunit / 41666 / 5.06 | | X | | |
| 124 | 0960 | 6200: succinyl-CoA synthetase, alpha subunit /30 548 / 6.00 | X | X | | |
| 125 | 0983 | 6223 : phosphoribosylaminoimidazolecarboxamide formyltransferase ... / 56 803 / 5.90 | | X | | |
| 126 | 0997 | 6237: D-lactate dehydrogenase / 64001 / 6.32 | | | X | |
| 127 | 1031 | 6270: 3-isopropylmalate dehydrogenase / 39172 / 4.91 | | X | | ** |
| 128 | 1044 | 6284: ferredoxin-NADP reductase / 29 314 / 5.72 | X | X | | * |
| 129 | 1046 | 6286: threonine synthase / 51870 / 5.31 | | X | | * |
| 130 | 1053 | 6294: class 5 outer membrane protein / 28 009 / 9.68 / 20 | X | X | X | |
| 131 | 1057 | 6297: gamma-glutamyltranspeptidase / 65 071 / 5.99 | X | X | | + |
| 132 | 1070 | 6309: 2-isopropylmalate synthase / 55 397 / 5.68 | | X | | ** |
| 133 | 1073 | 6313: conserved hypothetical protein / 42 032 / 4.60 | | X | | ** |
| 134 | 1127 | 6364: oxidoreductase, short chain dehydrogenase/reductase family / 25 917 / 5.99 | X | X | | ** |
| 135 | 1131 | 6368: chaperone protein HscA / 66166/ 5.18 | | X | | ** |
| 136 | 1150 | 6387: dihydroxy-acid dehydratase / 64 503 / 5,89 | X | X | | * |
| 137 | 1164/1 126 | 6400-6362: hypothetical protein / 22 025 / 8.03 (duplicated gene) | X | | X | + |
| 138 | 1199 | 6435: GTP binding protein / 67 260/5.04 | X | X | | + |
| 139 | 1201 | 6438: IMP dehydrogenase / 53 383 / 6.72 | X | | | * |
| 140 | 1206 | 6443: bacterioferritin B / 18075 / 4.60 | | X | | ** |
| 141 | 1228 | 6467: homoserine dehydrogenase /44 737 / 5.254 / 20 | X | X | | |
| 142 | 1231 | 6470: ATP-dependent protease La / 90590 / 6.18 | | X | X | * |
| 143 | 1238 | 6478: peptidyl-prolyl cis-trans isomerase-related protein / 53213 / 5.68 | X | X | | + |
| 144 | 1240 | 6480: ABC transporter, ATP-binding protein / 60 779/5.09 | X | X | | ** |

(continued)

| | NMB | ID (gi:722xxxx): name / MW / pl | Gel (Fig.) | | | Note |
|---|---|---|---|---|---|---|
| | | | 2 | 3 | 4 | |
| 145 | 1252 | 6492: phosphoribosylformylglycinamidine cyclo-ligase / 36 974 / 4.71 | X | | | ** |
| 146 | 1285 | 6525: enolase / 46 134 / 4,78 | X | X | | + |
| 147 | 1301 | 6541: 30S ribosomal protein / 61177 / 4.9 | X | X | X | * |
| 148 | 1302 | 6542: integration host factor, beta subunit / 11796 / 9.98 | | | X | ** |
| 149 | 1313 | 6554: trigger factor/ 48 325 4.72 | X | X | | ** |
| 150 | 1320 | 6562: 50S ribosomal protein L9 / 15 747 / 6.62 | X | X | X | |
| 151 | 1323 | 6565: 30S ribosomal protein S6 / 13 949 / 6.37 | X | X | X | ** |
| 152 | 1324 | 6566: thioredoxin reductase / 33961 / 5.16 | | X | | |
| 153 | 1328 | 6571: conserved hypothetical protein / 27021 / 6.41 | | | X | * |
| 154 | 1339 | 6583:prolyl-tRNA synthetase /62 99215.09 | X | X | X | ** |
| 155 | 1341 | 6585:pyruvate dehydrogenase, E1 component / 99 562 / 5.5 | X | X | X | ** |
| 156 | 1342 | 6586 : pyruvate dehydrogenase, E2, dihydrolipoamide acelyltransferase / 55 223 / 5,29 | X | X | | * |
| 157 | 1343 | 6587: hypothetical protein / 16 339/ 4.99 | X | | | ** |
| 158 | 1344 | 6588: pyruvate dehydrogenase, E3 component, lipoamide dehydrogenase / 62358 / 5.07 | | X | | |
| 159 | 1345 | 6590: hypothetical protein / 57143/5.13 | | X | | + |
| 160 | 1347 | 6591: extragenic suppressor protein SuhB / 28 469 / 5,82 | X | X | X | * |
| 161 | 1356 | 6601: Glu-tRNA(Gln)amidotransferase, subunit A / 51 280 / 5,44 | X | | | * |
| 162 | 1358 | 6603: Glu-tRNA(Gln) amidotransferase, subunit B / 51 912 / 5,05 | X | X | X | ** |
| 163 | 1372 | 6618:ATP-dependent Clp protease, ATP-binding subunit ClpX / 45100 / 5.18 | | X | | * |
| 164 | 1379 | 7413462: nifS protein / 4483215.62 | | X | | ** |
| 165 | 1390 | 6628: glucokinase / 34 951 / 8.59 | X | | X | |
| 166 | 1392 | 6630: glucose-6-phosphate 1-dehydrogenase / 5412415.30 | X | X | X | ** |
| 167 | 1425 | 6664: lysyl-tRNA synthetase, heat inducible / 57312 / 5.34 | X | X | | ** |
| 168 | 1429 | 6669: outer membrane protein PorA/40 129/8.73 | X | X | X | |
| 169 | 1445 | 6686: recA protein / 37 612 l 5.18 | | X | | * |
| 170 | 1452 | 6693: conserved hypothetical protein / 40 598/8.33 | X | | | * |
| 171 | 1457 | 6698: transketolase / 71 659 / 5.45 | X | X | X | * |
| 172 | 1471 | 6711: tryptophanyl-tRNA synthetase / 37 616 / 5.65 | X | X | | * |
| 173 | 1472 | 6713: clpB protein / 95 195 / 5.41 | | X | | ** |
| 174 | 1483 | 6723: lipoprotein NlpD, putative / 42991/9.55 | | | X | + |
| 175 | 1506 | 6749: arginyl-tRNA synthetase / 62 803 / 5.24 | | X | | |
| 176 | 1518 | 6762: acetate kinase / 42 410 / 5.76 | X | X | | |
| 177 | 1533 | 6778. H.8 outer membrane protein / 16 886/4.61/17 | X | X | | |
| 178 | 1536 | 6781: preprotein translocase SecA subunit / 103 295 / 5.05 | X | X | | * |

(continued)

| | NMB | ID (gi:722xxxx): name / MW / pI | Gel (Fig.) | | | Note |
|---|---|---|---|---|---|---|
| | | | 2 | 3 | 4 | |
| 179 | 1560 | 6807: glutaminyl-tRNA synthetase / 64 650 / 57.74 | X | X | | ** |
| 180 | 1567 | 6815: macrophage infectivity potentiator / 26 875 / 5.50 | X | X | | + |
| 181 | 1572 | 6819: aconitate hydratase 2 / 92 716 7 5.42 | X | X | | |
| 182 | 1574 | 6822: ketol-acid reductoisomerase / 36 439 / 5.65 | X | X | | ** |
| 183 | 1577 | 6825: acetolactate synthase III, large subunit / 62 813 / 5.88 | X | X | | |
| 184 | 1581 | 6829: histidinol dehydrogenase / 46324 / 5.07 | X | X | | |
| 185 | 1583 | 6831: imidazoleglycerol-phosphate dehydratase / 34002 / 8.61 | | | X | ** |
| 186 | 1584 | 6833:3-hydroxyaciddehydrogenase / 30 378 / 5.33 | | X | | + |
| 187 | 1594 | 6843: spermidine/putrescine ABC transporter, periplasmic ... / 40 234 5.52 | X | X | | + |
| 188 | 1595 | 6845:alanyl-tRNA synthetase / 96 038/5.54 | X | | X | ** |
| 189 | 1604 | 6853: phosphoglycerate mutase / 25 959 / 5.59 | X | X | | ** |
| 190 | 1612 | 6862: amino add ABC transporter, periplasmic amino acid-binding protein / 27 68 / 4.87 | X | X | | + |
| 191 | 1613 | 6863: fumarate hydratase, class I / 54 951 / 5,12 | X | X | | + |
| 192 | 1621 | 6872: glutathione peroxidase / 20129 / 5.18 | | X | | ** |
| 193 | 1636 | gi 7443356 opacity protein, authentic frameshift / 27180 / 9.52 | | | X | |
| 194 | 1640 | 6890: phosphoserine aminotransferase / 41651 / 5.20 | | X | | * |
| 195 | 1642 | 6892: N utilization substance protein A / 55 752 / 4.54 | X | X | | ** |
| 196 | 1655 | 6907: adenine specific methylase, putative / 33 956 / 4,36 | X | | | * |
| 197 | 1684 | 6938: seryl-tRNA synthetase / 47 883 / 5.60 | X | | | ** |
| 198 | 1691 | 6945: dihydropteroate synthase / 30 313/ 5.30 | | X | | |
| 199 | 1710 | 6965: glutamate dehydrogenase, NADP-specific / 48 490 / 5.98 | X | X | | ** |
| 200 | 1714 | 6969. multidrug efflux pump channel protein / 48 482 / 8.38 / lipo | X | | X | + |
| 201 | 1716 | 6971: membrane fusion protein / 40 209 8.60 | X | | X | |
| 202 | 1796 | 7050: conserved hypothetical protein/ 20 931 / 5.73 | X | X | | |
| 203 | 1809 | ----: putative; pilN protein, authentic frameshift / 22 258 / 9.00 | X | | | |
| 204 | 1810 | 7063: pilO protein / 19 870/4.74 | | X | | |
| 205 | 1811 | 7064: pilP protein / 20126 / 4.94 | | X | | |
| 206 | 1812 | ---.putative, pilQ protein, authentic frameshift / 83151 I 10.75 / 25 | X | X | X | |
| 207 | 1835 | 7090: tyrosyl-tRNA synthetase / 47437 / 15.76 | | X | | * |
| 208 | 1838 | 7093: GTP-binding protein, putative / 39 376 / 4,80 | X | X | | ** |
| 209 | 1839 | 7094: formate-tetrahydrofolate ligase / 59063 / 5.85 | X | X | | + |
| 210 | 1843 | 7099: transcriptional regulator, MarR family / 16 583 / 5.15 | | X | | ** |
| 211 | 1849 | 7105: carbamoyl-phosphate synthase, small subunit /40 587 / 5.54 | X | X | | |
| 212 | 1854 | 7110: hypothetical protein / 25 131 / 8.95 | X | | X | ** |

(continued)

| | NMB | ID (gi:722xxxx): name / MW / pI | Gel (Fig.) | | | Note |
|---|---|---|---|---|---|---|
| | | | 2 | 3 | 4 | |
| 213 | 1855 | 7111: carbamoyl-phosphate synthase, large subunit/ 117 377 / 5.10 | X | X | | |
| 214 | 1856 | 7112: transcriptional regulator, LysR family / 33367 / 5.55 | | X | | + |
| 215 | 1859 | 7116: S-adenosylmethionine:tRNA ribosyltansferase-isomerase / 38216 / 6.20 | | X | | * |
| 216 | 1861 | 7118: acetyl-CoA carboxylase, biotin carboxylase / 49 599 / 5,88 | X | X | X | ** |
| 217 | 1862 | 7119: ribosomal protein L11 methyltransferase / 32144 / 4.36 | | X | | |
| 218 | 1864 | 7121:glutamate-1-semialdehyde 2,1-aminomutase / 45 315 / 5.33 | | X | | * |
| 219 | 1869 | 7127: fructose-bisphosphate aldolase / 38 337 / 5.48 | X | X | X | + |
| 220 | 1870 | 7128: hypothetical protein / 26 964 / 7.23 | X | | X | + |
| 221 | 1903 | 7160: chromosomal replication initiator protein DnaA/58 029 5.93 | | X | | ** |
| 222 | 1920 | 7178: GMP synthase / 57686 / 5.53 | X | X | | |
| 223 | 1921 | 7179: 3-oxoacyl-(acyl-carrier-protein) reductase / 26 068 / 5.95 | X | X | X | * |
| 224 | 1930 | 7187: glycyl-tRNA synthetase, beta chain 174 574 / 5,86 | X | X | | |
| 225 | 1934 | 7192: ATP synthase F1, beta subunit / 50 391 / 5.01 | X | X | X | |
| 226 | 1936 | 7194: ATP synthase F1, alpha subunit / 55 291 / 5.43 | X | X | X | * |
| 227 | 1937 | 7195: ATP synthase F1, delta subunit / 19 526 / 5.02 | | X | | ** |
| 228 | 1938 | 7196: ATP synthase F0, B subunit / 17128 / 5.64 | | X | | |
| 229 | 1946 | 7205: outer membrane lipoprotein / 29 258 / 5.01 | X | X | X | + |
| 230 | 1953 | 7212: stringent starvation protein A / 23 165 / 6.23 | X | X | X | ** |
| 231 | 1966 | 7226 :ABC transporter, ATP-binding protein / 29 286 / 5.58 | | X | | * |
| 232 | 1968 | 7228: aldehyde dehydrogenase A / 52257 / 5.20 | X | X | | |
| 233 | 1972 | 7233: chaperonin, 60 kDa/57 423 4.9 | X | X | X | + |
| 234 | 1982 | 7243: DNA polymerase I/103 184/5.21 | | X | | * |
| 235 | 1996 | 7259: phosphoribosylformylglycinamidine synthase / 143 854 / 5.31 | X | X | | * |
| 236 | 2000 | 7264: conserved hypothetical protein / 33468 / 4.77 | | X | | ** |
| 237 | 2039 | 7300 : major outer membrane protein PIB / 33 786 / 6.54 | X | X | X | |
| 238 | 2057 | 7319: 50S ribosomal protein L13 / 16212 / 9.72 | | | X | ** |
| 239 | 2079 | 7341: aspartate-semialdehyde dehydrogenase / 39 858 / 5.40 | | X | | * |
| 240 | 2086 | 7348: GTP-binding protein / 41 930/5.52 | | X | | * |
| 241 | 2091 | 7353: hemolysin, putative / 21804/9.81 | | | X | + |
| 242 | 2096 | 7359: malate:quinone oxidoreductase / 53 9687 / 5.51 | X | X | | + |
| 243 | 2101 | 7364: 30S ribosomal protein S2 / 26 899 / 9.04 | X | | | ** |
| 244 | 2102 | 7365. elongation factor TS (EF-TS) / 30 330 / 5.30 | X | X | | ** |
| 245 | 2103 | 7366: uridylate kinase / 25896 / 6.17 | | | X | * |

EP 1 562 982 B1

(continued)

| | NMB | ID (gi:722xxxx): name / MW / pl | Gel (Fig.) | | | Note |
|---|---|---|---|---|---|---|
| | | | 2 | 3 | 4 | |
| 246 | 2129 | 7385: argininosuccinate synthase / 49 664 / 5.18 | X | X | | * |
| 247 | 2138 | 7395: peptide chain release factor 2 / 41 487 5.18 | X | | | ** |
| 248 | 2154 | 7412: electron transfer flavoprotein, alpha subunit / 32 579 / 4.99 | | X | | * |
| 249 | 2155 | 7413. electron transfer flavoprotein, beta subunit / 26 948 / 6.08 | X | X | X | ** |
| 250 | 2159 | 7417: glyceraldehyde 3-phosphate dehydrogenase / 35 845 / 5.40 | X | X | | * |
| | | TOTALS 7 | 156 | 194 | 87 | |

**REFERENCES**

[1] Tettelin et al. (2000) Science 287:1809-1815.

[2] Pizza et al. (2000) Science 287:1816-1820.

[3] Parkhill et al. (2000) Nature 404:502-506.

[4] Cole (2002) Eur Respir J Suppl. 36:78s-86s.

[5] McClelland et al. (2001) Nature 413:852-856.

[6] De Groot et al. (20001) Vaccine 19:4385-4395.

[7] Stephens (2000) J Infect Dis 181 Suppl 3:S521-523.

[8] Tettelin et al. (2001) Science 293:498-506.

[9] Weinstock et al. (2000) Res Microbiol 151:151-158.

[10] Wizemann et al. (2001) Infect Immun 69:1593-1598.

[11] Grifantini et al. (2002) Nat Biotechnol. 20:914-921.

[12] *http:llwww.ncbi.nlm.nih.govlentrez*

[13] WO01/64922.

[14] WO01/64920.

[15] WO03/020756.

[16] Bjune et al. (1991) Lancet 338(8775):1093-1096.

[17] International patent application WO00/25811.

[18] International patent application WO01/52885.

[19] de Kleijn et al. (2001) Vaccine 20:352-358.

[20] US patent 5597572.

[21] US patent 5747653.

[22] European patent application 0680512.

[23] International patent application WO01/09350.

[24] International patent application WO02/09746.

[25] European patent 0449958

[26] International patent application WO01/91788.

[27] Gennaro (2000)-Remington: The Science and Practice of Pharmacy. 20th edition, ISBN: 0683306472.

[28] Vaccine design: the subunit and adjuvant approach, eds. Powell & Newman, Plenum Press 1995 (ISBN 0-306-44867-X).

[29] WO90/14837.

[30] WO02/26212.

[31] WO98/33487.

[32] WO00/07621.

[33] WO99/27960.

[34] WO98/57659.

[35] European patent applications 0835318, 0735898 and 0761231.

[36] Krieg (2000) Vaccine 19:618-622; Krieg (2001) Curr opin Mol Ther 2001 3:15-24; WO96/02555, WO98/16247, WO98/18810, WO98/40100, WO98/55495, WO98/37919 and WO98/52581 *etc*.

[37] WO99/52549.

[38] WO01/21207.

[39] WO01/21152.

[40] WO00/62800.

[41] WO00/23105.

[42] WO99/11241.

[43] Del Giudice et al. (1998) Molecular Aspects of Medicine, vol. 19, number 1.

*[44]* Vaccine Design... (1995) eds. Powell & Newman. ISBN: 030644867X. Plenum.

[45] Johnson et al. (1999) Bioorg Med Chem Lett 9:2273-2278.

[46] Covacci & Rappuoli (2000) J. Exp. Med. 19:587-592.

[47] WO93/18150.

[48] Covacci et al. (1993) Proc. Natl. Acad. Sci. USA 90: 5791-5795.

[49] Tummuru et al. (1994) Infect. Immun. 61:1799-1809.

[50] Marchetti et al. (1998) Vaccine 16:33-37.

[51] Telford et al. (1994) J. Exp. Med. 179:1653-1658.

[52] Evans et al. (1995) Gene 153:123-127.

[53] WO96/01272 & WO96/01273, especially SEQ ID NO:6.

[54] WO97/25429.

[55] WO98/04702.

[56] Costantino et al. (1992) Vaccine 10:691-698.

[57] Costantino et al. (1999) Vaccine 17:1251-1263.

[58] International patent application WO03/007985.

[59] Watson (2000) Pediatr Infect Dis J 19:331-332.

[60] Rubin (2000) Pediatr Clin North Am 47:269-285, v.

[61] Jedrzejas (2001) Microbiol Mol Biol Rev 65:187-207.

[62] Bell (2000) Pediatr Infect Dis J 19:1187-1188.

[63] Iwarson (1995) APMIS 103:321-326.

[64] Gerlich et al. (1990) Vaccine 8 Suppl:S63-68 & 79-80.

[65] Gustafsson et al. (1996) N. Engl. J. Med. 334:349-355.

[66] Rappuoli et al. (1991) TIBTECH 9:232-238.

[67] Vaccines (1988) eds. Plotkin & Mortimer. ISBN 0-7216-1946-0.

[68] Del Guidice et al. (1998) Molecular Aspects of Medicine 19:1-70.

[69] Hsu et al. (1999) Clin Liver Dis 3:901-915.

[70] International patent application WO99/24578.

[71] International patent application WO99/36544.

[72] International patent application WO99/57280.

[73] International patent application WO02/079243.

[74] International patent application WO02/02606.

[75] Kalman et al. (1999) Nature Genetics 21:385-389.

[76] Read et al. (2000) Nucleic Acids Res 28:1397-406.

[77] Shirai et al. (2000) J. Infect. Dis. 181(Suppl 3):S524-S527.

[78] International patent application WO99/27105.

[79] International patent application WO00/27994.

[80] International patent application WO00/37494.

[81] International patent application WO99/28475.

[82] Ross et al. (2001) Vaccine 19:4135-4142.

[83] Sutter et al. (2000) Pediatr Clin North Am 47:287-308.

[84] Zimmerman & Spann (1999) Am Fam Physician 59:113-118, 125-126.

[85] Dreesen (1997) Vaccine 15 Suppl:S2-6.

[86] MMWR Morb Mortal Wkly Rep 1998 Jan 16;47(1):12, 19.

[87] Vaccines (1988) eds. Plotkin & Mortimer. ISBN 0-7216-1946-0.

[88] McMichael (2000) Vaccine 19 Suppl 1:S101-107.

[89] Schuchat (1999) Lancet 353(9146):51-6.

[90] International patent application WO02/34771.

[91] Dale (1999) Infect Dis Clin North Am 13:227-43, viii.

[92] Ferretti et al. (2001) PNAS USA 98: 4658-4663.

[93] Kuroda et al. (2001) Lancet 357(9264):1225-1240; see also pages 1218-1219.

[94] J Toxicol Clin Toxicol (2001) 39:85-100.

[95] Demicheli et al. (1998) Vaccine 16:880-884.

[96] Stepanov et al. (1996) J Biotechnol 44:155-160.

[97] Ingram (2001) Trends Neurosci 24:305-307.

[98] Rosenberg (2001) Nature 411:380-384.

[99] Moingeon (2001) Vaccine 19:1305-1326.

[100] EP-A-0372501

[101] EP-A-0378881

[102] EP-A-0427347

[103] WO93/17712

[104] WO94/03208

[105] WO98/58668

[106] EP-A-0471177

[107] WO00/56360

[108] WO91/01146

[109] WO00/61761

[110] WO01/72337

[111] *Research Disclosure,* 453077 (Jan 2002)

[112] Molloy et al. (2000) Eur J Biochem 267(10):2871-81

[113] Molloy et al. (2001) Electrophoresis 22(9):1686-96

[114] Phadke et al. (2001 ) Proteomics 1 (5):705-20.

[115] Nouwens et al. (2000) Electrophoresis 21(17):3797-809.

[116] Chevallet et al. (1998) Electrophoresis 19(11):1901-9.

[117] Herbert et al. (1998) Electrophoresis 19(5):845-51.

[118] Doherty et al. (1998) Electrophoresis 19(2):355-63.

[119] Wilm et al. (1996) Nature 379(6564):466-9.

[120] WO99/24578.

[121] WO99/36544.

[122] WO99/57280.

[123] Nakai & Kanehisa Proteins 11(2):95-110

[124] Nakai (2000) Adv Proteins Chem 54:277-344.

[125] WO01/38350

**SEQUENCE LISTING**

EP 1 562 982 B1

| SED ID | NMB | Sequence |
|---|---|---|
| 1 2 | 0010 | MAFLKLTEQNVQGKTVLIRADMNVPFKDGKISDDTRIRASLASIKYCVDNGASVIVMTHLGRPTEGEFHPEDDVAP VAAHLGSLLGKDVKVLNDWRENKPALNAGDVVMLQNVRINKGEKKNDLELGKAYASLCDVFVNDAFGTAHRAQAST EAVAQAAPVACAGVLMAGELDALGKALKQPARPMVAIVAGSKVSTKLTILESLADKVDQLIVGGGIANTFLLAEGK AIGKSLAEHDLVEESKKIMAKMAAKGGSVPLPTDVVVAKAFAADAEAVVKDIAADAEAVVKDIADVAEDEMILDIG PKSAAALADLLKAAGTVVWNGPVGVFEFDQFAGGTKALAEAIAQSKAFSIAGGGDTLAAIAKFGVTEQIGYISTGG GAFLEFLEGKELPAVAALEKRGA |
| 2 | 0018 | MNTLQKGFTLIELMIVIAIVGILAAVALPAYQDYTARAQVSEAILLAEGQKSAVTEYYLNHGEWPGNNTSAGVATS SEIKGKYVKSVEVKNGVVTAQMASSNVNNEIKGKKLSLWAKRQNGSVKWFCGQPVTRDKAKAANDDVTAAAAANGK KIDTKHLPSTCRDASDAS |
| 3 | 0030 | MTRKILVTSALPYANGSIHLGHMVEHIQTDVWVRFQKLRGHACHYCCADDTHGTPVMLAAQKQGIAPEDMIAKVRE EHLADFTGFFIGYDNYYSTHSPENKQFSQDIYRALKANGKIESRVIEQLFDPEKQMFLPDRFVKGECPKCHAQDQY GDNCEVCGTTYSPTELINPYSAVSGTKPELRESEHFFFKLGECADFLKAWTSGNNPHDGKPHLQAEALNKMKEWLG EGEFTLSDWDISRDAPYFGFEIPDAPGKYFYVWLDAPVGYMASFKNLCDRIGVDFDEYFKADSQTEMYHFIGKDI LYFHALFWPAMLHFSGHRAPYGVPRHGELTVDGQKMSKSRGTFITAKSYLEQGLNPEWMRYYIAAKLNSKIEDIDL NLQDFISRVNSDLVGKYVNIAARASGFIAKRFEGRLKDVADSELMKLTAQSEAIAECYESREYAKALRDIMALAD IVNEYVDANKPWELAKQEGQDERLHEVCSELINAFTMLTAYLAPVLPQTAANAAKFLNLEAITWANTRDTLGKHAI NKYEHLMQRVEQKQVDDLIEANKQSIAAAAAPAAEEGKYEKVAEQASFDDFMKIDMRVAKVLNCEAVEGSTKLLKF DLDFGFEKRIIFSGIAASYPNPAELNGRMVIAVANFAPRKMAKFGVSEGMILSAATADGKLKLLDVDTGAQPGDKV G |
| 4 | 0038 | MPQNTLNIVILAAGKGTRMYSKMPKVLHRIGGKPMVGRVIDTAAALNPQNICVVIGHGKEQVLDTVKRDVVWVEQT EQLGTGHAVKTALPHLSAEGRTLVLYGDVPLIDVETLETLLEAAGNEVGLLTDVPNDPTGLGRIIRDSNGSVTAIV EEKDADAVQKAVKEINTGILVLPNAKLENWLNSLSSNNAQGEYYLTDLIAKAVADGIKVHPVQVRASHLAAGVNNK LQLTELERIFQTEQAQELLKAGVTLRDPARFDLRGRLKHGQDVVIDVNCIFEGDIELGDNVEIGANCVIKNAKIGA NSKIAPFSHLESCEVGENNRIGPYARLRPQARLADDVHVGNFVEIKNAAIGKGTKANHLTYIGDAEVGCKTNFGAG TIIANYDGVHKHKTVIGDEVRIGSNCVLVAPVTLGNKVTTGAGSTITRNVEDNKLALARARQTVIEGWVRPEKDKQ |

| SED ID | NMB | Sequence |
|---|---|---|
| 5 | 0045 | MFSFFRRKKKQETPALEEAQIQETAAKAESELAQIVENIKEDAESLAESVKGQVESAVETVSGAVEQVKETVAEML<br>SEAEEAAEKAAEQVEAAKEAVAETVGEAVGQVQEAVATTEEHKLGWAARLKQGLTKSRDKMAKSLAGVFGGGQIDE<br>DLYEELETVLITSDMGMEATEYLMKDVRDRVSLKGLKDGNELRGALKEALYDLIKPLEKPLVLPETKEPFVIMLAG<br>INGAGKTTSIGKLAKYFQAQGKSVLLAAGDTFRAAAREQLQAWGERNNVTVISQTTGDSAAVCFDAVQAAKARGID<br>IVLADTAGRLPTQLHLMEEIKKVKRVLQKAMPDAPHEIIVVLDANIGQNAVNQVKAFDDALGLTGLIVTKLDGTAK<br>GGILAALASDRPVPVRYIGVGEGIDDLRPFDARAFVDALLD |
| 6 | 0051 | MNTDNLHDILDEMVQVYSQKKQSRSETPAEIGAHFHPLLDRLCETAEAQNASDILISKGFPPSLKINSALTPQPQK<br>ALTGEETAAIAASTMNAEQSEIFRRDGEINYSVQSRSGTRYRANAYHSQGSAGLVLRRINHVIPQMQELGLPEKLK<br>DLAVAPRGLLIIVGPTGSGKSTTMATMLEHRNKTLPSHIVTIEDPIEFIYKPRRCIFTQREIGVDTINWQTAVQNA<br>MRQSPDVVCIGEVRSRESMEYAMQLAQTGHLCIFTLHANTAPQSLERILNFYPKEQHNQILIDIALNLTGIICQRL<br>ALKQDKTGRTAVVDLLINTPAIQDFILKGDLMNISKIMETAKTDGMQTMDQNLFELYRHGIISYEEALRQSVSANN<br>LRLHIQLHKEGKTPELLYDRVNGLNLIS |
| 7 | 0052 | MQITDLLAFGAKNKASDLHLSSGISPMIRVHGDMRRINLPEMSAEEVGNMVTSVMNDHQRKIYQQNLEVDFSFELP<br>NVARFRVNAFNIGRGPAAVFRTIPSTVLSLEELKAPSIFQKIAESPRGMVLVTGPTGSGKSTTLAAMINYINETQP<br>AHILTIEDPIEFVHQSKKSLINQRELHQHTLSFANALRSALREDPDVILVGEMRDPETIGLALTAAETGHLVFGTL<br>HTTGAAKTVDRIVDVFPAGEKEMVRSMLSESLTAVISQNLLKTHDGNGRVASHEILIANPAVRNLIRENKITQINS<br>VLQTGQASGMQTMDQSLQSLVRQGLIAPEVARRRAQNSESMSF |
| 8 | 0068 | MQNNNEFKIGNRSVGYNHEPLIICEIGINHEGSLKTAFEMVDAAYNAGAEVVKHQTHIVEDEMSDEAKQVIPGNAD<br>VSIYEIMERCALNEEDEIKLKEYVESKGMIFISTPFSRAAALRLQRMDIPAYKIGSGECNNYPLIKLVASFGKPII<br>LSTGMNSIESIKKSVEIIREAGVPYALLHCTNIYPTPYEDVRLGGMNDLSEAFPDAIIGLSDHTLDNYACLGAVAL<br>GGSILERHFTDRMDRPGPDIVCSMNPDTFKELKQGAHALKLARGGKKDTIIAGEKPTKDFAFASVVADKDIKKGEL<br>LSGDNLWVKRPGNGDFSVNEYETLFGKVAACNIRKGAQIKKTDIE |

EP 1 562 982 B1

| SED ID | NMB | Sequence |
|---|---|---|
| 9 | 0089 | MNQTSRDLTRISHNTKIVATLGPGSNNVELLEDMIRVGGLNVVRFNFSHGTPEFHQENALIVREAAKRAGQEIAII ADLQGPKIRVGKIAGGGIELNKGETLVLDAALEGEGTREAVGLDYRDLPDDVAAGDVLWLDDGLLTLTVESVEGSR IITRVENSHVLKSNKGINKRGGGLSAGALTEKDFRDLKTAIAIGCDYLAISFVKSAEDLHIARAKVEEEMKGSTAV RPGLVSKIERVEAIENLDEIILAGDGIMVARGDLAVEVGHAAVPALQKRMIRRARELRRFSITATQMMESMITNPV PTRAEVSDVANAVLDGTDAVMCSAETAVGAYPFETVSQMAIICAAAEKEQDSLNGVAEQVEYPEAVSTNLAVAGGA VSVARAVHAKAIVALTESGSTAFEISRHNITLPIFALTPSVSAQRRMAMYRGVRPLILATSTDHDTALNEVETMLV EHNILHSGDQYIITSGSQMRESGSTNTLEVLRVK |
| 10 | 0109 | MLKCGTFFITRHIPRGCRRFFQPNQARQTEIYQIRGTVMQRRIITLLCAAGMAFSTQTLAANLEVRPNAPERYTVK |
| | | QGDTLWGISGKYLYSPWQWGRLWDANRDQIHNPDLIYPDQVLVLRHVDGEPRLGLEQTDGIPVVKMSPDKEVSGYG IPAIDVNFYRIFMRHPQIVSRKETAAAPRLLSGPEGRLLYTKGTRVYTKGLKEPGRYLTYRINKNITDPDTGKFLG QEVAFSGIVRSLDYTDSVLEQRSKQAGERPKDNEYHTRTHPLITPLRTPSIQPLVVETAISEIQQGDYLMKMPEDT DRFNMMPHEPSRPVQAKIVSVFEGTRIAGQFQTITIDKGEADGLDKGTVLSLYKRKKTMQVDLSNNFKSRDTVELI STPAEEVGLAMVYRTSEHLSSAIILENISDISVGDTAANPGRDLDNIPDQGRSRVKFGFNRSE |
| 11 | 0115 | MRSSDILIVDDEIGIRDLLSEILQDEGYSVALAENAEEARKLRHQARPAMVLLDIWMPDCDGITLLKEWAKNGQLN MPVVMMSGHASIDTAVEATKIGAIDFLEKPISLQKLLSAVENALKYGAAQTETGPVFDKLGNSAAIQEMNREVGAA VKCASPVLLTGEAGSPFETVARYFHKNGTPWVSPARVEYLIDMPMELLQKAEGGVLYVGDIAQYSRNIQAGIAFIV GKAEHRRVRVVASGSRAAGSDGIACEEKLAELLSESVVRIPPLRMQHEDIPFLIQGIACNVAESQKIAPASFSEEA LAALTRYDWPGNFDQLQSVVATLLLEADGQEIGAGAVSSLLGQNVPAEGAEDMVGGFNFNLPLRELREEVERRYFE YHIAQEGQNMSQVAQKVGLERTHLYRKLKQLGIGVSRRAGEKTEE |

EP 1 562 982 B1

(continued)

| SED ID | NMB | Sequence |
|---|---|---|
| 12 | 0124 | MAKEKFERSKPHVNVGTIGHVDHGKTTLTAALTTILAKKFGGAAKAYDQIDNAPEEKARGITINTSHVEYETETRH YAHVDCPGHADYVKNMITGAAQMDGAILVCSAADGPMPQTREHILLARQVGVPYIIVFMNKCDMVDDAELLELVEM EIRDLLSSYDFPGDDCPIVQGSALKALEGDAAYEEKIFELAAALDSYIPTPERAVDKPFLLPIEDVFSISGRGTVV TGRVERGIIHVGDEIEIVGLKETQKTTCTGVEMFRKLLDEGQAGDNVGVLLRGTKREDVERGQVLAKPGTITPHTK FKAEVYVLSKEEGGRHTPFFANYRPQFYFRTTDVTGAVTLEEGVEMVMPGENVTITVELIAPIAMEEGLRFAIREG GRTVGAGVVSSVIA |
| 13 | 0126 | MSKKWYVVQAYSGFEKNVQRILEERIAREEMGDYFGQILVPVEKVVDIRNGRKTISERKSYPGYVLVEMEMTDDSW HLVKSTPRVSGFIGGRANRPTPISQREAEIILQQVQTGIEKPKPKVEFEVGQQVRVNEGPFADFNGVVEEVNYERN KLRVSVQIFGRETPVELEFSQVEKIN |
| 14 | 0128 | MAKVSKRLKALRSSVEANKLYAIDEAIALVKKAATAKFDESVDVSFNLGVDPRKSDQVIRGSVVLPKGTGKITRVA VFTQGANAEAAKEAGADIVGFEDLAAEIKAGNLNFDVVIASPDAMRIVGQLGTILGPRGLMPNPKVGTVTPNVAEA VKNAKAGQVQYRTDKAGIVHATIGRASFAEADLKENFDALLDAIVKAKPAAAKGQYLKKVAVSSTMGLGIRVDTSS VNN |
| 15 | 0130 | MSLNIETKKVAVEEISAAIANAQTLVVAEYRGISVSSMTELRANARKEGVYLRVLKNTLARRAVQGTSFAELADQM VGPLVYAASEDAVAAAKVLHQFAKKDDKIVVKAGSYNGEVMNAAQVAELASIPSREELLSKLLFVMQAPVSGFARG LAALAEKKAGEEAA |
| 16 | 0131 | MAITKEDILEAVGSLTVMELNDLVKAFEEKFGVSAAAVAVAGPAGAGAADAEEKTEFDVVLASAGDQKVGVIKVVR AITGLGLKEAKDIVDGAPKTIKEGVSKAEAEDIQKQLEEAGAKVEIK |

| SED ID | NMB | Sequence |
|---|---|---|
| 17 | 0132 | MCMNYSFTEKKRIRKSFAKRENVLEVPFLLATQIDSYAKFLQLENAFDKRTDDGLQAAFNSIFPIVSHNGYARLEF VHYTLGEPLFDIPECQLRGITYAAPLRARIRLVILDKEASKPTVKEVRENEVYMGEIPLMTPSGSFVINGTERVIV SQLHRSPGVFFEHDKGKTHSSGKLLFSARIIPYRGSWLDFEFDPKDLLYFRIDRRRKMPVTILLKALGYNNEQILD IFYDKETFYLSSNGVQTDLVADRLKGETAKVDILDKEGNVLVAKGKRITAKNIRDITNAGLTRLDVEPESLLGKAL AADLIDSETGEVLASANDEITEELLAKFDINGVKEITTLYINELDQGAYISNTLRTDETAGRQAARVAIYRMMRPG EPPTEEAVEQLFNRLFFSEDSYDLSRVGRMKFNTRTYEQKLSEAQQNSWYGRLLNETFAGAADKGGYVLSVEDIVA SIATLVELRNGHGEVDDIDHLGNRRVRSVGELTENQFRSGLARVERAVKERLNQAESENLMPHDLINAKPVSAAIK EFFGSSQLSQFMDQTNPLSEVTHKRRVSALGPGGLTRERAGFEVRDVHPTHYGRVCPIETPEGPNIGLINSLSVYA RTNDYGFLETPYRRVIDGKVTEEIDYLSAIEEGRYVIAQANADLDSDGNLIGDLVTCREKGETIMATPDRVQYMDV ATGQVVSVAASLIPFLEHDDANRALMGANMQRQAVPCLRPEKPMVGTGIERSVAVDSATAIVARRGGVVEYVDANR VVIRVHDDEATAGEVGVDIYNLVKFTRSNQSTNINQRPAVKAGDVLQRGDLVADGASTDFGELALGQNMTIAFMPW NGYNYEDSILISEKVAADDRYTSIHIEELNVVARDTKLGAEDITRDIPNLSERMQNRLDESGIVYIGAEVEAGDVL VGKVTPKGETQLTPEEKLLRAIFGEKASDVKDTSLRMPTGMSGTVIDVQVFTREGIQRDKRAQSIIDSELKRYRLD LNDQLRIFDNDAFDRIERMIVGQKANGGPMKLAKGSEITTEYLAGLPSRHDWFDIRLTDEDLAKQLELIKVSLQQK REEADELYEIKKKKLTQGDELQPGVQKMVKVFIAIKRRLQAGDKMAGRHGNKGVVSRILPVEDMPYMADGRPVDIV LNPLGVPSRMNIGQILEVHLGWAAKGIGERIDRMLKEQRKAGELREFLNRLYNGSGKKEDLDALTDEEIIELASNL RKGASFASPVFDGAKESEIREMLNLAYPSDDPEVEKLGFNDSKTQITLYDGRSGEAFDRKVTVGVMHYLKLHHLVD EKMHARSTGPYSLVTQQPLGGKAQFGGQRFGEMEVWALEAYGAAYTLQEMLTVKSDDVNGRTKMYENIVKGEHKID AGMPESFNVLVKEIRSLGLDIDLERY |

(continued)

| SED ID | NMB | Sequence |
|---|---|---|
| 18 | 0133 | MNLLNLFNPLQTAGMEEEFDAIKIGIASPETIRSWSYGEVKKPETINYRTFKPERDGLFCAKIFGPVKDYECLCGK<br>YKRLKFKGVTCEKCGVEVTLSKVRRERMGHIELAAPVAHIWFLKSLPSRLGMVLDMTLRDIERVLYFEAFVVTDPG<br>MTPLQRRQLLTEDDYYNKLDEYGDDFDAKMGAEGIRELLRTLNVAGEIEILRQELESTGSDTKIKKIAKRLKVLEA<br>FHRSGMKLEWMIMDVLPVLPPDLRPLVPLDGGRFATSDLNDLYRRVINRNNRLKRLLELHAPDIIVRNEKRMLQEA<br>VDSLLDNGRRGKAMTGANKRPLKSLADMIKGKGGRFRQNLLGKRVDYSGRSVITVGPYLRLHQCGLPKKMALELFK<br>PFIFHKLEKQGLASTVKAAKKLVEQEVPEVWDILEEVIREHPIMLNRAPTLHRLGIQAFEPILIEGKAIQLHPLVC<br>AAFNADFDGDQMAVHVPLSLEAQMEARTLMLASNNVLSPANGEPIIVPSQDIVLGLYYMTRDRINAKGEGSLFADV<br>KEVHRAYHTKQVELGTKITVRLREWVKNEAGEFEPVVNRYETTVGRALLSEILPKGLPFEYVNKALKKKEISKLIN<br>ASFRLCGLRDTVIFADHLMYTGFGFAAKGGISIAVDDMEIPKEKAALLAEANAEVKEIEDQYRQGLVTNGERYNKV<br>VDIWGRAGDKIAKAMMDNLSKQKVIDRAGNEVDQESFNSIYMMADSGARGSAAQIKQLSGMRGLMAKPDGSIIETP<br>ITSNFREGLTVLQYFIATHGARKGLADTALKTANSGYLTRRLVDVTQDLVVVEDDCGTSDGFVMKAVVQGGDVIEA |
| | | LRDRILGRVTASDVVDPSSGETLVEAGTLLTEKLVDMIDQSGVDEVKVRTPITCKTRHGLCAHCYGRDLARGKLVN<br>AGEAVGVIAAQSIGEPGTQLTMRTFHIGGAASRAAAASQVEAKSNGTARFSSQMRYVANNKGELVVIGRSCEVVIH<br>DDIGRERERHKVPYGAILLVQDGMAIKAGQTLATWDPHTRPMITEHAGMVKFENVEEGVTVAKQTDDVTGLSTLVV<br>IDGKRRSSSASKLLRPTVKLLDENGVEICIPGTSTPVSMAFPVGAVITVREGQEIGKGDVLARIPQASSKTRDITG<br>GLPRVAELFEARVPKDAGMLAEITGTVSFGKETKGKQRLIVTDVDGVAYETLISKEKQILVHDGQVVNRGETIVDG<br>AVDPHDILRLQGIEALARYIVQEVQEVYRLQGVKISDKHIEVIIRQMLRRVNIADAGETGFITGEQVERGDVMAAN<br>EKALEEGKEPARYENVLLGITKASLSTDSFISAASFQETTRVLTEAAIMGKQDELRGLKENVIVGRLIPAGTGLTY<br>HRSRHQQWQEVEQETAETQVTDE |
| 19 | 0137 | MPRRREVPKRDVLPDPKFGSVELTKFMNVLMIDGKKSVAERIVYGALEQIEKKTGKVAIEVFNEAIANAKPIVEVK<br>SRRVGGANYQVPVEVRPSRRLALAMRWVRDAARKRGEKSMDLRLAGELIDASEGRGGALKKREEVHRMAEANKAFS<br>HFRF |

42

EP 1 562 982 B1

| SED ID | NMB | Sequence |
|---|---|---|
| 20 | 0138 | MARKTPISLYRNIGISAHIDAGKTTTTERILFYTGLTHKLGEVHDGAATTDYMEQEQERGITITSAAVTSYWSGMA<br>KQFPEHRFNIIDTPGHVDFTVEVERSMRVLDGAVMVYCAVGGVQPQSETVWRQANKYQVPRLAFVNKMDRQGANFF<br>RVVEQMKTRLRANPVPIVIPVGAEDNFSGVVDLLKMKSIIWNEVDKGTTFTYGDIPAELVETAEEWRQNMIEAAAE<br>ASEELMDKYLGGDELTEEEIVGALRQRTLAGEIQPMLCGSAFKNKGVQRMLDAVVELLPAPTDIPPVQGVNPNTEE<br>ADSRQASDEEKFSALAFKMLNDKYVGQLTFIRVYSGVVKSGDTVLNSVKGTRERIGRLVQMTAADRTEIEEVRAGD<br>IAAAIGLKDVTTGETLCAESAPIILERMEFPEPVIHIAVEPKTKADQEKMGIALNRLAKEDPSFRVRTDEESGQTI<br>ISGMGELHLEIIVDRMKREFGVEANIGAPQVAYRETIRKAVKAEYKHAKQSGGKGQYGHVVIEMEPMEPGGEGYEF<br>IDEIKGGVIPREFIPSVDKGIRDTLPNGIVAGYPVVDVRIRLVFGSYHDVDSSQLAFELAASQAFKEGMRQASPAL<br>LEPIMAVEVETPEEYMGDVMGDLNRRRGVVLGMDDDGIGGKKVRAEVPLAEMFGYSTDLRSATQGRATYSMEFKKY<br>SEAPAHIAAAVTEARKG |
| 21 | 0140 | MANQKIRIRLKAYDYALIDRSAQEIVETAKRTGAVVKGPIPLPTKIERFNILRSPHVNKTSREQLEIRTHLRLMDI<br>VDWTDKTTDALMKLDLPAGVDVEIKVQ |
| 22 | 0142 | MTLGLVGRKVGMTRVFDEQGVSVPVTVLDMSANRVTQVKSKDTDGYTAVQVTFGQKKANRVNKAEAGHFAKAGVEA<br>GRGLIEFALTEEKLAELKAGDEITVSMFEVGQLVDVTGTSKGKGFSGTIKRHNFGAQRTSHGNSRSHRVPGSIGMA<br>QDPGRVFPGKRMAGQYGNTKATVQKLEVVRVDAERQLLLVKGAVPGAVNSDVVVRPSVKVGA |
| 23 | 0143 | MELKVIDAKGQVSGSLSVSDALFAREYNEALVHQLVNAYLANARSGNRAQKTRAEVKHSTKKPWRQKGTGRARSGM<br>TSSPLWRKGGRAFPNKPDENFTQKVNRKMYRAGMATILSQLTRDERLFAIEALTAETPKTKVFAEQVKNLGLEQVL<br>FVTKQLDENVYLASRNLPNVLVLEAQQVDPYSLLRYKKVIITKDAVAQLEEQWV |
| 24 | 0144 | MNQQRLTQVILAPIVSEKSNVLAEKRNQMTFKVLANATKPEIKAAVELLFGVQVADVTTVTIKGKVKRFGRTLGRR<br>SDVKKAYVSLAAGQELDLEAAAAAADKE |

EP 1 562 982 B1

(continued)

| SED ID | NMB | Sequence |
|---|---|---|
| 25 | 0154 | MARLREFYKETVVPELVKQFGYKSVMEVPRIEKITLNMGVGEAVADKKVMEHAVSDLEKIAGQKPVVTVARKSIAG FKIRDNYPVGCKVTLRRDQMFEFLDRLITIALPRVRDFRGVSGKSFDGRGNYNMGVREQIIFPEIEYDKIDALRGL NITITTTAKTDEEAKALLSLFKFPFKG |
| 26 | 0156 | MSMHDPISDMLTRIRNAQRANKAAVAMPSSKLKCAIAKVLKEEGYIEDFAVSSDVKSILEIQLKYYAGRPVIEQIK RVSRPGLRIYKASSEIPSVMNGLGIAIVSTSKGVMTDRKARSQGVGGELLCIVA |
| 27 | 0157 | MSRVAKNPVTVPAGVEVKFGAEALVIKGKNGELSFPLHSDVAIEFNDGKLTFVANNSSKQANAMSGTARALVSNMV KGVSEGFEKRLQLIGVGYRAQAQGKILNLSLGFSHPIVYEMPEGVSVQTPSQTEIVLTGSDKQVVGQVAAEIRAFR APEPYKGKGVRYVGEVVVMKEAKKK |
| 28 | 0159 | MAKHEIEERGDGLIEKMVAVNRVTKVVKGGRIMAFSALTVVGDGDGRIGMGKGKSKEVPVAVQKAMDQARRSMIKV PLKNGTIHHEVIGRHGATKVFMQPAKEGSGVKAGGPMRLVFDAMGIHNISAKVHGSTNPYNIVRATLDGLSKLHTP ADIAAKRGLTVEDILGVNHG |
| 29 | 0168 | MQNSTTEFLKPRQIDVNTFSATRAKVSMQPFERGFGHTLGNALRRILLSSMNGFAPTEVAIAGVLHEYSTVDGIQE DVVDILLNIKGIVFKLHGRSQVQLVLKKSGSGVVSAGDIELPHDVEILNPGHVICHLADNGQIEMEIKVEQGRGYQ SVSGRQVVRDENRQIGAIQLDASFSPISRVSFEVEPARVEQRTDLDKLVLDIETDGSIDPEEAVRSAARILIDQMS IFADLQGTPVEEVEEKAPPIDPVLLRPVDDLELTVRSANCLKAEDIYYIGDLIQRTETELLKTPNLGRKSLNEIKE VLASKGLTLGSKLEAWPPVGLEKP |
| 30 | 0171 | MAKIIVVTSGKGGVGKTTTSASIATGLALRGYKTAVIDFDVGLRNLDLIMGCERRVVYDLINVIQGEATLNQALIK DKNCENLFILPASQTRDKDALTREGVEKVMQELSGKKMGFEYIICDSPAGIEQGALMALYFADEAIVTTNPEVSSV RDSDRILGILQSKSHKAEQGGSVKEHLLITRYSPERVAKGEMLSVQDICDILHIPLLGVIPESQNVLQASNSGEPV IHQDSVAASEAYKDVIARLLGENREMRFLEAEKKSFFKRLFGG |

(continued)

| SED ID | NMB | Sequence |
|---|---|---|
| 31 | 0173 | MTLTELRYIVAVAQERHFGRAARRCFVSQPTLSIAIKKLEEELAVSLFDRSSNDIITTEAGERIVAQARKVLEEAE LIRHLANEEQNELEGAFKLGLIFTVAPYLLPKLIVSLRRTAPKMPLMLEENYTHTLTESLKRGDVDAIIVAEPFQE PGIVTEPLYDEPFFVIVPKGHSFEELDAVSPRMLGEEQVLLTEGNCMRDQVLSSCSELAAKQRIQGLTNTLQGSS INTIRHMVASGLAISVLPATALTENDHMLFSIIPFEGTPPSRRVVLAYRRNFVRPKALSAMKAAIMQSQLHGVSFI CD |
| 32 | 0193 | MTHMIYPKTYDVIVVGGGHAGTEAALAAARMGAQTLLLSHNIETLGQMSCNPSIGGIGKGHLVRELDALGGAMALA |
| | | TDKSGIQFRRLNASKGAAVRATRAQADRILYKAAIREMLENQENLDLFQQAVEDVTLDGERISGVITAMGVEFKAR AVVLTAGTFLSGKIHIGLENYEGGRAGDPAAKSLGGRLRELKLPQGRLKTGTPPRIDGRTIDFSQLTEQPGDTPVP VMSVRGNADMHPRQVSCWITHTNTQTHDIIRSGFDRSPMFTGKIEGVGPRYCPSIEDKINRFADKDSHQIFLEPEG LTTHEYYPNGISTSLPFDIQIALVRSMKGLENAHLRPGYAIEYDYFDPRNLKASLETKTIAGLFFAGQINGTTGY EEAAAQGLLAGANAVQYVRGQDPLLLRREQAYLGVLVDDLITKGVNEPYRMFTSRAEYRLQLREDNADMRLTEDGY KIGLVSEAQWRMFNEKREAVEREIQRLKTTWYTPQKLAEGEQIRVFGQKLSREANLHDLLRRPNLDYAALMTLEGA MPSENLSAEVIEQVEIQVKYQGYIDRQNEEIDSRRDIETLKLPDGIDYGKVKGLSAEVQQKLNQHKPETVGQASRI SGVTPAAVALLMVHLKRGFKDAK |

(continued)

| SED ID | NMB | Sequence |
|---|---|---|
| 33 | 0196 | MKRMLFNATQAEELRVAIVDGQNLLDLDIETLGKEQRKGNIYKGIITRIEPSLEACFVDYGTDRHGFLPFKEVSRS YFQDYEGGRARIQDVLKEGMEVIVQVEKDERGNKGAALTTFISLAGRYLVLMPNNPRGGGVSRRIEGEERQELKAA MAELDIPNGMSIIARTAGIGRSAEELEWDLNYLKQLWQAIEEAGKAHHDPYLLFMESSLLIRAIRDYFRPDIGEIL VDNQEVYDQVAEFMSYVMPGNIGRLKLYEDHTPLFSRFQIEHQIESAFSRSVSLPSGGAIVIDHTEALVSIDVNSA RATRGADIEDTAFKTNMEAAEEVARQMRLRDLGGLVVIDFIDMENPKHQRDVENVLRDALKKDRARVQMGKLSRFG LLELSRQRLKPALGESSHVACPRCAGTGVIRGIESTALHVLRIIQEEAMKDNTGEVRAQVPVDVATFLLNEKRAEL FAMEERLDVNVVLIPNIHLENPHYEINRIRTDDVEEDGEPSYKRVAEPEEDESAKPFGGEKAKAARPEPAVKGVRH TSPAPTAAPEKKTSWWDSFKAWLKRIFGGSETQAAPAAETSEKRSTANRSGSRANNRRQNPRRSKREGSKVEVREV AGKTAGQEARADKAETRNNGNRRRNERGDRAAERANEAEIQSRNVQPAATVADAAPSETEVQTGKRRRNGSRSERG QTAPETATVAETTVQTAENTPSEPHTAEDKGSKPKSERNRRERDSRDAKERRERNNQRDRRQNGKKRNIPSAAKIE QYLNIHDTADKVRSAAAHVFGETDANAPITVSIADPVAERDLPTASPAVSNGDAPVYDAAEKIRRATAAILPEGAT PKAEAQEMPSETATFTAAAEQARETAQTGGLVLIETDPAALKAWAAQPEVQAGRGLRRSEQPKPSEVATVPAEEMI QVETRQG |
| 34 | 0212 | MTEQKHEEYGADSIQVLEGLEAVRKRPGMYIGDTQDGSGLHHMVFEVLDNAIDEALAGHCDKITVTIHADHSVSVA DNGRGMPTGIHPKEGRSAAEVIMTVLHAGGKFDNNSYKISGGLHGVGVSVVNALSDWVTLTIYRDGKEHFVRFVRG ETEEPLKIVGDSDKKGTTVRFLASTETFGNVEYSFDILAKRIRELSFLNNGVDIELTDERDGKHESFALSGGVAGF VQYMNRKKTPLHEKIFYAFGEKDGMSVECAMQWNDSYQESVQCFTNNIPQRDGGTHLTALRQVMTRTINNYIEANE VAKKAKVETAGDDMREGLTCVLSVKLPDPKFSSQTKDKLVSGEIGPVVNEVISQALTDFLEENPNEAKIITGKIVD AARAREAARKAREITRRKGVMDGLGLPGKLADCQEKDPALSELYLVEGDSAGGSAMQGRDRKFQAILPLKGKILNV EKARFEKMLASQEVATLITALGAGIGKEEFNAEKLRYHRIIIMTDADVDGAHIRTLLLTFFYRQMPELVERGYIYI AQPPLYKAKYGKQERYLKDELEKDQWLLGLALEKAKIISDGRTIEGAELADTAKQFLLAKTVIEQESRFVDELVLR AMLHASPIDLTSSENADKAVAELSGLLDEKEVALERIEGHEGHRFIKITRKLHGNVMVSYIEPKFLNSKAYQTLTQ TAAALKGMVGEGAKLYKGENGYDADSFETALDILMSVAQKGMSIQRYKGLGEMNPEQLWETTMDPAVRRLLKVRIE DAIAADEVFVTLMGDEVEPRRAFIENNALIAQNIDA |

EP 1 562 982 B1

46

(continued)

| SED ID | NMB | Sequence |
|---|---|---|
| 35 | 0219 | MSQRRVVITGLGQVSPVGNTVAEAWDTLLTGKSGIGAITRDTSDINSRVAGEVRGFDIGQYISAKEARRMDVFIHYGIAAALQAIADSGLDDVENLDKDRIGVNIGSGIGGLPGIEVTGKAVIEGGARKINPFFIPGSLINLISGHVTILKGYRGPSYGMVSACTTGAHAIGDSLRMIKYGDADIMVAGGAEGAISTLGVGGFAAMKALSTRNDDPATASRPWDKGRDGFVIGEGAGIIVLEELEHAKKRGAKIYAEIVGFGMSSDAYHITAPNEEGPALAVTRALKDAGINPEDVDYVNAHGTSTPLGDANETKALKRAFGEHAYKTVVSSTKSMTGHLLGAAGGVEAVYSILAIHDGKIPPTINIFEQDVEAGCDLDYCANEARDAEIDVAISNSFGFGGTNGTLVFKRFKG |
| 36 | 0246 | MAIYQSGVIFDQVDTANPDCWTLDEYVKRGGYTALRKILSENISQTDVIDEVKTSGLRGRGGAGFPTGLKWSFMPRSFPGEKYVVCNTDEGEPGTFKDRDIIMFNPHALIEGMIIAGYAMGAKAGYNYIHGEIFEGYQRFEAALEQARAAGFLGKNILGSDFEFELFAHHGYGAYICGEETALLESLEGKKGQPRFKPPFPASFGLYGKPTTNNTETFSSVPFIIRDGGQAFADKGIPNAGGTKLFCISGHVERPGNYEVPLGTPFAEVLKMAGGMRGGKKLKAVIPGGSSAPVLPADIMMQTNMDYDSISKAGSMLGSGAIIVMDEDVCMVKALERLSYFYYDESCGQCTPCREGTGWLYRIVHRIVEGKGMEDLDLLDSVGNQMAGRTICALADAAVFPVRSFTKHFRDEFVHYIEHGGPMKEHKWGGW |
| 37 | 0286 | MNFTRLLNQVLSTVQKKGNTFSDSPLNSFGGGALVAGVASMLLNGKNRKTITKIGSTAALGYLAYRGYQMWQQNKGRATVTQSDFQPAGETEETYSRTVLRTMIAAAASDGMIDEAERRTIEQESGTDPETAAWLAAEYRLPASIEDIAAAVGNDEALAAEAYLAARLVCADLSRKETVFLARLSQALKLDDNLVESLERQLGF |
| 38 | 0294 | MKLKTLALTSLTLLALAACSKQAETSVPADSAQSSSSAPAPAELNEGVNYTVLSTPIPQQQAGKIEVLEFFGYFCPHCAHLEPVLSEHIKTFKDDTYMRREHVWGDEMKPLARLAAAVEMAGESDKANSHIFDAMVNQKINLADTDTLKKWLSEQTAFDGKKVLAAFEAPESQARAAQMEELTNKFQISGTPTVIVGGKYQVEFKDWQSGMTTIDQLVDKVREEQKKPQ |

(continued)

| SED ID | NMB | Sequence |
|---|---|---|
| 39 | 0329 | MSVGLLRILVQNQVVTVEQAEHYYNESQAGKEVLPMLFSDGVISPKSLAALIARVFSYSILDLRHYPRHRVLMGVL TEEQMVEFHCVPVFRRGDKVFFAVSDPTQMPQIQKTVSAAGIEVELVIVEDDQLAGLLDWVGSRSTSLLQELGEGQ EEEESHTLYIDNEEAEDGPVPRFIHKTLSDALRSGASDIHFEFYEHNARIRFRVDGQLREVVQPPIAVRGQLASRI KVMSRLDISEKRIPQDGRMQLTFQKGGKPVDFRVSTLPTLFGEKVVMRILNSDAASLNIDQLGFEPFQKKLLLEAI HRPYGMVLVTGPTGSGKTVSLYTCLNILNTESVNIATAEDPAEINLPGINQVNVNDKQGLTFAAALKSFLRQDPDI IMVGEIRDLETADIAIKAAQTGHMVFSTLHTNNAPATLSRMLNMGVAPFNIASSVSLIMAQRLLRRLCSSCKQEVE RPSASALKEVGFTDEDLAKDWKLYRAVGCDRCRGQGYKGRAGVYEVMPISEEMQRVIMNNGTEVDILDVAYKEGMV DLRRAGILKVMQGITSLEEVTANTND |
| 40 | 0335 | MSLQNIIETAFENRADITPTTVTPEVKEAVLETIRQLDSGKLRVAERLGVGEWKVNEWAKKAVLLSFRIQDNEVLN DGVNKYFDKVPTKFADWSEDEFKNAGFRAVPGAVARRGSFVAKNVVLMPSYVNIGAYVDEGAMVDTWATVGSCAQI GKNVHLSGGVGIGGVLEPLQAAPTIIEDNCFIGARSEIVEGVIVEEGSVISMGVFIGQSTKIFDRTTGEIYQGRVP AGSVVVSGSMPSKDGSHSLYCAVIVKRVDAQTRAKTSVNELLRGI |
| 41 | 0336 | MGFLQGKKILITGMISERSIAYGIAKACREQGAELAFTYVVDKLEERVRKMAAELDSELVFRCDVASDDEINQVFA DLGKHWDGLDGLVHSIGFAPKEALSGDFLDSISREAFNTAHEISAYSLPALAKAARPMMRGRNSAIVALSYLGAVR AIPNYNVMGMAKASLEAGIRFTAACLGKEGIRCNGISAGPIKTLAASGIADFGKLLGHVAAHNPLRRNVTIEEVGN TAAFLLSDLSSGITGEITYVDGGYSINALSTEG |
| 42 | 0337 | MSRPVPAVFGSVFHSQMPVLAYREGKWQPTEWQSSQDLSLAPGAHALHYGSECFEGLKAFRQADGKIVLFRPTANI ARMRQSADILHLPRPETEAYLDALIKLVKRAADEIPDAPAALYLRPTLIGTDPVIGKAGSPSETALLYILASPVGD YFKVGSPVKILVETEHIRCAPHMGRVKCGGNYASAMHWVLKAKAEYGANQVLFCPNGDVQETGASNFILINGDEII TKPLTDEFLHGVTRDSVLTVAKDLGYTVSERNFTVDELKAAVENGAEAILTGTAAVISPVTSFVIGGKEIEVKSQE RGYAIRKAITDIQYGLAEDKYGWLVEVC |

EP 1 562 982 B1

48

| SED ID | NMB | Sequence |
|---|---|---|
| 43 | 0351 | MTILSDVKALGQQIWLDNLSRSLVQSGELAQMLKQGVCGVTSNPAIFQKAFAGDALYADEIAALKQQNLSPKQRYE TMAVADVRAACDVCLAEHESTGGKTGFVSLEVSPELSKDAQGTVEEARRLYAAIGCKNAMIKVPATDAGIDALETL VSDGISVNLTLLFSRAQTLKAYAAYARGIAKRLAAGQSVAHIQVVASFFISRVDGALDTTLPDHLKGKIAIALAKA AYQDWAQYFGSPEFAALETKGANRVQLLWASTGVKNPAYPDTLYVDSLIGAHTVNTVPDATLKAFIDHGTAKATLT EGVEEAQAQLAETAALGIDVETLATRLQEDGLKQFEEAFEKLLAPLA |
| 44 | 0359 | MSIKNAVKLIEESEARFVDLRFTDTKGKQHHFTVPARIVLEDPEEWFENGQAFDGSSIGGWKGIQASDMQLRPDAS TAFVDPFYDDATVVLTCDVIDPADGQGYDRDPRSIARRAEAYLKSSGIGETAYFGPEPEFFVFDGIEFETDMHKTR YEITSESGAWASGLHMDGQNTGHRPTVKGGYAPVAPIDCGQDLRSAMVNILEELGIEVEVHHSEVGTGSQMEIGTR FATLVKRADQTQDMKYVIQNVAHNFGKTATFMPKPIMGDNGSGMHVHQSIWKDGQNLFAGDGYAGLSDTALYYIGG IIKHAKALNAITNPSTNSYKRLVPHFEAPTKLAYSAKNRSASIRIPSVNSSKARRIEARFPDPTANPYLAFAALLM AGLDGIQNKIHPGDPADKNLYDLPPEEDALVPTVCASLEEALAALKADHEFLLRGGVFSKDWIDSYIAFKEEDVRR IRMAPHPLEFEMYYSL |
| 45 | 0382 | MTKQLKLSALFVALLASGTAVAGEASVQGYTVSGQSNEIVRNNYGECWKNAYFDKASQGRVECGDAVAAPEPEPEP EPAPAPVVVVEQAPQYVDETISLSAKTLFGFDKDSLRAEAQDNLKVLAQRLSRTNVQSVRVEGHTDFMGSDKYNQA LSERRAYVVANNLVSNGVPVSRISAVGLGESQAQMTQVCEAEVAKLGAKVSKAKKREALIACIEPDRRVDVKIRSI VTRQVVPAHNHHQH |
| 46 | 0390 | MYTRIMEISPWTLRSAKLEKEHKRLQESLTSLGNGYMGMRGSFEETYSADSHLGTYIAGVWFPDKTRVGWWKNGYP KYFGKAINAFNFSKVKIFVDGQEVDLAKNDVAGFSVELDMQHGVLRRSFTVFGVRFNVCKFLSVAQKELAVIRWEA VSVDGKTHQVRIDSIIDADVKNEDSNYEEKFWQVLDKGVSDSLSYIAAQTVANPFGVEQFIVNAEQTFAGSFKALG GSQTDWQVSNSFESEVGSTPETFEKRVIVTTSRDYQSLEAVKAAGRALSEKIAGVAFETLLDAHKAGWLHRWEIAD VVIEGSDEAQQGIRFNLFQLFSTYYGEDARLNIGPKGFTGEKYGGATYWDTEAYAVPLYLALAEPEVTRNLLQYRR NQLPQAQHNAREQGLAGALYPMVTFTGIECHNEWEITFEEIHRNGAIPYAIYNYTNYTGDEGYLAKEGLEVLVEVS RFWADRVHFSKRNGKYMIHGVTGPNEYENNINNNWYTNTLAAWVLDYTREALAKYPRPDLNVRADELEKWADISAN MYRPHDEELGVFVQHDGFLDKDIRPVSALSPDDLPLNQKWSWDKILRSPFIKQADVLQGIYFFSDRFNIDEKRRNF DFYEPMTVHESSLSPCIHSILAAELGKEEKAVEMYQRTARLDLDNYNNDTEDGLHITSMTGSWLAIVQGFAQMKTW GGKLSFAPFLPSAWTGYAFHINYRGRLIKVAVGKENVVFTLLKGESLDLQVYGKDITLDGSHTVALEK |

EP 1 562 982 B1

49

| SED ID | NMB | Sequence |
|---|---|---|
| 47 | 0391 | MTFTAVLFDLDGVITDTAEYHYRAWKKLAEELGISIDRKFNEQLKGVSRDDSLKRILAHGGKTVSEAEFAELTRRK NDNYVEMIQAVKPEDVYPGILPLLEALRANGKKIALASASKNGPFLLERMGLTHFFDAIADPAAVAHSKPAPDIFL AAAEGVDADIRQCIGIEDAAAGVAAIKAAGALPIGVGKAEDLGSDIALVSGTAELTYAYLQSVWEQSGR |
| 48 | 0426 | MEQQQRYISVLDIGTSKVLALIGEVQDDDKINIVGLGQAPSRGLRAGMVTNIDATVQAIRQAVNDAELMADTKITH VTTGIAGNHIRSLNSQGVVKIKDGEVTQADIDRAIETAKAINIPPDQKILDAVVQDYIIDTQLGVREPIGMSGVRL DTRVHIITGASTAVQNVQKCIERCGLKSDQIMLQPLASGQAVLTEDEKDLGVCVIDIGGGTTDIAVYMNGAIRHTS VIPAGGNLITKDLSKSLRTPLDAAEYIKIHYGVASCDTEGLGEMIEVPGVGDRTSRQVSSKVLAAIISARIQEIFG VVLGELQKSGFPKEVLNAGIVLTGGVSMMTGIVEFAEKIFDLPVRTGAPQEMGGLSDRVRTPRFSTAIGLLHAACK LEGNLPQPENGAVQEREGGGGLLARLKRWIENSF |
| 49 | 0427 | MEFVYDVAESAVSPAVIKVIGLGGGGCNAINNMVANNVRGVEFISANTDAQSLAKNHAAKRIQLGTNLTRGLGAGA NPDIGRAAAQEDREAIEEAIRGANMLFITTGMGGGTGTGSAPVVAEIAKSLGILTVAVVTRPFAYEGKRVHVAQAG LEQLKEHVDSLIIIPNDKLMTALGEDVTMREAFRAADNVLRDAVAGISEVVTCPSEIINLDFADVKTVMSNRGIAM MGSGYAQGIDRARMATDQAISSPLLDDVTLDGARGVLVNITTAPGCLKMSELSEVMKIVNQSAHPDLECKFGAAED ETMSEDAIRITIIATGLKEKGAVDFVPAREVEAVAPSKQEQSHNVEGMIRTNRGIRTMNLTAADFDNQSVLDDFEI PAILRRQHNSDK |
| 50 | 0446 | MSQTIDELLLPHRNAIDTIDAEILRLLNERAQHAHAIGELKGTGAVYRPEREVAVLRRIQDLNKGPLPDESVARLF REVMSECLAVERPLTIAYLGPQGTFTQQAAIKHFGHAAHTMACPTIDDCFKQVETRQADYLVAPVENSTEGSVGRT LDLLAVTALQACGEIVLRIHHNLLRKNNGSTEGIAKVFSHAQALAQCNDWLGRHLPNAERIAVSSNAEAARLVAES DDGTVAAIAGRTAAEIYGLDMVAECIEDEPNNTTRFLVMGHHETGASGSDKTSLAVSAPNRAGAVASLLQPLTESG |
|  |  | ISMTKFESRPSKSVLWEYLFFIDIEGHRRDAQIQTALERLGERASFVKVIGSYPTAVL |

(continued)

| SED ID | NMB | Sequence |
|---|---|---|
| 51 | 0462 | MQAFSLYPPVGHPDSAKKRQNRADVFLPFWKQDNFLGKSVQYRFEFAQIYFTMPKPCAGVTMGRGDFFFSNLFHQE SARMKKSVLAVLAALSLAACGGSEKNAVQPQADAASAANAEAAATDTLNIYNWSNYVDESTVEDFKKANNLKLTYD LYENNETLEAKMLTGKSGYDLVVPGIAFLPRQIEAGAYQKVNKDLIPNYKNIDPELLKMLETADPGNQYAVPYFSG VNTIAITAKGKELLGGKLPENGWDLLFKPEYTHKLKSCGIALWDTPSEMFPILLNYLGKDPKGSNPEDLKAAAEVL KSIRPDVKRFSPSIIDELARGDICLAAGNGGDLNLAKARSEEVKNNVGIEVLTPKGMGFWIESWLIPADAKNVANA HKYINYTLDPEIAAKNGIAVTFAPASKPAREKMPAELVNTRSIFPNEQDMKDGFVMPQMSTDAKKLSVSLWQKIKV GTN |
| 52 | 0466 | MRTNYCGLISEQYLDQTVTVKGWVHRRRDHGGVIFIDLRDREGIVQVVIDPDTPEAFAAADSSRNEYVLSITGRVR NRPEGTTNDKMISGKIEILAKEIEVLNAAATPPFQIDDENISENVRLTNRVIDLRRPVMQRNLRLRYQVAMGVRRY LDAQGFIDIETPMLTRSTPEGARDYLVPSRVHPGEFFALPQSPQLFKQLLMVAGFDRYYQITKCFRDEDLRADRQP EFTQIDLETSFLNEDEIMDITEGMAKQVFKDALNVDLGDFPRMPYSEAMFYYGSDKPDMRINLKFTELTDLMKTEE FKVFRGAADMKGGRVVALRVPNGAEFSRKEIDEYTKFVGIYGAKGLAYIKVNDVSNLSNGEDSGLQSPIVKYLSEN ALKEIIARTGAQNGDIIFFGADKAKVVNEAIGALRIKVGLEHGKDNGYFTDEWKPLWVVDFPMFEYDEEADRYVAV HHPFTAPKEGHEDLMVSDPANCLARAYDMVLNGWEIGGGSIRIHRADVQEKVFAALKISPEEQQEKFGFLLDNLKF GAPPHGGLAFGLDRLVTLMTGAESIRDVIAFPKTQRAQCLLTNAPNSVDDKQLRELSLRLRQKATETKEV |
| 53 | 0477 | MRENKIIFTGPVGVGKTTAIAAISDEALVQTDASASDMTLDRKRNTTVAMDYGAISLDEDTKVHLYGTPGQERFNF MWEILSQGSMGLVLLLDNARTNPLKDLEFFLHSFRGLLEKAPVVVGITKMDIRSQPGIDVYHKYLAKHNLNVPVFE IDARKEDDVKQLVSAMLFSIDPGLEV |
| 54 | 0530 | MTVPHIPRGPVMADIAAFRLTEEEKQRLLDPAVGGIILFRRNFQNIEQLKTLTAEIKALRTPELIIAVDHEGGRVQ RFIEGFTRLPAMSTLGEIWDKDGASAAETAAGQVGRVLATELSACGIDLSFTPVLDLDWGNCPVIGNRSFHRNPEA VARLALALQKGLTKGGMKSCGKHFPGHGFVEGDSHLVLPEDWRSLSELETADLAPFRIMSREGMAAVMPAHVVYPQ VDTKPAGFSEIWLKQILRRDIGFKGVIFSDDLTMEGACGAGGIKERARISFEAGCDIVLVCNRPDLVDELREDFRI PDNPTLAQRWQYMANTLGSAAAQAVMQTADFQAAQAFVAGLASPQDTAGGVKVGEAF |

| SED ID | NMB | Sequence |
|---|---|---|
| 55 | 0540 | MFFKHIEAAPADPILGLGEAFKAETRPEKVNLGIGVYKDASGATPLVKAVKEAEKRLLESETTKNYLTIDGVADYN AQTQILLFGKDHEIIASRRAKTAQSLGGTGALRIAAEFAKRQLNAQTIWISNPTWPNHNAIAKAVGIQDKPYRYYD AAKHGLDWDGMIEDLSQAQKGDIVLLHGCCHNPTGIDPTPEQWETLAKLSAEKGWLPLFDFAYQGFGNGLEEDAYG LRVFLKHNTELLIASSYSKNFGMYNERVGAFTLVAEDEETAARAHSQVKTIIRTLYSNPASHGANTIALVLKNDDL KAQWIAELDEMRGRIKAMRQKFVGLLKAKGASQNFDFIIKQNGMFSFSGLTPEQVDRLKNEFAIYAVRSGRINVAG ITDNNIDYLCESIVKVL |
| 56 | 0546 | MKMQAVVVNKNVAGDVEVIEREVRPLEYGEALVEVEYCGVCHTDLHVAAGDYGEKPGRVLGHEGIGLVKEVADGVK NLKVGDRVSIAWLFQSCGSCEYCNTGRETLCRSVLNAGYTADGGMATHCIVSADYAVKVPEGLDPAQASSITCAGV TTYKAIKVSGVRPGQWIAIYGAGGLGNLGVQYAKKVFGAHVVAIDINDDKLAFAKETGADLVVNAAKEDAAKVIQE KTGGAHAAVVTAVSAAAFNSAVNCVRAGGRVVAIGLPPESMDLSIPRLVLDGIEVVGSLVGTRKDLEEAFQFGAEG LVVPKVQLRALDEAPAIFQEMREGKITGRMVIDMKKECGCGHHH |
| 57 | 0564 | MEIILGIVMFTVIVLVLALMILFAKSKLVSEGDITIKVNGEKELTMPAGGKLLGALANEGIFIPSACGGGGSCGQC RVVVKSGGGDILPTELSHISKREAREGCRLSCQVNVKTDMDIEVPEEVFGVKKWECTVISNDNKATFIKELKLAIP EGEEVPFRAGGYIQIEAPPHTVAYKDFDIPKEYHEDWDKYNLWQYVSKVDEPILRAYSMASYPEEKGIIMLNVRIA TPPPRVPDAPPGQMSSYIWSLKPGDKVTISGPFGEFFAKDTDAEMVFIGGGAGMAPMRSHIFDQLKRLNSKRKITF WYGARSKREMFYVEDFDQLAAEFPNFTWHVALSDPLPEDNWDGYTGFIHNVVYENHLKNHEAPEDCEFYMCGPPIM NQSVIKMLKDLGVEDENILLDDFGG |
| 58 | 0589 | MNLIQQLEQEEIARLNKEIPEFAPGDTVVVSVRVVEGTRSRLQAYEGVVIARRNRGLNSNFIVRKISSGEGVERTF QLYSPTVEKIEVKRRGDVRRAKLYYLRGLTGKAARIKEKLPARKG |
| 59 | 0595 | MSRVLLVDDDALLTELLTEYLSAEGLNVRSVPDGEAGVQEILSGQYDVVVLDSMMPKMNGLDVLKNVRARSTVPII MLTAKGDDIDRIIGLEMGADDYVPKPCTPRELLARINAILRRAQHSGEQNNAPNSISVSDVVLYPAKRQASVKDMP LELTSTEFNLLEVLMRHAGQVVSKETLSVEALDRKLAKFDRSIDVHISSIRHKLGDASLIQTVRGLGYLFVKN |

EP 1 562 982 B1

| SED ID | NMB | Sequence |
|---|---|---|
| 60 | 0604 | MKAARFYDKGDIRIEDIPEPTVAPGTVGINVAWCGICGTDLHEFMEGPIFIPPCGHPHPISGESAPVTMGHEFSGV VYAVGEGVDDIKVGQHVVVEPYIIRDDVPTGEGSNYHLSKDMNFIGLGGCGGGLSEKIAVKRRWVHPISDKIPLDQ AALIEPLSVGHHAYVRSGAKEGDVALVGGAGPIGLLLAAVLKAKGIKVIITELSKARKDKARESGVADYILDPSEV DVVAEVKKLTNGEGVDVAFECTSVNKVLDTLVEACKPAANLVIVSIWSHPATINVHSVVMKELDVRGTIAYCNDHA ETIKLVEEGKINLEPFITQRIKLDELVSKGFERLIHNNESAVKIIVSPNL |
| 61 | 0610 | MLPVRFTRVSDGIKRLTPASNQTAFYHPFENPFCRYSSFYWSIAIMTATTASSAKPYLKIQGLVKKFGDNYAVDNI DLDIYQHEIFALLGSSGSGKSTLLRMLAGMESPNQGKIILDGQDITKLAPYDRPINMMFQSYALFPHMTVEQNIAF GLKQDKMPKGEIAARVEEMLRLVQMTKFAKRKPHQLSGGQQQRIALARSLAKRPKILLLDEPLGALDKKLRQQTQL ELVNTLEQVGVTCIMVTHDQEEAMTMATRIAIMSDGQLQQVGTPSDVYDYPNSRFTAEFIGETNIFDGVVIEDHAD YAVIECEGLENHVRIDHGLGGPSEQDLWVSIRPEDIDLYKEKPEYLGDYNWAKGTVKEIAYLGSFAIYHIKLGNGR VVKSQVPAPYWYVRNITPPTWDETVYISWPENQPTPLFR |
| 62 | 0617 | MHVSELQTLHISKLLELAEEHGIENANRFRKQDLVFAIVRQMMKKGEGFTCSGTLEILPDGFGFLRSADTSYLAGP DDIYVSPTQIRRFNLHTGDTIEGSVRVPKDNERYFALVRLDTINGDHPEVCRHKILFENLTPLFPTEQLKLERDLK SEENLTGRAIDLISPIGKGQRALLVAPPKSGKTVMLQNIAHAVTANYPEVELIVLLIDERPEEVTEMSRSVRGEVV SSTFDEPAQRHVQVAEMVLEKAKRMVEHKKDVVILLDSITRLARAYNTVVPTSGKILTGGVDANALHRPKRFFGAA RNVEEGGSLTIIATALVETGSRMDDVIYEEFKGTGNMELHLDRRMAEKRLFPAININKSGTRREELLVPNDQLQRM WLLRKFLHPMDEIEAAEFLIGKIKASKNNDDFFELMRGK |

EP 1 562 982 B1

(continued)

| SED ID | NMB | Sequence |
|---|---|---|
| 63 | 0618 | MADNYVIWFENLRMTDVERVGGKNASLGEMISQLTEKGVRVPGGFATTAEAYRAFLAHNGLSERISAALAKLDVEDVAELARVGKEIRQWILDTPFPEQLDAEIEAAWNKMVADAGGADISVAVRSSATAEDLPDASFAGQQETFLNINGLDNVKEAMHHVFASLYNDRAISYRVHKGFEHDIVALSAGVQRMVRSDSGASGVMFTLDTESGYDQVVFVTSSYGLGENVVQGAVNPDEFYVFKPTLKAGKPAILRKTMGSKHIKMIFTDKAEAGKSVTNVDVPEEDRNRFSITDEEITELAHYALTIEKHYGRPMDIEWGRDGLDGKLYILQARPETVKSQEEGNRNLRRFAINGDKTVLCEGRAIGQKVGQGKVRLIKDASEMDSVEAGDVLVTDMTDPDWEPVMKRASAIVTNRGGRTCHAAIIARELGIPAVVGCGNATELLKNGQEVTVSCAEGDTGFIYAGLLDVQITDVALDNMPKAPVKVMNVGNPELAFSFANLPSEGIGLARMEFIINRQIGIHPKALLEFDKQDDELKAEITRRIAGYASPVDFYVDKIAEGVATLAASVYPRKTIVRMSDFKSNEYANLVGGNVYEPHEENPMLGFRGAARYVADNFKDCFALECKALKRVRDEMGLTNVEIMIPFVRTLGEAEAVVKALKENGLERGKNGLRLIMMCELPSNAVLAEQFLQYFDGFSIGSNDMTQLTLGLDRDSGLVSESFDERNPAVKVMLHLAISACRKQNKYVGICGQGPSDHPDFAKWLVEEGIESVSLNPDTVIETWLYLANELNK |
| 64 | 0623 | MKKTLVAAAILSLALTACGGGSDTAAQTPSAKPEAEQSGKLNIYNWSDYVDPETVAAFEKETGIKTRSDYYDSNETLEAKVLTGKSGYDLTAPSIANVGRQIKAGAYQKIDKAQIPHYGNIDKDLLKMMEAVDPGNEYAVPYFWGINTLAINTQQVKKALGTDKLPENEWDLVFKPEYTAKLKSCGISYFDSAIEQIPLALHYLGKDPNSENPEDIKAAVDMKAVRGDVKRFSSSGYIDDMAAGNLCAAIGYGGDLNIAKTRAEEAANGVEIKVLTPKTGVGWVDSFMIPRDAQNVANAHRYIDYTLRPEVAAKNGSFVTYAPASRPARELMDEKYTSDASIFPNKELMEKSFIVSPKSAESVKLGVKLWQGLKAGK |
| 65 | 0631 | MAKVLIVPVSAGLDASAAAQAFAKALDAQIFQAVDATAETLLAQGKSDDWFDALVGKVAALDAANLVIEGIAPDADKIYLAGKNVELALSLDAAAVFAVRSDNADADELANRVNLAKQFFAAAPGVLEGFVVDGAAASVAEAAAEKTGLTFFGSSDALKDVSVLAGREAKRLSPAQFRYNLIDFARQADKRIVLPEGAEPRTVQAAAICHEKGIARCVLLAKREEVEAVAKERGISLPDSLEIIDPASLVEQYVEPMCELRKSKGLTPEDARKQLQDTVLGTMMAQNDVDGLVSGAVHTTANTIRPALQLIKTAPGASLVSSVFMLLPNQVLVFGDCAVNPNPTAQQLADIAIQSADSAKAFGIDPKVAMISYSTVNSGSGPDVDTVIEATKLAREKRPDLAIDGPLQYDAATVPGVGKSKAPGSPVAGQATVLVFPDLNTGNCTYKAVQRNANVLSVGPLLQGLRKPVNDLSRGALVEDIVFTIALTAVQAKQMEG |
| 66 | 0634 | MKTSIRYALLAAALTAATPALADITVYNGQHKEAAQAVADAFTRATGIKVKLNSAKGDQLAGQIKEEGSRSPADVFYSEQIPALATLSAANLLEPLPASTINETRGKGVPVAAKKDWVALSGRSRVVVYDTRKLSEKDLEKSVLNYATPKWKNRIGYAPTSGAFLEQVVAIVKLKGEAAALKWLKGLKEYGKPYAKNSVALQAVENGEIDAALINNYWHAFAREKGVQNVHTRLNFVRHRDPGALVTYSGAAVLKSSQNKDEAKKFVAFLASKEGQRALTAVRAEYPLNPHVVSTFNLEPIAKLEAPQVSATTVSEKEHATRLLEQAGMK |

(continued)

| SED ID | NMB | Sequence |
|---|---|---|
| 67 | 0637 | MHDKTWSGRFNEPVSELVKQYTASIGFDRRLAEWDIQGSLAHAQMLKETGVLDEGDLADIRRGMAEILEEIRSGKI<br>EWSSDLEDVHMNIERRLTDKIGDAGKRLHTGRSRNDQVATDIRLWLRDQITVIQSLIQSLQTALLDLAEQNAETVM<br>PGFTHLQVAQPVSFGHHMLAYVEMLGRDNERMADCRCRVNRMPLGAAALAGTTYPIQREITAELLGFEQICQNSLD<br>AVSDRDFAIEFTAAASLVMVHLSRLSEELILWMSPRFGFIDIADRFCTGSSIMPQKKNPDVPELVRGKSGRVIGHL<br>IGLITLMKSQPLAYNKDNQEDKEPLFDTADTLIDTLRIYADMMRGVTVKPDNMRAAVMQGFATATDLADYLVKKGM<br>PFRDAHEVVAQAVRHADQAGVDLSELPLEVLQGFSDLIADDVYGVLTPEGSLNARNHLGGTAPEQVRFQVKRWREM<br>LA |
| 68 | 0641 | MADFNQILTPGDVDGGIINVVNEIPAGSNHKIEWNRKLAAFQLDRVEPAIFAKPTNYGFIPQTLDEDGDELDVLLV<br>TEQPLATGVFLEARVIGVMKFVDDGEVDDKIVCVPADDRNNGNAYKTLSDLPQQLIKQIEFHFNHYKDLKKAGTTK<br>VESWGDAEEAKKVIKESIERWNKQA |
| 69 | 0663 | MKKALATLIALALPAAALAEGASGFYVQADAAHAKASSSLGSAKGFSPRISAGYRINDLRFAVDYTRYKNYKAPST<br>DFKLYSIGASAIYDFDTQSPVKPYLGARLSLNRASVDLGGSDSFSQTSIGLGVLTGVSYAVTPNVDLDAGYRYNYI<br>GKVNTVKNVRSGELSAGVRVKF |
| 70 | 0697 | MKEHKARKRFGQNFLQDTRIISDIVNAVRPQADDVVIEIGPGLAAITEPLAKKLNRLHVVEIDRDIVCRLKTLPFA<br>DKLVIHEGDVLQFDFNGIAGKKKIVGNLPYNISTPLLFKLAEVADDVVDMHFMLQKEVVERMVAAPKSNDYGRLGV<br>MLQYFFDMEMLIDVPPESFDPAPKVDSAVVRMIPVKHRIGKADDFEHFAKLVKLAFHQRRKTIRNNLKELAGDDDL<br>QAVGINPQDRAEHIAPEKYVALSNYLAGKVV |
| 71 | 0703 | MKKILLTVSLGLALSACATQGTVDKDAQITQDWSVEKLYAEAQDELNSSNYTRAVKLYEILESRFPTSRHAQQSQL<br>DTAYAYYKDDEKDKALAAIDRFRRLHPQHPNMDYALYLRGLVLFNEDQSFLNKLASQDWSDRDPKANREAYQAFAE<br>LVQRFPNSKYAADATARMVKLVDALGGNEMSVARYYMKRGAYIAAANRAQKIIGSYQNTRYVEESLAILELAYKKL<br>DKPRLAADTRRVLETNFPKSPFLKQPWRSDDMPWWRYWH |
| 72 | 0711 | MSSGNIHIHSMTGFANAAAECGSKRINLDLRAVNHRFLDIQIRMPDDLRYLESGIREKISSHIARGKVECKIQIQD<br>AENGSQSLELNRDLVGQLAEINKDLRKHHDLAKLGVADILRFPGVLASQRENTEELAKSITELTEKALKDFTAARK<br>REGKKLGEHLLQRLEAMEEIIDALSELFPTLLETHKEKIRARLVEAVGSIDNDRLQQEFALFIQKSDIDEEFSRLR |

| SED ID | NMB | Sequence |
|---|---|---|
|  |  | THIAEVRRIVTEHKGSSGKRLDFLMQELNREANTLGSKSIAAECTQASVELKVLIEQMREQVQNIE |
| 73 | 0720 | MLNITLPDGSVRQYESPVTVAQIAASIGAGLAKATVAGRVNGKLVDACDPIVEDSAVQIITPKDQEGIEIIRHSCA HLVGHAVKQLYPNAKMVIGPVIEEGFYYDIATEKPFTPEDVAAIEARMKELIAQDYDVVKIMTPRAEAIKIFQERG EEYKLRLIDDMPEVEAMGMYHHQEYVDMCRGPHVPNTRFLKNFKLTKLAGAYWRGDSNNEMLQRIYGTAWATKDEL KAYIQRIEEAEKRDHRKLGKQLDLFHLQDEAPGMVFWHPKGWALWQVIEQHMRKELNAAGYKEVKTPQIMDKTFWE KSGHWDNYKDNMFVTSSEKREYAVKPMNCPGHVQIFNNGLRSYRDLPMRLAEFGSCHRNEPSGALHGLMRVRGFVQ DDAHIFCTEDQIVSEARAFNELLIRIYKQFGFHDVSVKLSLRPEKRAGSDDVWDKAEQGLREALTACGVEWGELPG EGAFYGPKIEYHVRDALGRSWQCGTLQLDFVLPERLNAEYVTENNDRARPVMLHRAILGSLERFIGILIENHAGSF PLWLAPVQLVIMNITENQADYCREVAAKLQAAGFRAELDLRNEKIGYKIRDNSQYRFPYQIVVGDKEKQENKVAVR RKAEDLGSLDLDDFIAQLQQEITDALVNH |
| 74 | 0724 | MENVNRIVAEGIAAVEAAQDFNALEQIKARYLGKTGELTGLLKTLGQMSPEERKTIGAHINECKNRFQTAFNAKRE ALNEVKLQARLAAEALDITLPGRAQEGGSLHPVTLTLQRVVELFHGMGFEVADGPEIEDDFHNFQALNIPANHPAR AMQDTFYVENGDVLRTHTSPIQIRYMLDKKEPPIRIIAPGRVYRVDSDATHSPMFHQAEGLWVEEGVTFADLKAVF TDFIRRFFERDDLQVRFRPSFFPFTEPSAEIDIMGENGKWLEVGGCGMVHPNVLKNVNIDPEKYTGFAFGIGLDRF AMLRYNVNDLRLFFDNDLNFLKQFAK |
| 75 | 0728 | MQFSYSWLKTQADTELSSDKLEHLLTMSGLEVEEAETAAPAFAGVVIAEVKSVEKHPDADRLNVTRVDAGTGGLVQ IVCGAPNVKAGIKVPCSLPGAVLPGNFKIKPTKMRGEVSDGMLCSTDELGLPDDGVNGLHILPEDAPVGTNIREYL DLDDTLFTLKITPNRADCLSIKGIAREVSALTGCAFRQPEIHTAPITGSRKQPVQINAPADCGRFISRVIENVNAR ATTPDWMKQRLERSGIRSISALVDIGNYVMLEIGQPMHVFDADKLSGSLHIRRAREGETLECLNEKTVSLSENTLV VADEKGVLSLAGLMGGAASAVSDGTQNIVLEAAWFAPEIIAGKSRQYGFGSDSSFRFERGVDYRLQADAIERATEL VLQICGGAAGEMVEAQGELPEAKQVGLRLDRLKTVLGVDIPAEQVETILQHLGLQPEKTAEGFRVTAPSFRFDIEI EADLIEEIGRVYGYENIPDDYTSGRLKMLELPETRRPRFAVYNEMAARGYREVVSYAFVDEQWEQDFAANADPIRL QNPLAAQYAVMRSTLIGGLVEILQNNLNRKQNRVCVFEIARVFSKGSDGQFVQNERIGGLWYGAVMPEQWGGKTRN ADFYDIKADVENLLKNKAVEFVKTGHPALHPGRAANIVSDGKVIGFVGELHPKWLQKYDLPQAPLVFEIDMAAVLE CGKTRYRVVSKFQPVRRDLAFVMPEAMSHDDLLLVLKGAANKLVQEISVFDVYRGTGLPEGMKSVAVKVILQDMEN TLTDEAVEPLIGKLIGAATAAGARLRS |

EP 1 562 982 B1

| SED ID | NMB | Sequence |
|---|---|---|
| 76 | 0743 | MGGQKTHFGFSTVNEDEKAGKVAEVFHSVAKNYDIMNDVMSAGLHRVWKHFTINTAHLKKGDKVLDIAGGTGDLSR GWAKRVGKEGEVWLTDINSSMLTVGRDRLLNEGMILPVSLADAEKLPFPDNYFNLVSVAFGLRNMTHKDAALKEMY RVLKPGGTLLVLEFSKIYKPLEGAYDFYSFKLLPVMGRLIAKDAESYQYLAESIRMHPDQETLKQMMLDAGFDSVD YHNMSAGIVALHKGVKF |
| 77 | 0757 | MSEIGLVKIYFGKVRDLYEIDDKRMLMVASDRLSAFDVILDDPIPSKGEILTQISNFWFKKLAHIMPNHFTGQTVY DVLPENEAKALEKRAVVAKKLTPVKVEAIVRGYLAGSGWKDYQKTGSVCGIQLPEGMQEAQQLPEVIFTPSTKAAV GDHDENISFEECGRIIGKELAEEVRAKAVRLYTEAAEYAKSRGIIICDTKFEFGLDENGTLTLMDEVLTPDSSRFW PADQYKVGTNPPSFDKQFVRDWLEQSGWNKKAPAPKVPADVIQKTVEKYREALTLLTQD |
| 78 | 0758 | MMFDKHVKTFQYGNQTVTLETGEIARQAAAAVKVSMGDTVVLVAVTTNKEVKEGQDFFPLTVDYLERTYAAGKIPG GFFKREGKQSEKEILTSRLIDRPIRPLFPEGFYHDIQIVAMVVSVDPEIDSDIPAMLGASAALVLSGVPFAGPIGA ARVGYVNGVYVLNPTKAELAKSQLDLVVAGTSKAVLMVESEAKILPEDVMLGAVVYGHDQMQVAINAINEFADEVN PELWDWKAPETNEELVAKVRGIAGETIKEAFKIRQKQARSAKLDEAWSAVKEALITEETDTLAANEIKGIFKHLEA DVVRSQILDGQPRIDGRDTRTVRPLNIQTSVLPRTHGSALFTRGETQALAVATLGTSRDEQIIDALSGEYTDRFML HYNFPPYSTGEVGRMGAPKRREIGHGRLAKRALLAVLPKPEDFSYTMRVVSEITESNGSSSMASVCGGCLSLLSAG VPLKAHVAGIAMGLILEGNKFAVLTDILGDEDHLGDMDFKVAGTTEGVTALQMDIKIQGITKEIMQIALAQAKEAR LHILDQMKAAVAGPQELSAHAPRLFTMKINQDKIREVIGKGGETIRSITAETGTEINIAEDGTITIAATTQEAGDA AKKRIEQITAEVEVGKVYEGTVVKILDNNVGAIVSVMPGKDGLVHISQIAHERVRNVGDYLQVGQVVNVKALEVDD RGRVRLSIKALLDAPAREENAAE |
| 79 | 0763 | MKIANSITELIGNTPLVKLNRLTEGLKAEVAVKLEFFNPGSSVKDRIAEAMIEGAEKAGKINKNTVIVEATSGNTG VGLAMVCAARGYKLAITMPESMSKERKMLLRAFGAELILTPAAEGMAGAIAKAKSLVDAHPDTYFMPRQFDNEANP EVHRKTTAEEIWRDTDGKVDVFVAGVGTGGTITGVGEVLKKYKPEVKVVAVEPEASPVLSGGEKGPHPIQGIGAGF IPTVLNTKIYDSITKVSNEAAFETARAIAEKEGILVGISSGAAVWSALQLAKQPENEGKLIVVLLPSYGERYLSTP LFADLA |

EP 1 562 982 B1

| SED ID | NMB | Sequence |
|---|---|---|
| 80 | 0778 | MPSETSSPRKENETEVHIPAAPFIVKQSGSNALAVCALVLAALGLGTSGFLFVQGQNVLKNQELAFNQKIDKAALG ESENAALLKDNLNRQAAIQSELDRLDGNVKANGEQILEMQKSYRELTKGRADWLVDETETILNLAAQQLVLTGNIQ TAVGVLEHIDSRLSRFNQAELLPIKQAVSSDLAELKNRPYVDISGTALRLDRLETAVSGLPLMLDGVLKPGVQVKN EAASASWWQNVWEKSLGTLKGLVEIRRLENNDAMLISPEQAYFVRENLRLRLLDARTALMQRNSEVYQGDLNNAEA AVRQYFDAKSPATQSWLKELAELKALDVRMTADDGLKNSLNAVRAYRDGTRMTAAENQEAEQAASEPANEKTASEP AAASDVKTIEAPSLPSERKPEQPAKKQTVPEKAGRSPSAKGERA |
| 81 | 0791 | MIILHTNKGDIKIELDFDKAPVTAKNFEQYVKDGFYDGVIFHRVIKGFMIQGGGMDENMNEKETRDPIQNEASNGL PNDKYTIAMARTSDPHSASAQFFINTADNAFLNFRSKELYGKTVVQDWGYAVFGKVVDGFDVVDAIEGVSTKRHGY HDDVPSEPVVIIKAEAV |
| 82 | 0804 | MTVLSKEQVLSAFKNRKSCRHYDAARKISAEDFQFILELGRLSPSSVGSEPWQFIVVQNPEIRQAIKPFSWGMADA LDTASHLVVFLAKKNARSDSPFMLESLKRRGVTEPDAVAKSLARYQAFQADDIKILDDSRALFDWCCRQTYIALAN MMTGAAMAGIDSCPVEGFNYAEMERILSGQFGLFDAAEWGVSVAATFGYRVQEIATKARRPLEETVIWA . |
| 83 | 0814 | MQTWQLPEHIADVLPTNARQLESAREQLLALFRVHGYELVQPPLMEYAHSLLTHIDAGLSLKTILVTDRLSGRQLG IRADITPQVARIDAHLLSANQGINRLCYAGPVLHAQPDGLLNMREPLQAGAEMYGFADIRGDIELIDLMLKSMKIA DMGKVLLSLGHIGIFRALSDAAHLDAGQSATLLALMQDKDTGAVEAQVKAWKLDGMWAKAFSLLPRLYGGREVLSD ARGRLPDLSAVGGALGELQAVCDAFPDCEIHIDLSELRVDNYHTGLLYAAYAADFHDAVARGGRYDGLGGYFGRAR PATGFSFDLRSFIGRLPAIERQPAVLVDAEDAEAAHEAVEALREQGQCVVIDYGIGHNVSEELAGRLKKTDGVWQV VKR |
| 84 | 0815 | MAMAKNVVVIGAQWGDEGKGKIVDWLAEEAGGVVRFQGGHNAGHTLVVGGKKTILRLIPSGILHESLDCFIGSGVV VSPEALLGEIDELNAAGVKNVEGRLKIAPTCPLILPYHIALDQAREASRGKGKIGTTGRGIGPAYEDKVARRAIRA ADLLHPEKLREKLDAVLAYYNVQLQHLHNAEPVKAEDVMAVIEKVAPRIAPMITDVSRVLNEKNKNGEKLLFEGAQ GALLDIDYGTYPFVTSSNCLAGAASAGAGVGPQMLDYVLGIVKAYTTRVGSGPFPTELFDEVGVGLAERGHEFGSV TGRARRCGWFDAAALKRSIQINGISGMCITKLDVMDGVETINICVGYELPDGGKTDILPCGSDAVETCKPIYETMP GWRESTFGVKDYGALPENAKAYLKRIEEVCGAPVAIVSTGPDREETIVLHHPFA |

EP 1 562 982 B1

(continued)

| SED ID | NMB | Sequence |
|---|---|---|
| 85 | 0828 | MTIIVTGAAGFIGSNIVKALNQRGITDIVAVDNLSKGEKFKNLAECEIAHYLDKHEFIRQVREHILPYQNIEAVFH<br>QGACSDTMNHDGLYMMDNNYQYTLDLLDWCQDERIPFLYASSAAVYGKGEIFREERELEKPLNVYGYSKFLFDQVL<br>RRRMKEGLTAQVVGFRYFNVYGQHEQHKGRMASVAFHHFHQYREHGYVNLFGSNDGYGNGEQTRDFVSVEDVAKVN<br>LYFFDHPELSGIYNLGTGRSQQFNELAAATVNACRAAEGKPEMSLKELVEEELIRYIPFPDALKGKYQSFTQADIT<br>KLREAGYKEEFFDVKSGVDRYVKWMLENLA |
| 86 | 0854 | MAQKIQSVKGMNDLLPVKQKDFKLTAAFWQAFEDTVGRWTRAYGYQQIRTPIVEQTGLFVRSIGEETDVVGKEMYT<br>FSDSNDSLSLSLRPEGTASCLRAVVEHNLLYNSPQKLWYMGPMFRRERPQKGRYRQFHQVGIEALGFEGPDIDAEI<br>IAMSADLWEKLGIREYLTLEINSLGNREERAAHRAALVEYLTRYEDKLDEDSKRRLKTNPLRVLDTKNPDLQEICN<br>AAPRLVDYLGEDSQNHYVRFKAMLDGLGIQYIENPRLVRGLDYYNQTVFEWTTDKLGAQATVCGGGRYDGLIEELG<br>GKPAPSIGFAMGIERLLLLVSEYGSLEVNAAPDVYAMHQGEGADLQVMKYAQALRAQGFNVMQHSGYQSLKAQMKK<br>ADNSGARFALIVAQDELANGTVTLKDMNGAHGQQTVAAEDLTPTLQQWKNA |
| 87 | 0875<br>0876 | MAAYDSLMVFTGNANPELAQRVVRHLDISLGNASVSKFSDGEVAVELLENVRGRDVFILQPTCAPTNDNLMEILTM<br>ADALKRASAGRITTAIPYFGYARQDRRPRSVRVPISAKLVANMLYSAGIDRVLTVDLHADQIQGFFDIPVDNIYAT<br>PILLNDIKQQRIENLTVVSPDIGGVVRARAVAKSLNADLAIIDKRRPKANVAEVMNIIGDIQGRTCLIVDDMIDTA<br>NTLCKAAVALKERGAERVLAYASHAVFSGEAVSRIASSEIDQVVVTDTIPLSEAAKNCDRIRQVTIAGLLAETVRR<br>ISNEESVSYLFNEEVMTGSMLLP<br><br>MTYEIQASVREAQGTGASRRLRREGQIPGILYGEGQEPVAIAVDHKTVFYALEKESFHTALIKLSLNGETKDVIVR<br>DFQMHPFRREVQHIDFQAVKADQLVRIRVPLHIVNAENSQAVKLQGGRVSLLNTSVEVVALPANIPAFLDLDCAEV<br>VAGDILHLSDIKLPEGVESVSLKRNENLAVATVTGKKR |

EP 1 562 982 B1

| SED ID | NMB | Sequence |
|---|---|---|
| 88 89 90 | 0878<br>0884 | MNTPLPYSDYLIRILTASVYDVAVETPLEPARSLSVRLKNNILLKREDLQPVFSFKIRGAYNKMSKLPKDALACGV<br>IAASAGNHAQGVALSAQRLGCRAVIVMPETTPKIKVDAVKSHGGEVVLRGVSYNDAYDYAMELAEKEGLTYIAPFD<br>DPDVIAGQGTVGMEIVSQHPDPIRAVFVPIGGGGLAAGVAAFIKQVRPEIKVIGVQTNDSCCMKQSVEAGEIVHLK<br>DVGLFSDGTAVKVVGNETFRLCKELLDEIITVDTDAVCGAVKDIFDDTRSITEPAGALALAGLKAYIAREGAENQT<br>LIAVTSGANMNFHRLRHVSERSELGEGNEGIFAVTIPEERGSFLKFVNILGNRNITEFNYRYGDDEKAHIFVGLQA<br>AGPQDLAVIGSRLDEAGLPNVDLTNNEIAKIHIRYMVGGRTDKVENERLVSFEFPERPGALARFLNHMQGGWNITL<br>FHYRNHGADYGRILVGIDVPPHDAAAFDGFLESLGYSYHEETQNAAYKLFLA<br><br>MEHKLPQLPYELDALSPHLSKETLEFHYGKHHQTYITNLNNQIKGTEFENLPLEEIVKKSSGGVFNNAAQTWNHTF<br>YWLGFTSKGQGKPAGELAAAIDAKWGSFEKFQEAFNACAAGTFGSGWAWLVKTPAGGLDLVSTSNAATPLTTENTP<br>LLTCDVWEHAYYIDYRNSRPNYLKGFWEIVNWDEVAKRFAALS |
| 91 | 0885 | MNDYAAMPSEDREVGALSLPPHSMEAEQSVLGGLMLENPAWDRIADVVSGEDFYRHEHRLIFRSIAKLINESRPAD<br>VITVQEDLQRNEELEAAGGFEYLITLAQNTPSAANIRRYAEIVRERSIMRQLAEVGTEIARSAYNPQGRDAGQLLD<br>EAENKVFQIAESTAKSKQGFLEMPDLLKEVVQRIDMLYSRDNPDEVTGVPTGFIDLDKKTSGLQPGDLIIVAGRPS<br>MGKTAFSINIAEHVAVEGRLPVAVFSMEMGGAQLVMRMLGSVGRLDQSVLKTGRLEDEHWGRLNEAVVKLSDAPVY<br>IDETPGLTALELRARARRLARQFNNKLGLIVIDYLQLMAGSGRSDNRASELGEISRSLKALAKELQVPIIALSQLS<br>RTVESRTDKRPMMSDLRESGAIEQDADLIMFMYRDEYYNQDSPMKGLAECIIGKHRNGPVGKIFLTWTGQFTKFDN<br>AAYIPEEAKIED |
| 92 | 0894 | MNTLLNQLKPYPFARLREAMQGISAPEGLEAVPLHIGEPKHPTPKVITDALTASLHELEKYPLTAGLPELRQACAN<br>WLKRRYDGLTVDADNEILPVLGSREALFSFVQTVLNPVSDGIKPAIVSPNPFYQIYEGATLLGGGEIHFANCPAPS<br>FNPDWRSISEEVWKRTKLVFVCSPNNPSGSVLDLDGWKEVFDLQDKYGFIIASDECYSEIYFDGNKPLGCLQAAAQ<br>LGRSRQKLLMFTSLSKRSNVPGLRSGFVAGDAELLKNFLLYRTYHGSAMSIPVQRASIAAWDDEQHVIDNRRMYQE<br>KFERVIPILQQVFDVKLPDASFYIWLKVPDGDDLALARNLWQKAAIQVLPGRFLARDTEQGNPGEGYVRIALVADV<br>ATCVKAAETIVSLYR |
| 93 | 0920 | MTQKSTIVYTHTDEAPALATQSLLPIVQAFARHADIDVKTSDISLSGRILAAFPEYLTEAQRVPDALAELGELVKQ |

EP 1 562 982 B1

60

| SED ID | NMB | Sequence |
|---|---|---|
| | | PDANVIKLPNISASVPQLTAAIKELQSKGFAVPDYPADPQTDEEKAVRERYDRIKGSAVNPVLREGNSDRRAPKAV<br>KNFAKKNPHSMGAWTKDSKTHVATMQSGDFFHNEQSVIVPEATSVSIVFTDKQGNKKELREPVALKAGEIIDATVM<br>SKKALLAFLAEQVKDAKAKGVLFSLHMKATMMKVSDPIIFGHAVKVFFAPVFEKFGDKLAAAGVNVNNGFGNLLAN<br>LDKLDADTRTAVEAEIAAVYAANPDLAMVDSDKGITNLHVPSDVIVDASMPAMIRNSGRMWDKNGKAQDTKAVIPD<br>SSYAGVYQATIDFCREHGAFDPTTMGTVPNVGLMAQAAEEYGSHNKTFEIEADGQVQVIDAAGKVLMQHDVEAGDI<br>WRMCQTKDAPVKDWVQLAVNRARLSNTPAVFWLDENRPHDKSLLAKVKAYLAELDTNGLDIRVLAPEEAAKFSLGR<br>LKNGEDTISVTGNVLRDYLTDLFPILELGTSAKMLSIVPLMNGGGMFETGAGGSAPKHVQQFLEENHLRWDSLGEF<br>LALAVSFEHLAQKTGNAKAQVLADTLDAATEKLLLNDKSPKRKAGELDNRGSHFYLTLYWAQELAAQDKDAELKAA<br>FTPLAAALTADEAKIVAELSAVQGKAADIGGYYAANPEKAAQVMRPSVTFNQALNAL |
| 94 | 0944 | MTTLHFSGFPRVGAFRELKFAQEKYWRKEISEQELLAVAKDLREKNWKHQVAANADFVAVGDFTFYDHILDLQVAT<br>GAIPARFGFDSQNLSLEQFFQLARGNKDQFAIEMTKWFDTNYHYLVPEFHADTEFKANAKHYVQQLQEAQALGLKA<br>KPTVVGPLTFLWVGKEKGAVEFDRLSLLPKLLPVYVEILTALVEAGAEWIQIDEPALAVDLPKEWVEAYKDVYATL<br>SKVSAKILLSTYFGSVAEHAALLKALPVDGLHIDLVRAPEQLDAFADYDKVLSAGVIDGRNIWRANLNKVLETVEP<br>LQAKLGDRLWISSSCSLLHTPFDLSVEEKLKANKPDLYSWLAFTLQKTQELRVLKAALNEGRDSVAEELAASQAAA<br>DSRANSSEIHRADVAKRLADLPANADQRKSPFADRIKAQQAWLNLPLLPTTNIGSFPQTTEIRQARSAFKKGELSA<br>ADYEAAMKKEIALVVEEQEKLDLDVLVHGEAERNDMVEYFGELLSGFAFTQYGWVQSYGSRCVKPPIIFGDVSRPE<br>AMTVAWSTYAQSLTKRPMKGMLTGPVTILQWSFVRNDIPRSTVCKQIALALNDEVLDLEKAGIKVIQIDEPAIREG<br>LPLKRADWDAYLNWAGESFRLSSAGCEDSTQIHTHMCYSEFNDILPAIAAMDADVITIETSRSDMELLTAFGEFQY<br>PNDIGPGVYDIHSPRVPTEAEVEHLLRKAIEVVPVERLWVNPDCGLKTRGWKETLEQLQVMMNVTRKLRAELAK |
| 95 | 0946 | MALQDRTGQKVPSVVFRTRVGDTWKDVSTDDLFKGKKVVVFSLPGAFTPTCSSSHLPRYNELFGAFKENGVDAIYC<br>VSVNDTFVMNAWAAEEESDNIYMIPDGNGEFTEGMGMLVGKEDLGFGKRSWRYSMLVNDGVVEKMFIEPEEPGDPF<br>KVSDADTMLQFVAPDWKAQESVAIFTKPGCQFCAKAKQALQDKGLSYEEIVLGKDATVTSVRAITGKMTAPQVFIG<br>GKYIGGSEDLEAYLAKN |

EP 1 562 982 B1

61

62

(continued)

| SED ID | NMB | Sequence |
|---|---|---|
| 96 | 0947 | MKKIQADVVVIGGGTAGMGAFRNARLHSDNVYLIENNVFGTTCARVGCMPSKLLIAAAEARHHALHTDPFGVHLDK DSIVVNGEEVMQRVKSERDRFVGFVVADVEEWPADKRIMGSAKFIDEHTVQIDEHTQITAKSFVIATGSRPVILPQ WQSLGNRLIINDDVFSWDTLPKRVAVFGPGVIGLELGQALHRLGVKVEIFGLGGIIGGISDPVVSDEANAVFGEEL KLHLDAKTEVKLDADGNVEVHWEQDGEKGVFVAEYMLAAVGRRPNVDNIGLENINIEKDARGVPVADPLTMQTSIP HIFIAGDASNQLPLLHEAADQGKIAGDNAGRYPNIGGGLRRSTIGVVFTSPQIGFVGLKYAQVAAQYQADEFVIGE VSFKNQGRSRVMLVNKGHMRLYAEKATGRFIGAEIVGPAAEHLAHLLAWAHQMKMTVPQMLDMPFYHPVIEEGLRT ALRDADAKLKA |
| 97 | 0955 | MMDEKLNFSYLFGSNAPYIEELYEAFLENPDAVDEKWKQYFTDLSKQPGTVAVDVAHTPIRESFVTLAKKKIASAV AGGADEAMLKKQVSVLRLISAYRIQGVGAAQLDPLKRIPPRDIEALDPKFHGLSDADMALQFNMGEGDFANRGKLP LSQIISNLKQTYCGHIALEYIYIPNTEERRWVRNYFESVLSTPHYNADQKRRILKEMTAAETLERYLHTKYVGQKR FGVEGGESAIAGLNYLIQNAGKDGVEEVIIGMAHRGRLNVLVNILGKKPGDLFAEFEGRAEIKLPSGDVKYHMGFS SDIATPHGPMHVSLAFNPSHLEIVNPVVEGSARAKQKRLGENGRDKVLPVLIHGDSAFIGLGVNQATFNLSKTRGY TTGGTVHIVINNQIGFTTSDIRDTRSTVHCTDIAKMVSAPVIHVNGDDPERVCFAIQAALDYRKKFHKDIVIDVVC YRKWGHNEGDDPTLTQPMMYKKVSQHPGARALYTEQLIAEGVVTQAEADGYIQAYRDALDKGEHVEQTTLSNFQRT QIDWSKYQGKDWREHIETGLPAADIERLTEKFTAVPEGFALHPTAKRVIEARKAMASGKQAIDWGMAETLAYASLV TKGHGVRISGEDSGRGTFSHRHAVLHDQKREKWDDGTYVPLRHMGEGMGEFLVIDSILNEEAVMAFEYGFACSAPD KLTIWEAQFGDFANGAQVTIDQFLSSGETKWGRLCGLTTILPHGYDGQGPEHSSARVERWLQLCSENNMQVIMPSE ASQMFHLLQRQVLGSYRKPLVIFMSKRLLRFKGAMSPLENFTEGSTFRPVIGDTAERASNDSVKRVVLCAGQVYYD LEAGRAERKLEDDVAIVRVEQLYPFPYDEVKAELAKYPNAKSVVWAQEEPKNQGAFYQIRHRIEDVISEEQKLSYA GRPSSASPAVGYSSKHIAQLKQLVEDALAL |
| 98 | 0956 | MIIDVKVPMLSESVSEGTLLEWKKKVGEAVARDEILIDIETDKVVLEVPSPQAGVLVEIVAQDGETVVADQVLARV DTAATAAAEAPAAAPAEAAPAAAPAATQNNAAMPAAAKLAAETGVDVNALQGSGRDGRVLKEDVQNAAAKPAGAAA PAVALPAGARPEERVPMSRLRARVAERLLASQQENAILTTFNEVNMKPIMDLRAKYKEKFEKEHGVKLGFMSFFVK AAVAALKKYPVVNASVDGKDIVYHGYFDIGIAIGSPRGLVVPILRDADQMSIADIEQAIVDYAKKAKDGKIAIEDL TGGTFSITNGGTFGSMMSTPIINPPQSAILGMHATKERAVVENGQVVVRPMMYLALSYDHRIIDGREAVLTLVAIK DALEDPARLLLDL |

(continued)

| SED ID | NMB | Sequence |
|---|---|---|
| 99 | 0959 | MNLHEYQAKELLASYGLPVQGGILAHNGEEAAAAYDKLGGKFAVVKAQVHAGGRGKAGGVKVVKSREEAKEVAESL<br>IGTNLVTYQTDANGQPVNSVLVCEDMYPVQTELYLGAVVDRSTRRITFMASTEGGVEIEKVAAETPEKIFKVTVDP<br>LVGLQPCQAREVAFQLGLKDKQINEFVKLMTGAYKAFVENDFALFEVNPLAVRENGALACVDGKIGIDSNALYRLP<br>KIAELRDKSQENERELKASEFDLNYVALEGNIGCMVNGAGLAMATMDIIKLKGGQPANFLDVGGGATKDRVVEAFK<br>LILEDKSVQGVLINIFGGIVRCDMIAEAIVAAVKEINVNVPVVVRLEGNNAELGAKILNESGLKLTSADGLNDAAE<br>KIVAAVNA |
| 100 | 0960 | MSVLINKDTKVLVQGFTGKNGTFHSEQALAYGTKVVGGVTPGKGGQTHLNLPVFNTMKEAVKETGADASVIYVPAP<br>FVLDSIVEAVDSGVGLVVVITEGVPTLDMLKAKRYLETNGNGTRLVGPNCPGVITPGECKIGIMPGHIHTPGRIGI<br>ISRSGTLTYEAVAQTTKLGLGQSTCIGIGGDPIPGMNQIDALKLFQEDPDTDAIIMIGEIGGTAEEEAAEYIQSNV<br>SKPVVGYIAGVTAPKGKRMGHAGAIISGGKGTAEEKFAAFEKAGIAYTRSPAELGTTMLEVLKAKGLA |
| 101 | 0983 | MSSIKRALISLSDKTGAVEFAQTLHKLGVEILSTGGTAKLLADAGVPVIEVADYTGFPEMLDGRVKTLHPKIHGGI<br>LGRRDLDEHVAKMEEHGIGNIDLVCVNLYLFAATIAKPNCTLEDAIENIDIGGPTMVRSAAKNWKHVAIVTDTADF<br>PAIAAELEANNGALSDKTRFNLSRKAFSHTAQYDGMISNYLTSLSDDVLSGTPEIAGFPGRFNQSWIKVQDMRYGE<br>NPHQRAAFYRDIDPAAGSLAAYKQLQGKELSYNNIADADAAWEAVKSFDVPACVIVKHANPCGVAIASNTLDAYKL<br>AYATDTTSAFGGIIAFNREVDGATVKQITDNQFMEVLMAPKFTAEALEIAAAKKNVRVLEVPLEAGANRFELKRVG<br>GGLLVQTPDIHRISRADLKVVSKRQPTEQEWNDLLFVWNVAKYVKSNAIVFGKGGQTYGIGAGQMSRVDSTRIAAR<br>KAQDAGLDLNGACAASDAFFPFRDGVDVIAEQGIKAIIHPAGSMRDQEVFDAADEHGIAMVVTGIRHFRH |
| 102 | 0997 | MSASQLLSRLTQTVGEKYIITDPAKTEQYRQGYRFGEGKALAVVRPGSILEMWKILQACVEADVIVITQAANTGLT<br>GGSTPDGNDYDRDIVIVNTMRMNIIQTINNNEQVVCLPGSTLNQLELLLKPLGREPHSVIGSSCIGASVLGGVCNN<br>SGGALVQRGPAYTEMALFAQINEEGRLELVNHLGIDLGNTPEEILTNLQGHHYQNKDITQDAGKGHDHAYCEHVRQ<br>VDEPTAARFNADPARHYEASGCAGKLMVFAVRLDTFPQEKQTAVFYIGTNDINELTDIRRAALGEFESLPVSGEYI<br>HRHAFDIADVYGKDTFYVIKKFGTHQLPKLFDLKARVDRFGKKVSFLPKHFSDKAMQFVSKFLPDHLPKSMRDYRD<br>KYEHHLILKMGGKGVDEARAFLKEYFAHHGGAFFECNAEETQAAMLHRFAVASAAIRYRAVHDDEVEDLVALDIAL<br>RRDDRDWFEKLPPEIDNKIIHKLYYGHFMCHVFHQDYIIKKGNDCMALEHEMLHLLDQRGAQYPAEHNVGHLYEAK<br>PALKQFYRKLDPTNSFNPGVGKTSKKKNWAE |

| SED ID | NMB | Sequence |
|---|---|---|
| 103 | 1031 | MTKHIAILRGDGIGPEIVAETVRVLDKLIAQGLDAGYEYAPLGGEAYDEYGHPYPEFTQNLCRKADAVLLGAVGSP QYDNLDRPLRPERGLLAIRKDLNLFANLRPAVLYPELANASTLKPEIVAGLDILIVRELTGDIYFGEPRGIRVLEN GEREGYNTMKYSESEIRRIAHVAFQSAQKRSKKVCSVGKANVLETTELWREIFEEIGKEYPDVELSHMYVDNAAMQ LVRAPKQFDVIATGNIFGDILSDEASMLTGSIGMLPSASLDENGKGLYEPSHGSAPDIAGQNKANPLATILSLAML LRYSLNDEARAQQVENAVQKVLQQGLRTSDIYEEGTKLVSCSEMGDAVLAAL |
| 104 | 1044 | MAAFNTQKVLSVHHWTDAYFTFTCTRDESLRFENGQFVMVGLMVDGKPLMRAYSVASANWEEHLEFFSIKVQDGPL TSRLQHLKVGDDVLISKKPTGTLVAGDLNPGKHLYLLSTGTGIAPFLSITKDPEIYEQFEKIILVHGVRYKKDLAY YDRFTKELPEHEYLGDLVKEKLIYYPIVSREEFEHHGRLTDLMVSGKLFEDIGLPKINPQDDRAMLCGSPAMLKDT CKVLDDFGLTVSPKTGVRGDYLIERAFVDQ |
| 105 | 1046 | MKYISTRGETAHKPFSEVLLMGLAPDGGLMLPEHYPQIGRETLDKWRGLAYPELAFEIMRLFVTDIPEDDLRDILN RTYTEAAFGTKEITPVRTLSDGIKIQALSNGPTLAFKDMAMQFLGNAFEYVLNKEGKKLNILGATSGDTGSAAEYA LRGKKGVNVFMLSPDGKMSAFQRAQMYSLQDENIHNIAVKGMFDDCQDIVKAVQNDAAFKEKYHIGTVNSINWGRI VAQVVYYFAGYFNATSSNDETVSFCVPSGNFGNVCAGHIAKQMGLPIRRLIVATNENDVLDEFFKTGAYRPRNSAH TYVTSSPSMDISKASNFERFVFDLMDRDPAEINTLWAEVAAGKGFDLRFALDKVGGKYGFTSGKSTHADRLATIKQ VYEQDQELIDPHTADGVKVAREVREEGEMVVCLETALAAKFDATIREAVGDAAIPRPAALEGLENLPQRVQTVPNS ADAVKGIIEQTLA |
| 106 | 1053 | MKKTVFTCAMIALTGTAAAAQELQTANEFTVHTDLSSISSTRAFLKEKHKAAKHISVRADIPFDANQGIRLEAGFG RSKKNIINLETDENKLGKTKNVKLPTGVPENRIDLYTGYTYTQTLSDSLNFRVGAGLGFESSKDSIKTTKHTLHSS RQSWLAKVHADLLSQLGNGWYINPWSEVKFDLNSRYKLNTGVTNLKKDINQKTNGWGFGLGANIGKKLGESASIEA GPFYKQRTYKESGEFSVTTKSGDVSLTIPKTSIREYGLRVGIKF |
| 107 | 1070 | MTQTNRVIIFDTTLRDGEQSPGAAMTKEEKIRVARQLEKLGVDIIEAGFAAASPGDFEAVNAIAKTITKSTVCSLS RAIERDIRQAGEAVAPAPKKRIHTFIATSPIHMEYKLKMKPKQVIEAAVKAVKIAREYTDDVEFSCEDALRSEIDF LAEICGAVIEAGATTINIPDTVGYSIPYKTEEFFRELIAKTPNGGKVVWSAHCHNDLGLAVANSLAALKGGARQVE CTVNGLGERAGNASVEEIVMALKVRHDLFGLETGIDTTQIVPSSKLVSTITGYPVQPNKAIVGANAFSHESGIHQD GVLKHRETYEIMSAESVGWATNRLSLGKLSGRNAFKTKLADLGIELESEEALNAAFARFKELADKKREIFDEDLHA LVSDEMGSMNAESYKFISQKISTETGEEPRADIVFSIKGEEKRASATGSGPVDAIFKAIESVAQSGAALQIYSVNA VTQGTESQGETSVRLARGNRVVNGQGADTDVLVATAKAYLSALSKLEFSAAKPKAQGSGTI |

EP 1 562 982 B1

| SED ID | NMB | Sequence |
|---|---|---|
| 108 | 1073 | MIKISTRFDAGSVVVKDLTDPSNIRLALRPDNASDFAQWFYFRLQGAAYQNCIMHFENAAEAAYPKGWEGYQACAS<br>YDRRNWFRVPTSYENGVLTVNHTPLSNSVYYAYFEPYSEEQHLNLLGDAQGSGLCRIDDLGSTVQGRDINLLTIGN<br>QVESDLKIWITARQHPGETMAEWFIEGLLGRLLDPQDPTARALLDRATFYIVPNMNPDGSALGNLRTNAAGANLNR<br>EWENPTVEKSPEVFFVREKMLETGVDLFLDIHGDEGLPFVFVAGTEGVPNYNPRIAALEAQFKNALLNASPDFQDE<br>YGYEKDAPGEANMTLATNWVGNRFNCLAYTLEMPFKDNANLPDDDFGWNGQRSLRLGEAALSAILNVIGDLR |
| 109 | 1127 | MATLSDKTILVTGASQGLGEQVAKAYAAAGATVILVARHQKKLEKVYDAIVEAGYPEPFAICFDLISAEEKEFEHF<br>AATIAEATQGKLDGIVHCAGYFYALSPLDFQTVAEWVNQYRINTVAPMGLTRALFPLLKQSPDASVIFVGESHGET<br>PKAYWGGFGASKAALNYLCKVAADEWERFGNLRANVLVPGPINSPQRIKSHPGEAKSERKSYGDVLPAFVWWASAE<br>SKGRSGEIVYL |
| 110 | 1131 | MALLQISEPGMSAAPHRHRLAAGIDLGTTNSLVATVRSGSAACLPDAEGRVTLPSVVRYLENGGIEVGKTALSAQK<br>TDPLNTVSSAKRLIGRTLADLHQNTHYLPYRFGDNQRVIELHTRQGVKTPVEVSAEILKTLKSRAEETLGGDLVGV<br>VITVPAYFDDAQRQATKDAARLAGLNVLRLLNEPTAAAIAYGLDNASEGTFVVYDLGGGTFDVSVLQLTKGLFEVK<br>ATGGNSALGGDDFDHRLFCRLLEQNGLSQLNEQDSQLLLSLVRAAKEQLTTQTEARIQATLSDGMAIDTSISRAEF<br>HNLTQHLVMKTLEPVTQALKDAGVGKNEVKGVIMVGGSTRMLHVQQAVATFFGQTPLNNLNPDEVVALGAAIQANV<br>LAGNKTDGEWLLLDVTPLSLGLETYGGLAEKIIPRNSTIPTARAQDFTTFKDGQTAMTIHVVQGERELVADCRSLA<br>KFTLRGIPPMAAGAARIRVTFQIDADGLLSVSAQEQSTGVQAQIEVKPSYGLDDDTITQMLKDSMSNAAEDMAARA |
|  |  | RAEAVVEAESLTDAVNAALELDSDLLDAEELQQIRQGIADLQGRLKDGKAEDIRAAAAKLGSITDNFAAKRMNRNI<br>QRALTGQSVDNI |

65

EP 1 562 982 B1

| SED ID | NMB | Sequence |
|---|---|---|
| 111 | 1150 | MPEYRSKTSTHGRNMAGARALWRATGVMETDFGKPIIAVANSFTQFVPGHVHLHNMGQLVAREIEKAGAIAKEFNT IAIDDGIAMGHSGMLYSLPSRDLIADSIEYMVNAHCADALVCISNCDKITPGMLIAAMRLNIPTIFVSGGPMEAGK VIGVANIQPERRLDLIDAMIESADDNVSNRQVEEVEQNACPTCGSCSGMFTANSMNCLTEALGLSLPGNGSYLATH AGRKELFLEAGRMIVEITKRYYEQNDETVLPRSIATKKAFENAMTMDIAMGGSTNTILHLLAVANEAGVDFKMADI DRLSRVVPCICKTAPNNHDYYMEDVHRAGGIFAILKELDKAGKLHTDVHTIHAPTLKDAIEQWDVTNPENTRAIER FKAAPGGVRTTQAFSQNRMWKTLDLDREKGCIRDVAHAYSQDGGLAVLFGNIAERGCVVKTAGVDESILKFTGRAR VFESQEDAVEGILGNQIVAGDIVIIRYEGPKGGPGMQEMLYPTSYLKSKGLGKACALLTDGRFSGGTSGLSIGHAS PEAAEGGAIGLVHEGDTVEIDIPNRSIHLAISDEELAARRAEMEARGSKAWKPKNRDRYVSAALRAYGAMATSADK GAVRDVAQIER |
| 112 | 1201 | MRIVEKAYTFDDVLLVPAHSTVLPRDVKLQTKLTREITLNLPLLSAAMDTVTEARLAISMAQEGGIGIIHKNMPPE MQARAVSKVKRHESGVVKDPVTVAPTTLIREVLEMRAQRKRKMSGLPVVENGKVVGIVTNRDLRFENRVDLPVSAI MTPRERLVTVPEGTSIDEARELMHTHKVERVLVLNEKDELKGLITVKDILKTTEFPNANKDSEGRLRVGAAVGTGG DTEERVKALVEAGVDVIVVDTAHGHSQGVIDRVRWVKETYPHIQVIGGNIATAKAALDLVAAGADAVKVGIGPGSI CTTRIVAGVGVPQLTAIHNVAEALKGTGVPLIADGGIRFSGDIAKALAAGAYSVMLGGMFAGTEEAPGEIELYQGR SYKSYRGMGSLGAMSQGSADRYFQDKTDSTDKYVPEGIEGRVPYKGPIVNIIHQLTGGLRSSMGYLGCANIAEMHE KAEFVEITSAGMSESHVHDVQITKEAPNYHR |
| 113 | 1206 | MKGDRLVIRELNKNLGLLLVTINQYFLHARILKNWGFEELGEHFFKQSIVEMKAADDLIERILFLEGLPNLQELGK LLIGESTEEIIACDLTKEQEKHEALLAAIATAEAQQDYVSRDLLEKQKDTNEEHIDWLETQQELIGKIGLPNYLQT AAQED |
| 114 | 1228 | MKPVNIGLLGLGTVGGGTAAVLRDNAEEISRRLGREIRISAVCDLSEEKARQTCPSAAFVKDPFELVAREDVDVVV ELFGGTGIAKDAVLKAIENGKHIVTANKKLLAEYGNEIFPLAEKQNVIVQFEAAVAGGIPIIKALREGLAANRIKS IAGIINGTSNFILSEMREKGSAFADVLKEAQALGYAEADPTFDIEGNDAGHKITIMSALAFGTPMNFSACYLEGIS KLDSRDIKYAEELGYRIKLLGITRKTGKGIELRVHPTLIPESRLLANVNGVMNAVRVNADMVGETLYYGAGAGALP TASAVVADIIDIARLVEADTAHRVPHLAFQPAQVQAQTILPMDEITSSYYLRVQAKDEPGTLGQIAALLAQENVSI EALIQKGVIDQTTAEIVILTHSTVEKHIKSAIAAIEALDCVEKPITMIRMESLHD |

EP 1 562 982 B1

| SED ID | NMB | Sequence |
|--------|-----|----------|
| 115 | 1231 | MTQKEKHFEEYAALATLPLRDVVVYPHMVLPLFVGRPKSIAALENAITREEPVFLLAQTDAAVEEPIAADLYQTGT VAQVLQVLKLPDGTVKVLVEGLYRGRVLTIEDTGGLFVSHIETVVEEDTGGNTDLEAVRRTLLAQFEQYAKLNKKI PAEIIGSINGIAENSRLTDTVAAHLQLKLAQRQQILEIPEIGKRMEFLLAQLESELDIMQAEKRIRGRVKRQMEKS QREYYLNEQIKAIHKELGEEDENGELDALEADIKKAGMTKEAEEKCLSELKKLKMMPPMSAESTVVRNYIDTLLEL PWKKKSRVSKDIAKAGLVLDADHYGLEKVKERILEYLAVQKRMDKLKGPILCLVGPPGVGKTSLGESIAKATGRKY VRMALGGVRDESEIRGHRRTYIGSMPGKILQNMAKAGVKNPLFLLDEIDKLGNDFRGDPASALLEVLDPEQNNKFA DHYAEVDYDLSDVMFIATSNSLNIPTPLLDRMEIIRLSGYTEDEKINIAMQYLVPKQMKRNGVKEGELAIEESAVR DIIRYYTREAGVRSLDREIAKICRKVVMQITLDEDKKRLSETKKTSKAKPKAVKVNEKNLHDYLGVRRFDYGVAES ENRIGQVTGLAWTEVGGELLTVEAAALPGKGVIQCTGQLGDVMKESVSAAWSVVRSRAESVGLAPDFYEKKDIHIH VPEGATPKDGPSAGIAMTLAAVSAFTKIPVRADVAMTGEITLRGEVLPIGGLKEKLLAALRGGIKHVLIPKDNVKD LEEIPENVKTGLTIHPVKWIDEVLALGLESQPEPWAEPSGAEAAAESASKPKPRSRATKH |
| 116 | 1240 | MISTNGITMQFGAKPLFENVSVKFGEGNRYGLIGANGSGKSTFMKILGGDLEQTAGEVAIENGVRLGKLRQDQFAY EDMRVLDVVMMGHTEMWAAMTERDAIYANPEATEDDYMKAAELEAKFAEYDGYTAEARAAELLSGVGISEDLHNAK MAEVAPGFKLRVLLAQALFSKPDVLLLDEPTNNLDINTIRWLEGVLNQYDSTMIIISHDRHFLNEVCTHMADLDYN TITIYPGNYDDYMLASAQSRERALKDNAKAKEKLQELQEFVARFSANKSKARQATSRLKQADKIKSEMVEVKPSTR QNPYIRFEADEKAKLHRQAVEVEKLAKRFETQLFKNLNFILEAGQRLAIIGPNGAGKSTLLKLLAGAYNPEYSDGL LPDEGTIKWAEKASVGYYPQDHENDFDVDMDLSEWMRQWGQEGDDEQVIRGTLGRLLFGSNDVVKKVKVLSGGEKG RMLYGKLLLLKPNVLVMDEPTNHMDMESIESLNMALEKYNGTLIFVSHDRQFVSSLATQIIELDGKGGYEHYLGDY ESYLEKKGVA |
| 117 | 1252 | MSTSLSYRDAGVDIDAGDQLVENIKPFAKRTMRPEVLGDLGGFGALVEIGKKYQNPVLVSGTDGVGTKLKLAFDWD KHDTVGIDLVAMSVNDILVQGAEPLFFLDYFACGKLDVPRATDVIKGIAQGCEESGCALIGGETAEMPGMYPVGEY DLAGFAVGVVEKENVITGRSIGVGDVVLGLASNGAHSNGYSLIRKIIERDNPDLDAEFDNGKTLREAVIAPTRLYV KPILAALEKFTIKGMAHITGGGITENVPRVLPENTVAQIDAKSWELPKLFQWLQKAGNVETQEMYRTFNCGIGMVV IVAAEDADAVQGLLGEQGETVYRLGLIRERQGDEHQTQVA |

EP 1 562 982 B1

| SED ID | NMB | Sequence |
|---|---|---|
| 118 | 1301 | MSMENFAQLLEESFTLQEMNPGEVITAEVVAIDQNFVTVNAGLKSESLIDVAEFKNAQGEIEVKVGDFVTVTIESV ENGFGETKLSREKAKRAADWIALEEAMENGDILSGIINGKVKGGLTVMISSIRAFLPGSLVDVRPVKDTSHFEGKE IEFKVIKLDKKRNNVVVSRRAVLEATLGEERKALLENLQEGSVIKGIVKNITDYGAFVDLGGIDGLLHITDLAWRR VKHPSEVLEVGQEVEAKVLKFDQEKQRVSLGMKQLGEDPWSGLTRRYPQGTRLFGKVSNLTDYGAFVEIEQGIEGL VHVSEMDWTNKNVHPSKVVQLGDEVEVMILEIDEGRRRISLGMKQCQANPWEEFAANHNKGDKISGAVKSITDFGV FVGLPGGIDGLVHLSDLSWTESGEEAVRKYKKGEEVEAVVLAIDVEKERISLGIKQLEGDPFGNFISVNDKGSLVK GSVKSVDAKGAVIALSDEVEGYLPASEFAADRVEDLTTKLKEGDEVEAVIVTVDRKNRSIKLSVKAKDAKESREAL NSVNAAANANAGTTSLGDLLKAKLSGEQE |
| 119 | 1302 | MTKSELMVRLAEVFAAKNGTHLLAKDVEYSVKVLVDTMTRSLARGQRIEIRGFGSFDLNHRPARIGRNPKTGERVE VPEKHVPHFKPGKELRERVDLALKENAN |
| 120 | 1313 | MMSVTVETLENLERKVVLSLPWSEINAETDKKLKQTQRRAKIDGFRPGKAPLKMIAQMYGASAQNDVINELVQRRF YDVAVAQELKVAGFPRFEGVEEQDDKESFKVAAIFEVFPEVVIGDLSAQEVEKVTASVGDAEVDQTVEILRKQRTR FNHVEREARNGDRVIIDFEGKIDGEPFAGGASKNYAFVLGASQMLPEFEAGVVGMKAGESKDVTVNFPEDYHGKDV AGKTAVFTITLNNVSEATLPEVDADFAKALGIADGDVAKMREEVQKNVSREVERRVNEQTKESVMNALLKAVELKA PVALVNEEAARLANEMKQNFVNQGMADAANLDLPLDMFKEQAERRVSLGLILAKLVDENKLEPTEEQIKAVVANFA ESYEDPQEVIDWYYADPSRLQAPTSLAVESNVVDFVLGKAKVNEKALSFDEVMGAQA |
| 121 | 1320 | MQIILLEKIGGLGNLGDIVTVKNGYARNFLIPAGKAKRATEANMKEFEARRAELEAKQAEILADARVRQEKLDGQT VTVAQKAGVDGRLFGSVTNADIAAAIVAAGIEAVKANVRLPNGPLKAVGEYEVEVALHTDAVAKITVAVVAATE |
| 122 | 1323 | MRHYEIVFIVHPDQSEQVPAMVERYKTMIAEANGKIHRLEDWGRRQLAYPINKIHKAHYVLMNIETTPEVVEELET AFRFNDAILRHLTIKTKHAVTEASPMLGGEKAKNLLSGASEEAVAQ |

EP 1 562 982 B1

68

(continued)

| SED ID | NMB | Sequence |
|---|---|---|
| 123 | 1324 | MSQHRKLIILGSGPAGYTAAVYAARANLNPVIITGIAQGGQLMTTTEVDNWPADADGVQGPELMARFLAHAERFGT<br>EIIFDQINAVDLQKRPFTLKGDMGEYTCDALIVATGASAKYLGLPSEEAFAGKGVSACATCDGFFYKNQDVAVVGG<br>GNTAVEEALYLANIAKTVTLIHRRSEFRAEKIMIDKLMKRVEEGKIILKLESNLQEVLGDDRGVNGALLKNNDGSE<br>QQIAVSGIFIAIGHKPNTDIFKGQLEMDEAGYLKTKGGTADNVGATNIEGVWAAGDVKDHTYRQAITSAASGCQAA<br>LDAERWLGSQNI |
| 124 | 1328 | MTDTAENQTQNNWQAGHPRSIRSFVLRQSHMTAAQQRAIDTLWDSFGIDYQATPADLDARFGSSRPKILEIGFGMG<br>TATAEIARRLPETDFLAIDVHGPGVGNLLKLIDENHLENIRVMRHDAVEVVENMLQDGSLDGIHIFFPDPWHKKRH<br>HKRRLIQAPFIAKLLPKLKTGGYIHLATDWEEYAQQMLEVLSSFDSLQNTAADYAPTPDYRPETKFEARGKRLGHG<br>VWDLVFKRIG |
| 125 | 1339 | MKASQFFISTLKEAPAEAALASHKLMIRAGLIKANASGLYTWMPMGLRVLRKVENVVREEMARAGSVELLMPVVQP<br>AELWQESGRWEFYGKELLRLKDRHDRDFCMGPTCEEVIADIVRKEINSYKQLPKNFYHIQTKFRDEVRPRFGVMRA<br>REFVMKDAYSFHADYASLQTTYQDMYDAYCRIFTRLGLAFRPVAADTGSIGGTGSHEFQVLAESGEDVIAYSDTSD<br>YAANIELAPTLPLKGERAAAQAELVKVHTPNVKTIDSLVDFLSIPIEKTLKSIVVEGENEGELILLLLRGDHEFND<br>IKAEKLAGVKSPLTMASPAAIVEQFGANGGSLGPVGFAGKVYADFATEKGADWVIGANEDDYHYTGFNFGRDAAEP<br>EFVDLRNVVEGDESPDGQGRLKLARGIEVGHVFQLRDKYTQAMNVSFLDNNGKSQIMEMGCYGIGITRVVAAAIEQ<br>NNDEKGIIWTKAMAPFEVVIVPMNYKKSDTVREAADKIYAELLAAGADVLLDDRDERAGVLLNDSELLGIPHRIVI<br>GDRALKEGNVEYAERRDNEAQAVAIGEIVARVTASLNA |

| SED ID | NMB | Sequence |
|---|---|---|
| 126 | 1341 | MSTQLHDVDPIETQEWLDALSSVLEYEGGERAQYLLENLVKYCRDKGVRMPHGTTTPYLNTVSVENEKGIPGDQNI EHRIRAFVRWNAAAIVLRAGKKDLELGGHIASFQSAATMYEVGFNHFWKAKGEGEEGDLVFFQGHVAPGIYARAFV EGRLTEDQLNNFRQEVDGHGLPSYPHPHLLPDFWQFPTVSMGLGPIMAIYQARFLKYLESRGLAKTKGRKVWCFCG DGEMDEPESQGAIALAAREGLDNLIFVINCNLQRLDGPVRGNGKIIQELEGNFAGAGWNVVKVIWGRRWDRLLAKD KDGILRQRMEECLDGDYQTYKSKDGAYVREHFFNTPELKALVADMTDEQLWALNRGGHDPQKVYNAYDRAANHADG KPTVILAKTIKGYGMGASGEGQNVAHQAKKMDKASLKQFRDRFDIPVTDEQIESGDLPYLTFAPDTEEYKYLHARR DALGGYLPQRKPTQEVLEVPELSAFDAQLKSSGEREFSTTMAFVRILSTLLKDKKIGKRVVPIVPDESRTFGMEGM FRQYGIWNPKGQQYTPQDKDQLMFYKESVDGQILQEGINEPGAMADWIAAATSYANSNFAMIPFYIYYSMFGFQRI GDLAWAAGDMHARGFLLGGTAGRTTLNGEGLQHEDGHSHIQADLIPNCVSYDPTFQYEVAVIVQDGLRRMYANNED VFYYITLMNENYTHPDMPEGAEQDILKGMYLLKAGGKGDKKVQLMGSGTILQEVIAGAELLKADFGVEADIWSCPS FNLLHRDAVEVERFNRLHPLEAEKVPFVTSQLQGHDGPVIAATDYIRSYADRIRAYIPNDYHVLGTDGFGRSDSRA NLRRFFEVDRYNVAVAALAALAEQGKVSKETVQQAIEKYGIKADSAPSWKR |
| 127 | 1342 | MSIVEIKVPDIGGHENVDIIAVEVKAGDTIAVDDTLITLETDKATMDVPADAAGVVKEVKVKVGDKISEGGVILTV ETGAAAAEAAPAAAEAQPAPAAAPAAAGGATVQVAVPDIGGHTDVDVIAVEIKVGDTVAEDDTLITLETDKATMDV PCTAAGVVKAVFLKVGDKVSEGSAIIEVETVGSAAAAPAQAAQAAAPAAAPPPTAAAAPAAAPAPSAPAAAKIDEA AFAKAHAGPSARKLARELGVDLGQVKGTGLKGRIMGDDIKAFVKSVMQGGAAKPAAASASLGGGLDLLPWPKVDFS KFGNVEVKELSRIKKISGQNLSRNWVVIPHVTVHEEADMTELEEFRKQLNKEWEREGVKLSPLAFIIKASVSALKA FPEFNASLDGDNLVLKNYFNIGFAADTPNGLVVPVIKDVDQKGLKQISQELTELSKKAREGKLKPQEMQGACFTIS SLGGIGGTGFTPIVNAPEVAILGVCKSQIKPVWNGKEFAPRLMCPLSLSFDHRVIDGAAGMRFTVFLAKLLKDFRR ITL |
| 128 | 1343 | MGNFLYRGISCQQDEQNNGQLKPKGNKAEVAIRYDGKFKYDGKATHGPSVKNAVYAHQIETGLYDGCYISTTTDKE IAKKFATSSGIENGYIYVLNRDLFGQYSIFEYEVEHPENPNEKEVTIRAEDCGCIPEEVIIAKELIEIN |

EP 1 562 982 B1

70

| SED ID | NMB | Sequence |
|---|---|---|
| 129 | 1344 | MALVELKVPDIGGHENVDIIAVEVNVGDTIAVDDTLITLETDKATMDVPAEVAGVVKEVKVKVGDKISEGGLIVVV EAEGTAAAPKAEAAAAPAQEAPKAAAPAPQAAQFGGSADAEYDVVVLGGGPGGYSAAFAAADEGLKVAIVERYKTL GGVCLNVGCIPSKALLHNAAVIDEVRHLAANGIKYPEPELDIDMLRAYKDGVVSRLTGGLAGMAKSRKVDVIQGDG QFLDPHHLEVSLTAGDAYEQAAPTGEKKIVAFKNCIIAAGSRVTKLPFIPEDPRIIDSSGALALKEVPGKLLIIGG GIIGLEMGTVYSTLGSRLDVVEMMDGLMQGADRDLVKVWQKQNEYRFDNIMVNTKTVAVEPKEDGVYVTFEGANAP KEPQRYDAVLVAAGRAPNGKLISAEKAGVAVTDRGFIEVDKQMRTNVPHIYAIGDIVGQPMLAHKAVHEGHVAAEN CAGHKAYFDARVIPGVAYTSPEVAWVGETELSAKASGRKITKANFPWAASGRAIANGCDNGFTKLIFDAETGRIIG |
| | | GGIVGPNGGDMIGEVCLAIEMGCDAADIGKTIHPHPTLGESIGMAAEVALGTCTDLPPQKKK |
| 130 | 1347 | MNPFLNTAFKAARRAGQMMIRAAGNLDAVKTDSKAFNDFVSDVDRNSEIILVEALKEAYPHHKITCEESGSHGKAA AEYEWIIDPLDGTTNFLHGHPQYAISMALLHKGVLQEALVYAPERNDVYMASRGKGALLNDRRIRVSNRIELNRCL IGTGFPVVDQSMMDKYLAILKDFLAKTAGGRREGAASLDLCAVATGRFDGFFEFNLKPWDIAAGALIVQEAGGIVT DMSGEDGWLESGDIVAANPKVLAQMLKIISAHV |
| 131 | 1356 | MTQYTLKQASVLLQSKQISAVELASAYLAAIAEKNPALNGYITIDQDKTLAEARAADERIAQGNASALTGVPVAYK DIFCQTGWRSACASKMLDNFISPYTATVVQNLLDEGMVTLGRTNMDEFAMGSTNENSFYGAAKNPWNLEHVPGGSS GGSAAVVAARLAPAALGSDTGGSIRQPASHCGITGIKPTYGTVSRFGMVAYASSFDQTGPMAQTAEDCAILLNAMA GFDPKDSTSLEREKEDYTRDLNQPLKGLKIGLPKEYFGEGNSADVLTALQNTIDLLKAQGAELIEVSLPQTKLSIP AYYVLASAEASTNLSRYDGVRYGHRAAQFADLEEMYGKTRAEGFGSEVKRRIMIGTYVLSHGYYDAYYLKAQKLRR LVADDFQTAFARCDLILAPTAPTAAPKIGADASPVETYLSDIYTIAVNLAGLPALTLPAGFSGGGLPVGVQLVGNY FAEAKILGAAHQIQLNSDWHGKRPE |
| 132 | 1358 | MTWETVIGLEIHVQLNTKSKIFSGASTAFGAEPNAHASVVECALPGVLPVMNREVVEKAIKLGLALDAKINQKNVF DRKNYFYPDLPKGYQISQLDLPIVEHGKLEIVVGDDVKTINVTRAHMEEDAGKSVHEGLNGATGIDLNRAGTPLLE VVSEPEMRSAAEAVAYAKALHSLVTWLDICDGNMAEGSFRVDANVSVRPKGQEEFGTRREIKNLNSFRFLEQAINY EAEAQIEILEDGGKVQQATMLFDPEKGETRVMRLKEDAHDYRYFPDPDLLPVIISDAQMQKAKAEMPELPKEMAAR FVADYGVSEYDARLLTASRAQAAYFEEAAKESGQGKLTANWMNGELAAALNKEGMELADSPITAPRLAALVGKIAD GTLSSKLAKKAFEAMWAEPEATIAEIIEKHGLQQMTDTGEIEAMVDEVLANNAKAVEQFKSGNEKALNAIVGQVMK ASKGKANPAQVQELIKAKLA |

EP 1 562 982 B1

71

| SED ID | NMB | Sequence |
|---|---|---|
| 133 | 1372 | MSNENRTCSFCGKSKSHVKHLIEGENAFICDECVSNCIEILHEDGNDGTPSESAGGEPEESGKLPTPAEIVANLND HVIGQEQAKKALAVSVYNHYKRLRHPKAGANVELSKSNILLIGPTGSGKTLLAQSLARKLDVPFVMADATTLTEAG YVGEDVEQIITKLLGKCDFDVEKAQRGIVYIDEIDKISRKSDNPSITRDVSGEGVQQALLKLIEGTVASVPPQGGR KHPNQEFINVDTTNILFICGGAFAGLEKVIRQRTEKGGIGFGASVHSKDENADITKLFGIVEPEDLIKFGLIPELI GRLPVIATLEELDEDALINILTEPKNALVKQYQALFGMENVELEFEEGALRSIARQAMERKTGARGLRSIVERCLL DTMYRLPDLKGLKKVVVGKAVIEEGREPELVFES |
| 134 | 1379 | MTVKTPVYLDYAATPPVDKRVAEKMIPYLTETFGNPASNSHSFGWEAEEAVEKARADIAALINADSKEIVFTSGAT ESNNLAIKGAAHFYKSKGNHLITVKTEHKAVLDTMRELERQGYEVTYLDVQENGLVDLDVLKAAIREDTILVSVMW VNNEIGVVQDIPAIGEICRERKIIFHVDAAQACGKVPVDVEAAKVDLLSMSGHKVYGPKGIGALYVRRKPRVRLEA QMHGGGHERGFRSGTLPTHQIVGMGEAFRIAKEELAQDTAHYLKLRDIFLKGIEGIEEVYINGDLEHRVPNNLNVS FNFVEGESLIMAVKELAVSSGSACTSASLEPSYVLRALGRNDELAHSSLRITFGRMTTEEEVQFAAELIKSKIGKL RELSPLWEMFKDGIDLNSIEWAAH |
| 135 | 1390 | MSSTPNKQAGYPRLVADIGGTNARFALETAPRVIEKAAVLPCKDYDTVTDAVRAYLNQSGATAVRHAAFAIANPIL GDWVQMTNHHWAFSIETTRQTLGLDTLILLNDFTAQALAVTQTSSKDLMQVGGQKPVEFAPKAVIGPGTGLGVSGL VHSHAGWVALAGEGGHTSFPPFDDMEVLIWQYAKNKYGHVSAERFLSGAGLSLVYEALAAKQKAKPAKLMPSEITE KALSGASPLCRQTLDIFCAMLGTVASNLALTLGARGGVYLCGGIIPRVLEYFKTSPFRSRFENKGRFEAYLAAIPV YVVLSEFPGISGAAAALDNHLRNV |
| 136 | 1392 | MSTQTNFDLVLFGATGDLAMRKLLPCLYQAHVAGLLHPEGRILGVSRSELDTEGFLAKVETSSKIHVKENFSDEAW ASFVERFAYLKVDVTQPDDFAALGDLVKARKETDNVVIYLSTAPKFFAQACENLAAIGLNADNVRVVLEKPLGTDL ASSQQINTDVARYFKEGQIYRIDHYLGKESLQNLLALRFANVMFEPLWNNKYIESVQLTIAEQLGVEERGEFYDIT GALRDMVQNHLMQMLCMTAMEAPASLDADAVRDEKVKVIKSLKPLTVESVNENVVRGQYTAARGMNGYLEEINVPQ DSFTETYVAIKAEIENERWKGVPFYLRTGKRMAGKVAEIVLNFKDLNSHIFEGSRTAPNRLVIELQPYESVRLYTQ MKTPGAGNKVETVPLATDLGKALEGRRAEAYERLLLDVINGKLALFNRRDELEAAWEYVMPILENWTNNTTPPHGY GAHSWGPEAARELLARDGHKWHEEQ |

| SED ID | NMB | Sequence |
|---|---|---|
| 137 | 1425 | MSEQNHPQTEPQLDENQIIALRREKLHNIRQQRNAYPNDFKRDSFAADLHAQYGEIGKEELDPQGIPVKVAGRMML KRQMGKASFATIQDVSGQIQLYLNNKGVSQEVLDDFNHWDLGDIVGAEGTLFKTNHGELTVRVSGIRLLSKSLRPL PDKHKGLSDQETKYRQRYVDLIANEESRNTFIKRSQIIQSVRNFMVGEHYLEVETPMMHPIPGGATAKPFVTHHNA LDIPLYLRIAPELYLKRLVVGGLERVFEINRSFRNEGMSVRHNPEFTMIEFYEAFSDYERMMQMAEDIIRNASRTV NGTANITYNGKEVDLESPFERLTILEAIKKYNPHYTDEQLNDAEWLKKEIVKHGESLPPSPGIGSLQLALFEGCAE GKLWNPTFIVDYPVEVSPLARASDTKQGLTERFELFVVGRELANGYSELNDPEDQAERFKAQVVQKDAGDDEAMHY DADYIRAMEFGLPPTGGCGIGIDRLVMLLTDSQTIRDVILFPQMRPE |
| 138 | 1429 | MRKKLTALVLSALPLAAVADVSLYGEIKAGVEGRNYQLQLTEAQAANGGASGQVKVTKVTKAKSRIRTKISDFGSF IGFKGSEDLGDGLKAVWQLEQDVSVAGGGATQWGNRESFIGLAGEFGTLRAGRVANQFDDASQAIDPWDSNNDVAS QLGIFKRHDDMPVSVRYDSPEFSGFSGSVQFVPIQNSKSAYTPAYYTKNTNNNLTLVPAVVGKPGSDVYYAGLNYK NGGFAGNYAFKYARHANVGRNAFELFLIGSGSDQAKGTDPLKNHQVHRLTGGYEEGGLNLALAAQLDLSENGDKTK NSTTEIAATASYRFGNAVPRISYAHGFDFIERGKKGENTSYDQIIAGVDYDFSKRTSAIVSGAWLKRNTGIGNYTQ INAASVGLRHKF |
| 139 | 1445 | MSDDKSKALAAALAQIEKSFGKGAIMKMDGSQQEENLEVISTGSLGLDLALGVGGLPRGRIVEIFGPESSGKTTLC LEAVAQCQKNGGVCAFVDAEHAFDPVYARKLGVKVEELYLSQPDTGEQALEICDTLVRSGGIDMVVVDSVAALVPK |
| | | AEIEGDMGDSHVGLQARLMSQALRKLTGHIKKTNTLVVFINQIRMKIGVMFGSPETTTGGNALKFYSSVRLDIRRT GSIKKGEEVLGNETRVKVIKNKVAPPFRQAEFDILYGEGISWEGELIDIGVKNDIINKSGAWYSYNGAKIGQGKDN VRVWLKENPEVANEIDAKIRALNGVEMHITEGTQDETDGERPEE |
| 140 | 1452 | MQNIFDPLVIRGKSLTPIVQGGMGVGVSASGLSSAVARENGIGTIASVDLRHLHEDLLAESQINPSEEKYTSLNCT ALDREIQKAKSASEGKGLIAVNVMKAVKDHAAYVRQACESGADAVVMGAGLPLDLPEMTEGYHKDVALLPILSESR GINIVLKRWMKKGILPDAIVVEHPAHAAGHLGASTVEGVNDAKFDFKRVIEETFEVFKSLGLESEKIPLILAGGMA NFEKVKTALKNWGASAVQIGTAFAVTEEGDAHLNFKKTLAGAETEKVVEFMSVAGLPARGVRTKFLDSYIKRESKL QTNAKADPRRCTQGLNCLTSCGLRDGLSKAGQFCIDIQLAAAFRGEVDKGLFFRGKDRCPSAMPSAPSARRYNIC |

EP 1 562 982 B1

73

(continued)

| SED ID | NMB | Sequence |
|---|---|---|
| 141 | 1457 | MSQLANAIRFLSADAVQKANSGHPGAPMGMAEMAETLWTKFLNHNPANPKFYNRDRFVLSNGHASMLLYSLLHLTG YNLSIEDLKNFRQLHSKTPGHPEYGYTDGVETTTGPLGQGIANAVGMALAEKILAAEFNKDGLNIVDHYTYVFMGD GCLMEGVSHEACSLAGTLGLGKLIVLYDDNNISIDGKVDGWFTENIPQRFESYGWHVVPNVNGHDTAAIQAAIEAA RAETGKPSIICCKTLIGKGSANKEGSHKTHGAPLGADEIEATRKHLGWTYPAFEIPQEIYDAWNAKEQGAKLEADW NELFAQYQAKYPAEAAEFVRRMDKKLPDNFDEYVQAALKEVCAKAETIATRKASQNSIEILAKELPELVGGSADLT PSNLTDWSNSVSVTRDKGGNYIHYGVREFGMGAIMNGLVLHGGVKPFGATFLMFSEYERNALRMAALMKINPVFVF THDSIGLGEDGPTHQPIEQTATLRLIPNMDVWRPCDTAESLVAWAEAVKAADHPSCLIFSRQNLKFQARSEQQLND IKRGGYVISEAQGNAQAVIIATGSEVELALEAQKALAAQNIAVRVVSMPSTNVFDRQDAAYQAAVLPEGLPRIAVE AGHADGWYKYVGLNGAVVGINRFGESAPADLLFKAFGFTVDNVVDTVKSVL . |
| 142 | 1471 | MSKKRVLTGVTTTGIPHLGNYVGAIRPAVRAAQNLDTESFLFLADYHGIIKCHEPEMIHQSTQAVAATWLACGLDP ERTTFYRQSDTPEVMELNWILTCITAKGLMNRAHAYKAAVQANAENGQEDPDFGVEMGLFSYPILMTADILMFNAN EVPVGRDQIQHVEMARDIAGRFNHRFRELFTLPEVKIDENVELLVGLDGRKMSKSYGNTIPLWENDKKTQKSVNKI ITNMKEPGEPKQPDESPLFEIYKAFSTPSETVEFTKMLADGLAWGEAKKLLAAKINAELAEPRERYNELTADPSQI EEILQAGAAKARKEARELLDKVRDAVGIRPLK |
| 143 | 1472 | MRYDKLTAKFQQALAEAQSLALAADGSYLEAGFVLKALLDDQNSGAAALLAHAGVNVPQVKQRLQQHLNSLPKVSG QGGDILPSRELQAVLNLMDKAATKRSDAYIASELFLLALVQQNDATGKILKEAGATEQNINAAIDAVRGGQNVNDA NAEDQRDALKKYTLDLTQRARDGKLDPVIGRDDEIRRAIQVLQRRTKNNPVLIGEPGVGKTAIVEGLAQRIVNGEV PESLRNKRLLVLDLAALIAGAKYRGEFEERLKGVLNDLAKDDGNTLIFIDEIHTLVGAGKTDGAMDAGNMLKPALA RGELHCIGATTLDEYRQYIEKDAALERRFQKVLVGEPSVEDTIAILRGLQERYEIHHGIDITDPAIVAAAELSDRY ITDRFLPDKAIDLIDEAASRVKMEKETKPEAMDKIDRRLIQLRMEKAHVEKEKDDASKKRLELIDEEINGLQKEYA DLDEIWKAEKAISDGAANIKKQIDEVKIKIEQAKRQGDLALASKLMYEDLEHLEKQRAAAERADTDSTKPANKLLR NNVGAEEIAEVVSRMTGIPVSKMMEGERDKLLKMEEVLHRRVVGQDEAVRAVSDAIRRSRSGLADPNKPYGSFLFL GPTGVGKTELCKALAGFLFDSEDHLIRIDMSEYMEKHAVARLIGAPPGYVGYEEGGYLTEQVRRKPYSVILLDEVE KAHPDVFNILLQVLDDGRLTDGQGRTVDFKNTVIVMTSNIGSQHIQQMGIQDYEAVKEVVMEDVKEHFRPEMINRI DEVVVFHGLDQDNIRNIAKIQLKGLEKRLEKQNLRLAVSDAALDIIAKAGFDPIYGARPLKRAIQSEIENPLAKAL LAGNYAPESEIRVEADGDRLKFA |

EP 1 562 982 B1

74

| SED ID | NMB | Sequence |
|---|---|---|
| 144 | 1506 | MNLHQTVEHEAAAAFAAAGIADSPIVLQPTKNAEHGDFQINGVMGAAKKAKQNPRELAQKVAEALADNAVIESAEV<br>AGPGFINLRLRPEFLAQNIQTALNDARFGVAKTDKPQTVVIDYSSPNLAKEMHVGHLRSSIIGDSISRVLAFMGNT<br>VIRQNHVGDWGTQFGMLVAYLVEQQKDNAAFELADLEQFYRAAKVRFDEDPAFADTAREYVVKLQGGDETVLALWK<br>QFVDISLSHAQAVYDTLGLKLRPEDVAGESKYNDDLQPVVDDLVQKGLAVEDDGAKVVFLDEFKNKEGEPAAFIVQ<br>KQGGGFLYASTDLACLRYRIGRLKADRLLYVVDHRQALHFEQLFTTSRKAGYLPENVGAAFIGFGTMMGKDGKPFK<br>TRSGDTVKLVDLLTEAVERATALVKEKNPELGADEAAKIGKTVGIGAVKYADLSKNRTSDYVFDWDAMLSFEGNTA<br>PYLQYAYTRVQSVFRKAGEWDANAPTVLTEPLEKQLAAELLKFEDVLQSVADTAYPHYLAAYLYQIATLFSRFYEA<br>CPILKAEGASRNSRLQLAKLTGDTLKQGLDLLGIDVLDVM |
| 145 | 1518 | MSQKLILVLNCGSSSLKGAVLDNGSGEVLLSCLAEKLNLPDAYITFKVNGEKHKVDLSAHPDHTGAVEALMEELKA<br>HGLDSRIGAIGHRVVSGGELYSESILVDDEVIAGIEKCIPLAPLHNPAHLLGLRAAQSIFKGLPNVVVFDTSFHQT<br>MPEVAYKYAVPQELYEKYGLRRYGAHGTSYRFVADETARFLGKDKKDLRMVIAHLGNGASITAVANGESRDTSMGL<br>TPLEGLVMGTRSGDIDPSVFGFLAENANMTIAQITEMLNKKSGLLGISGLSNDCRTIEEEAAKGHKGAKLALDMFI<br>YRLAKYIGSMAVAAGGLDALVFTGGIGENSDIIRERVIGYLGFLGLNIDQEANLKARFGNAGVITTADSKAVAVVI<br>PTNEELMIAHDTARLSGL |
| 146 | 1533 | MKAYLALISAAVIGLAACSQEPAAPAAEATPAAEAPASEAPAAEAAPADAAEAPAAGNCAATVESNDNMQFNTKDI<br>QVSKACKEFTITLKHTGTQPKASMGHNLVIAKAEDMDGVFKDGVGAADTDYVKPDDARVVAHTKLIGGGEEASLTL<br>DPAKLADGEYKFACTFPGHGALMNGKVTLVD |
| 147 | 1536 | MLTNIAKKIFGSRNDRLLKQYRKSVARINALEEQMQALSDADLQAKTAEFKQRLADGQTLDGILPEAFAVCREASR<br>RTLGMRHFDVQLIGGMVLHDGKIAEMRTGEGKTLVATLAVYLNALAGKGVHVVTVNDYLASRDAGIMEPLYNFLGL<br>TVGVIISDMQPFDRQNAYAADITYGTNNEFGFDYLRDNMVTDQYDKVQRELNFAVVDEVDSILIDEARTPLIISGQ<br>ADDNIQLYQIMNTVPPHLVRQETEEGEGDYWVDEKAHQVILSEAGHEHAEQILTQMGLLAENDSLYSAANIALMHH<br>LMAALRAHSLFHKDQHYVIQDGEIVIVDEFTGRLMSGRRWSEGLHQAVEAKEGVEIKRENQTLASITFQNYFRLYT<br>KLSGMTGTADTEAFEFQSIYNLETVIIPTNRPVQRKDFNDQIFRSAEEKFEAVVKDIEECHKRGQPVLVGTTSIEN<br>SELVSKLLTQAGLPHNVLNAKEHEREALIVAQAGKVGAITVATNMAGRGTDIVLGGNLKHQTDAIRADETLSDEEK<br>QAQIAALEDGWQAEHDKVMEAGGLHIIGTERHESRRIDNQLRGRSGRQGDPGSSRFYLSFEDPLLRLFALDRAAAI |

EP 1 562 982 B1

| SED ID | NMB | Sequence |
|--------|-----|----------|
|  |  | LNRLAPERGVAIEHNLLTRQIEGAQRKVEGRNFDMRKQVLEYDDVANEQRKVIYSQRNEILTSKDISDLMQEIRSD VVSDLVDTYMPPDSMEEQWDIPTLENRLAAEFRLHEDIQSWLKADNAIDGQDIKERLIERIENEYAAKTELVGKQA MADFERNVMLQVIDNQWREHLAAMDYLRQGIHLRSYAQKNPKQEYKREAFTMFQDLWNGIKFHIASLLTSVQIEQN PVAVVEEQPIGNIQSIHSESPDMEELLGQSQTDLVTEAFNPDGTDFSPEALEARGQIVHRNDPCPCGSGLKYKQCH GKLA |
| 148 | 1560 | MLNKDQFADNHFIRTIIEEDLESGKHTAVQTRFPPEPNGYLHIGHAKSICLNFGLAYIYDGLCNLRFDDTNPEKEN DEYVNAIKEDVEWLGFHWAGEPRFASNYFDQLYDYAVGLIKDGKAYVDDLTPEEMREYRGTLTEAGKNSPYRDRSV EENLDLFTRMKNGEFPDGSKTLRLKIDMASGNINMRDPVIYRIRRAHHHNTGDKWCIYPMYDYTHCISDAIEGITH SLCTLEFEAHRPLYDCVLDNIPAPHATRPRQYEFSRLELLYTITSKRKLNQLVVEKHVSGWDDPRMPTISGMRRRG YTPEGLRLFAKRAGISKSENIVDMSVLEGAIREELENSAPRLMAVLNPLKVTLTNFETGRTQSRRAAFHPNHEEMG EREVPISQTIYIEADDFAENPPKGFKRLIPGGEVRLRHGYVIKCDEVVKDEAGNVVELKCSIDHDTLGKNPEGRKV KGVIHWVSAEHAAEIKVRLYDRLFTVERPDAVRGEDGEYLPFTDFLNPESVKEITAYAEPAAKDLPAESRWQFERI GYFVTDRKDHGKDTPVFNRTVTLKDSWQPK |
| 149 | 1572 | MLEAYRKAAAERAALGIPALPLNAQQTADLVELLKSPPAGEGEFLVELLAHRVPPGVDDAAKVKASFLAAVAEGSA SSPLISPEYATELLGTMLGGYNIHALIELLDDDKLASIAAKGLKHTLLMFDSFHDVQEKAEKGNKYAQEVLQSWAD AEWFASRAKVPEKITVTVFKVDGETNTDDLSPAPDAWSRPDIPLHALAMLKNPRDGITPDKPGEVGPIKLLEELKA KGHPVAYVGDVVGTGSSRKSATNSVIWHTGEDIPFVPNKRFGGVCLGGKIAPIFFNTQEDSGALPIEVDVSALKMG DVVDILPYEGKIVKNGETVAEFELKSQVLLDEVQAGGRINLIIGRGLTAKAREALKLPASTAFRLPQAPAESKAGF TLAQKMVGRACGLPEGQGVRPGTYCEPRMTTVGSQDTTGPMTRDELKDLACLGFSADMVMQSFCHTAAYPKPVDVK THKELPAFISTRGGVSLRPGDGVIHSWLNRLLLPDTVGTGGDSHTRFPIGISFPAGSGLVAFAAATGVMPLDMPES VLVRFSGKLQPGVTLRDLVNAIPLYAIKQGLLTVAKAGKKNIFSGRILEIEGLPDLKVEQAFELTDASAERSAAGC TVKLNKEPIIEYMKSNVVLMKNMIANGYQDPRTLERRIKAMEKWLANPELLEADKDAEYAAVIEINMDDIKEPIIA CPNDPDDVCFMSERSGTKIDEVFIGSCMTNIGHFRAASKLLEGKADTPVRLWIAPPTKMDAKQLSDEGHYGVLGRA GARMEMPGCSLCMGNQAQVREGATVMSTSTRNFPNRLGKNTFVYLGSAELAAICSKLGKIPTVEEYQANIGIINEQ GDKIYRYMNFNEIDSYNEVAETVNV |

EP 1 562 982 B1

76

(continued)

| SED ID | NMB | Sequence |
|--------|-----|----------|
| 150 | 1574 | MQVYYDKDADLSLIKGKTVAIIGYGSQGHAHAANLKDSGVNVVIGLRQGSSWKKAEAAGHVVKTVAEATKEADVVM LLLPDETMPAVYHAEVTANLKEGATLAFAHGFNVHYNQIVPRADLDVIMVAPKGPGHTVRSEYKRGGGVPSLIAVY QDNSGKAKDIALSYAAANGGTKGGVIETTFREETETDLFGEQAVLCGGVVELIKAGFETLTEAGYAPEMAYFECLH EMKLIVDLIFEGGIANMNYSISNNAEYGEYVTGPEVVNASSKEAMRNALKRIQTGEYAKMFIQEGNVNYASMTARR RLNADHQVEKVGAQLRAMMPWITANKLVDQDKN |
| 151 | 1577 | MQLSGAQIIVQSLKAEGVEYVFGYPGGAVIEIYDALFQLNKFKHILTRHEQAAVHAADAYARVSGKVGVALVTSGP GVTNALTGIATAYTDSIPMVVISGQVGNSLIGTDAFQEVDTVGITRPCVKHNFLVTDINELAETIKKAFQIAASGR PGPVVVDVPKDVTQAMAKFSYPQEDIFIRSYQPVVQGHIGQIKKAVQMLASAKRPVVYFGGGVVLGNASEELTRFV RMTGAPCTGTLMGLGAYPSGDRQFLGMLGMHGTYEANLAMQNADVVLAVGARFDDRVVSVPSKFFEKAKKVIHIDV DPSSIAKRVKVDIPIVGDVKNVLSEMVALWQKQESVPSEDALGKWWKTIEEWRSRDCLWFDNGSEIIKPQYVIQKL AEITGNSAIITSDVGQHQMFAAQYYPFERPRQWLNSGGLGTMGVGLPYAIGAKLAAPDQDVFCITGDGSIQMNIQE LSTCFQYRIPVNVITLNNGYLGMVRQWQEIYYGGRESETYFDSLPDFVKLAEAYGHIGIRVDKKSDVEGALLEALN QKDRLVFIDFLTDQKQNVMPMVGNGKGLDEMVLPPHMRADGKA |
| 152 | 1581 | MKKLNTQSPDFQAGLKALLAFETAQNPETERIVADICADVQKRGDAALIEYTNKFDQTNAKSIDDLILTQADLNAA FERIPNDVQTALQTAARRVESYHQRQKMESWSYTDEDGTLLGQQITPLDRVGIYVPGGKAAYPSSVIMNAMPAHVA GVKEIIMVVPTPKGERNDIVLAAAYVAGVTKVFTVGGAQAVAALAYGTETIPQVDKITGPGNAFVAAAKRRVFGVV GIDMVAGPSEILVIADGTTPADWVAMDLFSQAEHDEIAQAILIGTSQAYLDEVEAAMDRLIETMPRRDIIEASLGN RGAMILAKDLDEACEIANYISPEHLELSVENPQEWAKKIRHAGAIFMGRYTGESLGDYCAGPNHVLPTSRTARFSS PLGTYDFQKRSSLIQVSEQGAQKLGETASVLAHGESLTAHARAAEFRMK |
| 153 | 1583 | MNLTKTQRQLHNFLTLAQEAGSLSKLAKLCGYRTPVALYKLKQRLEKQAEDPDARGIRPSLMAKLEKHTGKPKGWL DRKHRERTVPETAAESTGTAETQIAETASAAGCRSVTVNRNTCETQITVSINLDGSGKSRLDTGVPFLEHMIDQIA RHGMIDIDISCKGDLHIDDHHTAEDIGITLGQAIRQALGDKKGIRRYGHSYVPLDEALSRVVIDLSGRPGLVYNIE FTRALIGRFDVDLFEEFFHGIVNHSMMTLHIDNLSGKNAHHQAETVFKAFGRALRMAVEHDPRMAGQTPSTKGTLT A |

77

EP 1 562 982 B1

(continued)

| SED ID | NMB | Sequence |
|---|---|---|
| 154 | 1595 | MKTSELRQKFLKFFETKGHTVVRSSSLVPHDDPTLLFTNAGMNQFKDVFLGFDKRPYSRATTAQKCVRAGGKHNDL<br>ENVGYTARHHTFFEMMGNFSFGDYFKRDAIHFAWEFLTSPEWLNIPKDKLLATVYAEDDEAYNIWLNEIGMPSERI<br>VRIGDNKGAKYASDNFWQMGDTGPCGPCSEIFYDHGEEIWGGIPGSPEEDGDRWIEIWNCVFMQFNRDEQGNMNPL<br>PKPSVDTGMGLERIAAVMQHVHSNYEIDLFQDLLKAVARETGAPFRMEEPSLKVIADHIRSCSFLIADGVLPSNEG<br>RGYVLRRIIRRAVRHGYKLGQSKPFFHKLVADLVKEMGGAYPELKEKQAQIEEALKNEESRFAQTLETGMALLENA<br>LVKGGKTLGGEIIFKLYDTYGFPYDLTADICRERNIEPDEAGFEREMEAQRARARAAQSFKANAQLPYDGQDTEFK<br>GYSERQTESKVLALYKDGEQVNELNEGDSGAVVIDFTPFYAESGGQVGDVGYIFSGENRFEVRDTQKIKAAVFGQF<br>GVQTSGRLKVGDSVTAKVDDEIRNANMRNHSATHLMHKALRDVLGRHVEQKGSLVTAESTRFDISHPQAVTAEEIA<br>EVERRVNEAILANVAVNAAIMSMEDAQKTGAMMLFGEKYGEEVRVLQMGGFSTELCGGTHVSRTGDIGLFKIISEG<br>GIAAGVRRIEAITGLNALKWAQEQERLVKDIIAETKAQTEKDVLAKIQAGAAHAKALEKELARAKAELAVHAGAKL |
| | | LDDAKDLGAAKLVAAQIEADAAALREIVTDLTGKSDNAVILLAAVNDGKVSLCAGVSKPLTGKVKAGDLVKFAAEQ<br>VGGKGGGRPDLAQAGGTDADKLPAVLDSVKDWVGAKLV |
| 155 | 1604 | MELVFIRHGQSEWNAKNLFTGWRDVKLSEQGLAEAAAAGKKLKENGYEFDIAFTSVLTRAIKTCNIVLEESDQLFV<br>PQIKTWRLNERHYGQLQGLDKKQTAEQYGDEQVRIWRRSYDTLPPLLDKDDEFSAHKDRRYAHLPADVVPDGENLK<br>VTLERVLPFWEDQIAPAILSGKRVLVAAHGNSLRALAKHIEGISDEDIMGLEIPTGQPLVYKLDDNLKVIEKFYL |
| 156 | 1621 | MGIYDFQMKDAEGNAVDLSGYRGKVLLIVNTATRCGLTPQYEALQKLYAQYTAEGLEILDFPCNQFREQAPESSGE<br>IAQVCMMKFGTKFKIFDKIEVNGANTAPLYAYLKSVKPQDKGNHLFKDFVLKLAALGEKRDEGDIKWNFTKFLVNR<br>DGEVVERFAPSVTPEEIEADIRALL |
| 157 | 1636 | LLFSSLLFSSLLFSSLLFSSAAQAASEDSSRSPYYVQADLAYAAERITHDYPKPTGTDKDKISTVSDYFRNIRAHS<br>YHPRVSVGYDFGGWRIAADYASYRKWNNNKYSVNIKKGRETQDNREELKTENQENGSFHAASSLGLSAIYDFKLND<br>KFDKFKPYIGARVAYGHVKHQVHSVETKTTIVTSKPAATSPQGGPIIET |

| SED ID | NMB | Sequence |
|--------|-----|----------|
| 158 | 1640 | MSLYPIYNFSAGPAVLPEAVLETARQEMLDYNGTGFPVMAMSHRSEMFLSILHHAEQDLRQLLKVPDNYKILFLQG GATTQFNMAAMNLAHGFRTADAVVTGNWSRIAYEQMSRLTDTEIRLAAHGGEQFDYLDLPPVETWDVAPDSAFVHF AVNETVNGLQYREVPCLSEGMPPLVCDMSSEILSREFDVADYGLIYAGAQKNIGPAGVTVVIVREDLLERCPNDIP DVFNYRSHINRDGMYNTPSTYAIYMSGLVFRWLQAQGGVKKIEAVNRLKAQTLYETIDGSDGFYINRIRPNARSKM NVVFQTGDEELDRRFVLEAELQGLCLLKGYKTVGGMRASIYNAMPLEGVRALADFMRDFQRRYG |
| 159 | 1642 | MSREMLQLAEALASEKNVDAEVVFQALEFALSTAAKKKADREHMDVRVQINRDTGEYQTFRRWLIVADEDYTYPDV EKTIEEIQEEIPGTTIQIGEYYEEQLPNEGFGRQAAQTAKQIILQRIRDAEREQNLNEFLAVKEDIVSGTVKRVER HGIIVEVVAGKLDALIPRDQMIPRENFRSGDRIRALFLRVEEIGNTGRKQVILSRTSGDFLVKLYANEVPEIADGM LEIRAVARDPGQRAKVAVKANDQRIDPQGTCIGVRGSRVNAVSNELSGERIDVVLWSPEPAQFVMSALSPAEVSRI VIDEDKHAVDVIVAEDQLALAIGRGGQNVRLASDLTGWQLNIMTSAEADERNAAEDAAIRRLFMDHLNVDEETADV LVQEGFATLEEVAYVPAAELLAIEGFDEEIVDMLRNRARDAILTMAIAAEEKLGEVSDDMRNLEGIDADMLRSLAE AGITTRDDLAELAVDELIEITGVNEETAKAVILTAREHWFTEDK |
| 160 | 1655 | MVHIMFNQAAQELTTIRDILRFAVSRFNEAGLFFGHGTDNAHDEAAYLILHTLNLPLDMLAPYLDAKLLEAEKEEV LAVIERRAVEHIPAAYLTHQAWQGEFDFYVDERVIIPRSFIYELLGDGLRPWIEYDELVHNALDLCTGSGCLAIQM AHHYPDAQIDAVDVSLDALEVAGINVEDYGLEERIRLIHTDLFEGLEGTYDLIVSNPPYVDAESVELLPEEYLHEP ELALGSGADGLDATRQILLNAAKFLNPKGVLLVEIGHNRDVLEAAYPELPFTWLETSGGDGFVFLLTREQLLGEE |
| 161 | 1684 | MLDIQLLRSNTAAVAERLARRGYDFDTARFDTLEERRKSVQVKTEELQASRNSISKQIGALKGQGKHEEAQAAMNQ VAQIKTDLEQAAADLDAVQKELDAWLLSIPNLPHESVPAGKDETENVEVRKVGTPREFDFEIKDHVDLGEPLGLDF EGGAKLSGARFTVMRGQIARLHRALAQFMLDTHTLQHGYTEHYTPYIVDDTTLQGTGQLPKFAEDLFHVTRGGDET KTTQYLIPTAEVTLTNTVADSIIPSEQLPLKLTAHSPCFRSEAGSYGKDTRGLIRQHQFDKVEMVQIVHPEKSYET LEEMVGHAENILKALELPYRVITLCTGDMGFGAAKTYDLEVWVPAQNTYREISSCSNCEDFQARRLKARFKDENGK NRLVHTLNGSGLAVGRTLVAVLENHQNADGSINIPAALQPYMGGVAKLEVK |
| 162 | 1691 | MARHVWQAGRFEIGLDKPKIMGIVNLTPDSFSDGGVYSQNAQTALAHAEQLLKEGADILDIGGESTRSGADYVSPE EEWARVEPVLAEVAGWGVPISLDTRRTVIMEKALALGGIDIINDVAALNDEGAVELLARQADTGICLMHMQGLPKT MQINPKYQDVVGEVARYLKARSAECIAAGIAPQRIILDPGFGSGFGKPLQHNIALMRHLPELMAETGFPLLIGVSR KSTIGELTGEANAAERVHGSVAAALASVARGAQIVRVHDVKATADALKVWEALGINL |

EP 1 562 982 B1

79

(continued)

| SED ID | NMB | Sequence |
|---|---|---|
| 163 | 1710 | MTDLNTLFANLKQRNPNQEPFHQAVEEVFMSLDPFLAKRNPKYTQQSLLERIVEPERVVMFRVTWQDDKGQVQVNRG YRVQMSSAIGPYKGGLRFHPTVDLGVLKFLAFEQVFKNALTTLPMGGGKGGSDFDPKGKSDAEVMRFCQAFMTELY RHIGADTDVPAGDIGVGGREIGYLFGQYKKIRNEFSSVLTGKGLEWGGSLIRPEATGYGCVYFAQAMLQTRNDSFE GKRVLISGSGNVAQYAAEKAIQLGAKVLTVSDSNGFVLFPDSGMTEAQLAALIELKEVRRERVATYAKEQGLQYFE KQKPWGVAAEIALPCATQNELLDEEAAKTLLANGCYVVAEGANMPSTLGAVEQFIKAGILYAPGKASNAGGVATSGL EMSQNAIRLSWTREEVDQRLFGIMQSIHESCLKYGKVGDTVNYVNGANIAGFVKVADAMLAQGF |
| 164 | 1716 | MAFYAFKAMRAAALAAAVALVLSSCGKGGDAAQGGQPAGREAPAPVVGVVTVHPQTVALTVELPGRLESLRTADVR AQVGGIIQKRLFQEGSSYVRAGQPLYQIDSSTYEAGLESARAQLATAQATLAKADADLARYKPLVAAEAVSRQEYDA AVTAKRSAEAGVKAAQAAIKSAGISLNRSRITAPISGFIGQSKVSEGTLLNAGDATVLATIRQTNPMYVNVTQSAS EVMKLRRQIAEGKLLAADGVIAVGIKFDDGTVYPEKGRLLFADPAVNESTGQITLRAAVPNDQNILMPGLYVRVLM DQVAVDNAFVVPQQAVTRGAKDTVMIVNAQGGMEPREVTVAQQQGTNWIVTSGLKDGDKVVVEGISIAGITGAKKV TPKEWASSENQAAAPQSGVQTASEAKPASEAK |
| 165 | 1796 | MIMAKKISILVGSLRRASFARKVALNAAEMFPEGWQAEIVEIGHLPLYNFDYDDPAVEDVPLPESYTAFRETIKAS DGILFVTSENNRTIPACLKNAVDIGSKPNADVAWKNKPAGIISHSVGKMGGYSSQKNLRLALSYFDMPVTGQPEVF LGNSPTLFDENGKLIDSARDFVQSYINQFVGLIERNAK |
| 166 | 1809 | MNNLIKINLLPYREEMNKRKQQQFKTLMYGAVLTGVAAVAATYLFIDNMINNQSERNTLLETSIAHLDTELSEIQK LKQEKDAFLIKKNKIEELQLKRLQAAKILDSLNEAVPGSTYLTSLDAVTADSYRLSGRTSSDNRVAAMMRAMPNTG IFKQPELLSIKKNNSHQEFTLQATLQPIVKAAESKENPASGNAQEAN |
| 167 | 1810 | MASKSSKTNLDLNNLHLLNLPARLFIALLAVAAVLGLGYAGLFKSQMESLEEYEAKETELKNTYKQKSIDAASLNN LRDELASIRSAFDIMLKQLPTDAEIPNLVQELHQAGSSNGLRLDSVMPQPPVDDGPIKRLPYSISITGNYEQISQF |
| | | TRDVGSLSRIITLESLKIAQSPENGGNPDGKSSIILNLSAIATTYQAKSVEELAAEAAQNAEQK |

(continued)

| SED ID | NMB | Sequence |
|---|---|---|
| 168 | 1811 | MKHYALLISFLALSACSQGSEDLNEWMAQTRREAKAEIIPFQAPTLPVAPVYSPPQLTGPNAFDFRRMETDKKGEN APDTKRIKETLEKFSLENMRYVGILKSGQKVSGFIEAEGYVYTVGVGNYLGQNYGRIESITDDSIVLNELIEDSTG NWVSRKAELLLNSSDKNTEQAAAPAAEQN |
| 169 | 1812 | MNTKLTKIISGLFVATAAFQTASAGNITDIKVSSLPNKQKIVKVSFDKEIVNPTGFVTSSPARIALDFEQTGISMD QQVLEYADPLLSKISAAQNSSRARLVLNLNKPGQYNTEVRGNKVWIFINESDDTVSAPARPAVKAAPAAPAKQQAA APSTKSAVSVSEPFTPAKQQAAAPFTESVVSVSAPFSPAKQQAAASAKQQAAAPAKQQAAAPAKQQAAAPAKQTNI DFRKDGKNAGIIELAALGFAGQPDISQQHDHIIVTLKNHTLPTTLQRSLDVADFKTPVQKVTLKRLNNDTQLIITT AGNWELVNKSAAPGYFTFQVLPKKQNLESGGVNNAPKTFTGRKISLDFQDVEIRTILQILAKESGMNIVASDSVNG KMTLSLKDVPWDQALDLVMQARNLDMRQQGNIVNIAPRDELLAKDKALLQAEKDIADLGALYSQNFQLKYKNVEEF RSILRLDNADTTGNRNTLISGRGSVLIDPATNTLIVTDTRSVIEKFRKLIDELDVPAQQVMIEARIVEAADGFSRD LGVKFGATGKKKLKNDTSAFGWGVNSGFGGDDKWGAETKINLPITAAANSISLVRAISSGALNLELSASESLSKTK TLANPRVLTQNRKEAKIESGYEIPFTVTSIANGGSSTNTELKKAVLGLTVTPNITPDGQIIMTVKINKDSPAQCAS GNQTILCISTKNLNTQAMVENGGTLIVGGIYEEDNGNTLTKVPLLGDIPVIGNLFKTRGKKTDRRELLIFITPRIM GTAGNSLRY |
| 170 | 1835 | MSVIQDLQSRGLIAQTTDIEALDALLNEQKIALYCGFDPTADSLHIGHLLPVLALRRFQQAGHTPIALVGGATGMI GDPSFKAAERSLNSAETVAGWVESIRNQLTPFLSFEGGNAAIMANNADWFGSMNCLDFLRDIGKHFSVNAMLNKES VKQRIDRDGAGISFTEFAYSLLQGYDFAELNKRHGAVLEIGGSDQWGNITAGIDLTRRLHQKQVFGLTLPLVTKSD GTKFGKTEGGAVWLNAKKTSPYQFYQFWLKVADADVYKFLKYFTFLSIEEIDAIEAKDKASGSKPEAQRILAEEMT RLIHGEEALAAAQRISESLFAEDQSSLTESDFEQLALDGLPAFEVSDGINVVEALVKTGLASSNKEARGFVNSKAV LLNGKPAEANNPNHAAERPDDACLLNGEHKRFGKYTILRRGKRNHALLVWK |
| 171 | 1838 | MSLKCGIVGLPNVGKSTLFNALTQSGIEAANYPFCTIEPNVGIVEVPDPRMAELAKIVNPQKMQPAIVEFVDIAGL VAGASKGEGLGNQFLANIRETDAIVNVVRCFDDDNIVHVAGRVDPIADIETIGTELALADLASVEKAIVREEKRAR SGDKDAQKLVDLCKKLLPHLDEGKPVRSFGLDAEERAMLKPLFLLTAKPAMYVGNVAEDGFENNPHLDRLKELAAK ENAPVVAVCAAMESEIAELEDDEKAEFLAEMGLEEPGLNRLIRAGYDLLGLQTYFTAGVKEVRAWTIHKGDTAPQA AGVIHTDFERGFIRAQVISYDDFVSLGGEAKAKEAGKMRVEGKEYVVQDGDVMHFLFNV |

EP 1 562 982 B1

81

| SED ID | NMB | Sequence |
|---|---|---|
| 172 | 1843 | MPTQSKHASINIGLIQAREALMTQFRPILNQANITDQQWRIIRLLAENGTLDFQDLANQACILRPSLTGILTRLEK AGLVVRLKPSNDQRRVFLKLTAEGEKLYEEIGEEVDERYDAIEEVLGREKMLLLKDLLAELAKIEDALNS |
| 173 | 1849 | MSTPALLVLADGSVFHGTSIGYEGSTSGEVVFNTSMTGYQEILTDPSYCKQIVTLTYPHIGNTGTNAEDEESRSVY AAGLIIRDLPLLHSNFRASESLHDYLVRNKTVAIADIDTRRLTTLLREKGAQGGAILTGADATIEKAQELIAAFGS MVGKDLAKEVSCTETYEWTEGEWALGKGFVTPDEQPYHVVAYDFGVKTNILRMLASRGCRLTVVPAQTSAEDVLAL NPDGVFLSNGPGDPEPCTYAIKAVQKLIESGKPIFGICLGHQLISLAIGAKTLKMRFSHHGANHPVQDLDSGKVVI TSQNHGFAVDADTLPANARITHKSLFDNTLQGIELTDKPVFCFQGHPEASPGPQDVGYLFDKFIGNMKAAKRA |
| 174 | 1854 | MSIPINFNNLKYLLNDMRNKNRIIEAFPFNYNQRQYAVILTRYKPDEPRPDDYAQAKLEFFNLNSENSIFAYADFY EVHFKSATDFINFFKINVQAGAAKIREIFQSFSNLFADFIPTQTKKDLDIIYKKIVATRLEPNSPNTIYCYDVRRN GKDKAGKPNRRSVENSEKAKILRPELYEKFKADSNYSFFFSDNPSDEKTDAEIIREVTNRQ |
| 175 | 1855 | MPKRTDLKSILIIGAGPIVIGQACEFDYSGAQACKALREEGYKVILVNSNPATIMTDPEMADVTYIEPIMWQTVEK IIAKERPDAILPTMGGQTALNCALDLARNGVLAKYNVELIGATEDAIDKAEDRGRFKEAMEKIGLSCPKSFVCHTM NEALAAQEQVGFPTLIRPSFTMGGSGGGIAYNKDEFLAICERGFDASPTHELLIEQSVLGWKEYEMEVVRDKNDNC IIICSIENFDPMGVHTGDSITVAPAQTLTDKEYQIMRNASLAVLREIGVDTGGSNVQFAVNPANGEMIVIEMNPRV SRSSALASKATGFPIAKVAAKLAVGFTLDELRNDITGGKTPASFEPSIDYVVTKIPRFAFEKFPAADDRLTTQMKS VGEVMAMGRTIQESFQKALRGLETGLCGFNPRSEDKAEIRRELANPGPERMLFVADAFRAGFTLEEIHEICAIDPW FLAQIEDLMKEEKAVSDGILSDLDFAALRRLKRKGFSDKRLAQLLNVSEKEVREHRYALKLHPVYKRVDTCAAEFA TETAYLYSTYEEECESRPSDRKKVMILGGGPNRIGQGIEFDYCCVHAALALRESGFETIMVNCNPETVSTDFDTSD RLYFEPLTLEDVLEIVRTENPWGVIVHYGGQTPLKLANALVENGVNIIGTSADSIDAAEDRERFQKVLNDLGLRQP PNRIAHNEEEALVKAEEIGYPLVVRPSYVLGGRAMQVVHSAEELQKYMREAVQVSEDSPVLLDFFLNNAIEVDVDC VSDGKDVVIGGIMQHVEQAGIHSGDSGCSLPPYSLSEEIQDEIRRQTKAMAYALGVVGLMNVQFAVQDGVVFVLEV NPRASRTVPFVSKATGVPLAKVGARCMAGISLKEQGVEKEVVPDFYAVKEAVFPFIKFPGVDTILGPEMRSTGEVM GVGASFGEAYYKAQLGAGERLNPTGKIFLSVREEDKERVIKTAKNFQVLGYGICATRGTAQYLTEHGLIVQTINKV PEGRPHIGDALKNGEIALVVNTVSSDPQSVSDSHIIRQSALQQRVPQYTTTAGGEAMSEGAKSRDHLGVYSVQELH GRLKNRN |

82

EP 1 562 982 B1

(continued)

| SED ID | NMB | Sequence |
|--------|-----|----------|
| 176 | 1859 | MDISDFDFTLPEKLIAQHPPEVRGSSRLLVALPDMPLQDRVFGDLPDYVEAGDVLVFNNTKVMKARLFGQKDSGGR IEALIERVLDNHTALAHIRSSKSPKPGMGLVFEGGIRAVTVGREGELFCLRFEGGETVYELLEQNGHLPLPPYIER AADADDDSRYQTVYAKYQGAVAAPTAGLHFTEELLHRLKDKGAVTAEVTLHVGAGTFQPVRVDKIEEHKMHSEWFE VPSETAAAVEAAKARGNKVWAVGTTSMRALESAARATGRLKAGQGDTDIFITPGYRFNVVDRLVTNFHLPKSTLLM LVSAFSGMGHIRAAYRHAVEREYRFFSYGDAMVLGRNEGVVR |
| 177 | 1861 | MLKKVLIANRGEIALRVLRACREMGIATVAVHSEADKDSLHVKLADESVCIGPAASAQSYLNVPAIIAAAEVSCAD AVHPGYGFLAENADFAEQVEQSGFTFIGPKPDTIRLMGDKVSAKHAMIAAGVPCVPGSDGALPDDGEEILKIADKV |
| | | GYPVIIKASGGGGGRGMRVVEKKEDLLQSVEMTKAEAGAAFGNPMVYMERYLQRPRHVEIQVIADEHGNAIYLAER DCSLQRRHQKVIEEAPAPFITEKERAKIGNACADACKRIGYRGAGTFEFLYEDGEFFFIEMNTRVQVEHPVTELIT GVDIVQEQLRIAAGLPLQYKQKDIQVEGHAFECRINAEDPYNFIPSPGLIESCHLPGGFGIRVDSHIYQGYRIPPY YDSLIGKICVHGKTREQAMAKMRVALAELAVTGIKTNTPLHRDLFADAGFQKGGVSIHYLEHWLEDRKAKQDK |
| 178 | 1862 | MPYQQITVNVNDAVAERLADALMEHGALSAAIEDAYAGTQNEQAIFGEPGMPAEQIWQQSKVIALFGEHDEAAAII QTATQECGLKDLAYTGETIEDQDWVRLTQSQFDPIRISDRLWITPSWHEVPEGSAVNLRLDPGLAFGTGSHPTTRL CLKWLDTQLKNGESVLDYGCGSGILTIAALKLGAGFAVGVDIDEQAVRAGKDNAAQNNVDAQFFLPDGLPQGQFDV VVANILANPLRMLGEMLAARTKQGGRIVLSGLLDEQAEELGGIYSQWFDLDPAETEEGWARLSGVKR |
| 179 | 1864 | MNRNEILFDRAKAIIPGGVNSPVRAFGSVGGVPRFIKKAEGAYVWDENGTRYTDYVGSWGPAIVGHAHPEVVETVC EAALGGLSFGAPTEGEIVIAEEIAKIMPSVERLRLVSSGTEATMTAIRLARGFTGRDKIIKFEGCYHGHSDSLLVK AGSGLLTFGNPSSAGVPADFTKHTLVLEYNNIAQLEEAFAQSGNEIACVIVEPFVGNMNLVRPTEAFVKALRGLTE KYGAVLIYDEVMTGFRVALGGAQSLHGITPDLTTMGKVIGGGMPLAAFGGRKDIMECISPLGGVYQAGTLSGNPIA VAAGLKTLEIIQREGFYENLTARTEQLVQGFRTAADAAGIEFTADSVGGMFGLYFAAHAPRNYADMARSNIEGFKQ FFHGMLDRNVAFGPSAYEAGFVSAAHTPELIDETVAVAVEVFKAMAE |

EP 1 562 982 B1

83

(continued)

| SED ID | NMB | Sequence |
|---|---|---|
| 180 | 1903 | MTLAEFWPLCLRRLHDMLPQGQFAQWIAPLTVGEEGGVWVVYGKNQFACNMLKSQFAGKIEAVREELAAGRSAFVF KPGEGVRYEMAAVEGAVEPAEPSLHAVSEGMPVQEVLLDELPSEEPVKPAASKTAADILAERMKNLPHEPRQAAGS ASRPESVAVAKARTDVQRDAEEARYEQTNLSPDYTFDTLVEGKGNRLAAAAAQAIAESPGQSYNPFFLYGSTGLGK THLVQAVGNELLKNRPDAKVRYMHSDDYIRSFMKAVRNNTYDVFKQQYKQYDLLIDDIQFIKGKDRTMEEFFYLY NHFHNEKKQLILTCDVLPAKIEGMDDRLKSRFSWGLTLELEPPELEMRIAILQKKAEAAGISIEDEAALFIANLIR SNVRELEGAFNRVGASSRFMNRPVIDIDLARTALQDIIAEKHKVITADIIIDAVAKYYRIKISDVLGKKRTRNIAR PRQVAMSLTKELTTLSLPSIGDSFGGRDHTVMHGIRAVAKLREEDPELAQDYEKLLLIQN |
| 181 | 1920 | MTQDKILILDFGSQVTQLIARRVREAHVYCELHSFDMPLDEIKAFNPKGIILSGGPNSVYESDYQADTGIFDLGIP VLGICYGMQFMAHHLGGEVQPGNQREFGYAQVKTIDSELTRGIQDGEPNTLDVWMSHGDKVSKLPDGFAVIGNTPS CPIAMMENAEKQFYGIQFHPEVTHTKQGRALLNRFVLDICGAQPGWTMPNYIEEAVAKIREQVGSDEVILGLSGGV DSSVAAALIHRAIGDQLTCVFVDHGLLRLNESKMVMDMFARNLGVKVIHVDAEGQFMAKLAGVTDPEKKRKIIGAE FIEVFDAEEKKLTNAKWLAQGTIYPDVIESAGAKTKKAHAIKSHHNVGGLPENMKLKLLEPLRDLFKDEVRELGVA LGLPREMVYRHPFPGPGLGVRILGEVKKEYADLLRQADDIFIQELRNTTDENGTSWYDLTSQAFAVFLPVKSVGVM GDGRTYDYVIALRAVITSDFMTAHWAELPYSLLGKVSNRIINEVKGINRVYDVSGKPPATIEWE |
| 182 | 1921 | MSTQDLNGKIALVTGASRGIGAAIADTLAAAGAKVIGTATSESGAAAISERLAQWGGEGRVLNSAEPETIESLIAD IEKAFGKLDILVNNAGITRDNLLMRMKEEWDDIMQVNLKSVFRASKAVLRGMMKQRSGRIINITSVVGMGNAGQ TNYAAAKAGLIGFSKSMAREVGSRGITVNCVAPGFIDTDMTRALPEETRQTFTAQTALGRFGDAQDIADAVLFLAS DQAKYITGQTLHVNGGMLMP |
| 183 | 1930 | MMTQTLLIELLTEELPPKALNNLGNHFAASVAEGLEKAQLVDGAAEFTAYASPRRLAVQVKNVKAVQADQKIVKKG PAVANAMKDGAPTKALEGFARGAGAKIEDLTIVHDGKQDVYAYEVVQIGKPLGGLLEDIINQAVKKLPIPKVMRWG SSTFTFVRPVHGLVVLHGGDIVNVSVLGLQSGNKTLGHRFLSDGEITIENADSYAAQMREQGKVVASFAERKAAIQ TVLEGQARRLNATAADEALLDEVTALVEWPVVLEAGFEEHFLAVPQECLILTMQQNQKYFPLLDQNGKLMNRFLL VSNLQTEDPSHIIQGNERVLRARLSDAEFFYKQDQKATLESRLPKLTNVVYHNKIGSQAERIERLQSIAAHIAKAL GADAAAAERAARLAKADLVTEMVGEFPELQGTMGKYYARLDGTEEITEAVEQHYQPRFAGDNLPEGKIAAAVALA DKLETLVGIWGIGLJPTGDKDPYALRRAALGILRMLMQYGLDVNELIQTAFNSFPQGLLNEKTPSETADFMQARLA VLLQNDYPQDIVAAVLAKQPRRLDDLTAKLQAVAAFKQLPEAAALAAANKRVQNLLKKADAELGAVNESLLQQDEE KALFAAAQGLQPKIAAAVAEGNFQTALSELASVKPQVDAFFDGVMVMAEDAAVKQNRLNLLNRLAEQMNAVADIAL LGE |

(continued)

| SED ID | NMB | Sequence |
|---|---|---|
| 184 | 1934 | MSQGKIVQIIGAVVDVEFPRDMIPRVYDALKLDENGLTLEVQQLLGDGVVRAIAMGSSDGLKRGMTVSNTGAPITV PVGKGTLGRIVDVLGTPVDEAGPIDTDKSRAIHQAAPKFDELSSTTELLETGIKVIDLLCPFAKGGKVGLFGGAGV GKTVNMMELINNIAKAHSGLSVFAGVGERTREGNDFYHEMKDSNVLDKVAMVYGQMNEPPGNRLRVALTGLTMAEY FRDEKDENGKGRDVLFFVDNIYRYTLAGTEVSALLGRMPSAVGYQPTLAEEMGRLQERITSTQTGSITSIQAVYVP ADDLTDPSPATTFAHLDATVVLSRDIASLGIYPAVDPLDSTSRQLDPMVLGQEHYDVARGVQSTLQKYKELRDIIA ILGMDELSDEDKLTVMRARKIQRFLSQPFHVAEVFTGSPGKYVALRDTIAGFKAILNGEYDHLPEQAFYMVGSIEE AVEKAKTLN |
| 185 | 1936 | MQLNPAEISDLIKAKIENLSVNAEVRTCGTVISVTDGIVRIHGLSDAMQGEMLEFPGNTFGLAMNLERDSVGAVVL GEYEHIKEGDTVTCTGRILEVPVGRELVGRVVDALGRPIDGKGPINTTLTAPIEKIAPGVIARKSVDQPMQTGLKA IDSMVPVGRGQRELIIGDRQTGKTAVALDAIVNQKGTGVICIYVAIGQKASSIANVVRKLEEHGAMEHTIVVAATA SEAAALQYIAPYSGCTMGEFFRDRGEDALIVYDDLSKQAVAYRQISLLLRRPPGREAYPGDVFYLHSRLLERAARV NEHEVEKLTNGEVKGKTGSLTALPIIETQAGDVSAFVPTNVISITDGQIFLETDLFNAGIRPAINAGISVSRVGGA AQTKVIKKLGGGIRLALAQYRELAAFSQFASDLDEATRKQLEHGEVVTELMKQKQFSTLNTAEMALTLWAINNGSY SDVPVAKALAFESEFLSFVRTQHPEVLEAVNASGAMSDESEKTLEAAMKSFKSSYAYQA |
| 186 | 1937 | MAEFATIARPYAKALFGLAQEKNQIESWLGGLEKLAAVVQEGKVASLIDRPETNASEKADILIDLVGLKDKELKNF VIVLAGQKRLSILPEVYAQYQDLTLSFNHIKSAVIYSAYPLTDKQVGELVQMLNKRFDSELKISVEIEPELIGGIK |
| | | VEVGDQVLDLSVQGKLSALYTTMTN |
| 187 | 1938 | MNINATLFAQIIVFFGLVWFTMKFVWPPIAKALDERAAKVAEGLAAAERGKSDFEQAEKKVAELLAEGRNQVSEMV ANAEKRAAKIVEEAKEQASSEAARIAAQAKADVEQELFRARESLREQVAVLAVKGAESILRSEVDASKHAKLLDTL KQEL |
| 188 | 1953 | MMTLYSGITCPFSHRCRFVLYEKGMDFEIKDVDIYNKPEDLAVMNPYNQVPVLVERDLVLHESNIINEYIDERFPH PQLMPGDPVMRGRGRLVLYRMEKELFNHVQVLENPAATNKEQAKAREAIGNGLTMLAPSFSKSKYILGEDFSMIDV ALAPLLWRLDHYDVKLGKSAAPLLKYAERIFQREAFIEALTPAEKAMRK |

(continued)

| SED ID | NMB | Sequence |
|---|---|---|
| 189 | 1966 | MSPSPFIEMKDVAFAYGDRPILKNINFSIPQGNFAAVMGGSGSGKTTLMRLLTGQIRPQSGQVLIEGRDLAGFSAD ELYEHRRRMGVLFQHGALFTDLSVFDNIAFPMRELTRLPEAVIRDLVLLKLNAVGLRGVENLMPSELSGGMSRRVA LARTIALDPEIMLYDEPFTGLDPISLGVIAHLISRVNKALRSTSIMVTHDIEKSLEIVDQVIFLAHGEIMFSGSPQ EMRELDSPWVRQFVGGLADGPVAYRYPAQTSLQQDLLG |
| 190 | 1968 | MKQLAMYINGRFENDFNGEWRDVLNPSTEEAIAREPKGGKADVDRAVAAARAAQPAWERLPAVERGAYLRKIAQGI RERADELTDTIVAEGGKTKDLLARVEVMFTADYLDYQAEWARRYEGEIIQSDRPRENILLFKRPLGVIAGILPWNFP FFLIARKMGPALIVTGNTIVVKPSSVTPINCHIFAEIVDAVGLPAGVFNVVNGPGAEIGNALSAHPQVDMVSLTGSV EAGRQVMEAASANITKVSLELGGKAPAIVLKDADLDLAVKSILASRVGNTGQICNCAERVYVHSSLKDAFIEKMTA AMKGVRYGNPAEEAEAGALEMGPLIEERAVKAVAEKVERAVKQGAKLVCGGKRAEGRGYFFEPTLLTDTDNSMDIMK EETFGPVLPVSAFDTLDQVIALANDCEFGLTSSVYTTNLNEAFYVTRRLQFGETYINRENFEAMQGFHAGWKKSGI GGADGKHGLEEYLQTQVVYLETDI |
| 191 | 1982 | MSNRPTLLLVDGSSYLYRAYHAMGQNLTAPDGAPTGALYGVINMLRRLRSEYPHDYCAVVFDAKGKNFRHQMFEEY KATRPPMPDLRPQAEALPDLVRLTGWPVIVIGQVEADDVIGTLAKQGAEHGLRVIVSTGDKDMAQLVDERVTLVN TMSSETLDIEGVKAKFGVRPDQIRDYLALMGDKVDNVPGVEKCGPKTAVKWLEAYGSLAGVMEHASEIKGKVGENL QAALPQLPLSYDLVTIKTDVDLHAELSDGIESLRRTTPKWAQLVVDFKRWGFRTWLKEAESNMNTGSTDDLFGSDS IGEQAALNAEMPFEKQAEKATAPEKLDYQAVTTEAQFAALLDKLSRADTIGIDTETTSLDAMNASLVGISIAFQAG EAVYIPVGHSLTAAPEQLDLQDVLGRLKPHLGNPALKKIGQNLKYDQHVFANYGIALNGIAGDAMLASYIIESHLG HGLDELSERWLGLETITYESLCGKGAKQIGFADVAIGQATEYAAQDADFALRLEAHLRAQMDEKQLEMYEKMELPV AQVLFEMERNGVQIDRAELARQSAELGAELMKLEQEAYAAAGQPFNLNSPKQLQEILFDKMGIPTKGLKTAKGGI STNEAVLEQLAPDYPLPKIILQNRSLAKILKSTYTDKLPEMISPKDGRVHTTYYAQAVAITGRLASNNPNLQNIPIRT EEGRKVRRAFTAPQGSVIVSADYSQELRIMAHLSGDKTLIAAFQNGEDVHRRTAAEVFGTAPENVSSEQRRYAKS INFGLIYGMQQYGLAKSLGIDNLSAKNFIDRYFARYPGVAEYMQRTKEQAAAQGYVETLFGRRLYLPDIRNKNANA RAGAERAAINAPMQGTASDLIKRAMIDVSRWLSECEASPWDELLQSKLIMQVHDELVLEVEVTELDFVKEKLPQIM AKVDGGLLDVPLVAEVGVGENWEEAH |

(continued)

| SED ID | NMB | Sequence |
|---|---|---|
| 192 | 1996 | MSVVLPLRGVTALSDFRVEKLLQKAAALGLPEVKLSSEFWYFVGSEKALDAATVEKLQALLAAQSVEQTPKAREGL HLFLVTPRLGTISPWASKATNIAENCGLAGIERIERGMAVWLEGRLNDEQKQQWAALLHDRMTESVLPDFQTASKL FHHLESETFSGVDVLGGGKEALVKANTEMGLALSADEIDYLVENYQALQRNPSDVELMMFAQANSEHCRHKIFNAD FILNGEKQPKSLFGMIRDTHNAHPEGTVVAYKDNSSVIEGAKIERFYPNAAENQGYRFHEEDTHIIMKVETHNHPT AIAPFAGAATGAGGEIRDEGATGKGSRPKAGLTGFTVSNLNIPDLKQPWEQDYGKPEHISSPLDIMIEGPIGGAAF NNEFGRPNLLGYFRTFEEKFDGQVRGYHKPIMIAGGLGSIQAQQTHKDEIPEGALLIQLGGPGMLIGLGGGAASSM DTGTNDASLDFNSVQRGNPEIERRAQEVIDRCWQLGGKNPIISIHDVGAGGLSNAFPELVNDARRGAVFKLREVPL EEHGLNPLQIWCNESQERYVLSILEKDLDAFRAICERERCPFAVVGTATDDGHLKVRDDLFANNPVDLPLNVLLGK LPKTTRTDKTVAPSKKPFHAGDIDITEAAYRVLRLPAVAAKNFLITIGDRSVGGLTHRDQMVGKYQTPVADCAVTM MGFNTYRGEAMSMGEKPTVALFDAPASGRMCVGEAITNIAAVNIGDIGNIKLSANWMAACGNEGEDEKLYRTVEAV SKACQALDLSIPVGKDSLSMKTVWQDGEEKKSVVSPLSLIISAFAPVKDVRKTVTPELKNVEDSVLLFVDLGFGKA RMGGSAFGQVYNNMSGDAPDLDDTGRLKAFYSVIQQLVAENKLLAYHDRSDGGLFAVLVEMAFAGRCGLDIDLNLL LAQTFITNHTALSQSLRTEEVKALAEWQETIARTLFNEELGAVIQVRKQDVADIINLFYQQQLHHNVFEIGTLTDE NTLIIRDGQTHLISDNLIKLQQTWQETSHQIQRLRDNPACADSEFALIGDNERSALFADVKFDVNEDIAAPFINSG AKPKIAILREQGVNGQIEMAAAFTRAGFDAYDVHMSDLMAGRIHLADFKMLAACGGFSYGDVLGAGEGWAKSILFH PALRDQFAAFFADPDTLTLGVCNGCQMVSNLAEIIPGTAGWPKFKRNLSEQFEARLSMVHVPKSASLILNEMQGSS LPVVVSHGEGRADFALHGGNISADLGIALQYIDGQNQVTQTYPLNPNGSPQGIAGVTNADGRITIMMPHPERVYRA AQMSWKPEGWTELSGWYRLFAGARKALG |
| 193 | 2000 | MNQTAINRADVRTRFIFDDMPVRGLHVRLENVWQHIVKQKNYPAAIRRALGELLAAGVLLSGNLKNEGTLIVQVQG RGRLKMLVAEAASDRTVRATARWDETAEIADDESLGDLLGEGGVFVLTLQPKDGEPWQGVVPLEGGGIAQMLVNYM KRSEQLDTHIVLSASDEAAGGLLVQRLPEEVLDEEAWEHVSTLARTLTAEELAGLDAQHVLYRLFHETPPRVFEPE TFEFSCTCSRGKVSDMLLMLGGEEVGGVVVEQGSIEVDCDFCHSKYVFDETDVNALFGEDVVGVAKGLPRHTVQ |
| 194 | 2039 | MKKSLIALTLAALPVAAMADVTLYGTIKAGVETSRSVFHQNGQVTEVTTATGIVDLGSKIGFKGQEDLGNGLKAIW QVEQKASIAGTDSGWGNRQSFIGLKGGFGKLRVGRLNSVLKDTGDINPWDSKSDYLGVNKIAEPEARLISVRYDSP EFAGLSGSVQYALNDNAGRHNSESYHAGFNYKNGGFFVQYGGAYKRHHQVQEGLNIEKYQIHRLVSGYDNDALYAS VAVQQQDAKLTDASNSHNSQTEVAATLAYRFGNVTPRVSYAHGFKGLVDDADIGNEYDQVVVGAEYDFSKRTSALV SAGWLQEGKGENKFVATAGGVGLRHKF |

(continued)

| SED ID | NMB | Sequence |
|--------|-----|----------|
| 195 | 2057 | MKTFSAKPHEVKREWFVIDAQDKVLGRVAAEVASRLRGKHKPEYTPHVDTGDYIIVINADKLRVTGAKFEDKKYFR |
| | | HSGFPGGIYERTFREMQEQFPGRALEQAVKGMLPKGPLGYAMIKKLKVYAGAEHAHAAQQPKVLELK |
| 196 | 2079 | MKVGFVGWRGMVGSVLMQRMKEENDFAHIPEAFFFTTSNVGGAAPDFGQAAKTLLDANNVAELAKMDIIVTCQGGD YTKSVFQALRDSGWNGYWIDAASSLRMKDDAIIVLDPVNRDVLDNGLKNGVKNYIGGNCTVSLMLMALGGLFQNDL VEWATSMTYQAASGAGAKNMRELISGMGAVHAQVADALADPAGSILDIDRKVSDFLRSEDYPKANFGVPLAGSLIP WIDVDLGNGQSKEEWKGGVETNKILGRSDNPTVIDGLCVRVGAMRCHSQAITLKLKKDLPVSEIETILAGANDWVK VIPNEKEASIHELTPAKVTGTLSVPVGRIRKLGMGGEYISAFTVGDQLLWGAAEPLRRVLRIVLGSL |
| 197 | 2086 | MKFIDEAKIEVAAGKGGNGATSFRREKFVPRGGPDGGDGGKGGSVWAEADENTNTLVEYRFVKRYQAKNGEKGHGS DRYGAGADDIVLKMPVGTLIRDLDTGETVADLTYHGQRVCLAKGGKGGLGNIHFKSSVNRAPKQSTPGEEGEARSL QLELKVLADVGLLGMPNAGKSTLITAVSAARPKIANYPFTTLHPNLGVVRIDENHSFVMADIPGLIEGAAEGAGLG HRFLKHLSRTGLLLHVVDLAPFDETVNPAEEALAIINELRKYDEELYGKPRWLVLNKLDMLDEEEAQTRTAAFLEA VGWDYPKPDDRFQFDMETPRLFQISALTHQGTQELVHQINQYLTEKKRIEAEKAEAEKAAANVEIIEQQPKTDTGV FKPE |
| 198 | 2101 | MSQITMRQMIEAGVHFGHQTRFWNPKMAQYIFGARNKIHIVNLEKTLPMFQDAQEAVRRLVANKGTVLFVGTKRQA RDIIREEATRAGMPFVDYRWLGGMLTNYKTVKQSIKRLEEKTAALENAAESGFSKKEILEMQRDVEKLERSLGGIK NMKGLPDAIFVIDTGYQKGTLVEAEKLGIPVIAVVDTNNSPDGVKYVIPGNDDSAKAIRLYCRGIADAVLEGKNQA LQETVAAAQEAAAE |
| 199 | 2102 | MAEITAKMVADLRAATGLGMMECKKALVEAEGNFDKAEEILRIKSGAKAGKLAGRTAAEGVLAYAINGNVGALVEV NCETDFVAKDAGFVEFANFVAKTAAEKKPASVEELSELVEAERKAIIAKLGENMSVRRFQVIDTANQLVAYIHGAL ATEGVLVEYKGSEDVARKIGMHIVAAKPQCVSEAEVDAETVEKERHIYTEQAIASGKPADIAAKMVEGRIRKFLAE ITLNGQAFVMNPDQTVAQFSKENGTEVISFVRYKVGDGIEKKAVDYAAEVAAAAKV |

| SED ID | NMB | Sequence |
|---|---|---|
| 200 | 2103 | MTQQIKYKRVLLKLSGESLMGSDPFGINHDTIVQTVGEIAEVVKMGVQVGIVVGGGNIFRGVSAQAGSMDRATADY MGMMATVMNALALKDAFETLGIKARVQSALSMQQIAETYARPKAIQYLEEGKVVIFAAGTGNPFFTTDTAAALRGA EMNCDVMLKATNVDGVYTADPKKDPSATRYETITFDEALLKNLKVMDATAFALCRERKLNIVVFGIAKEGSLKRVI TGEDEGTLVHC |
| 201 | 2129 | MSQNHTILQSLPVGQKVGIAFSGGLDTSAALLWMKLKGALPYAYTANLGQPDEDDYNAIPKKAMEYGAENARLIDC RAQLAHEGIAAIQCGAFHVSTGGIAYFNTTPLGRAVTGTMLVSAMKEDDVNIWGDGSTYKGNDIERFYRYGLLTNP ALKIYKPWLDQQFIDELGGRHEMSEFLIANGFNYKMSVEKAYSTDSNMLGATHEAKDLEFLNSGIKIVKPIMGVAF WDENVEVSPEEVSVRFEEGVPVALNGKEYADPVELFLEANRIGGRHGLGMSDQIENRIIEAKSRGIYEAPGMALFH IAYERLVTGIHNEDTIEQYRINGLRLGRLLYQGRWFDSQALMLRETAQRWVAKAVTGEVTLELRRGNDYSILNTES PNLTYQPERLSMEKVEDAAFTPLDRIGQLTMRNLDITDTRVKLGIYSQSGLLSLGEGSVLPQLGNKQ |
| 202 | 2138 | MEAEVINQLNNTLNDLEKRSEDIRVYMDYQGKKDRLEEVIGLSEDPELWNDPKRAQEIGKERKILEGIVLTLDNIA SGIEDNRMLIEMTVEENDEEGFAAVQEDVAGLEKQMADLEFKRMFNQPADPNNCFIDITAGAGGTEAEDWAGMLFR MYSRYAERKGFRIEILEEDDGEIAGINRATIRVEGEYAYGLLRTETGVHRLVRYSPFDSNNKRHTSFASVFVYPEI DDSIEIEINPADLRIDTYRASGAGGQHINKTDSAVRITHEPTGIVVQCQNDRSQHANKAAAMEMLKSKLYELEMRK RNEEKQALEEGKSDVGWGSQIRSYVLDSSRIKDLRTGYEVGNTKAVLDGDLDGFIEASLKQGV |
| 203 | 2154 | MSVLIIVEHDNKQLNPTTLHAVTAAAKLGKVDLLVAGNGASAVVEFAKQVAGVKKVLVADAAHYAEGLAEELAPLV VKLAADYRYVAATATTFGKNLLPRVAALLDVPQISDLTEIVDNTTFVRPIYAGNAFETVQADSEKLVLTFRATVFD AVAAQGGNAEVINVEATPAQNLSRFVNRQLSHSDRPELTQAKVIVSGGRALGSAEKFNEVLTPLADVLGAAIGASR AAVDAEYAPNDAQVGQTGKVVAPQLYFAIGISGAIQHVAGMQDSKVIVAINKDADAPIFNVADYGLVGDLFEIVPQ LTEVLKN |
| 204 | 2155 | MKALVAVKRVVDYNVKVRVKADGSDVDIGNVKMSMNPFDEIAVEEAVRLKEAGKVSEIVAVSLGEKKCEETLRTAL AMGADRAIHVETDTKLESLAVAKLLKAVADKENPQIFFLGKQAIDDDANQVAQMLAALLNAAQGTFASKVQIEGDE VQIVREIDGGEETIALKLPAVISADLRLNEPRFVKLPNIMAAKKKPLEKLTPDDLVADISPRLKTVKFAEPKARQA GVKVASVAELVEKLKNEAKVI |

EP 1 562 982 B1

89

| SED ID | NMB | Sequence |
|---|---|---|
| 205 | 2159 | MSIKVAINGFGRIGRLALRQIEKAHDIEVVAVNDLTPAEMLLHLFKYDSTQGRFQGTAELKDDAIVVNGKEIKVFA NPNPEELPWGELGVDVILECTGFFTNKTKAEAHIRAGARKVVISAPGGNDVKTVVYGVNQDILDGSETVISAASCT TNCLAPMAAVLQKEFGVVEGLMTTIHAYTGDQNTLDAPHRKGDLRRARAAALNIVPNSTGAAKAIGLVIPELNGKL DGSAQRVPVASGSLTELVSILERPVTKEEINAAMKAAASESYGYNEDQIVSSDVVGIEYGSLFDATQTRVMTVGGK QLVKTVAWYDNEMSYTCQLVRTLEYFAGKI |
| 206 | 1126 1164 | MKTVSTAVVLAAAAVSLTGCATESSRSLEVEKVASYNTQYHGVRTPISVGTFDNRSSFQKGIFSDGEDRLGSQAKT ILVTHLQQTNRFNVLNRTNLNALKQESGISGKAHNLKGADYVVTGDVTEFGRRDVGDHQLFGILGRGKSQIAYAKV ALNIVNVNTSEIVYSAQGAGEYALSNREIIGFGGTSGYDATLNGKVLDLAIREAVNSLVQAVDNGAWQPNR |
| 207 | 1799 | MSEYLFTSESVSEGHPDKVADQVSDAILDAILAQDPKARVAAETLVNTGLCVLAGEITTTAQVDYIKVARETIKRI GYNSSELGFDANGCAVGVYYDQQSPDIAQGVNEGEGIDLNQGAGDQGLMFGYACDETPTLMPFAIYYSHRLMQRQS ELRKDGRLPWLRPDAKAQLTVVYDSETGKVKRIDTVVLSTQHDPSIAYEELKNAVIEHIIKPVLPSELLTDETKYL INPTGRFVIGGPQGDCGLTGRKIIVDTYGGAAPHGGGAFSGKDPSKVDRSAAYACRYVAKNIVAAGLATQCQIQVS YAIGVAEPTSISIDTFGTGKISEEKLIALVREHFDLRPKGIVQMLDLLRPIYSKSAAYGHFGREEPEFTWERTDKA |
|  |  | AALRAAAGL |
| 208 | 0110 | MALLNILQYPDERLHTVAKPVEQVDERIRKLIADMFETMYESRGIGLAATQVDVHERVVVMDLTEDRSEPRVFINP VIVEKDGETTYEEGCLSVPGIYDTVTRAERVKVEALNEKGEKFTLEADGLLAICVQHELDHLMGIVFVERLSQLKQ GRIKTKLKKRQKHTI |

90

| SED ID | NMB | Sequence |
|---|---|---|
| 209 | 0957 | MSQYDVVVIGAGPGGYVAAIRAAQLGFKTACVDAGVNKAGNAPALGGTCLNVGCIPSKALLQSSEHFHAAQHEFAE HGITVGDVKFDAAKMIERKDAIVTKLTGGVKFLFQKNKVTSLFGTASFAGKNGDAYQIEVDNKGEKTVIEAKHVIV ATGSVPRPLPQVAIDNVNVLDNEGALNLTEVPAKLGVIGSGVIGLEMGSVWNRVGAEVTILEAAPTFLAAADQQIA KEAFKYFTKEQGLSIELGVKIGDIKSEGKGVSVAYETAAGEAKTEVFDKLIVAIGRIPNTKGLNAEAVGLEKDERG FIKVDGECRTNLPNVWAIGDVVRGPMLAHKASDEGVAVAERIAGQKPHIDFNNVPFVIYTDPEIAWVGKTEEQLKA EGVEYKKGTSGFGANGRALAMGKAKGTVKVLADAKTDRILGVHMIGPVVSELVTEGVTALEFFASSEDIARIIHAH PTLSEVVHEAALAADKRALHG |
| 210 | 1011 | MMIEKSISIVDGKEYSVFAVSHEFRYTFDEPILVADLISSLKAYETLTSSYLPAILNQLFDVKIQKIKVAVSEIER GSFLEKLIFNLFFKDEDAYNEFCLKIRKFLGTENQDGSINMSKIIMFAMTTLLGVGAGYLLFKNPPQEKQAITNNI VTVINADSSVALDGEHLVSVVKEVTGSSKQKTAENVAKVYAPASKNNGSITLGTDDVRIEPVAQQTVATLPKDVDL RDTPLTEDYTDIDVQIRATDRDKNSGWYAVIDQIVPSRVRLELPEDIDLNRLANNATIRANVTVEFDLKQNGSRKP KKIILTSLSTD |
| 211 | 1055 | MFSKSVTLAQYDPDLAAAIAQEDQRQQDHVELIASENYVSCAVMDAQGSQLTNKYAEGYPGKRYYGGCEYVDIVEQ LAIDRVKELFGAAYANVQPHSGSQANQAVYASVLKPGDTILGMSLAHGGHLTHGASVNISGKLYNAVTYGLDENEV LDYAEVERLALEHKPKMIVAGASAYALQIDWAKFREIADKVGAYLFVDMAHYAGLVAGGEYPNPVPFCDFVTTTTH KTLRGPRGGVILCRDNTHEKALNSSIFPSLQGGPLMHVIAAKAVAFKEALQPEFKQYAKQVKINAAAMAEELVKRG LRIVSGRTESHVFLVDLQPMKITGKAAEAALGKAHITVNKNAIPNDPEKPFVTSGIRIGSAAMTTRGFNEADARVL ANLVADVLSNPEDEANLAKVRKQVTALCNKYPVYGA |
| 212 | 1437 | MSARENILAKLKKADALPMEEPAVFDYYREMGVSWGSEVERLKHWAAAMRAVKTEIYWVTKSNWMQVFREAAEGKG LKNILLPLATEHGQIARAALADSNIEPIAFEREIDTWKTEFFTNIDAGFSGAQCGIARTGTLMLFSSPEEPRTLSL VPPVHFCLFDTSKMYNEFHNAVEGEKLVENGMPTNVFLISGPSKTADIQLTLAYGAHGPRDLVILAILPDHISPAD LEENA |
| 213 | 1460 | MSLNKVILIGRLGRDPEVRYMPNGEAVCNFSVATSETWNDRNGQRVERTEWHNITMYRKLAEIAGQYLKKGGLVYL EGRIQSRKYQGKDGIERTAYDIVANEMKMLGGRNENSGGAPYEEGYGQSQEAYQRPAQQSRQPASDAPSHPQEAPA APRRQPVPAAAPVEDIDDDIPF |

| SED ID | NMB | Sequence |
|--------|-----|----------|
| 214 | 1540 | MNKKHGFPLTLTALAIATAFPAYAAQAGGATPDAAQTQSLKEITVRAAKVGRRSKEATGLGKIVKTSETLNKEQVL<br>GIRDLTRYDPGVAVVEQGNGASGGYSIRGVDKNRVAVSVDGVAQIQAFTVQGSLSGYGGRGGSGAINEIEYENIST<br>VEIDKGAGSSDHGSGALGGAVAFRTKEAADLISDGKSWGIQAKTAYGSKNRQFMKSLGAGFSKDGWEGLLIRTERQ<br>GRETRPHGDIADGVEYGIDRLDAFRQTYDIKRKTREPFFSVEGERESKPVAKLAGYGKYLNNQLNRWVKERIEQNQ<br>PLSAEEEAQVREAQARHENLSAQAYTGGGRILPDPMDYRSGSWLAKLGYRFGGRHYVGGVFEDTKQRYDIRDMTEK<br>QYYGTDEAEKFRDKSGVYDGDDFRDGLYFVPNIEEWKGDKNLVRGIGLKYSRTKFIDEHHRRRRMGLLYRYENEAY<br>SDNWADKAVLSFDKQGVATDNNTLKLNCAVYPAVDKSCRASADKPYSYDSSDRFHYREQHNVLNASFEKSLKNKWT<br>KHHLTLGFGYDASKAISRPEQLSHNAARISESTGFDENNQDKYLLGKPEVVEGSVCGYIETLRSRKCVPRKINGSN<br>IHISLNDRFSIGKYFDFSLGGRYDRKNFTTSEELVRSGRYVDRSWNSGILFKPNRHFSVSYRASSGFRTPSFQELF<br>GIDIYHDYPKGWQRPALKSEKAANREIGLQWKGDFGFLEISSFRNRYTDMIAVADHKTKLPNQAGQLTEIDIRDYY<br>NAQNMSLQGVNILGKIDWNGVYGKLPEGLYTTLAYNRIKPKSVSNRPGLSLRSYALDAVQPSRYVLGFGYDQPEGK<br>WGANIMLTYSKGKNPDELAYLAGDQKRYSTKRASSSWSTADVSAYLNLKKRLTLRAAIYNIGNYRYVTWESLRQTA<br>ESTANRHGGDSNYGRYAAPGRNFSLALEMKF |
| 215 | 1554 | MTKFIFVTGGVVSSLGKGIAAASIAAILESRGLNVTMLKLDPYINVDPGTMSPFQHGEVFVTDDGAETDLDLGHYE<br>RFIDSTMTRRNSFSTGQVYENVIAKERRGDYLGGTVQVIPHITDEIKRRIHEGAAGYDVAIVEIGGTVGDIESLPF<br>LEAIRQMRSQLGRNNTLFAHLSYVPYIAAAGEIKTKPTQHTVKEMLSIGLQPDILICRMDRTMPADERRKIALFCN<br>VEERAIVGSYDVDSIYECPEMLHDQGIDNIITEQLQLNVQQADLTAWKKIVHAIQNPKHTVKIAMVGKYVDLTESY<br>KSLIEALKHAGIHTETDVQITFVDSENIEKNKGDVSMLKDMDAILVPGGFGSRGVEGKIAAVRYARENNVPYLGIC<br>LGMQIALIEYARDVAGLKGANSTEFDLKCAAPVVALIDEWQTADGSVETRDESTDLGGTMRLGAQEVELKAGSLAA<br>KIYGSGHIRERHRHRYEVNNNYVPTLEQAGLVIGGVSAGRERLVETIELPNHPWFFACQFHPEFTSNPRKGHPLFT<br>AFVKAALNNKKA |
| 216 | 1576 | MRHILSVLIENESGAMSRVVGLFSARDYNIDSLAVAPTEDKTLSRMTIVTHGDEQVIEQITKQLNKLIEVIKVVDL<br>NESRFVERELMLVKVRAAGKDRDEFLRLTEIYRGSIIDVTDRSYTIEITGSTDKLDSFLETVGRAQILETVRTGAA<br>GIGRGERILKI |

(continued)

| SED ID | NMB | Sequence |
|--------|-----|----------|
| 217 | 1808 | MRLFKSLKNPKKTDAKLPKKSSGLNNRAAIGIDIDQHSIKMVQLSGRSLNQIQLEKYVIAKLPKNIIQGNKVQNYD QLVTYLQQAYAKLGTSCKNIIASVPQNLATIEQLTYTDKDAELDLQGFVESSISEVSSISLEEANYDYQVLSQSAA GEAVLAVASRKDEIEPLIDAFNAAGMKLSALDVDIFGQYNAYALWINHFAPELAAEKVAIFGVYAAQTYALVIQDG KILYKQETSVSEEQLNQLIQRTYQVTEEKAEEIINSPQKPSDYQESVANYFNQQITQEIQRVLQFYYTTQTADDMT DIKHILLTGEAARQEGIAQTVASQTNADVQCVHPARYFADNLKTDKQQFELDAPTLTRAFGLAVRGL |

EP 1 562 982 B1

**Claims**

1. A composition comprising: (a) outer-membrane vesicles prepared from a first strain of Neisseria meningitidis; and (b) one or more proteins which are present in outer-membrane vesicles prepared from a second strain of Neisseria meningitidis, but which are not present in outer-membrane vesicles prepared from said first strain, wherein said one or more proteins includes NMB0007 disclosed by Tettelin et al. [Science (2000) 287:1809-1815] and deposited in the sequence databases, and having the sequence of SEQ ID NO. 218.

2. The composition of claim 1, wherein the protein(s) of component (b) are: (i) purified from the second strain and added to component (a); (ii) expressed recombinantly, purified, and added to component (a); or (iii) expressed by said first strain following either engineering of the first strain such that it expresses said protein(s), either (1) from its chromosomal DNA or from extrachromosomal DNA, or (2) such that existing expression of said protein(s) is up-regulated, or (3) such that trafficking of said protein(s) already expressed by the first strain is altered to direct it/them to a different cellular location, thereby causing it/them to be present in the outer-membrane vesicles.

3. A composition according to claim 1, comprising outer-membrane vesicles (OMVs) prepared from a genetically modified first strain of Neisseria meningitidis, wherein said OMVs include one or more proteins which are (a) not present in OMVs prepared from said first strain prior to its being genetically modified, but which are (b) present in OMVs prepared from a second strain of Neisseria meningitidis.

**Patentansprüche**

1. Zusammensetzung, umfassend: (a) Vesikel der äußeren Membran, die von einem ersten Stamm von *Neisseria meningitidis* hergestellt wurden; und (b) ein oder mehrere Proteine, die in Vesikeln der äußeren Membran vorhanden sind, welche von einem zweiten Stamm von *Neisseria meningitidis* hergestellt wurden, welche jedoch nicht in Vesikeln der äußeren Membran vorhanden sind, die von dem ersten Stamm von *Neisseria meningitidis* hergestellt wurden, wobei das Protein oder die Proteine NMB0007 umfasst/umfassen, das in Tettelin et al., [Science (2000) 287:1809-1815) offenbart wird und in der Sequenz-Datenbank hinterlegt ist und die Sequenz von SEQ ID NO:218 aufweist.

2. Zusammensetzung nach Anspruch 1, wobei das Protein/die Proteine von Bestandteil (b):

   (i) aus dem zweiten Stamm gereinigt und zu dem Bestandteil (a) zugefügt wird/werden;
   (ii) rekombinant exprimiert, gereinigt und zu Bestandteil (a) zugefügt wird/werden; oder
   (iii) von dem ersten Stamm exprimiert wird/werden, nachdem der erste Stamm so erstellt wurde, dass er das Protein/die Proteine entweder (1) ausgehend von chromosomaler DNA oder ausgehend von extrachromosomaler DNA exprimiert; oder (2) so, dass die bereits vorhandene Expression des Proteins/der Proteine hochreguliert wird, oder (3) so, dass der Transport des Proteins/der Proteine, welches/welche bereits von dem ersten Stamm exprimiert wird/werden, so verändert wird, dass dieses/diese zu einem anderen Ort in der Zelle geleitet wird/werden, was dazu führt, dass es/sie in den Vesikeln der äußeren Membran vorhanden ist/sind.

3. Zusammensetzung nach Anspruch 1, die Vesikel der äußeren Membran (outer-membrane vesicles, OMVs) umfasst, welche von einem genetisch modifizierten, ersten Stamm von *Neisseria meningitidis* hergestellt wurden, wobei die OMVs ein oder mehrere Proteine enthalten, die (a) nicht in OMVs vorhanden sind, welche von dem ersten Stamm hergestellt wurden, bevor dieser genetisch modifiziert wurde, welche aber (b) in OMVs vorhanden sind, die von einem zweiten Stamm von *Neisseria meningitidis* hergestellt wurden.

**Revendications**

1. Composition comprenant : (a) des vésicules de la membrane externe préparées à partir d'une première souche de *Neisseria meningitidis ;* et (b) une ou plusieurs protéines qui sont présentes dans les vésicules de la membrane externe préparées à partir d'une seconde souche de *Neisseria meningitidis,* mais qui ne sont pas présentes dans les vésicules de la membrane externe préparées à partir de ladite première souche, où ladite/lesdites une ou plusieurs protéines comprennent la protéine NMB0007 décrite par Tettelin *et al.* [Science (2000) 287 : 1809-1815] et déposée dans la base de données des séquences et ayant la séquence de SEQ ID NO : 218.

**2.** Composition selon la revendication 1, dans laquelle la/les protéine(s) du composant (b) sont : (i) purifiées à partir de la seconde souche et ajoutées au composant (a) ; (ii) exprimées de manière recombinante, purifiées et ajoutées au composant (a) ; ou (iii) exprimées par ladite première souche après soit modification de la première souche de telle manière qu'elle exprime ladite/lesdites protéine(s) soit (1) à partir de son ADN chromosomique ou à partir d'ADN extrachromosomique, soit (2) de telle manière que l'expression existante de ladite/desdites protéine(s) est régulée à la hausse, soit (3) de telle manière que le trafic de ladite/desdites protéine(s) déjà exprimée(s) par la première souche soit modifié pour la/les diriger vers une localisation cellulaire différent, provoquant de cette manière sa/leur présence dans les vésicules de la membrane externe.

**3.** Composition selon la revendication 1, comprenant des vésicules de la membrane externe (OMV) préparées à partir d'une première souche génétiquement modifiée de *Neisseria meningitidis,* où lesdites OMV comprennent une ou plusieurs protéines qui (a) ne sont pas présentes dans les OMV préparées à partir de ladite première souche avant sa modification génétique, mais (b) sont présentes dans les OMV préparées à partir d'une seconde souche de *Neisseria meningitidis.*

*FIGURE 1*

3　　　pH　　10

4　　　6　7

4　　pH　　7

6　　pH　10　11

*FIGURE 2*

## FIGURE 3

## FIGURE 4

# FIGURE 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5753235 A
- EP 127839 A
- EP 155476 A
- WO 89046699 A
- US 5693506 A
- US 5659122 A
- US 5608143 A
- US 4738921 A
- EP 0036776 A
- EP 0121775 A
- US 4689406 A
- US 4551433 A
- EP 0267851 A
- EP 0219237 A
- EP 0324647 A, Chey
- US 4336336 A
- EP 0244042 A
- EP 0127328 A
- EP 0036259 A
- EP 0063953 A
- WO 8404541 A
- EP 0136829 A
- EP 0136907 A
- US 4745056 A
- EP 0284044 A
- EP 0329203 A
- US 4876197 A
- US 4880734 A
- EP 0164556 A
- EP 0196056 A
- WO 88024066 A
- EP 0012873 A
- JP O62096086 B
- US 4588684 A
- EP 0060057 A
- US 4546083 A
- US 4870008 A
- EP 0324274 A
- WO 8902463 A
- US 4837148 A
- US 4929555 A
- WO 9820734 A
- WO 9014837 A
- US 5591624 A
- WO 9637626 A
- WO 9530763 A
- WO 9205266 A
- US 5288641 A
- EP 0176170 A, Roizman

- WO 9504139 A, Wistar
- WO 9009441 A
- WO 9207945 A
- EP 0453242 A
- US 5091309 A
- US 5217879 A
- WO 9210578 A
- US 08405627 B
- WO 9421792 A
- WO 9507994 A
- US SN08679640 A
- US 4603112 A
- US 4769330 A
- WO 8901973 A
- US 5166057 A
- EP 0386882 A
- EP 0440219 A
- US 08366787 B
- US 08240030 B
- US 08404796 B
- US 5149655 A
- US 5206152 A
- WO 9211033 A
- US 60023867 B
- WO 9011092 A
- US 5580859 A
- US 5422120 A
- WO 9513796 A
- WO 9423697 A
- WO 9114445 A
- EP 524968 A
- US SN60023867 P
- US 4762915 A
- EP 0524968 A
- WO 9314778 A
- WO 9806437 A, Zuckermann
- US 4683195 A
- US 4683202 A
- WO 0164922 A
- WO 0164920 A
- WO 03020756 A
- WO 0025811 A
- WO 0152885 A
- US 5597572 A
- US 5747653 A
- EP 0680512 A
- WO 0109350 A
- WO 0209746 A
- EP 0449958 A

- WO 0191788 A
- WO 0226212 A
- WO 9833487 A
- WO 0007621 A
- WO 9927960 A
- WO 9857659 A
- EP 0835318 A
- EP 0735898 A
- EP 0761231 A
- WO 9602555 A
- WO 9816247 A
- WO 9818810 A
- WO 9840100 A
- WO 9855495 A
- WO 9837919 A
- WO 9852581 A
- WO 9952549 A
- WO 0121207 A
- WO 0121152 A
- WO 0062800 A
- WO 0023105 A
- WO 9911241 A
- WO 9318150 A
- WO 9601272 A
- WO 9601273 A

- WO 9725429 A
- WO 9804702 A
- WO 03007985 A
- WO 9924578 A
- WO 9936544 A
- WO 9957280 A
- WO 02079243 A
- WO 0202606 A
- WO 9927105 A
- WO 0027994 A
- WO 0037494 A
- WO 9928475 A
- WO 0234771 A
- EP 0372501 A
- EP 0378881 A
- EP 0427347 A
- WO 9317712 A
- WO 9403208 A
- WO 9858668 A
- EP 0471177 A
- WO 0056360 A
- WO 9101146 A
- WO 0061761 A
- WO 0172337 A
- WO 0138350 A

**Non-patent literature cited in the description**

- **Tettelin et al.** *Science,* 2000, vol. 287, 1809-1815
- **Sambrook.** Molecular Cloning; A Laboratory Manual. 1989
- MOLECULAR CLONING: A LABORATORY MANUAL. 2001
- DNA Cloning. 1985, vol. I, ii
- Oligonucleotide Synthesis. 1984
- Nucleic Acid Hybridization. 1984
- Transcription and Translation. 1984
- Animal Cell Culture. 1986
- Immobilized Cells and Enzymes. IRL Press, 1986
- **B. Perbal.** A Practical Guide to Molecular Cloning. 1984
- Methods in Enzymology series. Academic Press, Inc, vol. 154, 155
- Gene Transfer Vectors for Mammalian Cells. Cold Spring Harbor Laboratory, 1987
- Immunochemical Methods in Cell and Molecular Biology. Academic Press, 1987
- **Scopes.** Protein Purification: Principles and Practice. Springer-Verlag, 1987
- Handbook of Experimental Immunology. 1986, vol. I-IV
- Expression of Cloned Genes in Mammalian Cells. **Sambrook et al.** Molecular Cloning: A Laboratory Manual. 1989
- **Maniatis et al.** *Science,* 1987, vol. 236, 1237

- **Alberts et al.** Molecular Biology of the Cell. 1989
- **Dijkema et al.** *EMBO J.,* 1985, vol. 4, 761
- **Gorman et al.** *PNAS USA,* 1982, vol. 79, 6777
- **Boshart et al.** *Cell,* 1985, vol. 41, 521
- **Sassone-Corsi ; Borelli.** *Trends Genet.,* 1986, vol. 2, 215
- **Birnstiel et al.** *Cell,* 1985, vol. 41, 349
- Termination and 3' end processing of eukaryotic RNA. **Proudfoot ; Whitelaw.** Transcription and splicing. 1988
- **Proudfoot.** *Trends Biochem. Sci.,* 1989, vol. 14, 105
- Expression of cloned genes in cultured mammalian cells. **Sambrook et al.** Molecular Cloning: A Laboratory Manual. 1989
- **Gluzman.** *Cell,* 1981, vol. 23, 175
- **Kaufman et al.** *Mol. Cell. Biol.,* 1989, vol. 9, 946
- **Shimizu et al.** *Mol. Cell. Biol.,* 1986, vol. 6, 1074
- **Luckow ; Summers.** *Virology,* 1989, vol. 17, 31
- **Miller et al.** *Ann. Rev. Microbiol.,* 1988, vol. 42, 177
- The Regulation of Baculovirus Gene Expression. **Friesen et al.** The Molecular Biology of Baculoviruses. 1986

- **Vlak et al.** *J. Gen. Virol.,* 1988, vol. 69, 765
- **Carbonell et al.** *Gene,* 1988, vol. 73, 409
- **Maeda et al.** *Nature,* 1985, vol. 315, 592
- **Lebacq-Verheyden et al.** *Molec. Cell. Biol.,* 1988, vol. 8, 3129
- **Smith et al.** *Proc. Nat'l Acad. Sci. USA,* 1985, vol. 82, 8404
- **Miyajima et al.** *Gene,* 1987, vol. 58, 273
- **Martin et al.** *DNA,* 1988, vol. 7, 99
- **Smith et al.** *Mol. Cell. Biol.,* 1983, vol. 3, 2156
- **Miller et al.** *Bioessays,* 1989, vol. 4, 91
- Current Protocols in Microbiology. 1990, vol. 2, 16.8
- **Carbonell et al.** *J. Virol.,* 1985, vol. 56, 153
- **Wright.** *Nature,* 1986, vol. 321, 718
- **Fraser et al.** *In Vitro Cell. Dev. Biol.,* 1989, vol. 25, 225
- **Zenk.** *Phytochemistry,* 1991, vol. 30, 3861-3863
- **Vaulcombe et al.** *Mol. Gen. Genet.,* 1987, vol. 209, 33-40
- **Chandler et al.** *Plant Molecular Biology,* 1984, vol. 3, 407-418
- **Rogers.** *J. Biol. Chem.,* 1985, vol. 260, 3731-3738
- **Rothstein et al.** *Gene,* 1987, vol. 55, 353-356
- **Whittier et al.** *Nucleic Acids Research,* 1987, vol. 15, 2515-2535
- **Wirsel et al.** *Molecular Microbiology,* 1989, vol. 3, 3-14
- **Yu et al.** *Gene,* 1992, vol. 122, 247-253
- **R.L. Jones ; J. MacMillin ; Gibberellins.** Advanced Plant Physiology. Pitman Publishing Limited, 1984, 21-52
- **Sheen.** *Plant Cell,* 1990, vol. 2, 1027-1038
- **Maas et al.** *EMBO J.,* 1990, vol. 9, 3447-3452
- **Benkel ; Hickey.** *Proc. Natl. Acad. Sci.,* 1987, vol. 84, 1337-1339
- **Wilmink ; Dons.** *Plant Mol. Biol. Reptr,* 1993, vol. 11 (2), 165-185
- **Reed ; Maniatis.** *Cell,* 1985, vol. 41, 95-105
- **Crossway.** *Mol. Gen. Genet,* 1985, vol. 202, 179-185
- **Krens et al.** *Nature,* 1982, vol. 296, 72-74
- **Klein et al.** *Nature,* 1987, vol. 327, 70-73
- **Knudsen ; Muller.** *Planta,* 1991, vol. 185, 330-336
- **Fraley et al.** *Proc. Natl. Acad Sci. USA,* 1982, vol. 79, 1859-1863
- **Fromm et al.** *Proc. Natl Acad Sci. USA,* 1985, vol. 82, 5824
- **Raibaud et al.** *Annu. Rev. Genet.,* 1984, vol. 18, 173
- **Chang et al.** *Nature,* 1977, vol. 198, 1056
- **Goeddel et al.** *Nuc. Acids Res.,* 1980, vol. 8, 4057
- **Yelverton et al.** *Nucl. Acids Res.,* 1981, vol. 9, 731
- The cloning of interferon and other mistakes. **Weissmann.** Interferon 3. 1981
- **Shimatake et al.** *Nature,* 1981, vol. 292, 128
- **Amann et al.** *Gene,* 1983, vol. 25, 167
- **de Boer et al.** *Proc. Natl. Acad. Sci.,* 1983, vol. 80, 21
- **Studier et al.** *J. Mol. Biol.,* 1986, vol. 189, 113
- **Tabor et al.** *Proc Natl. Acad Sci.,* 1985, vol. 82, 1074
- **Shine et al.** *Nature,* 1975, vol. 254, 34
- Genetic signals and nucleotide sequences in messenger RNA. **Steitz et al.** Biological Regulation and Development: Gene Expression. 1979
- Expression of cloned genes in Escherichia coli. **Sambrook et al.** Molecular Cloning: A Laboratry Manual. 1989
- **Nagai et al.** *Nature,* 1984, vol. 309, 810
- **Jia et al.** *Gene,* 1987, vol. 60, 197
- **Allen et al.** *J. Biotechnol.,* 1987, vol. 5, 93
- **Makoff et al.** *J. Gen. Microbiol.,* 1989, vol. 135, 11
- **Miller et al.** *Bio/Technology,* 1989, vol. 7, 698
- **Masui et al.** *Experimental Manipulation of Gene Expression,* 1983
- **Ghrayeb et al.** *EMBO J.,* 1984, vol. 3, 2437
- **Oka et al.** *Proc. Natl. Acad. Sci.,* 1985, vol. 82, 7212
- **Palva et al.** *Proc. Natl. Acad. Sci. USA,* 1982, vol. 79, 5582
- **Davies et al.** *Annu. Rev. Microbiol.,* 1978, vol. 32, 469
- **Amann et al.** *Gene,* 1985, vol. 40, 183
- **Powell et al.** *Appl. Environ. Microbiol.,* 1988, vol. 54, 655
- **Masson et al.** *FEMS Microbiol. Lett.,* 1989, vol. 60, 273
- **Miller et al.** *Proc. Natl. Acad. Sci.,* 1988, vol. 85, 856
- **Wang et al.** *J. Bacteriol.,* 1990, vol. 172, 949
- **Cohen et al.** *Proc. Natl. Acad. Sci.,* 1973, vol. 69, 2110
- **Dower et al.** *Nucleic Acids Res,* 1988, vol. 16, 6127
- An improved method for transformation of Escherichia coli with ColE1-derived plasmids. **Kushner.** Genetic Engineering: Proceedings of the International Symposium on Genetic Engineering. 1978
- **Mandel et al.** *J. Mol. Biol.,* 1970, vol. 53, 159
- **Taketo.** *Biochim. Biophys. Acta,* 1988, vol. 949, 318
- **Chassy et al.** *FEMS Microbiol. Lett.,* 1987, vol. 44, 173

- **Fiedler et al.** *Anal. Biochem,* 1988, vol. 170, 38
- **Augustin et al.** *FEMS Microbiol. Lett.,* 1990, vol. 66, 203
- **Barany et al.** *J. Bacteriol.,* 1980, vol. 144, 698
- Transformation of Streptococcus lactis by electroporation. **Harlander.** Streptococcal Genetics. 1987
- **Perry et al.** *Infect. Immun.,* 1981, vol. 32, 1295
- **Somkuti et al.** *Proc. 4th Evr. Cong. Biotechnology,* 1987, vol. 1, 412
- **Myanohara et al.** *Proc. Natl. Acad. Sci. USA,* 1983, vol. 80, 1
- **Cohen et al.** *Proc. Natl. Acad. Sci. USA,* 1980, vol. 77, 1078
- **Henikoff et al.** *Nature,* 1981, vol. 283, 835
- **Hollenberg et al.** *Curr. Topics Microbiol. Immunol.,* 1981, vol. 96, 119
- The Expression of Bacterial Antibiotic Resistance Genes in the Yeast Saccharomyces cerevisiae. **Hollenberg et al.** Plasmids of Medical, Environmental and Commercial Importance. 1979
- **Mercerau-Puigalon et al.** *Gene,* 1980, vol. 11, 163
- **Panthier et al.** *Curr. Genet.,* 1980, vol. 2, 109
- **Botstein et al.** *Gene,* 1979, vol. 8, 17-24
- **Brake et al.** *Proc. Natl. Acad Sci USA,* 1984, vol. 81, 4642-4646
- **Stinchcomb et al.** *J. Mol. Biol.,* 1982, vol. 158, 157
- **Orr-Weaver et al.** *Methods in Enzymol.,* 1983, vol. 101, 228-245
- **Rine et al.** *Proc. Natl. Acad. Sci. USA,* 1983, vol. 80, 6750
- **Butt et al.** *Microbiol, Rev.,* 1987, vol. 51, 351
- **Kurtz et al.** *Mol. Cell. Biol.,* 1986, vol. 6, 142
- **Kunze et al.** *J. Basic Microbiol.,* 1985, vol. 25, 141
- **Gleeson et al.** *J. Gen. Microbiol.,* 1986, vol. 132, 3459
- **Roggenkamp et al.** *Mol. Gen. Genet.,* 1986, vol. 202, 302
- **Das et al.** *J. Bacteriol.,* 1984, vol. 158, 1165
- **De Louvencourt et al.** *J. Bacteriol.,* 1983, vol. 154, 737
- **Van den Berg et al.** *Bio/Technology,* 1990, vol. 8, 135
- **Cregg et al.** *Mol. Cell. Biol.,* 1985, vol. 5, 3376
- **Hinnen et al.** *Proc. Natl. Acad. Sci. USA,* 1978, vol. 75, 1929
- **Ito et al.** *J. Bacteriol.,* 1983, vol. 153, 163
- **Beach ; Nurse.** *Nature,* 1981, vol. 300, 706
- **Davidow et al.** *Curr. Genet.,* 1985, vol. 10, 380471
- **Gaillardin et al.** *Curr. Genet.,* 1985, vol. 10, 49
- **De Louvencourt et al.** *J. Bacteriol.,* 1983, vol. 154, 1165
- **Davidow et al.** *Curr. Genet.,* 1985, vol. 10, 39
- Remington's Pharmaceutical Sciences. Mack Pub. Co, 1991
- **Robinson ; Torres.** *Seminars in Immunology,* 1997, vol. 9, 271-283
- **Donnelly et al.** *Annu Rev Immunol,* 1997, vol. 15, 617-648
- **Jolly.** *Cancer Gene Therapy,* 1994, vol. 1, 51-64
- **Kimura.** *Human Gene Therapy,* 1994, vol. 5, 845-852
- **Connelly.** *Human Gene Therapy,* 1995, vol. 6, 185-193
- **Kaplitt.** *Nature Genetics,* 1994, vol. 6, 148-153
- **Hartley ; Rowe.** *J Virol,* 1976, vol. 19, 19-25
- **Geller.** *Science,* 1988, vol. 241, 1667-1669
- **Fink.** *Human Gene Therapy,* 1992, vol. 3, 11-19
- **Evans.** *Nature,* 1989, vol. 339, 385
- **Sabin.** *J. Biol. Standardization,* 1973, vol. 1, 115
- **Arnold.** *J Cell Biochem,* 1990, L401
- **Fisher-Hoch.** *Proc Natl Acad Sci,* 1989, vol. 86, 317
- **Flexner.** *Ann NY Acad Sci,* 1989, vol. 569, 86
- **Flexner.** *Vaccine,* 1990, vol. 8, 17
- **Mulligan.** *Nature,* 1979, vol. 277, 108
- **Madzak.** *J Gen Virol,* 1992, vol. 73, 1533
- **Enami.** *Proc Natl Acad Sci,* 1990, vol. 87, 3802-3805
- **Enami ; Palese.** *J Virol,* 1991, vol. 65, 2711-2713
- **Luytjes.** *Cell,* 1989, vol. 59, 110
- **McMichael.** *NEJ Med,* 1983, vol. 309, 13
- **Yap.** *Nature,* 1978, vol. 273, 238
- *Nature,* 1979, vol. 277, 108
- **Buchschacher.** *J. Virol.,* 1992, vol. 66, 2731
- **Hamre.** *Proc Soc Exp Biol Med,* 1966, vol. 121, 190
- **Curiel.** *Hum Gene Ther,* 1992, vol. 3, 147-154
- **Wu.** *J Biol Chem,* 1989, vol. 264, 16985-16987
- **Philip.** *Mol Cell Biol,* 1994, vol. 14, 2411-2418
- **Woffendin.** *Proc Natl Acad Sci,* 1994, vol. 91, 1581-1585
- **Wu ; Wu.** *J. Biol. Chem.,* 1987, vol. 262, 4429-4432
- **Hucked.** *Biochem Pharmacol,* 1990, vol. 40, 253-263
- **Plank.** *Bioconjugate Chem,* 1992, vol. 3, 533-539

- **Woffendin et al.** *Proc. Natl. Acad. Sci. USA,* 1994, vol. 91 (24), 11581-11585
- **Stryer.** Biochemistry. W.H. Freeman, 1975, 236-240
- **Szoka.** *Biochem Biophys Acta,* 1980, vol. 600, 1
- **Bayer.** *Biochem Biophys Acta,* 1979, vol. 550, 464
- **Rivnay.** *Meth Enzymol,* 1987, vol. 149, 119
- **Wang.** *Proc Natl Acad Sci,* 1987, vol. 84, 7851
- **Plant.** *Anal Biochem,* 1989, vol. 176, 420
- **Hug ; Sleight.** *Biochim. Biophys. Acta,* 1991, vol. 1097, 1-17
- **Straubinger.** *Meth. Enzymol.,* 1983, vol. 101, 512-527
- **Felgner.** *Proc. Natl. Acad. Sci. USA,* 1987, vol. 84, 7413-7416
- **Malone.** *Proc. Natl. Acad. Sci. USA,* 1989, vol. 86, 6077-6081
- **Debs.** *J. Biol. Chem.,* 1990, vol. 265, 10189-10192
- **Szoka.** *Proc. Natl. Acad. Sci. USA,* 1978, vol. 75, 4194-4198
- **Straubinger.** *Meth. Immunol.,* 1983, vol. 101, 512-527
- **Papahadjopoulos.** *Biochim. Biophys. Acta,* 1975, vol. 394, 483
- **Wilson.** *Cell,* 1979, vol. 17, 77
- **Deamer ; Bangham.** *Biochim. Biophys. Acta,* 1976, vol. 443, 629
- **Ostro.** *Biochem. Biophys. Res. Commun.,* 1977, vol. 76, 836
- **Fraley.** *Proc. Natl. Acad. Sci. USA,* 1979, vol. 76, 3348
- **Enoch ; Strittmatter.** *Proc. Natl. Acad. Sci. USA,* 1979, vol. 76, 145
- **Fraley.** *J. Biol. Chem.,* 1980, vol. 255, 10431
- **Szoka ; Papahadjopoulos.** *Proc. Natl. Acad. Sci. USA,* 1978, vol. 75, 145
- **Schaefer-Ridder.** *Science,* 1982, vol. 215, 166
- **Breslow.** *Annu Rev. Biochem,* 1985, vol. 54, 699
- **Law.** *Adv. Exp Med. Biol.,* 1986, vol. 151, 162
- **Chen.** *J Biol Chem,* 1986, vol. 261, 12918
- **Kane.** *Proc Natl Acad Sci USA,* 1980, vol. 77, 2465
- **Utermann.** *Hum Genet,* 1984, vol. 65, 232
- *Meth. Enzymol.,* 1986, vol. 128
- **Pitas.** *J. Biochem.,* 1980, vol. 255, 5454-5460
- **Mahey.** *J Clin. Invest,* 1979, vol. 64, 743-750
- **Atkinson.** *Annu Rev Biophys Chem,* 1986, vol. 15, 403
- **Radding.** *Biochim Biophys Acta,* 1958, vol. 30, 443
- **Meinkoth ; Wahl.** *Anal. Biochem.,* 1984, vol. 138, 267-284
- **Matteucci et al.** *J. Am. Chem. Soc.,* 1981, vol. 103, 3185
- **Urdea et al.** *Proc. Natl. Acad. Sci. USA,* 1983, vol. 80, 7461
- **Agrawal ; Iyer.** *Curr Opin Biotechnol,* 1995, vol. 6, 12-19
- **Agrawal.** *TIBTECH,* 1996, vol. 14, 376-387
- **Corey.** *TIBTECH,* 1997, vol. 15, 224-229
- **Buchardt et al.** *TIBTECH,* 1993, vol. 11, 384-386
- **Mullis et al.** *Meth. Enzymol.,* 1987, vol. 155, 335-350
- **Pizza et al.** *Science,* 2000, vol. 287, 1816-1820
- **Parkhill et al.** *Nature,* 2000, vol. 404, 502-506
- **Cole.** *Eur Respir J Suppl.,* 2002, vol. 36, 78s-86s
- **McClelland et al.** *Nature,* 2001, vol. 413, 852-856
- **De Groot et al.** *Vaccine,* 2001, vol. 19, 4385-4395
- **Stephens.** *J Infect Dis,* 2000, vol. 181 (3), 521-523
- **Tettelin et al.** *Science,* 2001, vol. 293, 498-506
- **Weinstock et al.** *Res Microbiol,* 2000, vol. 151, 151-158
- **Wizemann et al.** *Infect Immun,* 2001, vol. 69, 1593-1598
- **Grifantini et al.** *Nat Biotechnol.,* 2002, vol. 20, 914-921
- **Bjune et al.** *Lancet,* 1991, vol. 338 (8775), 1093-1096
- **de Kleijn et al.** *Vaccine,* 2001, vol. 20, 352-358
- **Gennaro.** Remington: The Science and Practice of Pharmacy. 2000
- Vaccine design: the subunit and adjuvant approach. Plenum Press, 1995
- **Krieg.** *Vaccine,* 2000, vol. 19, 618-622
- **Krieg.** *Curr opin Mol Ther,* 2001, vol. 3, 15-24
- **Del Giudice et al.** *Molecular Aspects of Medicine,* 1998, vol. 19 (1
- Vaccine Design.. Plenum, 1995
- **Johnson et al.** *Bioorg Med Chem Lett,* 1999, vol. 9, 2273-2278
- **Covacci ; Rappuoli.** *J. Exp. Med.,* 2000, vol. 19, 587-592
- **Covacci et al.** *Proc. Natl. Acad. Sci. USA,* 1993, vol. 90, 5791-5795
- **Tummuru et al.** *Infect. Immun.,* 1994, vol. 61, 1799-1809
- **Marchetti et al.** *Vaccine,* 1998, vol. 16, 33-37
- **Telford et al.** *J. Exp. Med.,* 1994, vol. 179, 1653-1658
- **Evans et al.** *Gene,* 1995, vol. 153, 123-127
- **Costantino et al.** *Vaccine,* 1992, vol. 10, 691-698

- **Costantino et al.** *Vaccine,* 1999, vol. 17, 1251-1263
- **Watson.** *Pediatr Infect Dis J,* 2000, vol. 19, 331-332
- **Rubin.** *Pediatr Clin North Am,* 2000, vol. 47, 269-285v
- **Jedrzejas.** *Microbiol Mol Biol Rev,* 2001, vol. 65, 187-207
- **Bell.** *Pediatr Infect Dis J,* 2000, vol. 19, 1187-1188
- **Iwarson.** *APMIS,* 1995, vol. 103, 321-326
- **Gerlich et al.** *Vaccine,* 1990, vol. 8, 63-6879-80
- **Gustafsson et al.** *N. Engl. J. Med.,* 1996, vol. 334, 349-355
- **Rappuoli et al.** *TIBTECH,* 1991, vol. 9, 232-238
- Vaccines. 1988
- **Del Guidice et al.** *Molecular Aspects of Medicine,* 1998, vol. 19, 1-70
- **Hsu et al.** *Clin Liver Dis,* 1999, vol. 3, 901-915
- **Kalman et al.** *Nature Genetics,* 1999, vol. 21, 385-389
- **Read et al.** *Nucleic Acids Res,* 2000, vol. 28, 1397-406
- **Shirai et al.** *J. Infect. Dis.,* 2000, vol. 181 (3), S524-S527
- **Ross et al.** *Vaccine,* 2001, vol. 19, 4135-4142
- **Sutter et al.** *Pediatr Clin North Am,* 2000, vol. 47, 287-308
- **Zimmerman ; Spann.** *Am Fam Physician,* 1999, vol. 59, 125-126
- **Dreesen.** *Vaccine,* 1997, vol. 15 (S2-6
- *MMWR Morb Mortal Wkly Rep,* 16 January 1998, vol. 47 (1), 12, 19
- Vaccines
- **McMichael.** *Vaccine,* 2000, vol. 19 (1), 101-107
- **Schuchat.** *Lancet,* 1999, vol. 353 (9146), 51-6
- **Dale.** *Infect Dis Clin North Am,* 1999, vol. 13, 227-43viii
- **Ferretti et al.** *PNAS USA,* 2001, vol. 98, 4658-4663
- **Kuroda et al.** *Lancet,* 2001, vol. 357 (9264), 1225-1240
- *J Toxicol Clin Toxicol,* 2001, vol. 39, 85-100
- **Demicheli et al.** *Vaccine,* 1998, vol. 16, 880-884
- **Stepanov et al.** *J Biotechnol,* 1996, vol. 44, 155-160
- **Ingram.** *Trends Neurosci,* 2001, vol. 24, 305-307
- **Rosenberg.** *Nature,* 2001, vol. 411, 380-384
- **Moingeon.** *Vaccine,* 2001, vol. 19, 1305-1326
- **Molloy et al.** *Eur J Biochem,* 2000, vol. 267 (10), 2871-81
- **Molloy et al.** *Electrophoresis,* 2001, vol. 22 (9), 1686-96
- **Phadke et al.** *Proteomics,* 2001, vol. 1 (5), 705-20
- **Nouwens et al.** *Electrophoresis,* 2000, vol. 21 (17), 3797-809
- **Chevallet et al.** *Electrophoresis,* 1998, vol. 19 (11), 1901-9
- **Herbert et al.** *Electrophoresis,* 1998, vol. 19 (5), 845-51
- **Doherty et al.** *Electrophoresis,* 1998, vol. 19 (2), 355-63
- **Wilm et al.** *Nature,* 1996, vol. 379 (6564), 466-9
- **Nakai ; Kanehisa.** *Proteins,* vol. 11 (2), 95-110
- **Nakai.** *Adv Proteins Chem,* 2000, vol. 54, 277-344